# FASCICULE DE BREVET EUROPEEN

(11) **EP 1 001 943 B1**
(45) Date de publication et mention de la délivrance du brevet: **17.12.2003**
(21) Numéro de dépôt: 98939679.1
(22) Date de dépôt: 15.07.1998
(51) Int. Cl.: C07D 241/12, A61K 31/495, C07D 405/04, C07D 405/06, C07D 405/12, C07D 405/14

(54) **DERIVES DE POLYHYDROXYALKYLPYRAZINES, LEUR PREPARATION ET MEDICAMENTS LES CONTENANT**
POLYHYDROXYALKYLPYRAZINE DERIVATE,DEREN HERSTELLUNG UND DIESE ENTHALTENDE ARZNEIMITTELN
POLYHYDROXYALKYLPYRAZINE DERIVATIVES, PREPARATION AND MEDICINES CONTAINING THEM

(30) Priorité: 18.07.1997 FR 9709186
(43) Date de publication de la demande: 24.05.2000
(73) Titulaire: Aventis Pharma S.A., 92160 Antony (FR)
(72) Inventeur: BOUCHARD, Hervé, F-94320 Thiais (FR); COMMERCON, Alain, F-94400 Vitry sur Seine (FR); PEYRONEL, Jean-François, F-91120 Palaiseau (FR); TERRIER, Corinne, F-93190 Livry Gargan (FR)
(86) Numéro de dépôt international: FR9801545
(87) Numéro de publication internationale: WO99003843

(56) Documents cités:
- WO-A-97/28813
- CHEMICAL ABSTRACTS, vol. 74, no. 23, 1971 Columbus, Ohio, US; abstract no. 126009f, page 504; XP002060213 & JP 07 105310 A (C.M.R.I.)
- CHEMICAL ABSTRACTS, vol. 81, no. 6, 1974 Columbus, Ohio, US; abstract no. 116272s, H.TSUCHIDA ET AL.: "FORMATION OF DESOXYFRUCTOSAZINE." page 185; XP002060281 & AGR.BIOL.CHEM., vol. 37, no. 11, 1973, pages 2571-2578, JAPAN
- CHEMICAL ABSTRACTS, vol. 77, no. 5, 1972 Columbus, Ohio, US; abstract no. 34798w, E.FORLAND: "REACTION OF PENTA-O-NICOTINOYL" page 550; colonne 1; XP002060282 & CARBOHYDR.RES., vol. 23, no. 1, 1972, pages 111-119, US

## Description

Médicaments contenant en tant que principe actif au moins un composé de formule générale (V) (VI) (VII) dans laquelle.

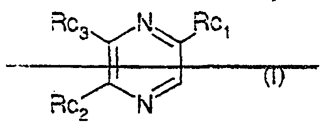

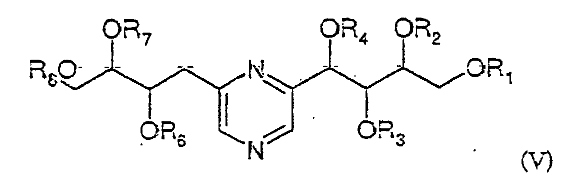

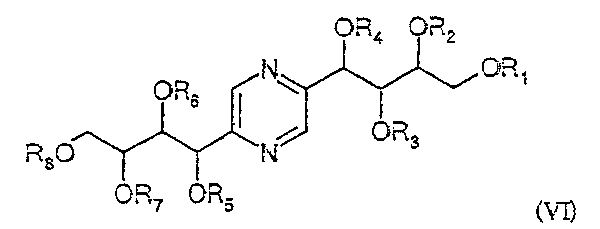

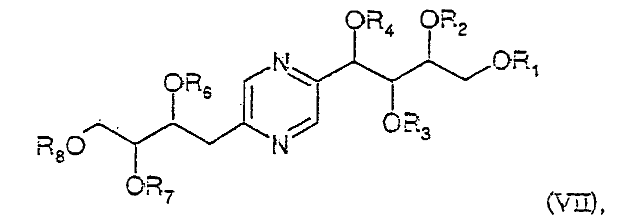

Les composés de formule (V),(VI),(VII) comportent des atomes de carbone asymétriques et présentent des formes stéréoisomères. Ces stéréoisomères font également partie de l'invention; les formes stéréoisomères des composés de formule (V), (VI), (VII) particulièrement représentatives de l'invention sont respectivement les composés suivants de formule générale (VIII), (IX), (X) : 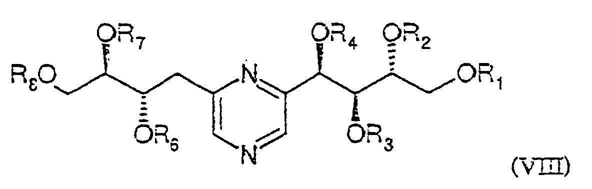 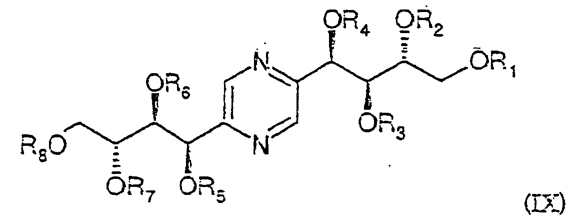 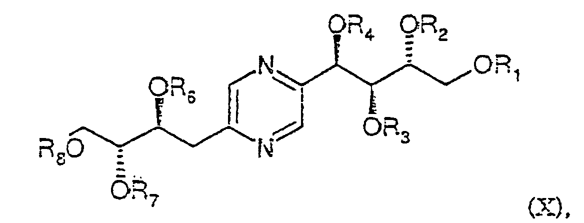 pour lesquels R₁, R₂, R₃, R₄, R₅, R₆, R₇, R₈ sont définis comme précédemment.

Les médicaments selon l'invention sont ceux contenant comme ingrédient actif au moins un des composés suivants :

### A - composés de formule générale :

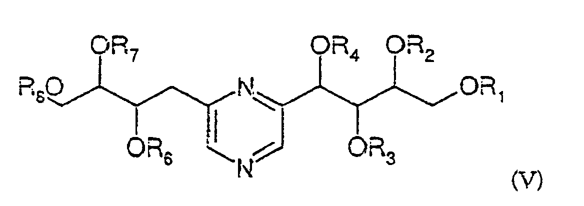

pour laquelle
(1) au moins un des substituants R₁, R₂, R₃, R₄, R₆, R₇, R₈ représente un radical choisi parmi la liste (L) suivante, les autres R₁, R₂, R₃, R₄, R₆, R₇, R₈ représentant un atome d'hydrogène : acétyl, 2,2-diméthylpropanoyl, benzoyl, 4-diméthylamino-benzoyl, 4-aminobenzoyl, 4-benzyloxyloxybenzoyl, 4-hydroxybenzoyl, 4-méthoxybenzoyl, 4-méthylbenzoyl, 3-méthylbenzoyl, 4-fluorobenzoyl, 3-hydroxybenzoyl, 4-chlorobenzoyl, 4-méthoxycarbonylbenzoyl, (4-acétyl)benzoyl, 4-nitro-benzoyl, 3,5-dichloro-benzoyl, N,N-diisopropylaminométhylènebenzoyl, N,N-diéthylaminométhylènebenzoyl, pentanoyl, (2-acétyloxy)-benzoyl, phénylacétyl, formyl, butanoyl, méthoxy-acétyl, méthoxycarbonylpropanoyl, carboxypropanoyl, carboxybutanoyl, ethoxycarbonylpropènoyl, éthoxycarbonylbutanoyl, benzyloxycarbonylpropanoyl, benzyloxycarbonylbutanoyl, N(phényl)aminocarbonyle, N(benzyl)aminocarbonyle, 2-thiénylcarbonyle, 1-pipéridinylméthylènebenzoyl, N-morpholinylméthylènebenzoyl, N,N-diméthylaminométhylènebenzoyl, N-4méthylpipérazinylméthylènebenzoyl, N(hydroxy-2 éth-1)ylaminométhylènebenzoyl, N-carbamoylméthylaminométhylènebenzoyl, N-éthylaminométhylènebenzoyl, aminométhylènebenzoyl, N-(2-hydroxyéthyl) N-(méthyl) aminométhylènebenzoyl, 2-furanylcarbonyle, phénoxyacétyle, éthoxycarbonyle, N-phénylcarbamoyle, N-benzylcarbamoyle, 4-diméthylaminobutanoyl;
   et plus particulièrement (L') : acétyl, 2,2-diméthylpropanoyl, benzoyl, 4-diméthylamino-benzoyl, 4-benzyloxyloxybenzoyl, 4-hydroxybenzoyl, 4-méthoxybenzoyl, 4-méthylbenzoyl, 3-méthylbenzoyl, 4-fluorobenzoyl, 4-chlorobenzoyl, 4-nitro-benzoyl, 3,5-dichloro-benzoyl, 4(N,N-diisopropylaminométhylène)benzoyl, 4(N,N-diéthylaminométhylène)benzoyl, 4(1-pipéridinylméthylène)benzoyl, 4(N-morpholinylméthylène)benzoyl, 4(N,N-diméthylaminométhylèns)benzoyl, pentanoyl, (2-acétyloxy)-benzoyl, phénylacétyl, formyl, butanoyl, méthoxy-acétyl, méthoxycarbonylpropanoyl, carboxypropanoyl, carboxybutanoyl, ethoxycarbonylpropènoyl, éthoxycarbonylbutanoyl, benzyloxycarbonylpropanoyl, benzyloxycarbonylbutanoyl, N(phényl)aminocarbonyle, N(benzyl)aminocarbonyle, 2-thiénylcarbonyle;
   de préférence :
   (1i) : soit un des substituants R₁ ou R₈ représente un radical choisi parmi la liste (L), préférentiellement (L'), et les autres R₁, R₂, R₃, R₄, R₆, R₇, R₈ représentent un atome d'hydrogène;
   (1ii) : soit les 2 substituants R₁ et R₈ représentent chacun un radical identique choisi parmi la liste (L), préférentiellement (L'), et les autres R₂, R₃, R₄, R₆, R₇ représentent un atome d'hydrogène;
   (1iii) : soit R₁, R₂, R₃, R₄, R₆, R₇, R₈ représentent chacun un radical identique choisi parmi la liste (L), préférentiellement (L');
(2) soit R₁ et R₂ forment ensemble le groupe -CO-, et R₃, R₄, R₆, R₇, R₈ représentent un atome d'hydrogène;
(3) soit R₇ et R₈ forment ensemble le groupe -CO-, et R₁, R₂, R₃, R₄, R₆ représentent un atome d'hydrogène
(4) soit R₁ et R₂ d'une part et R₇ et R₈ d'autre part forment deux à deux le groupe -CO- et R₃, R₄, R₆ représentent des atomes d'hydrogène;
(5) soit R₁ et R₂, R₃ et R_{4,} R₇ et R₈ forment deux à deux le groupe -CO- et R₆ représente un atome d'hydrogène;

A titre d'ingrédient actif avantageux selon l'invention, on peut notamment citer les composés pour lesquels
R₁, R₂, R₃, R₄, R₆, R₇, R₈ représentent chacun un radical acétyl, ou
R₁, R₂, R₃, R₄, R₆, R₇, R₈ représentent chacun un radical benzoyl, ou
R₁ et R₈ représentent chacun un radical benzoyl, et les autres R₂, R₃, R₄, R₆, R₇ représentent chacun un atome d'hydrogène.

### B - composés de formule générale :

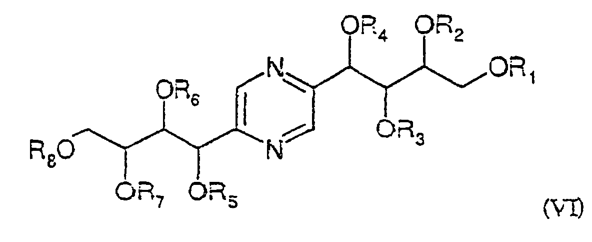

pour laquelle :
(1) au moins un des substituants R₁, R₂, R₃, R₄, R₅, R₆, R₇, R₈ représente un radical choisi parmi la liste (L) citée ci-dessus, et les autres subsubstituants R₁, R₂, R₃, R₄, R₅, R₆, R₇, R₈ représentent un atome d'hydrogène;
   avantageusement, les radicaux de la liste (L) peuvent-être choisis parmi les substituants de la liste (L') citée précédemment;
   de préférence ;
   (1i) : soit un des substituants R₁ ou R₈ représente un radical choisi parmi la liste (L), préférentiellement (L'), et les autres R₁, R₂, R₃, R₄, R₅, R₆, R₇, R₈ représentent un atome d'hydrogène;
   (1ii) : soit les 2 substituants R₁ et R₈ représentent chacun un radical identique choisi parmi la liste (L), préférentiellement (L'), et les autres R₂, R₃, R₄, R₅, R₆, R₇ représentent un atome d'hydrogène;
   (1iii) : soit R₁, R₂, R₃, R₄, R₅, R₆, R₇, R₈ représentent chacun un radical identique choisi parmi la liste (L), préférentiellement (L')
(2) soit R₁ et R₂ forment ensemble le groupe -CO-, et R₃, R₄, R₅, R₆, R₇, R₈ représentent un atome d'hydrogène;
(3) soit R₇ et R₈ forment ensemble le groupe -CO-, et R₁, R₂, R₃, R₄, R₅, R₆ représentent un atome d'hydrogène
(4) soit R₁ et R₂ d'une part et R₇ et R₈ d'autre part forment deux à deux le groupe -CO- et R₃, R₄, R₅, R₆ représentent des atomes d'hydrogène;
(5) soit R₁ et R₂, R₃ et R₄, R₇ et R₈ forment deux à deux le groupe -CO- et R₅, R₆ représente un atome d'hydrogène;
(6) soit R₁ et R₂, R₃ et R_{4,} R₅ et R_{6,} R₇ et R₈ forment deux à deux le groupe -CO-

A titre d'ingrédient actif avantageux selon l'invention, on peut notamment citer les composés pour lesquesl
R₁, R₂, R₃, R₄, R₅, R₆, R₇, R₈ représentent chacun un radical acétyl, ou
R₁, R₂, R₃, R₄, R₅, R₆, R₇, R₈ représentent chacun un radical benzoyl.

### C - composés de formule générale

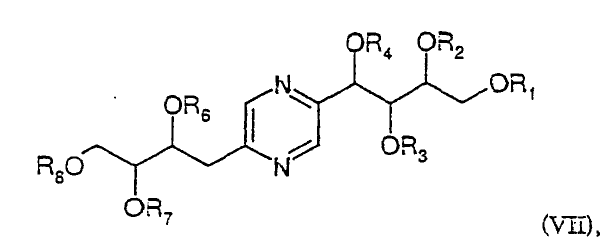

pour laquelle
(1) au moins un des substituants R₁, R₂, R₃, R₄, R₆, R₇, R₈ représente un radical choisi parmi la liste (L) citée ci-dessus, et les autres subsubstituants R₁, R₂, R₃, R₄, R₆, R₇, R₈ représentent un atome d'hydrogène;
   avantageusement, les radicaux de la liste (L) peuvent être choisis parmi les substituants de la liste (L') citée précédemment;
   (1i) : soit un des substituants R₁ ou R₈ représente un radical choisi parmi la liste (L), préférentiellement (L'), et les autres R₁, R₂, R₃, R₄, R₆, R₇, R₈ représentent un atome d'hydrogène;
   (1ii) : soit les 2 substituants R₁ et R₈ représentent chacun un radical identique choisi parmi la liste (L), préférentiellement (L'), et les autres R₂, R₃, R₄, R₆, R₇ représentent un atome d'hydrogène;
   (1iii) : soit R₁, R₂, R₃, R₄, R₆, R₇, R₈ représentent chacun un radical identique choisi parmi la liste (L), préférentiellement (L')
(2) soit R₁ et R₂ forment ensemble le groupe -CO-, et R₃, R₄, R₆, R₇, R₈ représentent un atome d'hydrogène;
(3) soit R₇ et R₈ forment ensemble le groupe -CO-, et R₁, R₂, R₃, R₄, R₆ représentent un atome d'hydrogène
(4) soit R₁ et R₂ d'une part et R₇ et R₈ d'autre part forment deux à deux le groupe -CO- et R₃, R₄, R₆ représentent des atomes d'hydrogène;
(5) soit R₁ et R₂, R₃ et R₄, R₇ et R₈ forment deux à deux le groupe -CO- et R₆ représente un atome d'hydrogène;
avantageusement, R₁ et R₂, R₃ et R₄, R₇ et R₈ forment deux à deux le groupe -CO- et R₆ représente un atome d'hydrogène;
également avantageusement, R₁ et R₂, R₇ et R₈ forment deux à deux le groupe -CO- et R₃ , R₄, R₆ représentent un atome d'hydrogène;
également avantageusement, ou R₁ ,R₂ ,R₆ ou R₄, R₆ ou R₁, R₃, R₄, R₆, R₈ ou R₂, R₃, R₄, R₆, ou R₁, R₃, R₄, R₆, R₇ ou R₂, ou R₁, R₂, R₈, ou R₁, R₂, R₆, R₇, R₈ ou R₆ représentent un radical benzoyl et les autres R₁, R₂, R₃, R₄, R₆, R₇, R₈ représentent un atome d'hydrogène;
également avantageusement, R₂, R₆, R₇ représentent un radical benzoyl et R₃, R₄ forment ensemble le radical -CO- et R₁, R₈ représentent un atome d'hydrogène;
également avantageusement, R₈ représente un radical pentanoyl ou acétyl et les autres R₁, R₂, R₃, R₄, R₆, R₇ représentent un atome d'hydrogène;
également avantageusement, les 2 substituants R₁ et R₈ représentent chacun un radical identique choisi parmi les radicaux acétyl, 2,2-diméthylpropanoyl, benzoyl, 4-diméthylamino-benzoyl, 4-benzyloxyloxybenzoyl, 4-hydroxybenzoyl, 4-méthoxybenzoyl, 4-méthylbenzoyl, 3-méthylbenzoyl, 4-fluorobenzoyl, 4-chlorobenzoyl, 4-nitro-benzoyl, 3,5-dichloro-bsnzoyl, 4(N,N-diisopropylaminométhylène)benzoyl, 4(N,N-diéthylaminométhylène)benzoyl, 4(1-pipéridinylméthylène)benzoyl, 4(N-morpholinylméthylène)benzoyl, 4(N,N-diméthylaminométhylène)benzoyl, pentanoyl, (2-acétyloxy)-benzoyl, phénylacétyl, formyl, butanoyl, méthoxy-acétyl, méthoxycarbonylpropanoyl, carboxypropanoyl, carboxybutanoyl, ethoxycarbonylpropènoyl, éthoxycarbonylbutanoyl, benzyloxycarbonylpropanoyl, benzyloxycarbonylbutanoyl, N(phényl)aminocarbonyle, N(benzyl)aminocarbonyle, 2-thiénylcarbonyle;
et les autres R₂, R₃, R₄, R₆, R₇ représentent un atome d'hydrogène;
également avantageusement, R₁, R₂, R₃, R₄, R₆, R₇, R₈ représentent chacun un radical identique choisi parmi les radicaux benzoyl, acétyl, 2,2-diméthylpropanoyl, (2-acétyloxy)-benzoyl, méthoxyacétyl, pentanoyl, formyl, méthoxycarbonylpropanoyl, carboxypropanoyl, éthoxycarbonylbutanoyl, carboxybutanoyl, éthoxycarbonylpropènoyl, benzyloxycarbonylpropanoyl, benzyloxybutanoyl;
encore plus préférentiellement, les 2 substituants R₁ et R₈ représentent chacun un radical benzoyl, et les autres R₂, R₃, R₄, R₆, R₇ représentent chacun un atome d'hydrogène;
de façon aussi préférentielle, R₁, R₂, R₃, R₄, R₆, R₇, R₈ représentent chacun un radical benzoyl.

Selon l'invention, on préfère plus particulièrement les médicaments contenant en tant que principe actif au moins un composé de formule générale (X) dans laquelle R₁, R₂, R₃, R₄, R₆, R₇, R₈ sont définis comme en formule générale (X).

Les composés suivants sont connus :
- le dérivé 1,2,2',3,3',4,4'-O,O,O,O,O,O,O-heptaacétyle-2-[(1R,2S,3R)(1,2,3,4-tétrahydroxybutyl)] 5-[(2'S,3'R)(2',3',4'-trihydroxybutyl)]pyrazine a été décrit, notamment dans Carbohydr.Res.77, 213-217 (1979), Prog. Food Nutr.Sci.(1981), 5(1-6, Maillard React.Food), 37-45 et Agr.Biol.Chem.37(11),2571-2578, 1973..
- le composé 1,1',2,2',3,3',4,4'-O,O,O,O,O,O,O,O-octaacétyle-2-[(1R,2S,3R)(1,2,3,4-tétrahydroxybutyl)] 5-[(1'R,2'S,3'R)(1',2',3',4'-tétrahydroxybutyl)]pyrazine a été décrit par AVALOS et coll., Tetrahedron, 49, 2655-2675 (1993)
- le composé 1,1',2,2',3,3',4,4'-O,O,O,O,O,O,O,O-octabenzoyle-2-[(1R,2S,3R)(1,2,3,4-tétrahydroxybutyl)] 5-[(1'R,2'S,3'R)(1',2',3',4'-tétrahydroxybutyl)]pyrazine est également connu (M.I.TAHA J.Chem.Soc. 2468-2472, 1961)
- le composé 1,2,2',3,3',4,4'-O,O,O,O,O,O,O-heptaacétyle-2-[(1R,2S,3R)(1,2,3,4-tétrahydroxybutyl)] 6-[(2'S,3'R)(2',3',4'-trihydroxybutyl)]pyrazine est décrit dans Agr.Biol.Chem.37(11), 2571-2578, 1973 et Carbohyd.Res. 23(11),111-119, 1972;
- le composé 1,2,2',3,3',4,4'-O,O,O,O,O,O,O-heptaacétyle-2-[(1R,2S,3S)(1,2,3,4-tétrahydroxybutyl)] b-[(2'S,3'S)(2',3',4'-trihydroxybutyl)]pyrazine a été décrit dans Carbohydr.Res. 26(2), 377-384, 1973, et

Cependant aucune activité biologique concernant ces dérivés n'a été découverte jusqu'à maintenant.

Un autre objet de la présente invention concerne donc également les composés de formule générale (I), à l'exception de 1,2,2',3,3',4,4'-O,O,O,O,O,O,O-heptaacétyle-2-[(1R,2S,3R)(1,2,3,4-tétrahydroxybutyl)] 3-[(2'S,3'R)(2',3',4'-trihydroxybutyl)]pyrazine, 1,1',2,2',3,3',4,4'-O,O,O,O,O,O,O,O-octaacétyle-2-[(1R,2S,3R)(1,2,3,4-tétrahydroxybutyl)] 5-[(1'R,2'S,3'R)(1',2',3',4'-tétrahydroxybutyl)]pyrazine, 1,1',2,2',3,3',4,4'-O,O,O,O,O,O,O,O-octabenzoyle-2-[(1R,2S,3R)(1,2,3,4-tétrahydroxybutyl)] 5-[(1'R,2'S,3'R)(1',2',3',4'-tétrahydroxybutyl)]pyrazine 1,2,2',3,3',4,4'-O,O,O,O,O,O,O-heptaacétyle-2-[(1R,2S,3R)(1,2,3,4-tétrahydroxybutyl)] 6-[(2'S,3'R)(2',3',4'-trihydroxybutyl)]pyrazine 1,2,2',3,3',4,4'-O,O,O,O,O,O,O-heptaacétyle-2-[(1R,2S,3S)(1,2,3,4-tétrahydroxybutyl)] 6-[(2'S,3'S)(2',3',4'-trihydroxybutyl)]pyrazine

Les composés de formule générale (I) particulièrement représentatifs selon l'invention sont notamment :

### A - composés de formule générale :

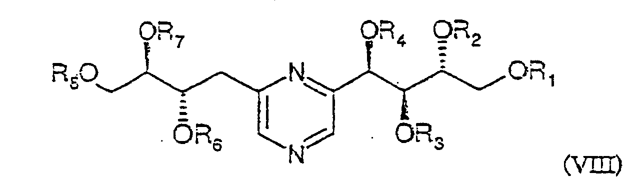

pour laquelle
(1) au moins un des substituants R₁, R₂, R₃, R₄, R₆, R₇, R₈ représente un radical choisi parmi la liste (L) citée ci-dessus, et les autres subsubstituants R₁, R₂, R₃, R₄, R₆, R₇, R₈ représentent un atome d'hydrogène.
   avantageusement, les radicaux de la liste (L) peuvent être choisis parmi les substituants de la liste (L'), citée précédemment;
   de préférence :
   (1i) soit un des substituants R₁ ou R₈ représente un radical choisi parmi la liste (L), préférentiellement (L'), et les autres R₁, R₂, R₃, R₄, R₆, R₇, R₈ représentent un atome d'hydrogène
   (1ii) soit les 2 substituants R₁ et R₈ représentent chacun un radical identique choisi parmi la liste (L), préférentiellement (L'), et les autres R₂, R₃, R₄, R₆, R₇ représentent un atome d'hydrogène
   (1iii) soit R₁, R₂, R₃, R₄, R₆, R₇, R₈ représentent chacun un radical identique choisi parmi la liste (L), préférentiellement (L');
(2) soit R₁ et R₂ forment ensemble le groupe -CO-, cyclohexyle ou cyclopentyle et R₃, R₄, R₆, R₇, R₈ représentent un atome d'hydrogène;
(3) soit R₇ et R₈ forment ensemble le groupe -CO-, cyclohexyle ou cyclopentyle et R₁, R₂, R₃, R₄, R₆ représentent un atome d'hydrogène
(4) soit R₁ et R₂ d'une part et R₇ et R₈ d'autre part forment deux à deux le groupe -CO- et R₃, R₄, R₆ représentent des atomes d'hydrogène;
(5) soit R₁ et R₂, R₃ et R₄, R₇ et R₈ forment deux à deux le groupe -CO- et R₆ représente un atome d'hydrogène;
à l'exception de :
1,2,2',3,3',4,4'-O,O,O,O,O,O,O-heptaacétyle-2-[(1R,2S,3R)(1,2,3,4-tétrahydroxybutyl)] 6-[(2'S,3'R)(2',3',4'-trihydroxybutyl)]pyrazine 1,2,2',3,3',4,4'-O,O,O,O,O,O,O-heptaacétyle-2-[(1R,2S,3S)(1,2,3,4-tétrahydroxybutyl)] 6-[(2'S,3'S)(2',3',4'-trihydroxybutyl)]pyrazine;

Les composés avantageux selon l'invention sont notamment ceux pour lesquels
R₁, R₂, R₃, R₄, R₆, R₇, R₈ représentent chacun un radical benzoyl, ou
R₁ et R₈ représentent chacun un radical benzoyl, et les autres R₂, R₃, R₄, R₆, R₇ représentent chacun un atome d'hydrogène.

### B - composés de formule générale :

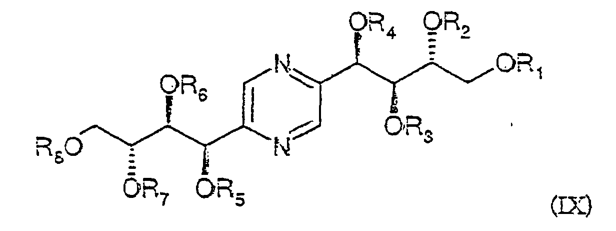

pour laquelle :
(1) au moins un des substituants R₁, R₂, R₃, R₄, R₅, R₆, R₇, R₈ représente un radical choisi parmi la liste (L) citée ci-dessus, et les autres subsubstituants R₁, R₂, R₃, R₄, R₅, R₆, R₇, R₈ représentent un atome d'hydrogène;
   avantageusement, les radicaux de la liste (L) peuvent être choisis parmi les substituants de la liste (L') citée précédemment;
   de préférence :
   (1i) : soit un des substituants R₁ ou R₈ représente un radical choisi parmi la liste (L), préférentiellement (L'), et les autres R₁, R₂, R₃, R₄, R₅, R₆, R₇, R₈ représentent un atome d'hydrogène;
   (1ii) : soit les 2 substituants R₁ et R₈ représentent chacun un radical identique choisi parmi la liste (L), préférentiellement (L'), et les autres R₂, R₃, R₄, R₅, R₆, R₇ représentent un atome d'hydrogène;
   (1iii) : soit R₁, R₂, R₃, R₄, R₅, R₆, R₇, R₈ représentent chacun un radical identique choisi parmi la liste (L), préférentiellement (L');
(2) soit R₁ et R₂ forment ensemble le groupe -CO-, et R₃, R₄, R₅, R₆, R₇, R₈ représentent un atome d'hydrogène;
(3) soit R₇ et R₈ forment ensemble le groupe -CO-, et R₁, R₂, R₃, R₄, R₅, R₆ représentent un atome d'hydrogène
(4) soit R₁ et R₂ d'une part et R₇ et R₈ d'autre part forment deux à deux le groupe -CO- et R₃, R₄, R₅, R₆ représentent des atomes d'hydrogène;
(5) soit R₁ et R₂, R₃ et R_{4,} R₇ et R₈ forment deux à deux le groupe -CO- et R₅, R₆ représente un atome d'hydrogène;
(6) soit R₁ et R₂, R₃ et R₄, R₅ et R₆, R₇ et R₈ forment deux à deux le groupe -CO-
à l'exception, de
1,1',2,2',3,3',4,4'-O,O,O,O,O,O,O,O-octaacétyle-2-[(1R,2S,3R)(1,2,3,4-tétrahydroxybutyl)]-5-[(1'R,2'S,3'R)(1',2',3',4'-tétrahydroxybutyl)]pyrazine,
1,1',2,2',3,3',4,4'-O,O,O,O,O,O,O,O-octabenzoyle-2-[(1R,2S,3R)(1,2,3,4-tétrahydroxybutyl)]5-[(1'R,2'S,3'R)(1',2',3',4'-tétrahydroxybutyl)]pyrazine

### C - composés de formule générale

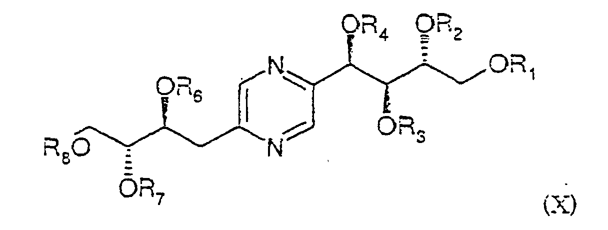

pour laquelle
(1) au moins un des substituants R₁, R₂, R₃, R₄, R₆, R₇, R₈ représente un radical choisi parmi la liste (L) citée ci-dessus, et les autres subsubstituants R₁, R₂, R₃, R₄, R₆, R₇, R₈ représentent un atome d'hydrogène;
   avantageusement, les radicaux de la liste (L) peuvent être choisis parmi les substituants de la liste (L') citée précédemment;
   de préférence :
   (1i) : soit un des substituants R₁ ou R₈ représente un radical choisi parmi la liste (L), préférentiellement (L'), et les autres R₁, R₂, R₃, R₄, R₆, R₇, R₈ représentent un atome d'hydrogène;
   (1ii) : soit les 2 substituants R₁ et R₈ représentent chacun un radical identique choisi parmi la liste (L), préférentiellement (L'), et les autres R₂, R₃, R₄, R₆, R₇ représentent un atome d'hydrogène;
   (1iii) : soit R₁, R₂, R₃, R₄, R₆, R₇, R₈ représentent chacun un radical identique choisi parmi la liste (L), préférentiellement (L');
(2) soit R₁ et R₂ forment ensemble le groupe -CO-, et R₃, R₄, R₆, R₇, R₈ représentent un atome d'hydrogène;
(3) soit R₇ et R₈ forment ensemble le groupe -CO-, et R₁, R₂, R₃, R₄, R₆ représentent un atome d'hydrogène
(4) soit R₁ et R₂ d'une part et R₇ et R₈ d'autre part forment deux à deux le groupe -CO- et R₃, R₄, R₆ représentent des atomes d'hydrogène;
(5) soit R₁ et R₂, R₃ et R_{4,} R₇ et R₈ forment deux à deux le groupe -CO- et R₆ représente un atome d'hydrogène;
à l'exception de 1,2,2',3,3',4,4'-O,O,O,O,O,O,O-heptaacétyle-2-[(1R,2S,3R)(1,2,3,4-tétrahydroxybutyl)] 5-[(2'S,3'R)(2',3',4'-trihydroxybutyl)]pyrazine,
avantageusement, R₁ et R₂, R₃ et R₄, R₇ et R₈ forment deux à deux le groupe -CO- et R₆ représente un atome d'hydrogène;
également avantageusement, R₁ et R₂, R₇ et R₈ forment deux à deux le groupe -CO- et R₃ , R₄, R₆ représentent un atome d'hydrogène;
également avantageusement, ou R₁ ,R₂ ,R₆ ou R₄, R₆ ou R₁, R₃, R₄, R₆, R₈ ou R₂, R₃, R₄, R₆, ou R₁, R₃, R₄, R₆, R₇ ou R₂, ou R₁, R₂, R₈, ou R₁, R₂, R₆, R₇, R₈ ou R₆ représentent un radical benzoyl et les autres R₁, R₂, R₃, R₄, R₆, R₇, R₈ représentent un atome d'hydrogène;
également avantageusement, R₂, R₆, R₇ représentent un radical benzoyl et R₃, R₄ forment ensemble le radical -CO- et R₁, R₈ représentent un atome d'hydrogène;
également avantageusement, R₈ représente un radical pentanoyl ou acétyl et les autres R₁, R₂, R₃, R₄, R₆, R₇ représentent un atome d'hydrogène;
également avantageusement, les 2 substituants R₁ et R₈ représentent chacun un radical identique choisi parmi les radicaux acétyl, 2,2-diméthylpropanoyl, benzoyl, 4-diméthylamino-benzoyl, 4-benzyloxyloxybenzoyl, 4-hydroxybenzoyl, 4-méthoxybenzoyl, 4-méthylbenzoyl, 3-méthylbenzoyl, 4-fluorobenzoyl, 4-chlorobenzoyl, 4-nitro-benzoyl, 3,5-dichloro-benzoyl, 4(N,N-diisopropylaminométhylène)benzoyl, 4(N,N-diéthylaminométhylène)benzoyl, 4(1-pipéridinylméthylène)benzoyl, 4(N-morpholinylméthylène)benzoyl, 4(N,N-diméthylaminométhylène)benzoyl, pentanoyl, (2-acétyloxy)-benzoyl, phénylacétyl, formyl, butanoyl, méthoxy-acétyl, méthoxycarbonylpropanoyl, carboxypropanoyl, carboxybutanoyl, ethoxycarbonylpropènoyl, éthoxycarbonylbutanoyl, benzyloxycarbonylpropanoyl, benzyloxycarbonylbutanoyl, N(phényl)aminocarbonyle, N(benzyl)aminocarbonyle, 2-thiénylcarbonyle;
et les autres R₂, R₃, R₄, R₆, R₇ représentent un atome d'hydrogène;
également avantageusement, R₁, R₂, R₃, R₄, R₆, R₇, R₈ représentent chacun un radical identique choisi parmi les radicaux benzoyl, 2,2-diméthylpropanoyl, (2-acétyloxy)-benzoyl, méthoxyacétyl, pentanoyl, formyl, méthoxycarbonylpropanoyl, carboxypropanoyl, éthoxycarbonylbutanoyl, carbaxybutanoyl, éthoxycarbonylpropènoyl, benzyloxycarbonylpropanoyl, benzyloxybutanoyl;
encore plus préférentiellement, les 2 substituants R₁ et R₈ représentent chacun un radical benzoyl, et les autres R₂, R₃, R₄, R₆, R₇ représentent chacun un atome d'hydrogène;
de façon aussi préférentielle, R₁, R₂, R₃, R₄, R₆, R₇, R₈ représentent chacun un radical benzoyl.

Selon l'invention, on préfère comme composés, les composés de formule (X) dans laquelle R₁, R₂, R₃, R₄, R₆, R₇, R₈ sont définis comme en formule générale (X).

Selon l'invention, le procédé de préparation peut être mis en oeuvre de la façon suivante:

Les composés de formule générale (V), (VI), (VII), peuvent être obtenus respectivement en faisant réagir sur les composés de formule (XI), resp.(XII), (XIII) : 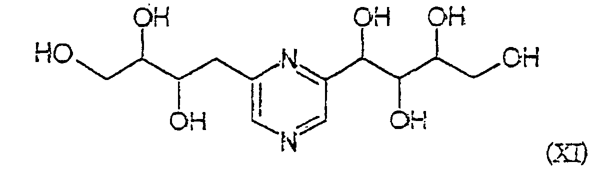 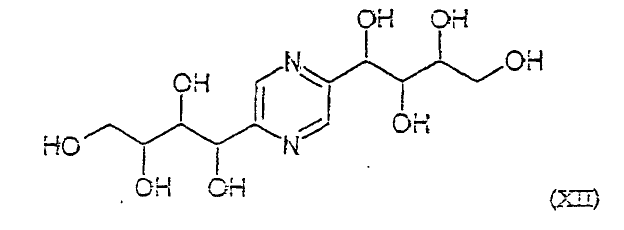 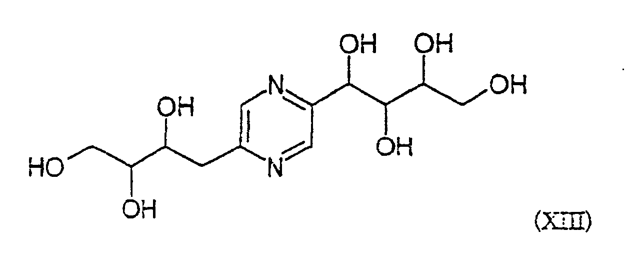 pour lesquels les fonctions hydroxyles -OH sont éventuellement protégées par des groupes protecteurs,
- soit un composé de formule R-X (XIV) dans laquelle R représente de préférence les groupements -COR_{9,} -COOR_{10,} -CR₁₁R₁₂OCOR_{13,} -CR₁₁R₁₂OR_{13,} -CONR₁₄R₁₅ et X représente un atome d'halogène de préférence le chlore ou le brome
- soit un composé de formule (R₉CO)₂O (XV)
- soit un composé de formule R₁₄N=C=O (XVI)
- soit un composé de formule CR₁₁R₁₂(OR₁₃)₂ (XVII). suivi éventuellement d'une déprotection totale, ou suivi éventuellement d'une déprotection sélective et d'une ou plusieurs autres fonctionnalisations à l'aide d'un des réactifs de formule (XIV), (XV), (XVI), (XVII) identiques ou différents du premier, les étapes de fonctionnalisation et de déprotection pouvant être répétées plusieurs fois, étant entendu que les réactifs de formule (XIV), (XV), (XVI) et (XVII) peuvent être identiques ou différents à chaque fonctionnalisation.

Les réactions de fonctionnalisation sont effectuées sur un ou plusieurs des groupes hydroxyles non protégés des composés de formule (XI), (XII), (XIII) et consistent en l'introduction de un ou plusieurs substituants R_{1,} R_{2,} R_{3,} R_{4,} R_{5,} R_{6,} R_{7,} R₈ identiques ou différents qui représentent un ou plusieurs des groupes fonctionnels de type -COR₉ _{,} -COOR_{10,} -CR₁₁R₁₂OCOR_{13,} -CR₁₁R₁₂OR_{13,} -(CO)-NR₁₄R₁₅. Ces réactions peuvent être effectuées selon toutes les méthodes connues de fonctionnalisation de fonctions hydroxyles et plus particulièrement et de manière non limitative, on préfèrera opérer sauf mention particulière dans les conditions suivantes :
- dans des solvants polaires, aprotiques, les éthers aliphatiques ou aromatiques, les nitriles, les solvants halogénés ou les milieux biphasiques; les solvants préférés sont notamment la pyridine, le tétrahrydrofurane (THF), l'éther diéthylique, le dioxane, le glyme, le diméthylformamide (DMF), le diméthylesulfoxide (DMSO).
- en milieu basique, en présence d'une base organique ou inorganique de type amine aliphatique ou aromatique et de préférence la triéthylamine ou la pyridine, un amidure, les alkyllithiens, les carbonates de métaux alcalins par exemple le carbonate de potassium (K₂CO₃), les hydroxydes de métaux alcalins, les alcoxydes de métaux alcalins, les hydrures de métaux alcalins et plus particulièrement l'hydrure de sodium (NaH), ou encore le 1,8-diazabicyclo[5.4.0]undec-7-ène (DBU).
- à une température comprise entre -78°C et +100°C.

On peut notamment introduire les fonctions
- ester de type -COR₉ (a),
   - par action d'un réactif R₉COX,
   - par action d'un anhydride de formule (R₉CO)₂O

Selon l'invention, on préfèrera opérer plus particulièrement par action de chlorure d'acyle dans la pyridine.
- carbonate de type -COOR₁₀ (b),
   par action d'un composé de formule R₁₀OCOX, suivi éventuellement d'une cyclisation en milieu basique pour obtenir le carbonate de type cyclique (f) correspondant pour lequel le groupe -C(O)- lie deux atomes d'oxygène proches.

D'un intérêt tout particulier sont les carbonates d'alkyle obtenus avec le chloroformiate d'alkyle correspondant en présence de pyridine.
- éther
   - de type -CR₁₁R₁₂OCOR₁₃ (c),
   par action d'un réactif de formule R₁₂OCOR₁₁R₁₂X ou
   - de type -CR₁₁R₁₂OR₁₃ (d),
en faisant réagir R₁₃OCR₁₁R₁₂X ou par action de CR₁₁R₁₂(OR₁₃)₂ en milieu acide : Un réactif particulièrement utilisé est de type chlorométhylalkyléther et l'acide utilisé peut être tout acide organique ou inorganique tel que l'acide méthanesulfonique, l'acide paratoluènesulfonique (APTS), l'acide camphresulfonique (CSA), le p-toluènesulfonate de pyridinium (PPTS), l'acide sulfurique (H₂SO₄) ou l'acide chlorhydrique (HCl).
- carbamate de type -CONR₁₄R₁₅ (e),
par action de R₁₄R₁₅NCOX ou R₁₄-N=C=O quand R₁₅ représente un atome d'hydrogène;
étant entendu que les quantités utilisées de réactif dépendent de la nature et du nombre de fonctionnalisations souhaitées.
Il est également possible, suivant les conditions opératoires utilisées, que les groupes fonctionnels d'alcools secondaires migrent vers les alcools primaires déprotégés (transestérification).
Les réactifs de formule (XIV), (XV), (XVI) et (XVII) sont soit disponibles commercialement soit peuvent être synthétisés par application ou adaptation des procédés de préparation connus, comme par exemple les méthodes décrites par J.March, Advanced Organic Chemistry, 4ème édition, 1992, J.Wiley (p.365, 400-401, 405,437,1053, 1290, 1294).

L'expression "les groupes protecteurs de fonctions hydroxyles" se rapporte à tous les groupes, qui, en fonction de la réaction pour laquelle ils sont utilisés, peuvent être éliminés sans affecter le reste de la molécule. En particulier, les groupes protecteurs et leur mise en oeuvre dans le procédé selon l'invention sont de façon non limitative ceux décrits par T.W.Greene, Protective Groups in Organic Synthesis, J.Wiley, Interscience publications (1991) et P.I.Kocienski, Protecting groups, Georg Thieme Verlag, 1994. De préférence, on utilisera les groupes protecteurs et les mises en oeuvre suivants :
- les groupements alkylènes, aralkylènes, diarylméthylènes formant avec les carbones et les oxygènes auxquels ils sont attachés un hétérocycle contenant de préférence 5 à 7 chaînons, dont 2 atomes d'oxygène. Les protections cycliques de type acétal ou cétal cycliques de formule 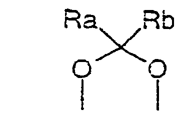 sont tout particulièrement préférées selon l'invention et peuvent être introduites de la façon suivante : 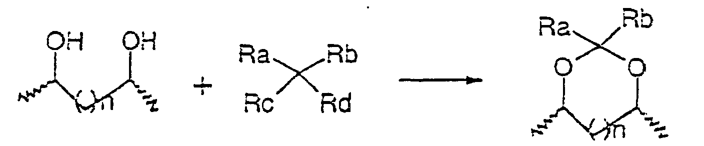 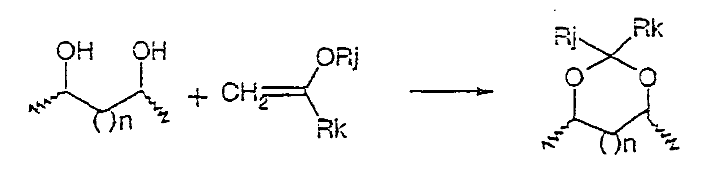
où Ra, Rb sont identiques ou différents et représentent indépendamment soit un atome d'hydrogène, un radical alkyle éventuellement substitué tel que méthyle, éthyle, un radical alkyloxy, soit Ra et Rb forment avec le carbone auquel ils sont rattachés un cycle de 4 à 7 atomes de carbone;
Rc, Rd identiques ou différents représentent un radical alkyloxy ou forment ensemble avec le carbone auquel ils sont attachés un groupe carbonyle -CO-; d'un intérêt tout particulier sont les réactifs tels que l'acétone, la pentan-3-one ou encore la cyclopentanone, cyclohexanone; on pourra utiliser éventuellement un agent activant tel que le trialkylorthoformiate, et notamment le triéthylorthoformiate;
Rj représente un radical alkyle, méthyle de préférence;
Rk représente un atome d'hydrogène ou un radical alkyle et méthyle en particulier; l'indice n est égal à 0 ou 1.
Cette réaction s'effectue par catalyse acide, par exemple en présence de PPTS, dans un solvant convenablement choisi, tel que les cétones et l'acétone en particulier, le THF, le dichlorométhane, le toluène, le chloroforme, à une température comprise entre -20°C et la température de reflux du milieu utilisé.

Ce type de protection acétonide peut particulièrement être utilisé pour former une protection simultanée des groupes hydroxyles en position 1-2, 1-3, 2-3, 2-4, 3-4, 1'-2', 1'-3', 2'-3', 2'-4', 3'-4'.
- silylés selon la réaction : 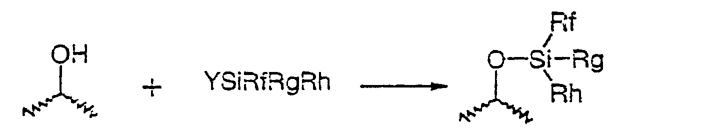
où Rf, Rg, Rh identiques ou différents sont alkyle, aryle et Y représente un atome d'halogène, en particulier l'atome de chlore, ou un groupe sulfonate tel que le trifluorométhanesulfonate. La réaction de silylation s'effectue à une tempéraure comprise entre -78°C et 100°C en milieu basique.
Les groupes protecteurs silylés -SfRfRgRh préférentiellement utilisés sont les radicaux trialkylsilyle, alkyldiphénylsilyle ou dialkylphénylsilyle et de préférence les radicaux tri-méthylsilyle, tert-butyl(diphényl)silyle (TBDPS), t-butyl(diméthyl)silyle (TBDMS) et diméthylphénylsilyle.
- éthers effectuées avec un réactif de formule ReZ, où Re représente des radicaux 2-triméthylsilyléthyle, alkyloxyméthyle, aralkyles de préférence le radical benzyle et Z représente un atome d'halogène, et en particulier un atome de chlore ou de brome. De façon préférentielle, on utilisera NaH, NaI comme base, dans le DMF. Eventuellement, on pourra opérer en présence d'un agent activant tel que un iodure de métal alcalin, de préférence NaI ou un iodure d'ammonium.
Egalement, une autre protection de type éther peut être effectuée par des groupes hétérocycliques, dont un exemple particulièrement représentatif est le tétrahydropyrane (THP), selon la réaction :

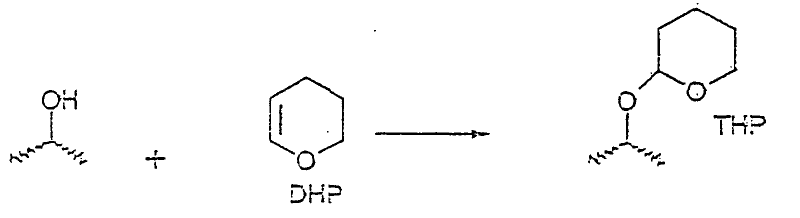

Cette réaction s'effectue de préférence dans le dichlorométhane en présence de PPTS.
- carbonates soit de type -OCORi, où Ri représente un radical alkyle, aryle, le phényle par exemple, ou aralkyle comme le benzyle de préférence, éventuellement substitué, soit de type cyclique tel que 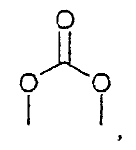
ces protections pouvant notamment être obtenues selon la séquence 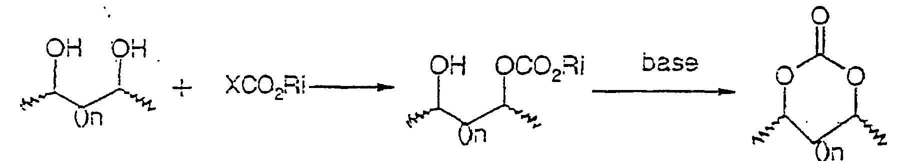 où n est défini comme précédemment;
- par greffage sur un polymère des radicaux alcools terminaux selon la méthode décrite par J.Y. Wong et C.C.Leznoff, Can.J.Chem., 51, 2452 (1973).

Les étapes de déprotection sont effectuées sur les groupes hydroxyles éventuellement protégés des composés de formule (XI), (XII), (XIII), c'est-à-dire les OH pour lesquels un ou plusieurs substituants R_{1,} R_{2,} R_{3,} R_{4,} R_{5,} R_{6,} R_{7,} R₈ identiques ou différents représentent des groupes protecteurs identiques ou différents de radicaux hydroxyles. Ces réactions peuvent être effectuées selon les méthodes habituelles connues de l'homme de l'art. Notamment, on pourra utiliser les méthodes citées par T.W.Greene, Protective Groups in Organic Synthesis, J.Wiley, Interscience publications (1991) et P.J.Kocienski, Protecting groups, Georg Thieme Verlag, 1994. Selon les groupes protecteurs utilisés dans les intermédiaires de formule (XI), (XII), (XIII), on pourra utiliser de préférence soit les bases organiques ou minérales, soit les acides organiques ou minéraux ou leurs dérivés tels que l'acide acétique, l'acide trifluoroacétique, l'acide fluorhydrique ou encore l'acide chlorhydrique en solution aqueuse ou organique, en quantités catalytique, stoechiométrique ou en excès, soit en présence d'ions fluorure F⁻ sous forme de fluorure de tétrabutylammonium, soit l'hydrogénation catalytique sur Pd/C par exemple, soit encore la réduction, par NaBH₄ de préférence.

Les schémas réactionnels suivants illustrent les méthodes de préparation des intermédiaires utilisés selon l'invention de façon préférentielle et non limitative; ils n'en donnent pas une vue exhaustive et ne sont donnés qu'à titre d'exemples.

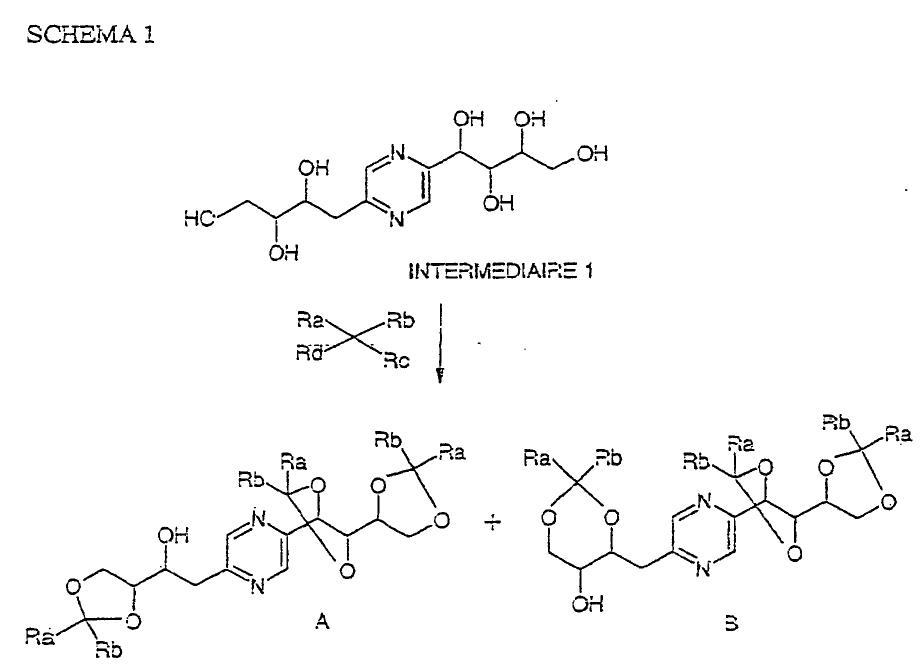

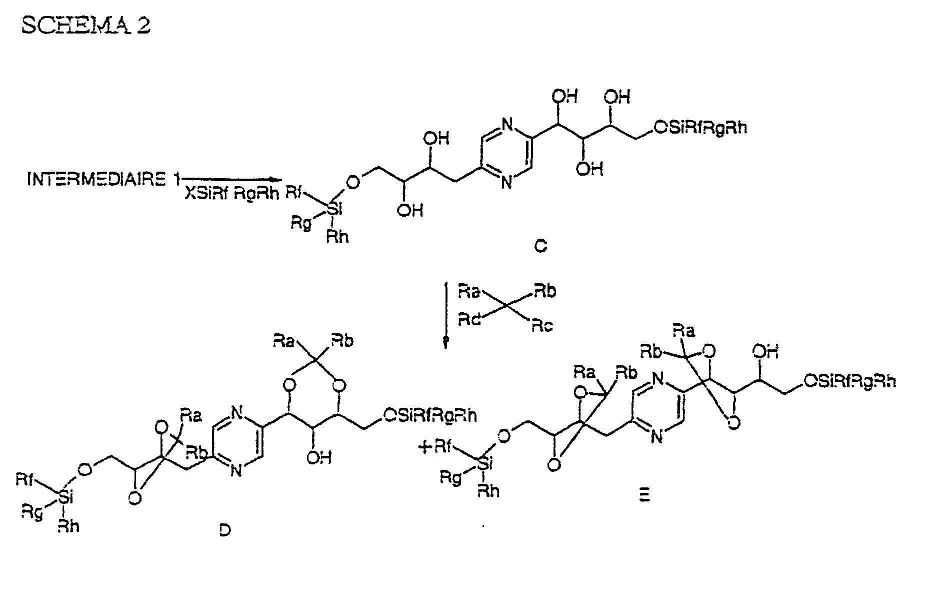

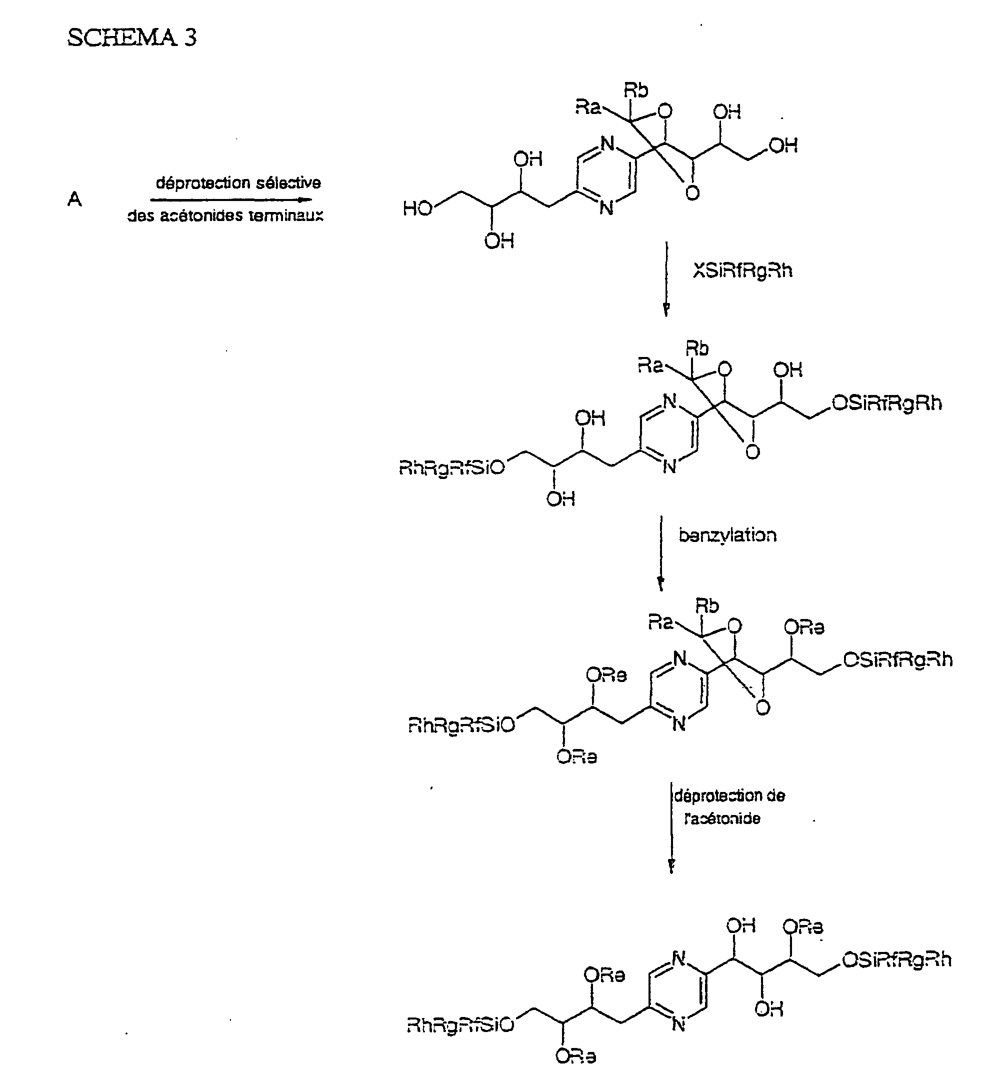

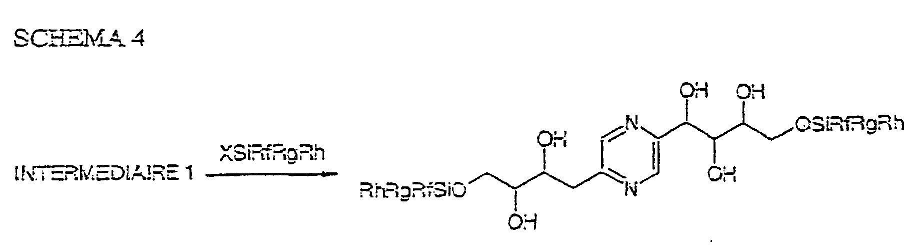

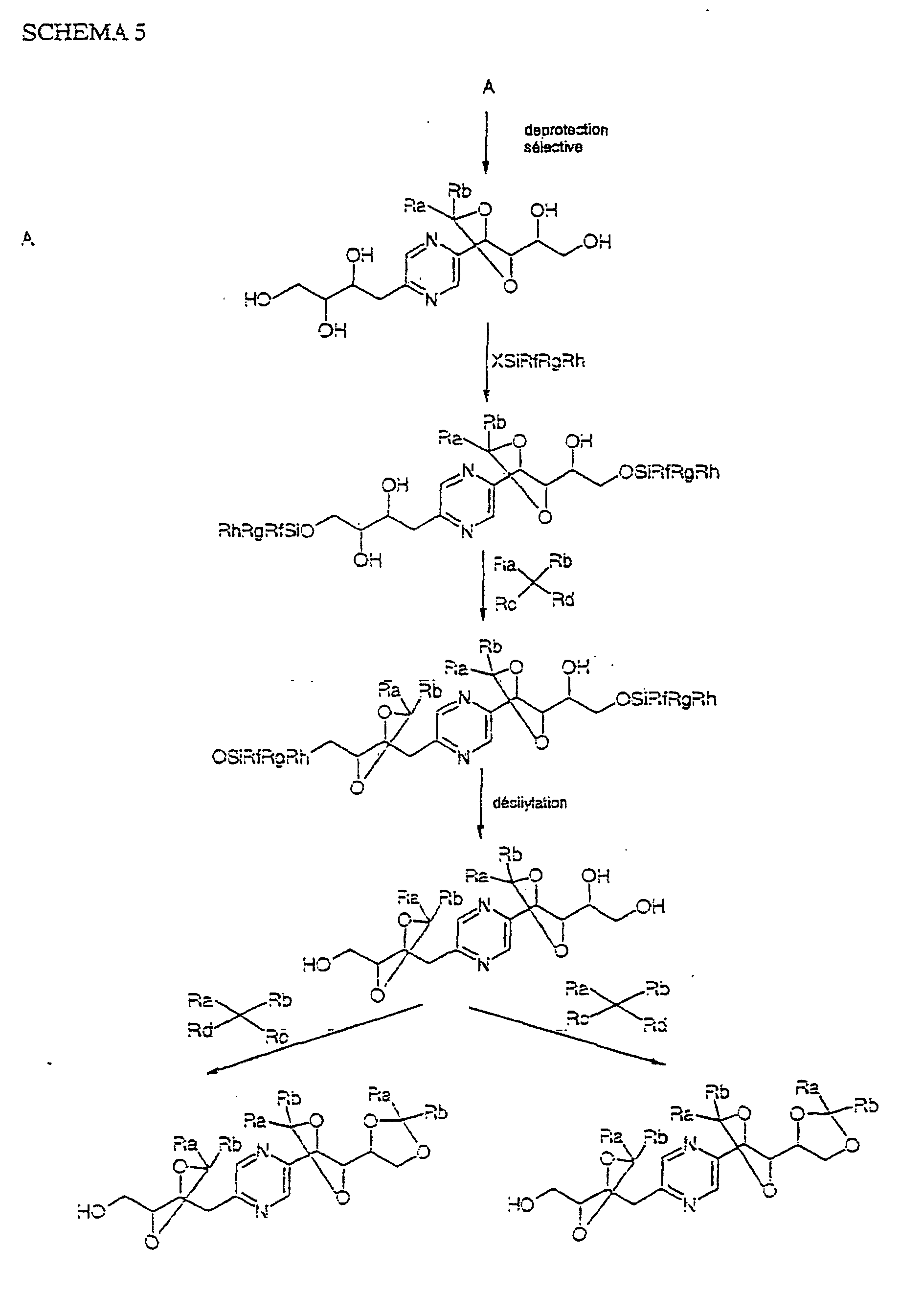

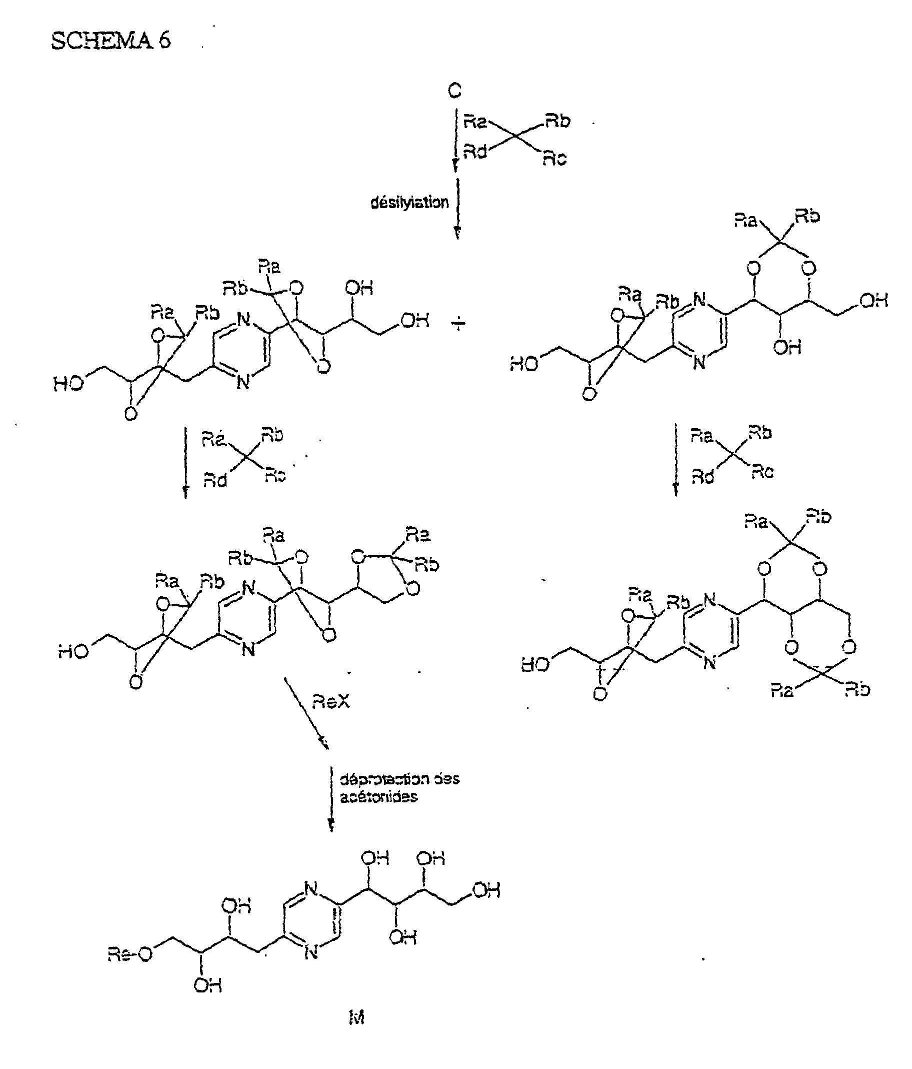

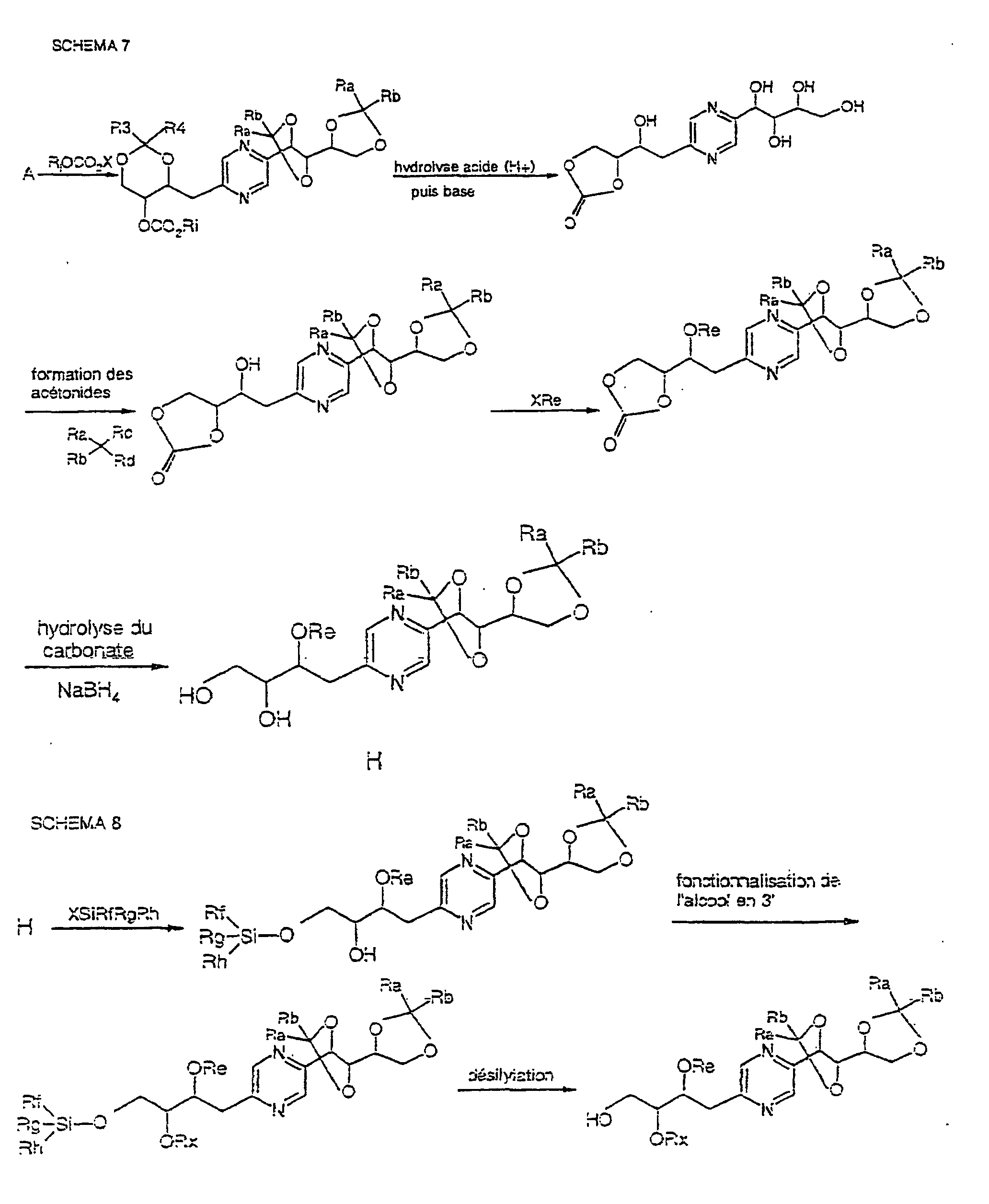

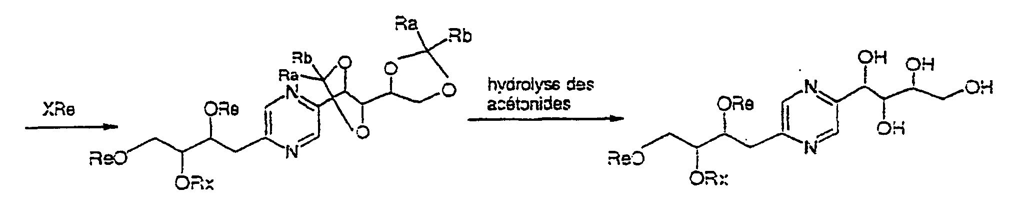

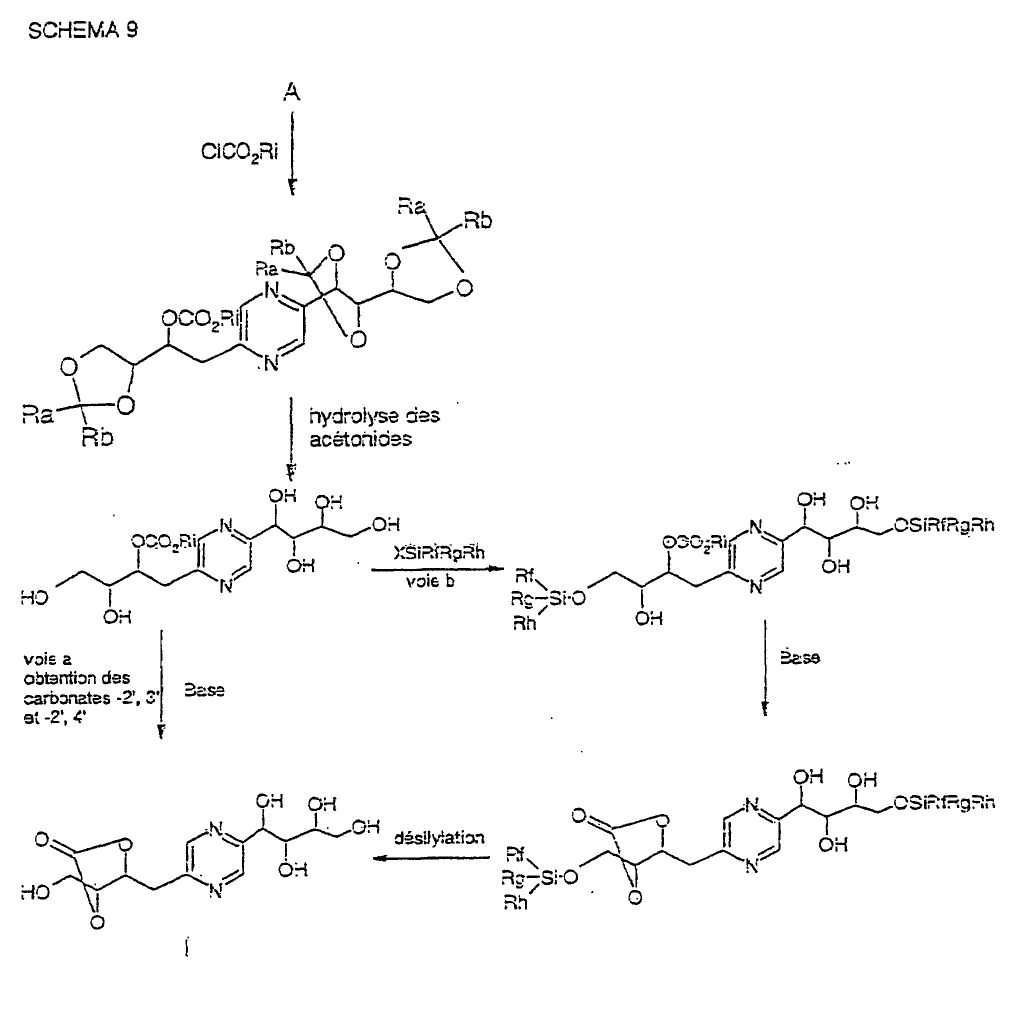

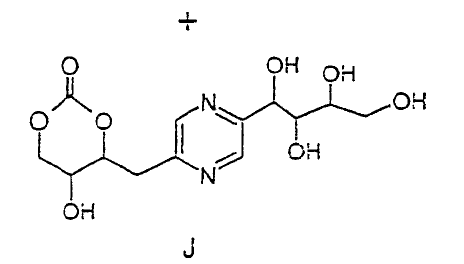

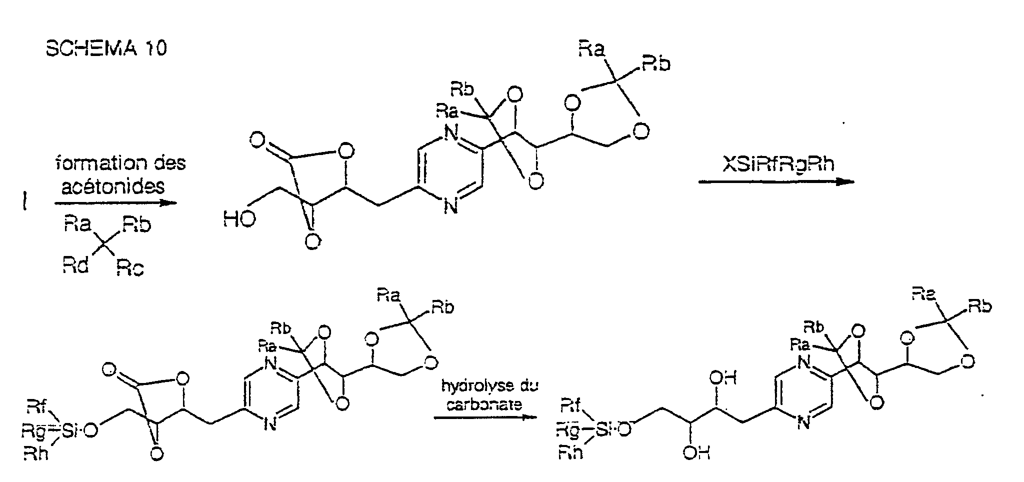

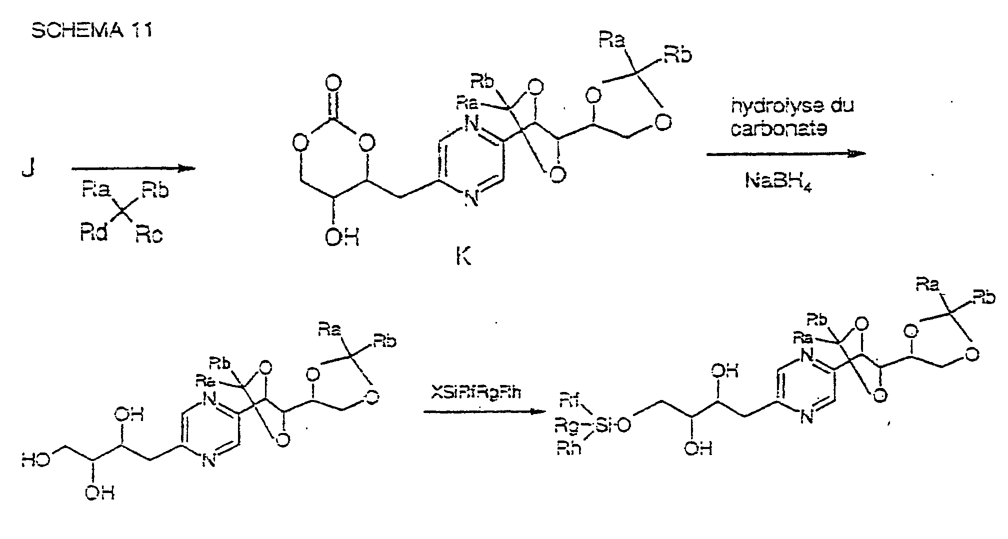

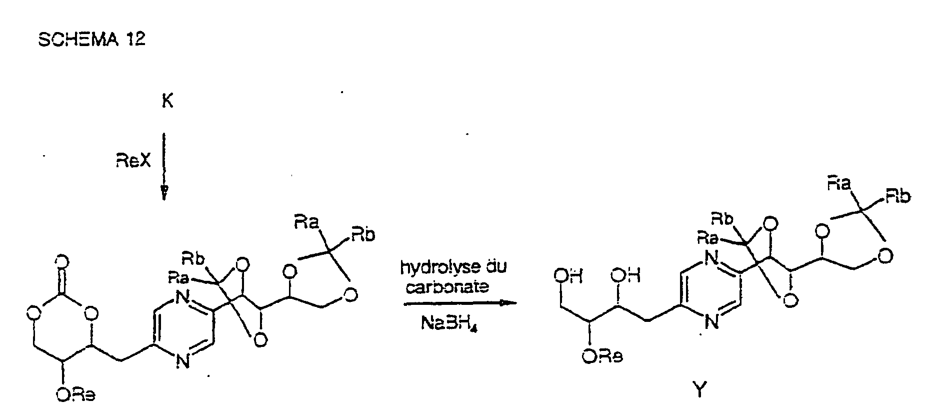

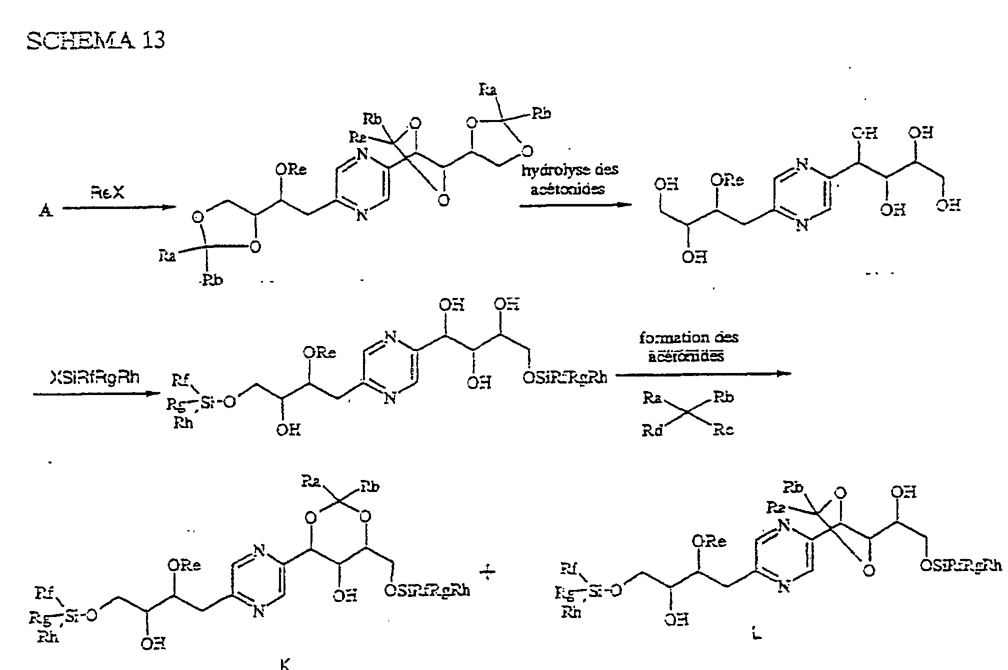

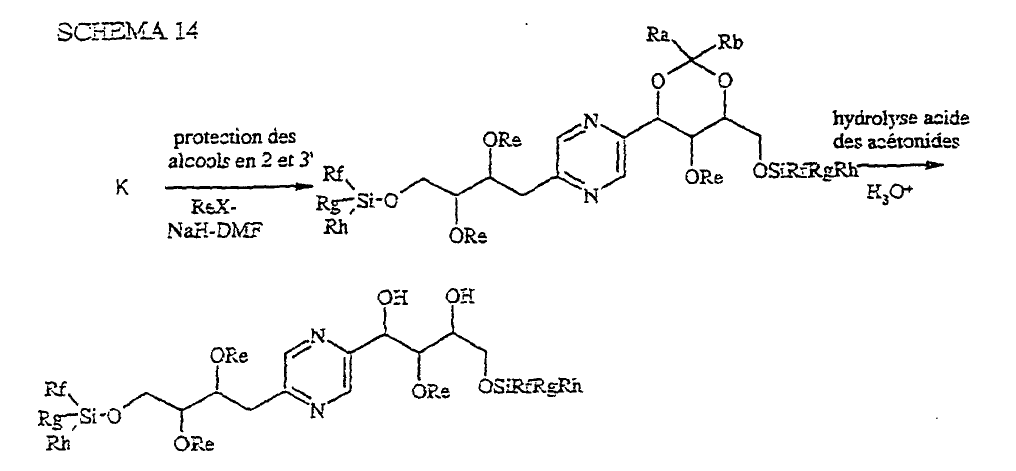

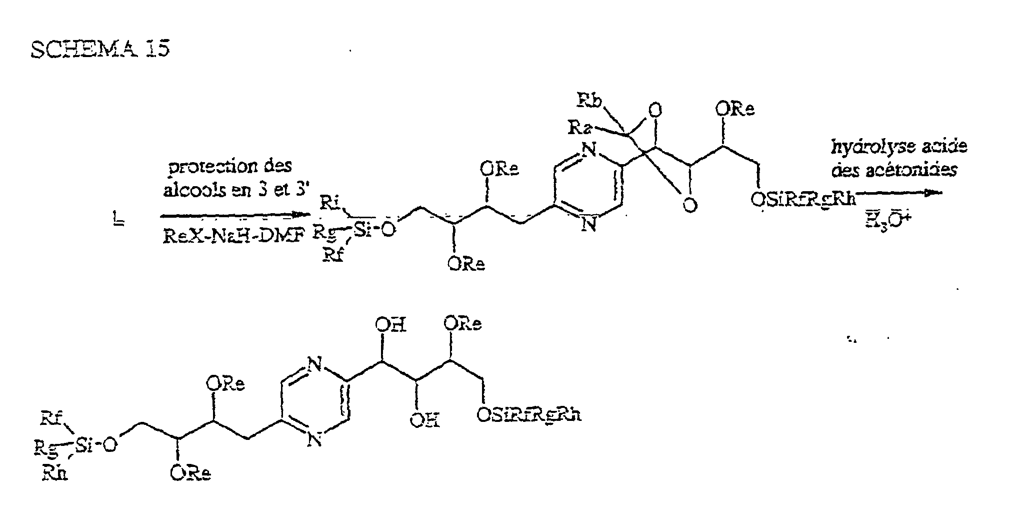

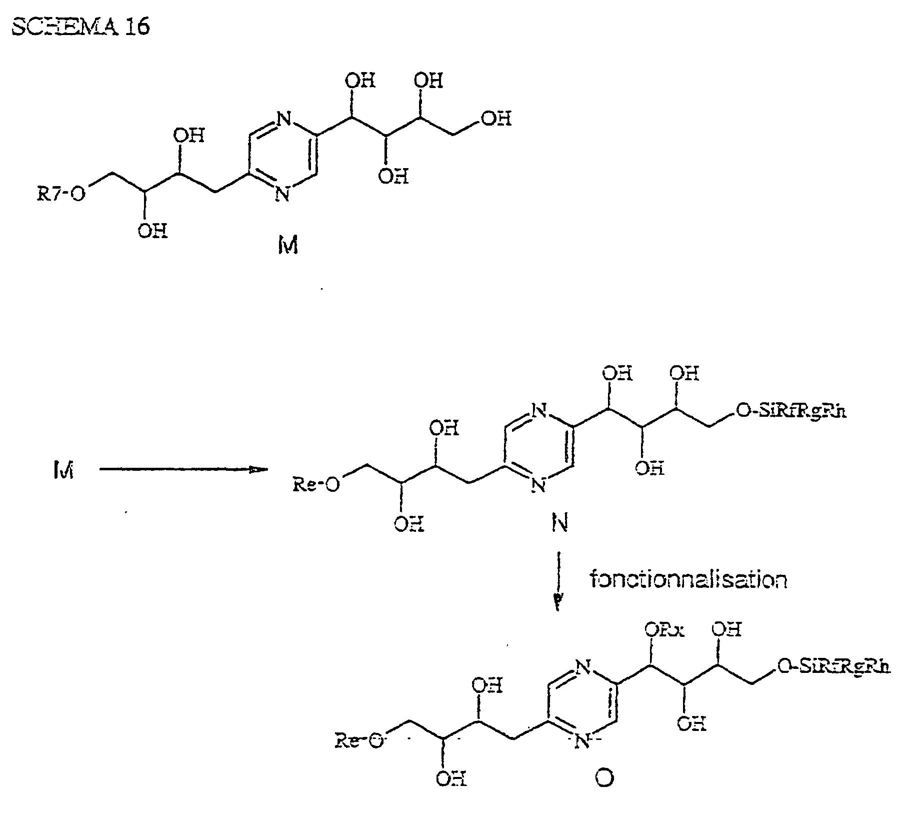

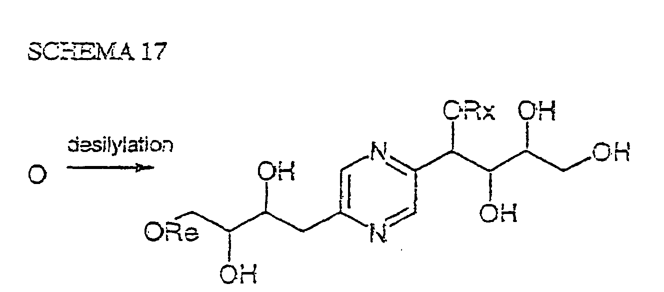

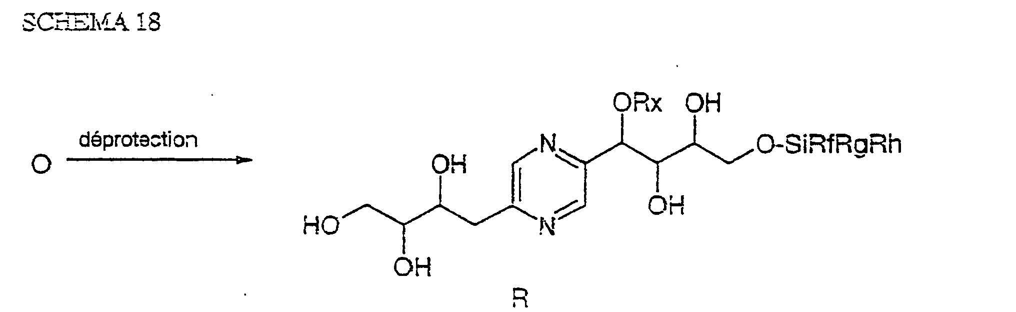

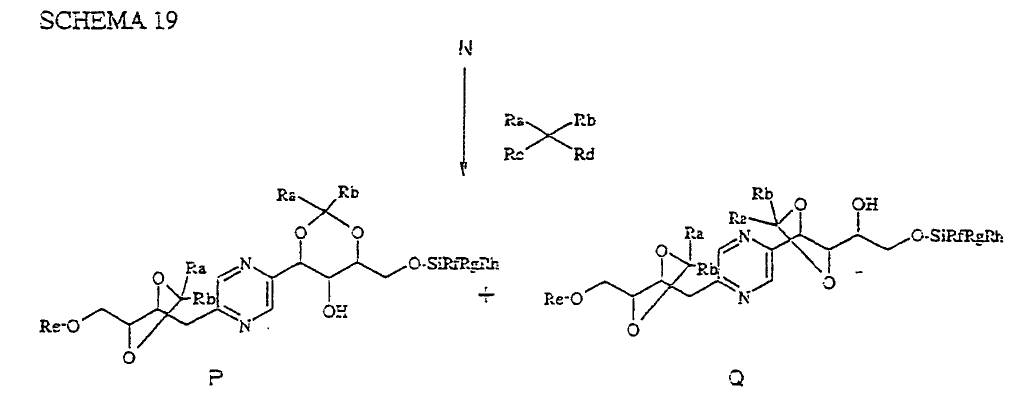

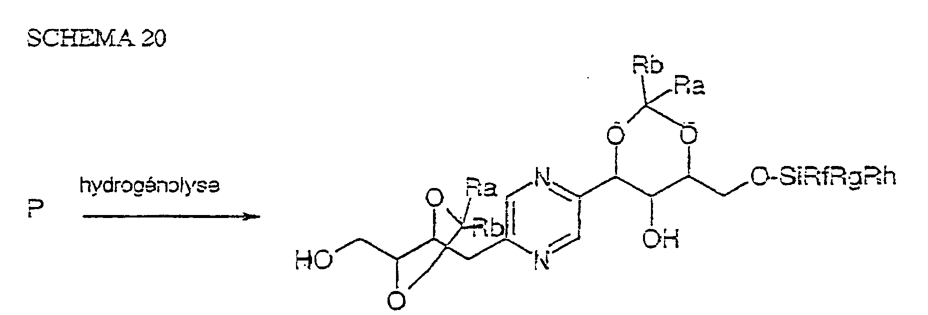

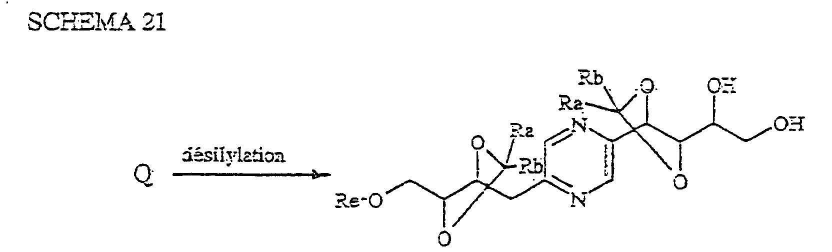

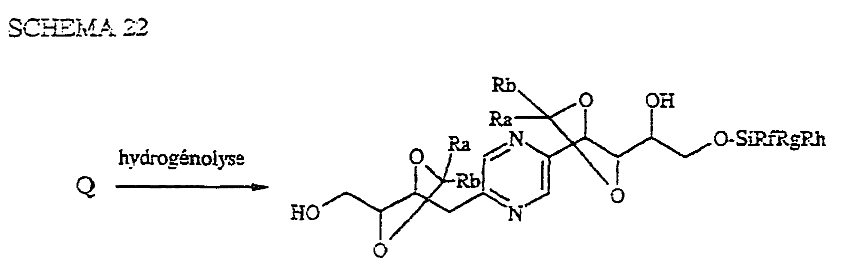

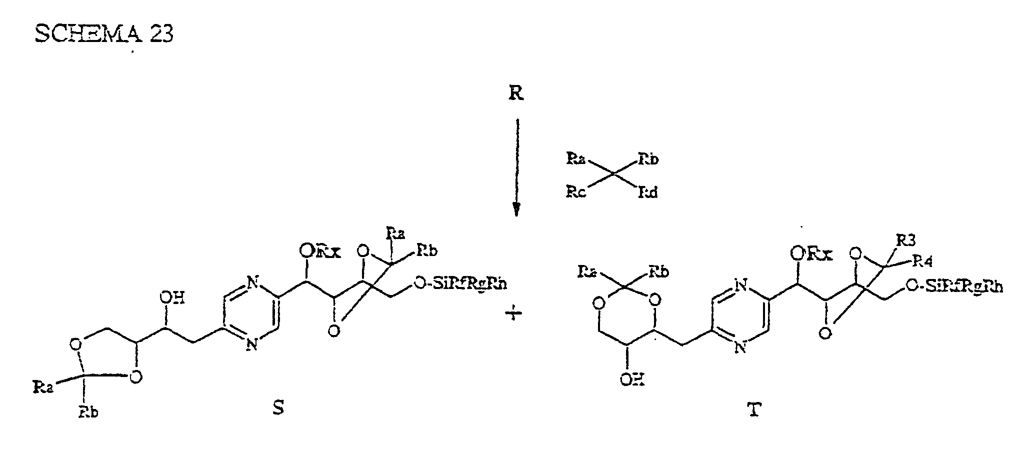

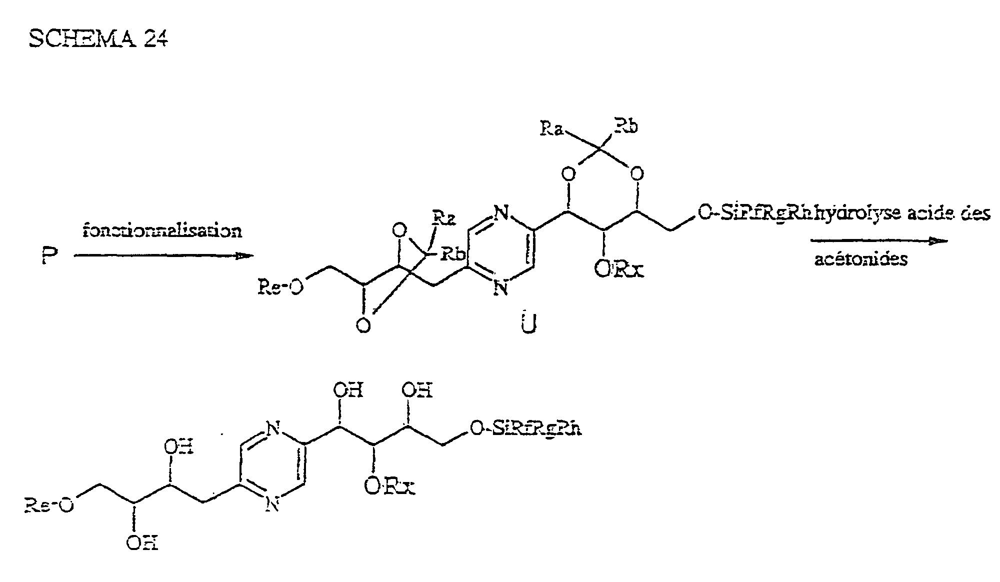

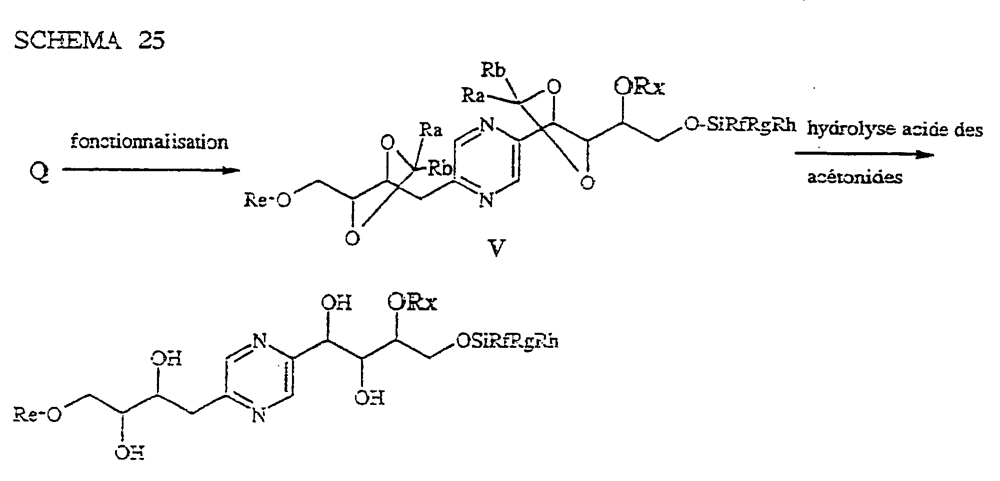

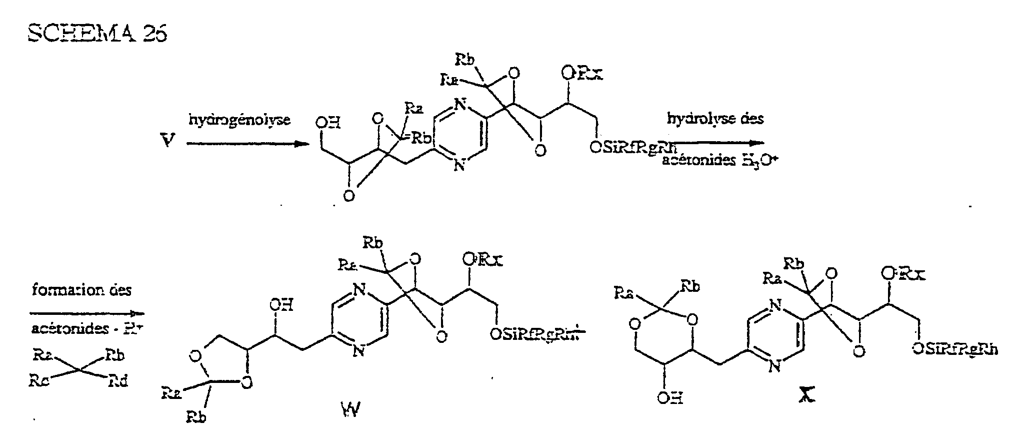

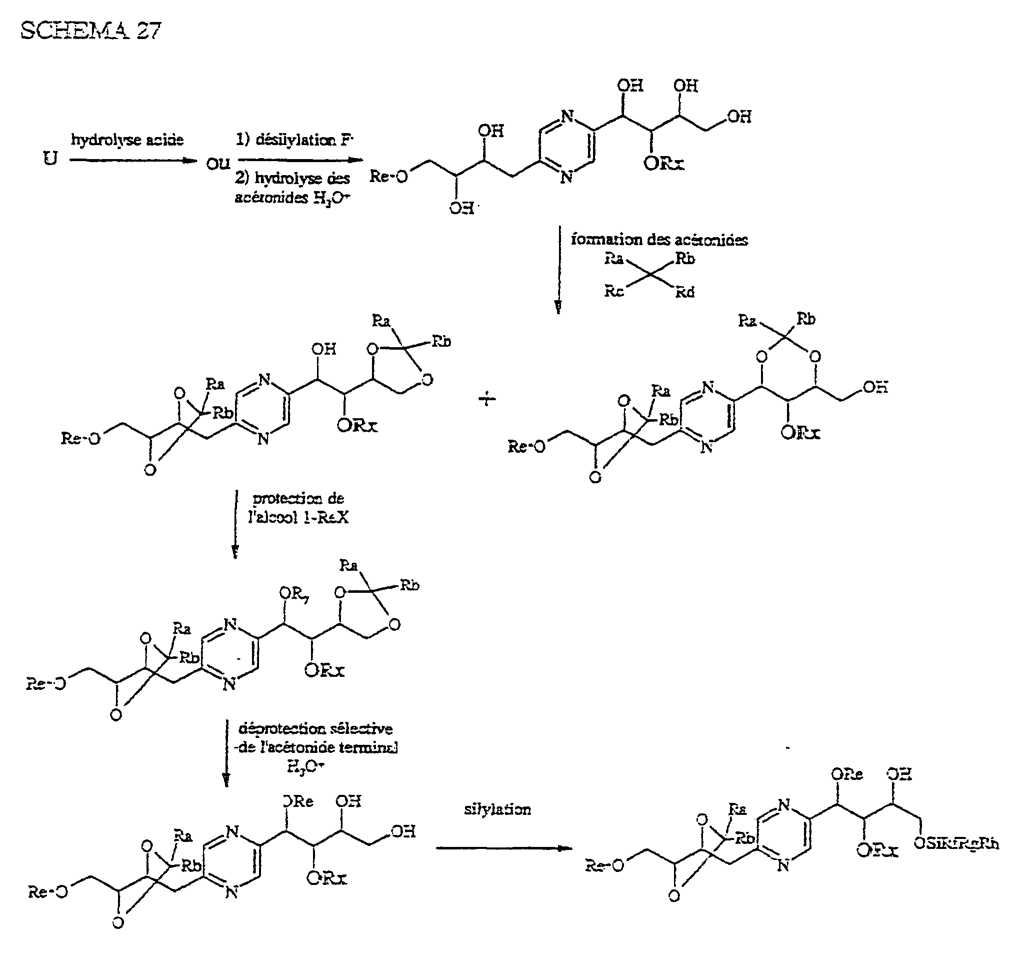

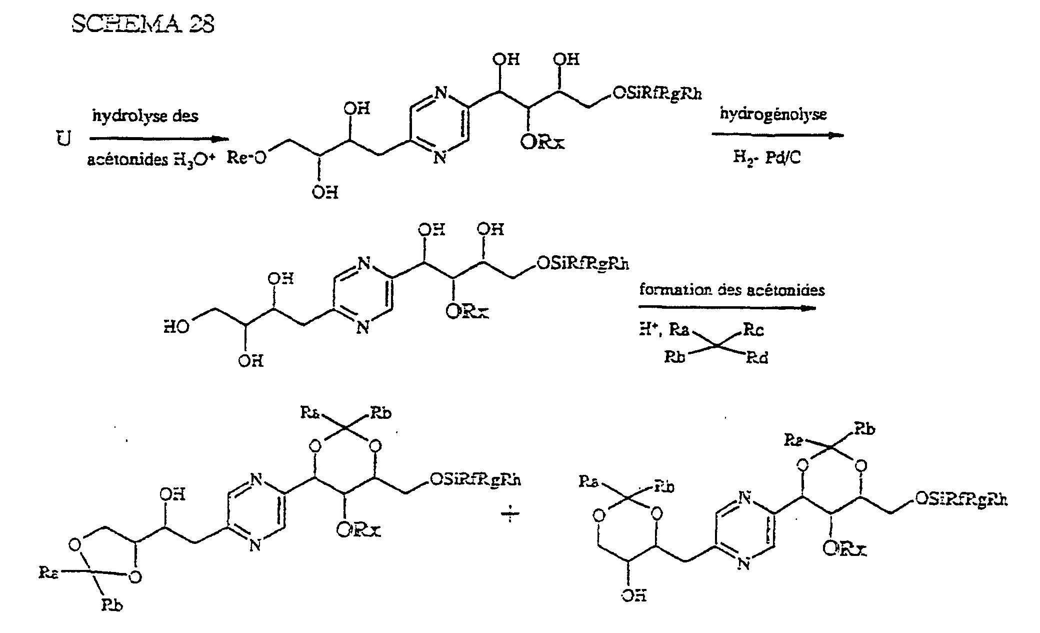

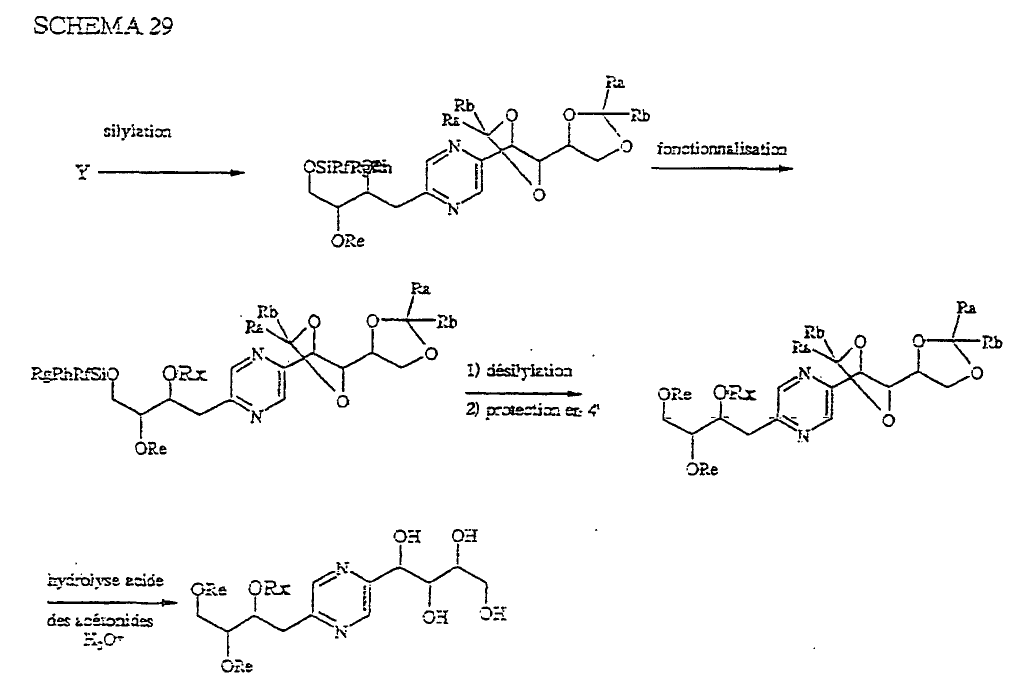

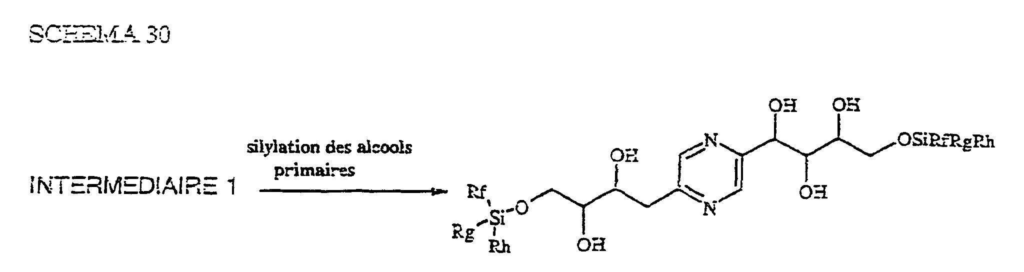

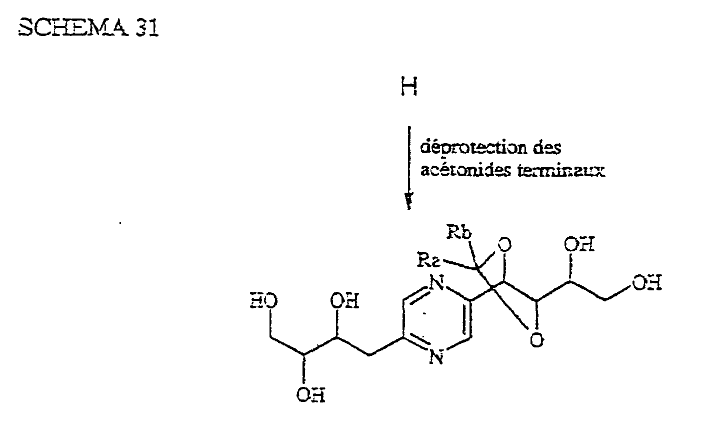

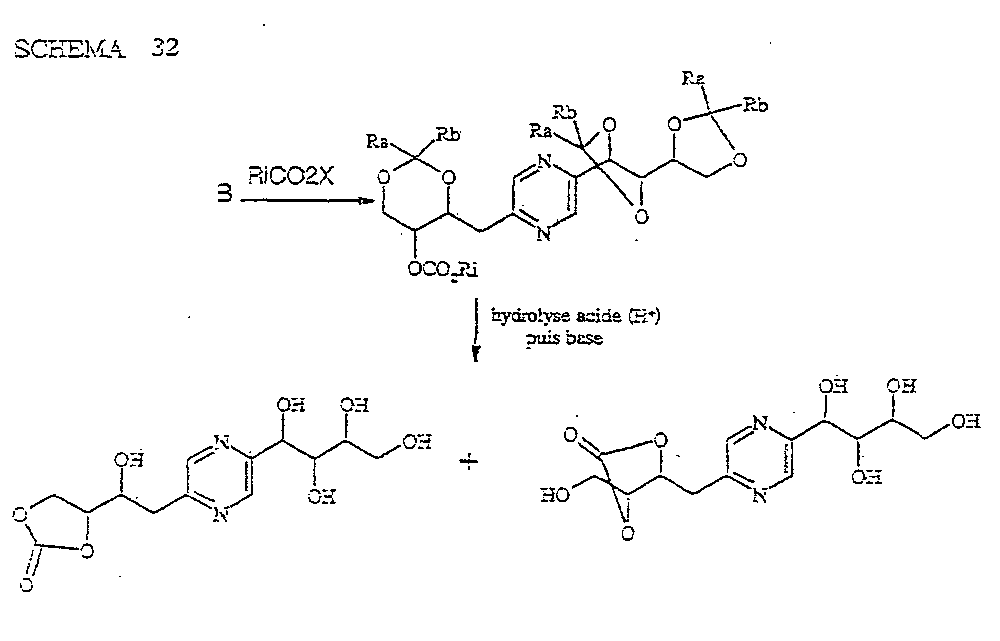

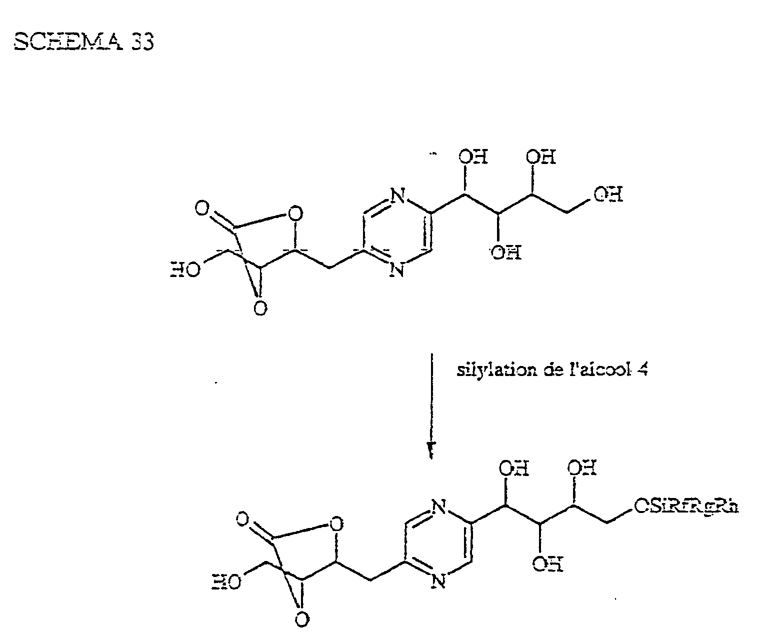

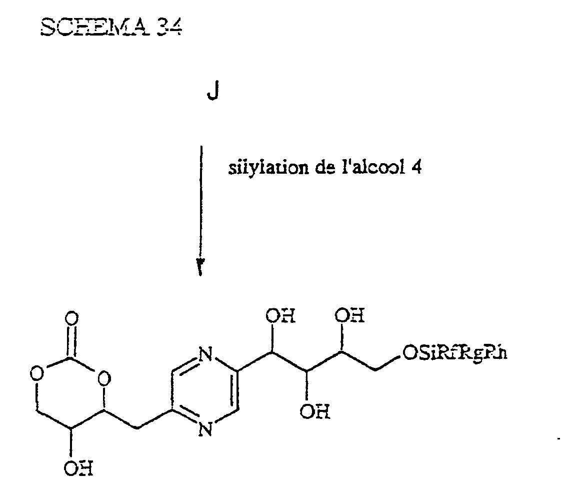

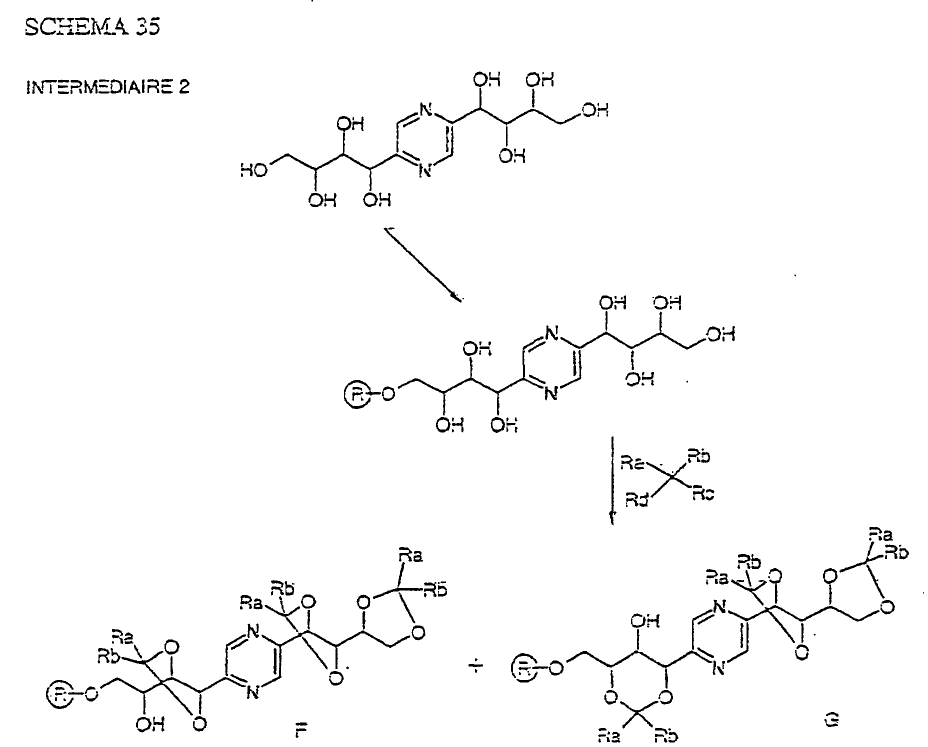

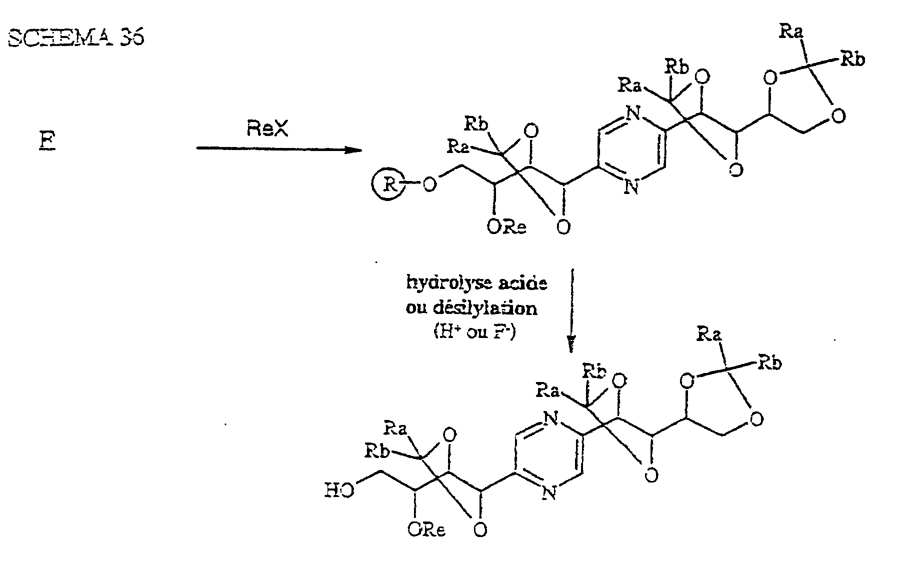

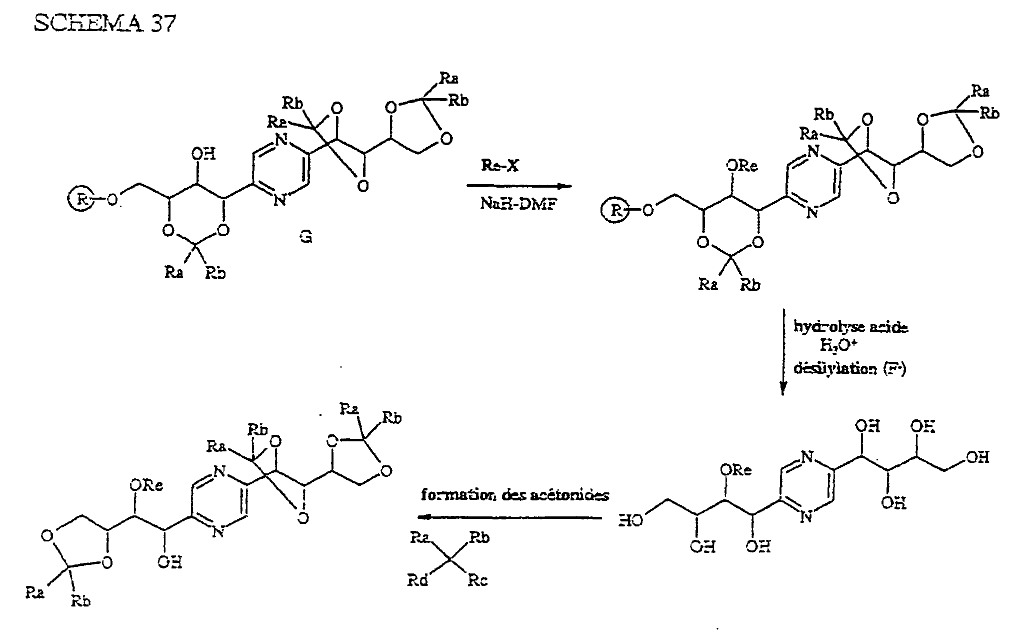

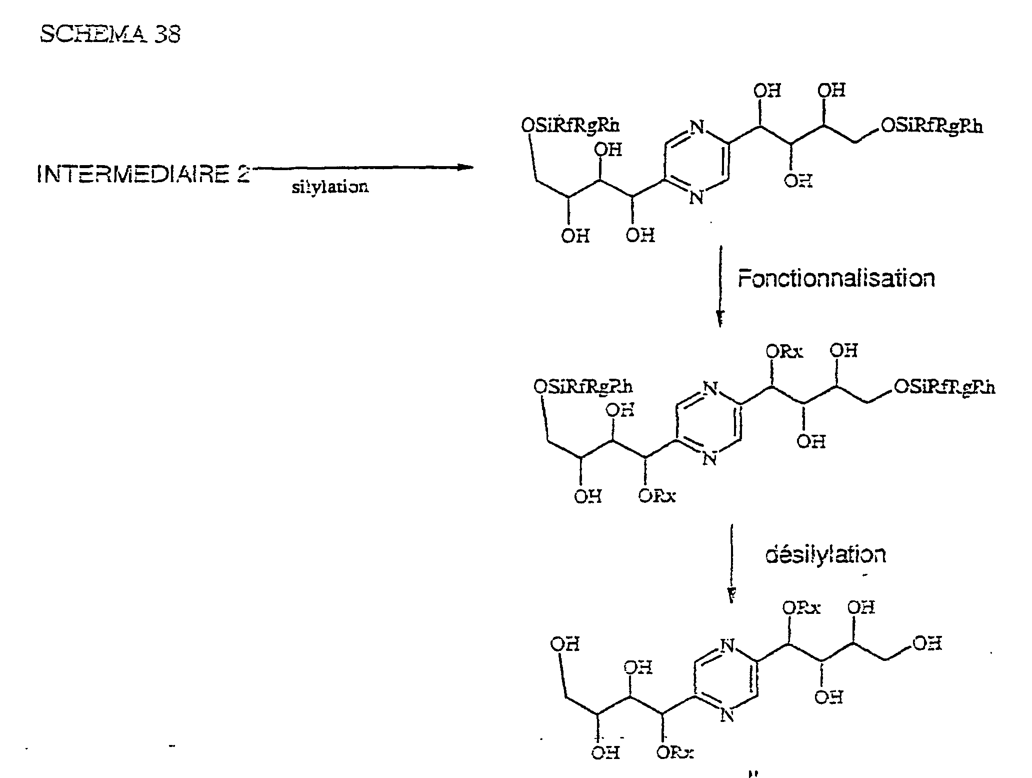

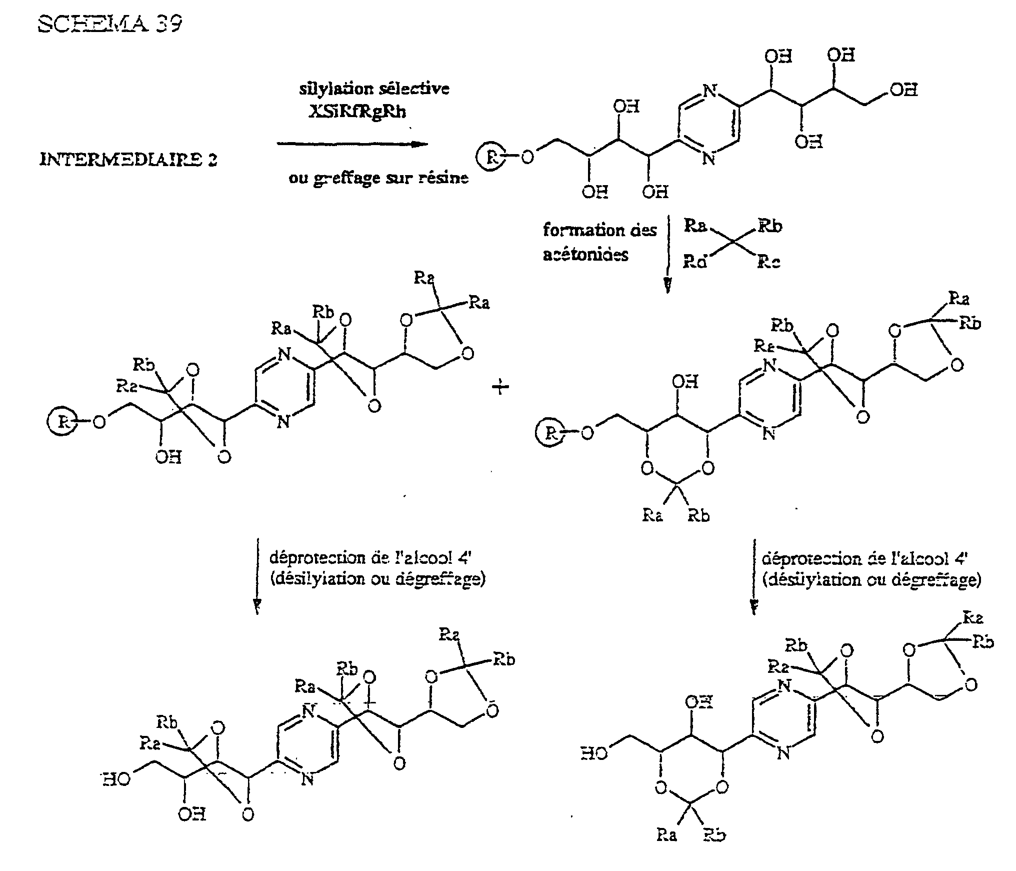

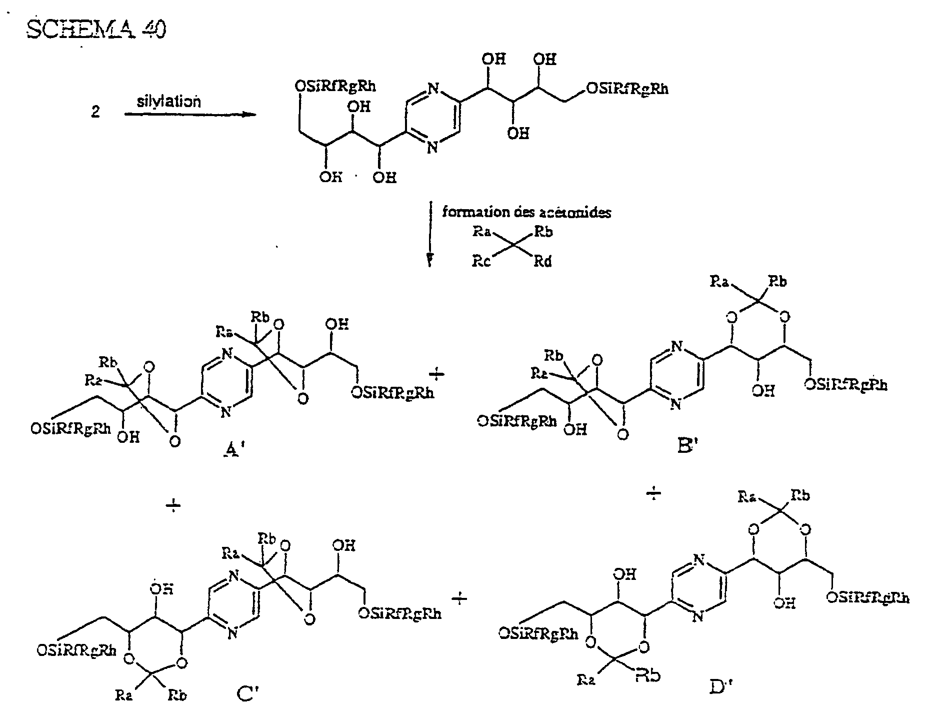

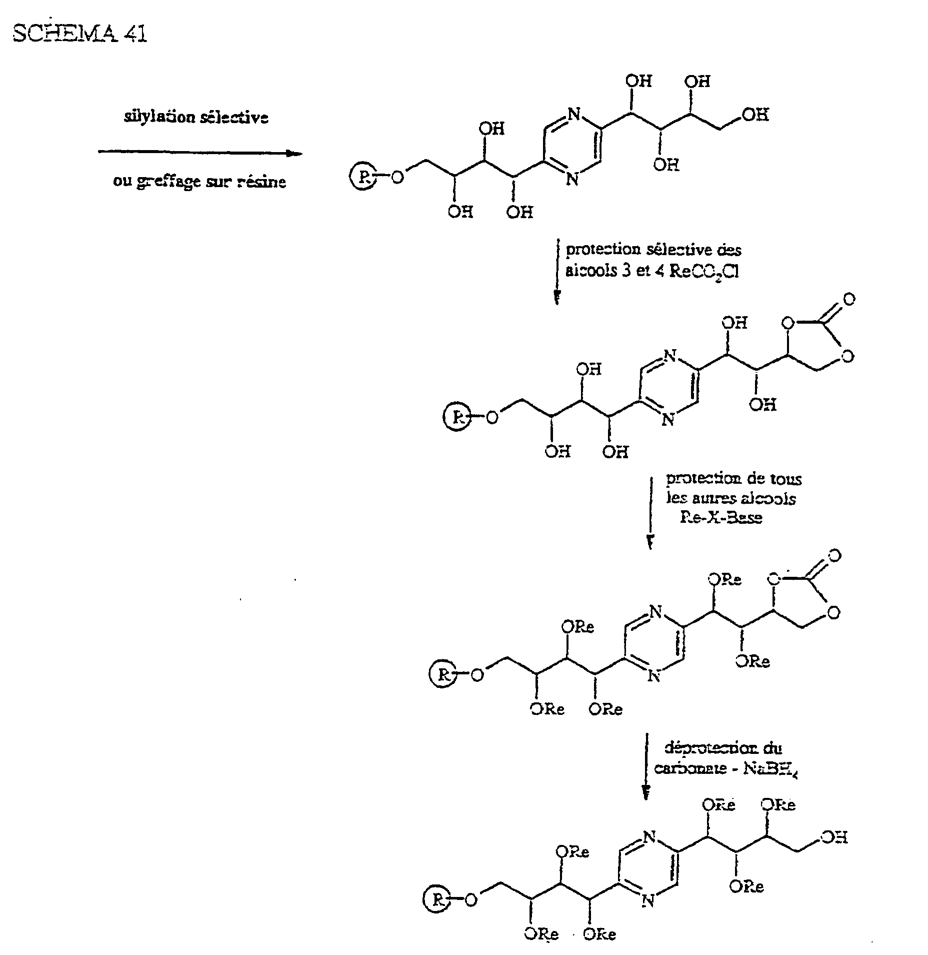

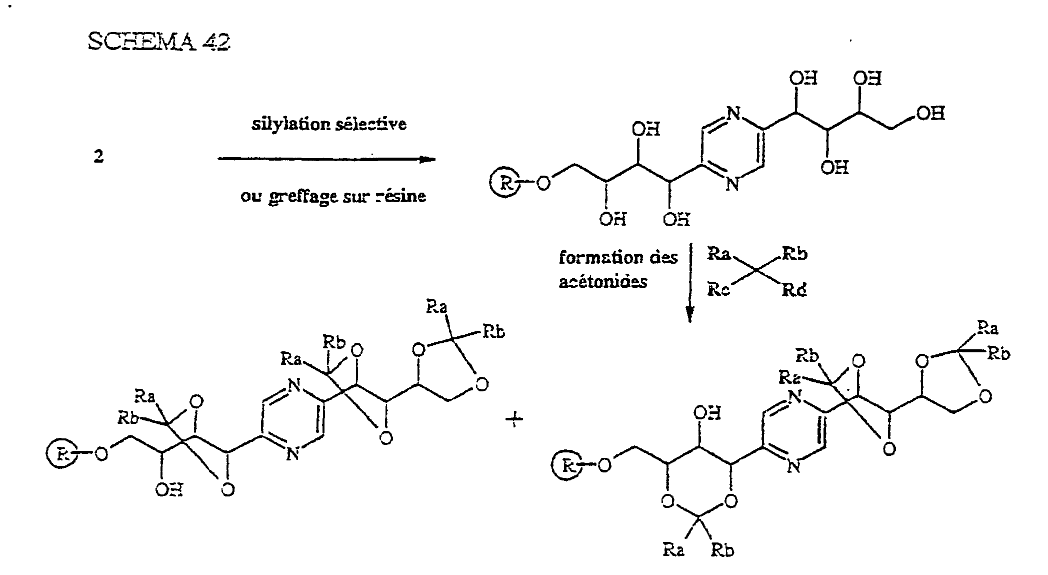

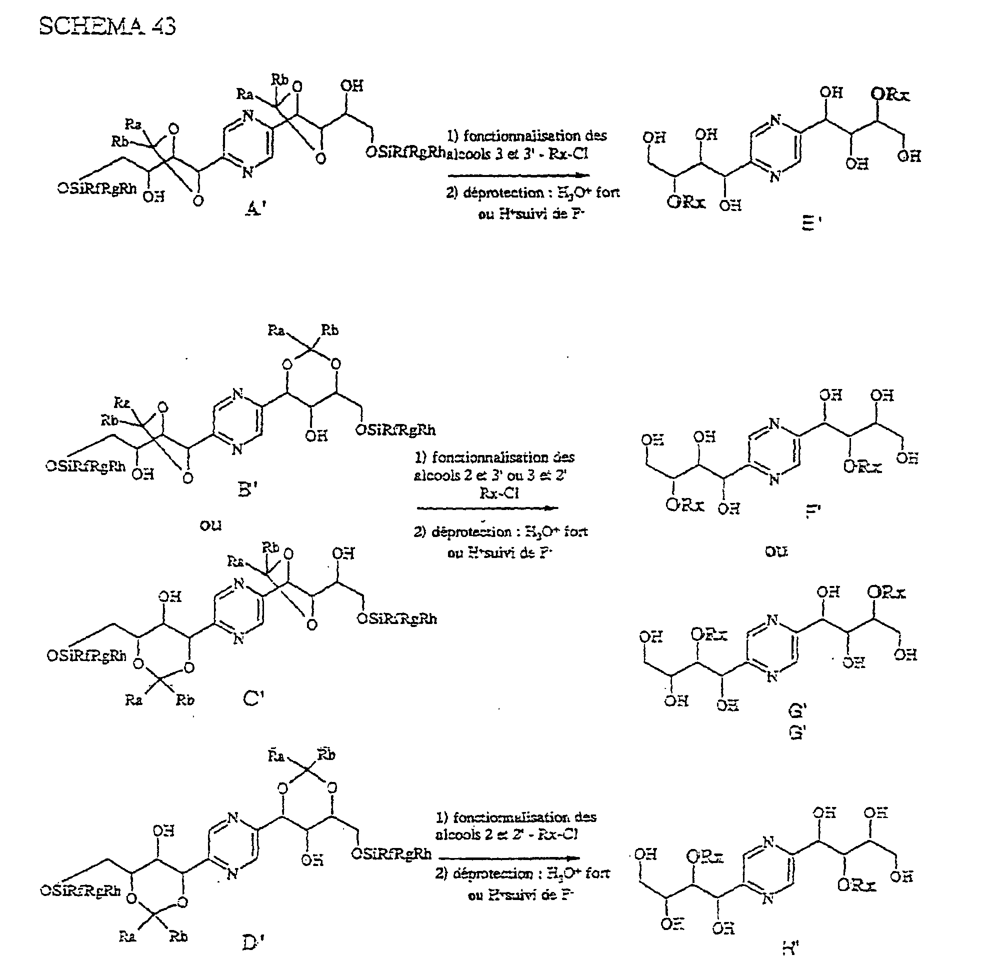

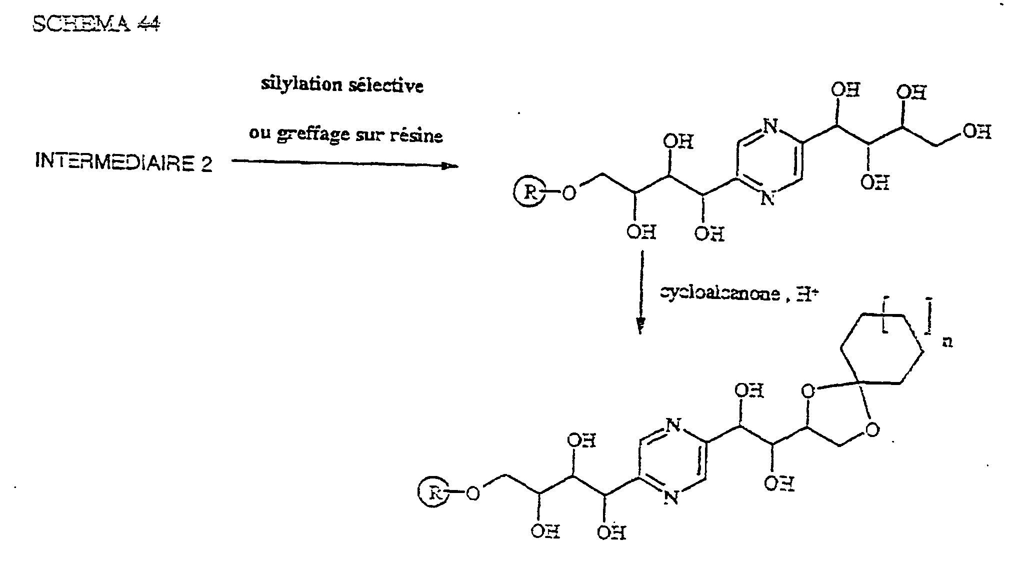

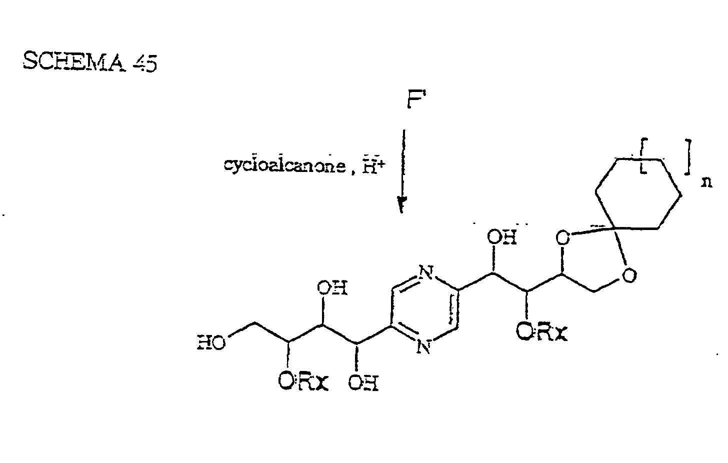

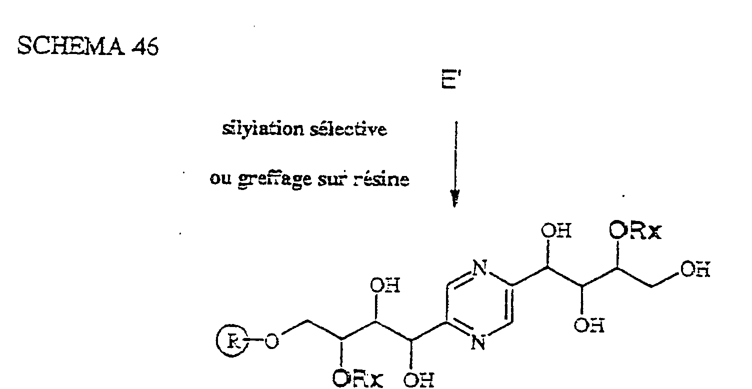

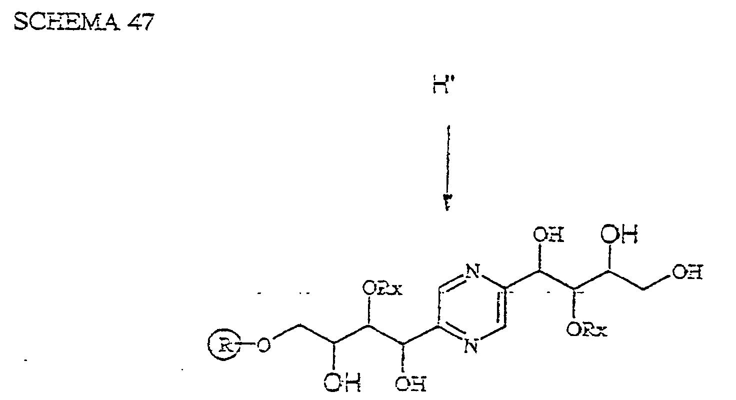

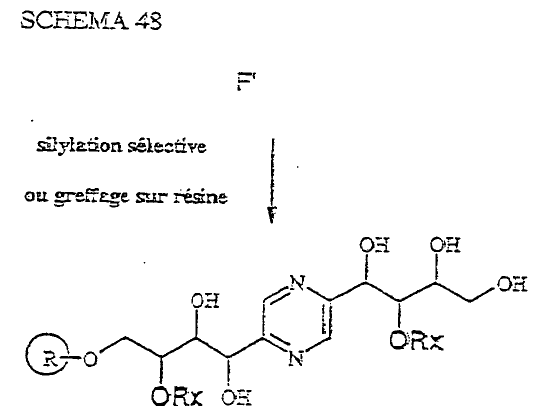

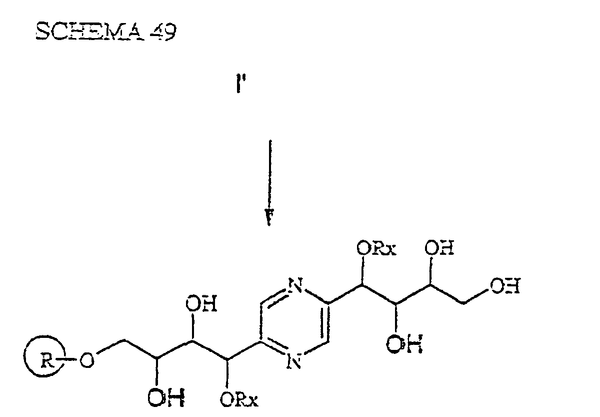

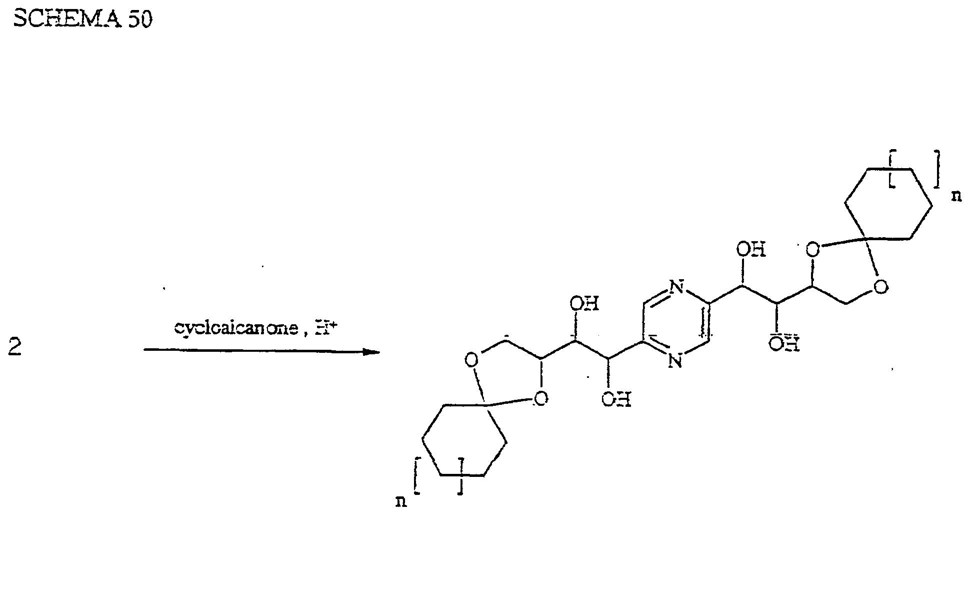

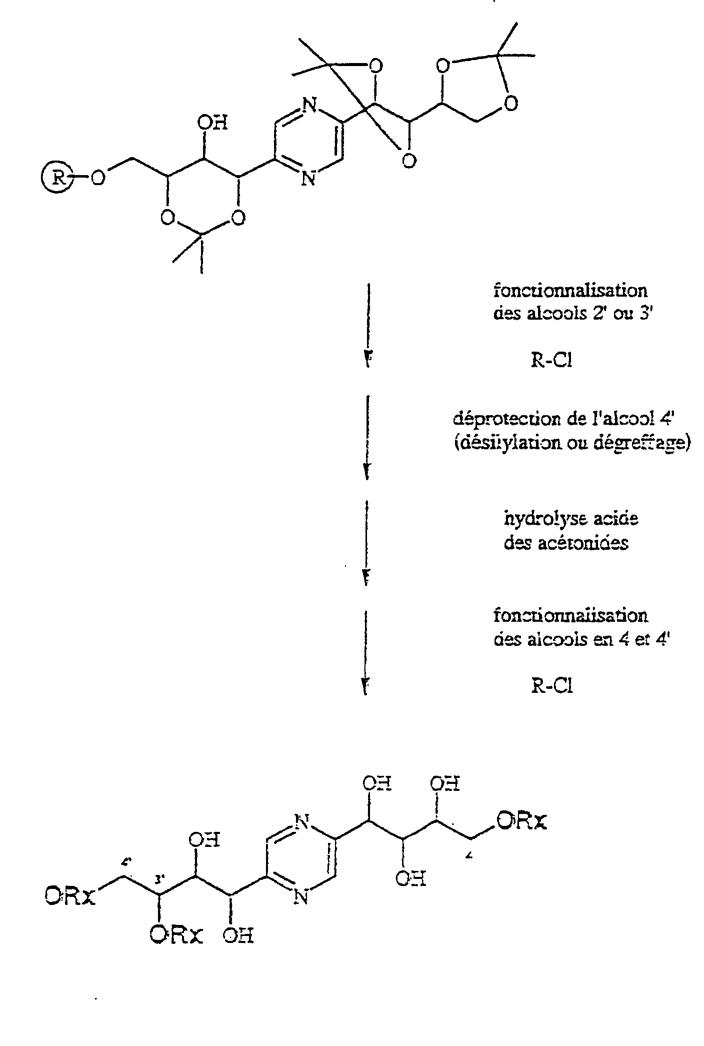

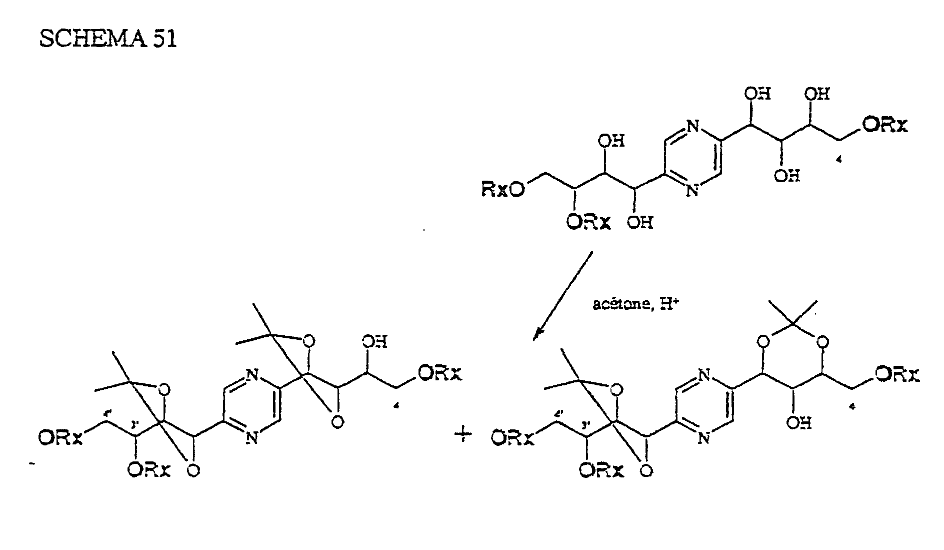

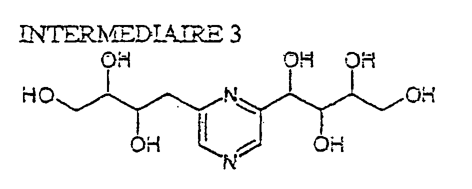

Les schémas réactionnels mettant en oeuvre l'intermédiaire 3 peuvent être établis en appliquant les mêmes schémas que ceux décrits plus haut pour l'intermédiaire 1.

Les mélanges réactionnels obtenus par les divers procédés décrits précédemment sont traîtés suivant des méthodes classiques physiques (évaporation, extraction, distillation, chromatographie, cristallisation par exemple) ou chimiques ( formation de sels par exemple).

Les composés de formule (I) peuvent être éventuellement transformés en sels d'addition avec un acide minéral ou organique par action d'un tel acide au sein d'un solvant tel qu'un alcool, une cétone, un éther ou un solvant chloré. Ces sels font également partie de l'invention.

Comme exemple de sels pharmaceutiquement acceptables, peuvent être cités les sels d'addition avec les acides minéraux ou organiques tels que acétates, propionate, succinate, benzoate, fumarate, maléate, oxalate, méthanesulfonate, iséthionate, théophyliinacétate, salicylate, méthylène-bis-b-oxynaphtoate, chlorhydrate, sulfate, nitrate et phosphate.

Les composés de formule (I) présentent des propriétés pharmacologiques intéressantes. Ce sont des hypoglycémiants.

L'activité hypoglycémiante des composés de formule (I) a été déterminée sur la réponse hyperglycémique à l'administration de glucose par la voie orale chez la souris normoglycémique, selon le protocole suivant :

Des souris Swiss albinos pesant entre 22 et 26 g sont laissées à jeun pendant 2 heures. A la fin de cette période, la glycémie est mesurée et, immédiatement après, une dose de glucose (2 g/kg) est administrée par voie orale. Trente minutes plus tard, la glycémie est mesurée encore une fois. Les souris qui répondent par une hyperglycémie supérieure à 170 mg/dl sont sélectionnées et utilisées pour détecter l'activité hypoglycémiante des composés selon l'invention.

Les souris ainsi choisies sont réparties en groupes d'au moins 10 animaux. Des groupes distincts reçoivent des doses de 3 à 50 mg/kg de produit dans un véhicule tel que l'eau ou un mélange de méthylcellulose/tween et eau une fois par jour par tubage gastrique. Le traitement dure 4 jours. Au 4 ^{ème} jour, après le dernier traitement, les animaux reçoivent une dose de glucose (2 g/kg) et la glycémie est mesurée 20 à 40 minutes plus tard. Le pourcentage d'inhibition de la réponse hyperglycémique à l'administration de glucose est calculée par rapport à la réponse mesurée dans le groupe traité par le véhicule.

Dans ce test, les composés selon l'invention présentent un pourcentage d'inhibition de la glycémie supérieur ou égal à 10%.

Les composés de formule générale (I) selon l'invention présentent une faible toxicité. Leur DL50 est supérieure à 2000 mg/kg par voie orale chez la souris.

En thérapeutique humaine, ces produits sont utiles dans la prévention et le traitement du diabète et notamment du diabète de type II (NID diabète), du diabète de l'obèse, du diabète de la cinquantaine, du diabète métapléthorique, du diabète du sujet âgé et du diabète léger. Ils peuvent être utilisés en complément de l'insulinothérapie dans le diabète insulino dépendant où ils permettent de diminuer progressivement la dose d'insuline, le diabète instable, le diabète insulinorésistant, en complément des sulfamides hypoglycémiants quand ceux-ci ne déterminent pas de baisse suffisante de la glycémie. Ces produits peuvent être utilisés également dans les complications du diabète telles que les hyperlipémies, les troubles du métabolisme lipidique, les dyslipémies, l'obésité. Ils sont aussi utiles dans la prévention et le traitement des lésions d'athérosclérose et leurs complications (coronopathies, infarctus du myocarde, cardiomyopathies, évolution de ces trois complications vers l'insuffisance ventriculaire gauche, artériopathies diverses, artérites des membres inférieurs avec claudication et évolution vers les ulcères et la gangrène, insuffisance vasculaire cérébrale et ses complications, impuissance sexuelle d'origine vasculaire), la rétinopathie diabétique et de toutes ses manifestations (augmentation de la perméabilité capillaire, dilatation, et thrombose capillaire, microanévrismes, shunt artérioveineux, dilatation veineuse, hémorragies ponctiformes et maculaires, exudats, oedèmes maculaires, manifestsions de la rétinopathie proliférante : néovaisseaux, cicatrices de rétinite proliférante, hémorragies du vitrée, décollement de la rétine), la cataracte diabétique, la neuropathie diabétique dans ses diverses formes (polyneuropathies périphériques et ses manifestations telles que paresthésies, hyperesthésies et douleurs, mononeuropathies, radiculopathies, neuropathies autonomes, amyotrophies diabétiques), les manifestations du pied diabétique (ulcéres des extrémités inférieures et du pied), la néphropathie diabétique dans ses deux formes diffuse et nodulaire, l'athéromatose (élévation des HDL lipoproteines favorisant l'élimination du cholestérol à partir des plaques d'athérome, baisse des LDL lipoprotéines, baisse du rapport LDL/HDL, inhibition de l'oxydation des LDL, diminution de l'adhésivité plaquettaire), des hyperlipémies et des dyslipémies (hypercholestérolémies, hypertriglycéridémies, normalisation du taux des acides gras, normalisation de l'uricémie, normalisation des apoprotéines A et B), de la cataracte, de l'hypertension artérielle et ses conséquences.

Les médicaments selon l'invention sont constitués par un composé selon l'invention ou une combinaison de ces produits, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouiliants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 150 mg et 600 mg par jour par voie orale pour un adulte avec des doses unitaires allant de 50 mg à 200 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Produit actif 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Produit actif 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 50 mg de produit actif ayant la composition suivante :
- Produit actif 50 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

L'invention concerne également l'utilisation des composés de formule générale (I) pour la préparation de compositions pharmaceutiques utiles pour le traitement ou la prévention du diabète et les complications du diabète.

Les exemples suivants illustrent plus particulièrement et à titre non limitatif le procédé de préparation utilisé selon l'invention :

### Exemple 1

### 4, 4'-O, O-dibenzoyl-2-[(1R, 2S, 3R) (1,2,3,4-tétrahydroxybutyl)] 5-[(2'S, 3'R) (2',3',4'-trihydroxybutyl)] pyrazine

A 500 mg de 2-[(1R,2S,3R) (1,2,3,4-tétrahydroxybutyl)] 5-[(2'S,3'R) (2', 3', 4'-trihydroxybutyl)] pyrazine en suspension dans 2,5 cm³ de pyridine séchée sur tamis moléculaire 4Å est additionné, goutte à goutte, à une température voisine de 20°C sous atmosphère d'argon, 0,48 cm³ de chlorure de benzoyle. La solution obtenue après deux minutes d'agitation est de nouveau agitée pendant 17 heures supplémentaires a une température voisine de 20°C. La pyridine est évaporée sous flux d'air à une température voisine de 20°C. Le résidu obtenu est repris avec un minimum d'un mélange dichlorométhane-méthanol (98-2 en volumes) et est purifié par chromatographie sous pression atmosphérique sur 40 g de gel de silice 60F254 Merck (0,063-0,200 mm) contenus dans une colonne de diamètre 2,5 cm. On utilise un gradient d'éluant constitué d'un mélange dichlorométhane-méthanol : 97,5-2,5 en volumes (500 cm³), 95-5 en volumes (500 cm³), 92,5-7,5 en volumes (500 cm³) puis 90-10 en volumes (500 cm³) en recueillant des fractions de 10 cm³. Les fractions ne contenant que le produit cherché sont réunies et concentrées à sec sous pression réduite (environ 2,7 kPa) à une température voisine de 40°C. On obtient ainsi 1,02 g d'un solide jaune contenant le produit attendu. Celui-ci est solubilisé dans un mélange dichlorométhane-méthanol. Après quelques minutes, un solide blanc se forme qui est filtré sur verre fritté en rinçant avec du dichlorométhane puis séché sous pression réduite (10 Pa) à une température voisine de 40°C.

On obtient ainsi 133 mg de 4, 4'-O, O-dibenzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'un solide blanc.

Le produit obtenu possède les caractéristiques suivantes :

Spectre de R.M.N. ¹ H (400 MHz, (CD₃)₂SO d6. δ en ppm) ; 2,81 (dd, J = 14 et 9 Hz, 1H : 1H du CH₂ 5α) ; 3,20 (dd, J = 14 et 3 Hz, 1H : l'autre H du CH₂ 5α) ; de 3,65 à 3,75 (mt, 1H : CH 5β) ; 3,72 (d large, J = 9 Hz, 1H : CH 2β) ; 3,89 (mt, 1H : CH 5γ) ; 4,01 (mt, 1H : CH 2γ) ; de 4,20 à 4,35 (mt, 2H : 1H du CH₂ 2δ et 1H du CH₂ 5δ) ; de 4,45 à 4,60 (mt, 2H : l'autre H du CH₂ 2δ et l'autre H du CH₂ 5δ) ; 5,02 (s large, 1H : CH 2α) ; 7.56 (t, J = 7,5 Hz, 4H : H en méta des 2 benzoyloxy) ; 7,67 (t, J = 7,5 Hz, 2H : H en para des 2 benzoyloxy) ; 8,04 (d, J = 7,5 Hz, 4H : H en ortho des 2 benzoyloxy) ; 8,47 (s, 1H : =CH en 6) ; 8,70 (s, 1H : =CH en 3).

La 2-[(1R, 2S, 3R) (1, 2, 3, 4-tétrahydroxybutyl)] 5-[(2'S, 3'R) (2', 3', 4'-trihydroxybutyl)] pyrazine (désoxyfructosazine) peut être préparée selon la méthode décrite dans le brevet JP 53-90401 ou par Kuhn et al, J.L.Ann.Chem.644,122-7,1961.

### Exemple 2

### 4, 4'-O, O-diacétyl-2-[(1R, 2S, 3R) (1, 2, 3, 4-tétrahydroxybutyl)] 5-[(2'S, 3'R) (2', 3', 4'-trihydroxybutyl)] pyrazine

A 50 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 0,5 cm³ de pyridine séchée sur potasse sont additionnés, goutte à goutte en 10 minutes, à une température voisine de 0°C, 0,034 cm³ d'anhydride acétique. La suspension blanche obtenue est agitée 2 heures à une température voisine de 0°C, puis le milieu réactionnel est ramené à une température voisine de 20°C. On ajoute quelques grains d'hydrogénocarbonate de magnésium, et la pyridine est évaporée à une température voisine de 20°C sous flux d'air. Le milieu réactionnel brut est repris avec un mélange dichlorométhane-méthanol, et la partie soluble du milieu est purifiée par chromatographie sur plaques de gel de silice 60F254 Merck (2 plaques, épaisseur = 0,5 mm, 20x20 cm) en éluant avec un mélange acétate d'éthyle-eau-acide acétique (30-10-12 en volumes). La fraction ne contenant que le produit cherché est extraite par un mélange dichlorométhane-méthanol, filtrée sur verre fritté puis concentrée à sec sous pression réduite (2,7 kPa) à une température voisine de 40°C. On obtient ainsi 2 mg de 4, 4'-O, O-diacétyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une laque incolore.

Le produit obtenu possède les caractéristiques suivantes :

Spectre de R.M.N. ¹ H (300 MHz, (CD₃)₂SO d6 avec ajout de quelques gouttes de CD3COOD d4, δ en ppm) : 1,91 et 2,02 (2 s, 6H : les 2 OCOCH₃) ; 2,75 (dd, J = 14 et 9 Hz, 1H : 1H du CH₂ 5α) ; 3,12 (dd, J = 14 et 3 Hz, 1H : l'autre H du CH₂ 5α) ; 3,55 (mt, 1H : CH 5γ) ; 3,60 (dd, J = 9 et 1,5 Hz, 1H : CH 2β) ; 3,77 (mt, 1H : CH 5β) ; 3,84 (mt, 1H : CH 2γ) ; de 3,90 à 4,05 (mt, 2H : 1H du CH₂ 2δ et 1H du CH₂ 5δ) ; de 4,20 à 4,35 (mt, 2H : l'autre H du CH₂ 2δ et l'autre H du CH₂ 5δ) ; 4,95 (s large, 1H : CH 2α) ; 8,43 (s, 1H ; =CH en 6) ; 8,66 (s, 1H : =CH en 3).

### Exemple 3

### 4, 4'-O, O-di-(2, 2-diméthylpropanoyl)-2-[(1R, 2S, 3R) (1, 2, 3, 4-tétrahydroxybutyl)] 5-[(2'S, 3'R) (2', 3', 4'-trihydroxybutyl)] pyrazine

A 100 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 2 cm³ de pyridine séchée sur hydroxyde de potassium est additionné, goutte à goutte à une température voisine de 20°C sous atmosphère d'argon, 0,102 cm³ de chlorure de pivaloyle. La suspension réactionnelle est agitée à une température voisine de 20°C pendant 15 minutes. Après homogénéisation du milieu, le solvant est évaporé sous pression réduite (2,7 kPa) à une température voisine de 30°C. On obtient une huile jaune clair qui est purifiée par chromatographie sur 2 plaques de gel de silice 60F254 Merck (épaisseur = 2 mm, 20x20 cm) en éluant avec un mélange dichlorométhane-méthanol (90-10 en volumes). La fraction ne contenant que le produit cherché est extraite par un mélange dichlorométhane-méthanol (90-10 en volumes), filtrée sur verre fritté puis concentrée à sec sous pression réduite (2,7 kPa) à une température voisine de 30°C.

On obtient ainsi 67 mg de 4, 4'-O, O-di-(2, 2-diméthylpropanoyl)-2-[(1R, 2S, 3R) (1, 2, 3, 4-tétrahydroxybutyl)] 5-[(2'S, 3'R) (2', 3', 4'-trihydroxybutyl)] sous la forme d'une poudre blanche.

Le produit obtenu possède les caractéristiques suivantes :

Spectre de R.M.N. ¹ H (400 MHz, (CD₃)₂SO d6, δ en ppm) : 1,20 (s, 18H : les 2 OCOC(CH₃)₃) ; 2,78 (dd, J = 14 et 9 Hz, 1H : 1H du CH₂ 5α) ; 3,14 (dd, J = 14 et 2,5 Hz, 1H : l'autre H du CH₂ 5α) ; de 3,50 à 3,65 (mt, 1H : CH 5γ) ; 3,63 (t large, J = 9 Hz, 1H : CH 2β) ; 3,81 (mt, 1H : CH 5β) ; 3,88 (mt, 1H : CH 2γ) ; de 3,95 à 4,05 (mt, 2H : 1H du CH₂ 2δ et 1H du CH₂ 5δ) ; 4,24 (dd, J = 12 et 3 Hz, 1H : l'autre H du CH₂ 5δ) ; 4,30 (dd, J = 12 et 2 Hz, 1H : l'autre H du CH₂ 2δ) ; 4,63 (d, J = 9 Hz, 1H : OH en 2β) ; 4,86 (d, J = 6,5 Hz, 1H : OH en 5β) ; 4,99 (d large, J = 6 Hz, 1H : CH 2α) ; 5,05 (d, J = 6 Hz, 1H : OH en 2γ) ; 5,10 (d, J = 6 Hz, 1H : OH en 5γ) ; 5,40 (d, J = 6 Hz, 1H : OH en 2α) ; 8,44 (s, 1H : =CH en 6) ; 8,67 (s, 1H : =CH en 3).

### Exemple 4

### 4, 4'-O, O-dipentanoyl-2-[(1R, 2S, 3R) (1, 2, 3, 4-tétrahydroxybutyl)] 5-[(2'S, 3'R) (2', 3', 4'-trihydroxybutyl)] pyrazine

A 200 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 4 cm³ de pyridine séchée sur hydroxyde de potassium est additionné, goutte à goutte à une température voisine de 20°C sous atmosphère d'argon, 0,196 cm³ de chlorure de pentanoyle. La solution obtenue est agitée à une température voisine de 20°C pendant 21 heures. Le solvant est évaporé sous pression réduite (2,7 kPa) à une température voisine de 30°C. Le brut réactionnel est purifié par chomatographie sur 4 plaques de gel de silice 60F254 Merck (épaisseur = 2 et 1 mm, 20x20 cm) en éluant avec un mélange dichlorométhane-méthanol (90-10 en volumes). La fraction ne contenant que le produit cherché est extraite par un mélange dichlorométhane-méthanol (90-10 en volumes), filtrée sur verre fritté puis concentrée à sec sous pression réduite (2,7 kPa) à une température voisine de 30°C.

On obtient ainsi 58 mg de 4, 4'-O, O-dipentanoyl-2-[(1R, 2S, 3R) (1, 2, 3, 4-tétrahydroxybutyl)] 5-[(2'S, 3'R) (2', 3', 4'-trihydroxybutyl)] pyrazine, sous la forme d'une poudre blanche.

La produit obtenu possède les caractéristiques suivantes :

Spectre de R.M.N. ¹ H (300 MHz, (CD₃)₂SO d6, δ en ppm) : 0,89 (t, J = 7,5 Hz, 6H : les CH₃ des deux chaînes butyle) ; 1,32 et 1,52 (2 mts, 8H : les 2 CH₂ centraux des deux chaînes butyle) ; 2,32 (t, J = 7,5 Hz, 4H : les OCOCH₂ des deux chaînes butyle) ; 2,75 (dd, J = 14 et 9 Hz, 1H : 1H du CH₂ 5α) ; 3,12 (dd, J = 14 et 3 Hz, 1H : l'autre H du CH₂ 5α) ; de 3,50 à 3,65 (mt, 1H : CH 5γ) ; 3,61 (t large, J = 9 Hz , 1H : CH 2β) ; de 3,70 à 3,95 (mt, 2H : CH 2γ et CH 5β) ; de 3,95 à 4,10 (mt, 2H : 1H du CH₂ 2δ et 1H du CH₂ 5δ) ; de 4,15 à 4,40 (mt, 2H : l'autre H du CH₂ 2δ et l'autre H du CH₂ 5δ) ; 4,64 (d, J = 9 Hz, 1H : OH en 2β) ; 4,88 (d, J = 6 Hz, 1H : OH en 5β) ; 4,96 (d large, J = 6 Hz, 1H : CH 2α) ; 5,06 (d, J = 6 Hz, 1H : OH en 2γ) ; 5,12 (d, J = 6 Hz, 1H : OH en 5γ) ; 5,42 (d, J = 6 Hz, 1H : OH en 2α) ; 8,42 (s, 1H : =CH en 6) ; 8,66 (s, 1H : =CH en 3).

### Exemple 5

### 4, 4'-O, O-di-(2-acétoxybenzoyl)-2-[(1R, 2S, 3R) (1, 2, 3, 4-tétrahydroxybutyl)] 5-[(2'S, 3'R) (2', 3',4'-trihydroxybutyl)] pyrazine

A 500 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 5 cm³ de pyridine séchée sur hydroxyde de potassium sont additionnés, à une température voisine de 20°C soits atmosphère d'argon, 816 mg de chlorure de l'acide 2-acétylsalicylique. La solution obtenue est agitée à une température voisine de 20°C pendant 15 minutes. Le solvant est évaporé sous pression réduite (2,7 kPa) à une température voisine de 30°C. Le brut réactionnel est purifié par chromatographie sous pression atmosphérique sur 40 g de gel de silice 60 Merck (0,040-0,063 mm) contenus dans une colonne de diamètre 2 cm en éluant avec un gradient d'éluant constitué d'un mélange de dichlorométhane-méthanol : 100-0 puis 95-5 en volumes. La chromatographie est suivie par chromatographie sur couche mince. Les fractions ne contenant que le produit attendu sont réunies et concentrées à sec sous pression réduite (2,7 kPa) à une température voisine de 30°C, pour donner 462 mg de produit attendu impur. Ce dernier est purifié par deux chromatographies sur plaques de gel de silice 60F254 Merck (épaisseur = 1 mm, 20x20 cm) en éluant avec un gradient d'éluant constitué d'un mélange dichlorométhane-méthanol (une fois 100-0 en volumes puis deux fois 90-10 en volumes). La fraction ne contenant que le produit cherché est extraite par un mélange dichlorométhane-méthanol (90-10 en volumes), filtrée sur verre fritte puis concentrée à sec sous pression réduite (2,7 kPa) à une température voisine de 30°C.

On obtient ainsi 108 mg de 4, 4'-O, O-di-(2-acétoxybenzoyl)-2-[(1R, 2S, 3R) (1, 2, 3, 4-tétrahydroxybutyl)] 5-[(2'S, 3'R) (2', 3', 4'-trihydroxybutyl)]pyrazine, sous la forme d'une poudre blanche.

Le produit obtenu possède les caractéristiques suivantes :

Spectre de R.M.N. ¹ H (400 MHz, (CD₃)₂SO d6, δ en ppm) : 2,31 (s, 6H : les 2 OCOCH₃) ; 2,80 (dd, J = 13,5 et 9 Hz, 1H : 1H du CH₂ 5α) ; 3,17 (dd, J = 13,5 et 2,5 Hz, 1H : l'autre H du CH₂ 5α) ; de 3,60 à 3,75 (mt, 1H : CH 5γ) ; 3,70 (t large, J = 9 Hz, 1H : CH 2β) ; 3,86 (mt, 1H : CH 5β) ; 3,97 (mt, 1H : CH 2γ) ; de 4,15 à 4,30 (mt, 2H : 1H du CH₂ 2δ et 1H du CH₂ 5δ) ; de 4,40 à 4,55 (mt, 2H : l'autre H du CH₂ 2δ et l'autre H du CH₂ 5δ) ; 4,74 (d, J = 9 Hz, 1H : OH en 2β) ; 4,96 (d, J = 6 Hz, 1H : OH en 5β) ; 5,01 (d large, J = 6 Hz, 1H : CH 2α) ; 5,23 (d, J = 6 Hz, 1H : OH en 2γ) ; 5,28 (d, J = 6 Hz, 1H : OH en 5γ) ; 5,46 (d, J = 6 Hz, 1H : OH en 2α) ; 7,24 (d large, J = 8 Hz, 2H : les 2 H aromatiques en 3) ; 7,43 (t large, J = 8 Hz, 2H :les 2 H aromatiques en 5) ; 7,69 (t large, J = 8 Hz, 2H : les 2 H aromatiques en 4) ; 8,04 (mt, 2H : les 2 H aromatiques en 6) ; 8,45 (s, 1H : =CH en 6) ; 8,69 (s, 1H : =CH en 3).

### Exemple 6

### 1, 2, 2', 3, 3', 4, 4'-O, O, O, O, O, O, O-heptabenzoyl-2-[(1R, 2S, 3R) (1, 2, 3,4-tétrahydroxybutyl)] 5-[(2'S, 3'R) (2', 3', 4'-trihydroxybutyl)] pyrazine

A 110 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 2,5 cm³ de pyridine séchée sur tamis moléculaire 4Å sont additionnés, goutte à goutte, à une température voisine de 20°C sous atmosphère d'argon, 0,63 cm³ de chlorure de benzoyle. La suspension blanche réactionnelle est agitée 16 heures à une température voisine de 20°C. Le milieu est dilué avec 2 cm³ d'un mélange dichlorométhane-méthanol (95-5 en volumes). Les solvants sont évaporés sous flux d'air à une température voisine de 20°C. Le résidu obtenu est dissous avec 20 cm³ de dichlorométhane. La phase organique est lavée avec 5 - cm³ d'eau, séchée sur sulfate de magnésium, filtrée sur verre fritté puis concentrée à sec sous pression réduite (0,2 kPa) à une température voisine de 40°C. On obtient 490 mg d'un solide blane qui est purifié par chromatographie sous pression atmosphérique sur 40 g de gel de silice 60F254 Merck (0,063-0,200 mm) contenus dans une colonne de diamètre 2,5 cm. On utilise un gradient d'éluant constitué d'un mélange dichlorométhane-méthanol : 100-0 en volumes (500 cm³) puis 98-2 en volumes (750 cm³) en recueillant des fractions de 12 cm³. Les fractions ne contenant que le produit cherché sont réunies et concentrées à sec sous pression réduite (environ 2,7 kPa) à une température voisine de 40°C.

On obtient ainsi 347 mg de 1, 2, 2', 3, 3', 4, 4'-O, O, O, O, O, O, O-heptabenzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue blanche.

Le produit obtenu possède les caractéristiques suivantes :

Spectre de R.M.N. ¹ H (400 MHz, (CD₃)₂SO d6, δ en ppm) : de 3,30 à 3,45 (mt, 2H : CH₂ 5α) ; 4,60 (dd, J = 12 et 5,5 Hz, 1H : 1H du CH₂ 2δ) ; 4,66 (dd, J = 12 et 7 Hz, 1H : 1H du CH₂ 5δ) ; 4,83 (mt, 2H : l'autre H du CH₂ 2δ et l'autre H du CH₂ 5δ) ; 5,76 (mt, 1H : CH 5γ) ; de 5,80 à 5,95 (mt, 2H : CH 2γ et CH 5β) ; 6,20 (dd, J = 7 et 4 Hz, 1H : CH 2β) ; 6,48 (d, J = 4 Hz, 1H : CH 2α) ; de 7,30 à 8,45 (mt, 35H : les H aromatiques des 7 OCOC₆H₅) ; 8,63 (s, 1H : =CH en 6) ; 8,70 (s, 1H : =CH en 3).

### Exemple 7

### 1, 2, 2', 3, 3', 4, 4'-O, O, O, O, O, O, O-heptaacétyle-2-[(1R, 2S, 3R) (1, 2, 3,4-tétrahydroxybutyl)] 5-[(2'S,3'R) (2', 3', 4'-trihydroxybutyl)]pyrazine

A 20 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 0,2 cm³ de pyridine séchée sur tamis moléculaire 4Å, est additionné, goutte à goutte à une température voisine de 20°C sous atmosphère d'argon, 0,0945 cm³ d'anhydride acétique. La solution obtenue après une heure d'agitation, est de nouveau agitée pendant 17 heures supplémentaires à une température voisine de 20°C. Le solvant est évaporé sous pression réduite (2,7 kPa) à une température voisine de 40°C. Le résidu ainsi obtenu est purifié par chromatographie sur 2 plaques de gel de silice 60F254 Merck (épaisseur = 0,5 mm, 20x20 cm) en éluant par un mélange dichlorométhane-méthanol (97-3 en volumes). La fraction ne contenant que le produit cherché est extraite par un mélange dichlorométhane-méthanol (85-15 en volumes), filtrée sur verre fritté puis concentrée à sec sous pression réduite (2,7 kPa) à une température voisine de 40°C.

On obtient ainsi 37,8 mg 1, 2, 2', 3, 3', 4, 4'-O, O, O, O, O, O, O-heptaacétyle-2-[(1R, 2S, 3R) (1, 2, 3, 4-tétrahydroxybutyl)] 5-[(2'S, 3'R) (2', 3', 4'-trihydroxybutyl)] pyrazine sous la forme d'une huile jaune pâle.

Le produit obtenu possède les caractéristiques suivantes :

Spectre de R.M.N. ¹ H (300 MHz, CDCl₃, δ en ppm) : 1,88 - 1,92 - 2,03 - 2,06 - 2,08 et 2,19 (6 s, respectivement 3H - 3H - 3H - 3H - 6H et 3H : les 7 OCOCH₃) ; 3,09 (AB limite, 2H : CH₂ 5α) ; de 4,05 à 4,40 (mt, 4H : CH₂ 2δ et CH₂ 5δ) ; de 5,20 à 5,35 et 5,50 (2 mts, respectivement 2H et 1H : CH 2γ - CH 5β et CH 5γ) ; 5,62 (dd, J = 9 et 2,5Hz, 1H : CH 2β) ; 6,14 (d, J = 2,5 Hz, 1H : CH 2α) ; 8,37 (s, 1H : =CH en 6) ; 8,44 (s, 1H : =CH en 3).

### Exemple 8

### 1, 2, 2', 3, 3', 4, 4'-O, O, O, O, O, O, O-heptaacétyle-2-[(1R, 2S, 3R) (1, 2, 3, 4-tétrahydroxybutyl)] 6-[(2'S, 3'R) (2', 3', 4'-trihydroxybutyl)] pyrazine

A 60 mg de 2-[(1R, 2S, 3R) (1, 2, 3, 4-tétrahydroxybutyl)] 6-[(2'S, 3'R) (2', 3', 4'-trihydroxybutyl)] pyrazine en suspension dans 0,6 cm³ de pyridine séchée sur tamis moléculaire 4Å, est additionné, goutte à goutte à une température voisine de 20 °C sous atmosphère d'argon, 0,285 cm³ d'anhydride acétique. La suspension réactionnelle est agitée 15 heures à une température voisine de 20 °C. Le solvant du milieu réactionnel ainsi obtenu est évaporé sous pression réduite (2,7 kPa) à une température voisine de 40°C. Le brut réactionnel est purifié par chromatographie sur 6 plaques de gel de silice 60F254 Merck (épaisseur = 0,5 mm, 20x20 cm) en éluant par un mélange dichlorométhane-méthanol (97-3 en volumes). La fraction ne contenant que le produit cherché est extraite par un mélange dichlorométhane-méthanol (85-15 en volumes), filtrée sur verre fritté puis concentrée à sec sous pression réduite (2,7 kPa) à une température voisine de 40°C.

On obtient ainsi 117 mg 1, 2, 2', 3, 3', 4, 4'-O, 0, O, 0, O, O, O-heptaacétyle-2-[(1R, 2S, 3R) (1, 2, 3, 4-tétrahydroxybutyl)] 6-[(2'S, 3'R) (2', 3', 4'-trihydroxybutyl)] pyrazine, sous la forme d'une meringue blanche.

Le produit obtenu possède les caractéristiques suivantes :

Spectre de R.M.N. ¹ H (300 MHz, CDCl₃, δ en ppm) : 1,90 - 1,99 - 2,05 - 2,10 - 2,12 et 2,20 (6 s, respectivement 3H - 3H - 3H - 3H - 6H et 3H : les 7 OCOCH₃) ; 3,16 (AB limite, 2H : CH₂ 6α) ; de 4,10 à 4,45 (mt, 4H : CH₂ 2δ et CH₂ 6δ) ; de 5,20 à 5,35 et 5,50 (2 mts, respectivement 2H et 1H : CH 2γ - CH 6β et CH 6γ) ; 5,64 (dd, J = 9 et 3 Hz, 1H : CH 2β) ; 6,10 (d, J = 3 Hz, 1H : CH 2α) ; 8,38 et 8,41 (2 s, 1H chacun : =CH en 3 et =CH en 5).

La 2-[(1R, 2S, 3R) (1, 2, 3, 4-tétrahydroxybutyl)] 6-[(2'S, 3'R) (2', 3', 4'-trihydroxybutyl)] pyrazine peut être préparée selon la méthode décrite dans le brevet JP 53-90401.

### Exemple 9

### 1, 1', 2, 2', 3, 3', 4, 4'-O, O, O, O, O, O, O, O-octaacétyle-2-[(1R, 2S, 3R) (1, 2, 3, 4-tétrahydroxybutyl)] 5-[(1'R, 2'S, 3'R) (1', 2', 3', 4'-tétrahydroxybutyl)] pyrazine

A 20 mg de 2-[(1R, 2S, 3R) (1, 2, 3, 4-tétrahydroxybutyl)] 6-[(1'R, 2'S, 3'R) (1', 2', 3', 4'-tétrahydroxybutyl)] pyrazine en suspension dans 0,2 cm³ de pyridine séchée sur tamis moléculaire 4Å, est additionné, goutte à goutte à une température voisine de 20 °C sous atmosphère d'argon, 0,104 cm³ d'anhydride acétique. La suspension réactionnelle est agitée 15 heures à une température voisine de 20 °C. Le solvant est évaporé sous pression réduite (2,7 kPa) à une température voisine de 40°C. On obtient 44,7 mg d'un solide blanc qui est purifié par chromatographie sur 2 plaques de gel de silice 60F254 Merck (épaisseur = 0,5 mm, 20x20 cm) en éluant par un mélange dichlorométhane-méthanol (97-3 en volumes). La fraction ne contenant que le produit cherché est extraite par un mélange dichlorométhane-méthanol (85-15 en volumes), filtrée sur verre fritté puis concentrée à sec sous pression réduite (2,7 kPa) à une température voisine de 40°C.

On obtient ainsi 36,1 mg de 1, 1', 2, 2', 3, 3', 4, 4'-O, O, O, O, O, O, O, O-octaacétyle-2-[(1R, 2S, 3R) (1, 2, 3, 4-tétrahydroxybutyl)] 5-[(1'R, 2'S, 3'R) (1', 2', 3', 4'-tétrahydroxybutyl)] pyrazine, sous la forme d'un solide blanc.

Le produit obtenu possède les caractéristiques suivantes :

Spectre de R.M.N. ¹ H (300 MHz, CDCl₃, δ en ppm) : 1,90 - 2,03 - 2,10 - et 2,22 (4 s, respectivement 6H - 6H - 6H et 6H : les 8 OCOCH₃) ; 4,14 (dd, J = 12,5 et 5,5 Hz, 2H : 1H du CH₂ 2δ et 1H du CH₂ 5δ) ; 4,29 (dd, J = 12,5 et 3 Hz, 2H : l'autre H, du CH₂ 2δ et l'autre H du CH₂ 5δ) ; 5,31 (mt, 2H : CH 2γ et CH 5γ) ; 5,67 (dd, J = 9 et 2 Hz, 2H : CH 2β et CH 5β) ; 6,17 (d, J = 2 Hz, 2H : CH 2α et CH 5α) ; 8,47 (s, 2H: =CH en 3 et =CH en 6).

La 2-[(1R, 2S, 3R) (1, 2, 3, 4-tétrahydroxybutyl)] 6-[(1'R, 2'S, 3'R) (1', 2', 3', 4'-tétrahydroxybutyl)] pyrazine peut être préparée selon la méthode décrite dans le brevet JP 53-90401 ou par Kuhn et al, J.L.Ann.Chem.644,122-7,1961.

### Exemple 10

### 1, 2, 2', 3, 3'-O, O, O, O, O-pentabenzyl-2-[(1R, 2S, 3R) (1, 2, 3, 4-tétrahydroxybutyl)] 5-[(2'S, 3'R) (2', 3', 4'-trihydroxybutyl)] pyrazine

### Préparation de : 4, 4'-O, O-di(diphényl-tert-butylsilyl)-2-[(1R, 2S, 3S) (1, 2, 3, 4-tétrahydroxybutyl)] 5-[(2'S, 3'S) (2', 3', 4'-trihydroxybutyl)] pyrazine

A 1,19 g de 2-[(1R, 2S, 3R) (1, 2, 3, 4-tétrahydroxybutyl)] 5-[(2'S, 3'R) (2', 3', 4'-trihydroxybutyl)] pyrazine en suspension dans 30 cm³ de pyridine séchée sur tamis moléculaire 4Å, sont additionnés goutte à goutte à une température voisine de 20°C sous atmosphère d'argon, 2,30 cm³ de chlorure de tert-butyldiphénylsilyle. La suspension réactionnelle est agitée pendant 2 jours et demi à une température voisine de 20°C au bout desquels le milieu s'est solubilisé. Le solvant est évaporé sous pression réduite (0,2 kPa) à une température voisine de 40°C. L'huile résiduelle est co-évaporée avec 2 fois 20 cm³ de toluène sous pression réduite (0,2 kPa) à une température voisine de 40°C. On obtient ainsi une huile jaune qui est purifiée par chromatographie sous pression atmosphérique sur 200 g de gel de silice 60F254 Merck (0,063-0,200 mm) contenus dans une colonne de diamètre 4,5 cm. On utilise un gradient d'éluant constitué d'un mélange dichlorométhane-méthanol : 98-2 en volumes (1000 cm³) puis 95-5 en volumes (2000 cm³) en recueillant des fractions de 25 cm³. Les fractions ne contenant que le produit cherché sont réunies et concentrées à sec sous pression réduite (2,7 kPa) à une température voisine de 40°C.

On obtient ainsi 2,568 g de 4, 4'-O, O-di(diphényl-tert-butylsilyl)-2-[(1R, 2S, 3S) (1, 2, 3, 4-tétrahydroxybutyl)] 5-[(2'S, 3'S) (2', 3', 4'-trihydroxybutyl)] pyrazine sous forme d'une meringue blanche.

Le produit obtenu possède les caractéristiques suivantes :

Spectre de R.M.N. ¹ H (250 MHz, (CD₃)₂SO d6, δ en ppm) ; 1,02 (s, 18H : les 2 C(CH₃)₃) ; 2,78 (dd, J = 14 et 9 Hz, 1H : 1H du CH₂ 5α) ; 3,08 (dd, J = 14 et 2,5 Hz, 1H : l'autre H du CH₂ 5α) ; de 3,55 à 4,00 (mt, 8H : CH 2β - CH 2γ - CH 5β -CH 5γ - CH₂O 2δ et CH₂O 5δ) ; 4,39 (d, J = 9 Hz, 1H : OH en 2β) ; 4,69 (d, J = 7 Hz, 1H : OH en 5β) ; de 4,85 à 5,00 (mt, 2H : OH en 2γ et OH en 5γ) ; 5,02 (d large, J = 6 Hz, 1H : CH 2α) ; 5,37 (d, J = 6 Hz, 1H : OH en 2α) ; de 7,30 à 7,60 et de 7,65 à 7,80 (2 mts, respectivement 12H et 8H : les H aromatiques des 4 phényles) ; 8,41 (d, J = 1 Hz, 1H : =CH en 6) ; 8,66 (d, J = 1 Hz, 1H : =CH en 3).

### Préparation de : 1, 2, 2', 3, 3'-O, O, O, O, O-pentabenzyl-4, 4'-O, O-di(diphényl-tert-butylsilyl)-2-[(1R, 2S, 3S) (1, 2, 3, 4-tétrahydroxybutyl)] 5-[(2'S, 3'S) (2', 3' , 4'-trihydroxybutyl)] pyrazine

A 100 mg de 4, 4'-O, O-di(diphényl-tert-butylsilyl)-2-[(1R, 2S, 3S) (1, 2, 3, 4-tétrahydroxybutyl)] 5-[(2'S, 3'S) (2', 3', 4'-trihydroxybutyl)] pyrazine en solution dans 0,5 cm³ de diméthylformamide sont successivement additionnés à une température voisine de 20°C et sous atmosphère d'argon, 1,15 cm³ de bromure de benzyle, 5 mg d'iodure de sodium et 61,5 mg d'hydrure de sodium (à 50% dans l'huile). Le milieu réactionnel est agité 3 heures 10 minutes à une température voisine de 20°C sous atmosphère d'argon. La suspension orange ainsi obtenue est diluée avec 30 cm³ d'éther éthylique, 5 cm³ d'une solution aqueuse saturée de chlorure d'ammonium et 5 cm³ d'eau distillée. La phase organique est lavée avec 3 fois 5 cm³ d'une solution aqueuse saturée en chlorure de sodium, séchée sur sulfate de magnésium, filtrée sur verre fritté puis concentrée à sec sous pression réduite (0,4 kPa) à une température voisine de 40°C. On obtient ainsi 1,34 g d'une huile orangée qui est purifiée par chromatographie sous pression atmosphérique sur 50 g de gel de silice 60F254 Merck (0,063-0,200 nm) contenue dans une colonne de diamètre 2,5 cm. On utilise un gradient d'éluant constitué d'un mélange dichlorométhane-cyclohexane allant de 0-100 à 100-0 en volumes en recueillant des fractions de 10 cm³. Les fractions ne contenant que le produit cherché sont réunies et concentrées à sec sous pression réduite (environ 2,7 kPa) à une température voisine de 40°C.

On obtient ainsi 39,8 mg de 1, 2, 2', 3, 3'-O, O, O, O, O-pentabenzyl-4, 4'-O, O-di(diphényl-tert-butylsilyl)-2-[(1R, 2S, 3S) (1, 2, 3, 4-tétrahydroxybutyl)] 5-[(2'S, 3'S) (2', 3', 4'-trihydroxybutyl)] pyrazine sous forme d'une huile jaune .

Le produit obtenu possède les caractérisitiques suivantes :

Spectre de R.M.N. ¹ H (400 MHz, CDCl₃ , δ en ppm) : 1,08 (s, 18H : les 2 C(CH₃)₃) ; 3,12 (AB limite, J = 5Hz, 2H : CH₂ 5α) ; de 3,65 à 3,70 (mt, 2H : CH 5γ et 1H d'un OCH₂Ar) ; de 3,75 à 3,95 (mt, 4H : CH 2γ - 1H du CH₂O 2δ - 1H du CH₂O 5δ et 1H d'un OCH₂Ar) ; 4,04 (d large, J = 9 Hz, 1H : l'autre H du CH₂O 5δ) ; de 4,05 à 4,15 (mt, 3H ; CH 2β - l'autre H du CH₂O 2δ et 1H d'un OCH₂Ar) ; 4,19 (mt, 1H : CH 5β) ; de 4,25 à 4,35 (mt, 3H : 3H correspondant aux OCH₃Ar) ; 4,43 (d, J = 11 Hz, 1H : 1H d'un OCH₂Ar) ; 4,50 (d, J = 12 Hz, 1H : 1H d'un OCH₂Ar) ; de 4,55 à 4,85 (mt, 3H : 1H d'un OCH₂Ar et OCH₂Ar) ; 5,03 (d, J = 3 Hz, 1H : CH 2α) ; de 6,65 à 7,80 (mt, 45H : H aromatiques des 9 phényles) ; 8,45 (s, 1H : =CH en 6) ; 8,80 (s, 1H : =CH en 3).

### Préparation de : 1, 2, 2', 3, 3'-O, O, O, O, O-pentabenzyl-2-[(1R, 2S, 3R) (1, 2, 3, 4-tétrahydroxybutyl)] 5-[(2'S, 3'R) (2', 3', 4'-trihydroxybutyl)] pyrazine

A 39 mg de 1, 2, 2', 3, 3'-O, O, O, O, O-pentabenzyl-4, 4'-O, O-di(diphényl-tert-butylsilyl)-2-[(1R, 2S, 3S) (1, 2, 3, 4-tétrahydroxybutyl)] 5-[(2'S, 3'S) (2', 3', 4'-trihydroxybutyl)] pyrazine en solution dans 0,5 cm³ de tétrahydrofurane séché sur tamis moléculaire 4Å, est additionné à une température voisine de 24°C, 0,127 cm³ de solution 1M de fluorure de n-tétrabutylammonium dans le tétrahydrofurane. Le milieu réactionnel devient immédiatemment rouge et est agité à une température voisine de 24°C pendant 40 mn. Le mélange réactionnel est directement purifié par chromatographie sur 3 plaques de silice 60F254 Merck (épaisseur = 0,5 mm, 20x20 cm) en éluant par un mélange dichlorométhane-méthanol (97-3 en volumes). La fraction ne contenant que le produit cherché est extraite par un mélange dichlorométhane-méthanol (85-15 en volumes), filtrée sur verre fritté puis concentrée à sec sous pression réduite (2,7 kPa) à une température voisine de 40°C.

On obtient ainsi 2,9 mg de 1, 2, 2', 3, 3'-O, O, O, O, O-pentabenzyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une laque incolore.

Le produit obtenu possède les caractéristiques suivantes :

Spectre de R.M.N. ¹H (400 MHz, (C-D₃)₂SO d6, δ en ppm) : 3,11 (AB limite, 2H : CH₂ 5α) ; de 3,50 à 3,75 (mt, 6H : CH 2γ - CH 5γ - CH₂O - 1H de l'autre CH₂O et 1H d'un OCH₂Ar) ; 3,87 (d large, J = 10 Hz, 1H : l'autre H du CH₂O) ; 3,93 (dd, J = 8 et 3,5 Hz, 1H : CH 2β) ; 4,13 (mt, 2H : CH 5β et 1H d'un OCH₂Ar) ; de 4,30 à 4,50 (mt, 5H : 5H correspondant aux OCH₂Ar) ; de 4,60 à 4,75 (mt, 4H : 1H d'un OCH₂Ar - OCH₂Ar et OH en δ) ; 4,78 (t, J = 5 Hz, 1H : OH en δ) ; 4,89 (d, J = 3,5 Hz, 1H : CH 2α) ; de 6,95 à 7,45 (mt, 25H : H aromatiques des 5 phényles) ; 8,56 (s, 1H : =CH en 6) ; 8,64 (s, 1H : =CH en 3).

### Exemple 11

### 4, 4'-O, O-diméthoxyacétyl-2-[(1R, 2S, 3R) (1, 2, 3, 4-tétrahydroxybutyl)] 5-[(2'S, 3'R) (2', 3', 4'-trihydroxybutyl)] pyrazine

A une suspension de 304 mg de 2-[(1R, 2S, 3R) (1, 2, 3, 4-tétrahydroxybutyl)] 5-[(2'S, 3'R) (2', 3', 4'-trihydroxybutyl)] pyrazine dans 8 cm³ de pyridine anhydre, sous atmosphère d'Argon, à une température voisine de 25°C, on ajoute goutte à goutte 0,226 cm³ de chlorure de méthoxyacétyle. Après 48 heures à une température voisine de 25°C, le mélange réactionnel est concentré à sec sous pression réduite (0,5 kPa) à une température voisine de 40°C. Le résidu obtenu est repris avec 2 cm³ d'un mélange dichlorométhane-méthanol (80-20 en volumes) et filtré sur verre fritté. Le filtrat est purifié par chromatographie préparative sur 6 plaques de gel de silice 60F254 Merck (épaisseur = 1 mm, 20x20 cm) en éluant par un mélange dichlorométhane-méthanol (80-20 en volumes). La fraction ne contenant que le produit cherché est extraite par un mélange dichlorométhane-méthanol (80-20 en volumes), filtrée sur verre fritté puis concentrée à sec sous pression réduite (0,5 kPa) à une température voisine de 35°C. On obtient ainsi 40 mg de 4, 4'-O, O-diméthoxyacétyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile beige visqueuse. Ce lot est regroupé avec 4 autres lots préparés d'une manière semblable (respectivement 40, 70, 45 et 45 mg), dissous dans un mélange dichlorométhane-méthanol (80-20 en volumes), filtré sur verre fritté puis concentré à sec sous pression réduite (0,5 kPa) à une température voisine de 35°C. On obtient ainsi 225 mg de 4, 4'-O, O-diméthoxyacétyl-2-[(1R, 2S, 3R) (1, 2, 3, 4-tétrahydroxybutyl)] 5-[(2'S, 3'R) (2', 3', 4'-trihydroxybutyl)] pyrazine. Ce lot est purifié par chromatographie préparative sur 7 plaques de gel de silice 60F254 Merck (épaisseur = 0,5 mm, 20x20 cm) en éluant par un mélange dichlorométhane-méthanol (90-10 en volumes). La fraction ne contenant que le produit cherché est extraite par un mélange dichlorométhane-méthanol (80-20 en volumes), filtrée sur verre fritté puis concentrée à sec sous pression réduite (0,5 kPa) à une température voisine de 20°C. On obtient ainsi 90 mg de 4, 4'-O, O-diméthoxyacétyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile beige épaisse. Ce lot est regroupé avec un autre lot (105 mg) préparé d'une manière semblable, dissous dans 5 cm³ de méthanol, filtré sur coton, puis concentré à sec sous courant d'Argon à une température voisine de 20°C, puis sous pression réduite (1 kPa) à une température voisine de 30°C. On obtient ainsi 170 mg de 4, 4'-O, O-diméthoxyacétyl-2-[(1R, 2S, 3R) (1, 2, 3, 4-tétrahydroxybutyl)] 5-[(2'S, 3'R) (2', 3',4'-trihydroxybutyl)] pyrazine sous forme d'une meringue beige.

Le produit obtenu possède les caractéristiques suivantes :

Spectre de R.M.N. ¹ H (400 MHz, (CD₃)₂SO d6, δ en ppm) : 2,75 et 3,14 (2dd, respectivement J = 14 et 9 Hz et J = 14 et 3 Hz, 1H chacun : CH₂ 5α) ; 3,35 (les 6H correspondants aux OCH₃) ; de 3,50 à 3,65 (mt, 2H : CH 5γ et CH 2β) ; 3,78 (mt, 1H : CH 5β) ; 3,87 (mt, 1H : CH 2γ) ; de 4,05 à 4,15 (mt, 2H : 1H du CH₂O 2δ et 1H du CH₂O 5δ) ; 4,08 (s, 4H : COCH₂O) ; 4,33 (dd, J = 12 et 3 Hz, 1H : l'autre H du CH₂O 5δ) ; 4,37 (dd, J = 11 et 2 Hz, 1H : l'autre H du CH₂O 2δ) ; 4,68 (d, J = 9 Hz, 1H : OH en 2β) ; 4,90 (d, J = 6,5 Hz, 1H : OH en 5β) ; 4,96 (d large, J = 6 Hz, 1H : CH 2α) ; 5,13 (d, J = 5,5 Hz, 1H : OH en 2γ) ; 5,18 (d, J = 5,5 Hz, 1H : OH en 5γ) ; 5,45 (d, J = 6 Hz, 1H : OH en 2α) ; 8,43 (s large, 1H : =CH en 6) ; 8,67 (s large, 1H : =CH en 3).

### Exemple 12

### 1, 2, 2', 3, 3', 4, 4'-O, O, O, O, O, O, O-heptaformyl-2-[(1R, 2S, 3R) (1, 2, 3,4-tétrahydroxybutyl)] 5-[(2'S, 3'R) (2', 3', 4'-trihydroxybutyl)] pyrazine

A 650 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 20 cm³ de pyridine séchée sur tamis moléculaire 4Å est additionné, sous atmosphère d'Argon, goutte à goutte en 30 minutes, à une température voisine de 0°C sous atmosphère d'argon, un mélange de 13 cm³ d'acide formique et 6 cm³ d'anhydride acétique. Le mélange réactionnel est ensuite concentré à sec puis coévaporé avec du toluène, sous pression réduite (environ 2,7 kPa), à une température voisine de 40°C. On obtient ainsi 1,1 g d'un solide blanc. Un échantillon de 500 mg de ce solide blanc est repris avec 7.5 cm³ de dichlorométhane et quelques gouttes de méthanol pour former une suspension. La partie soluble de cette suspension est purifiée par chromatographie préparative sur 5 plaques de gel de silice 60F254 Merck (épaisseur = 1 mm, 20x20 cm) en éluant par un mélange dichlorométhane-acétate d'éthyle (7-3 en volumes). La fraction ne contenant que le produit cherché est extraite par un mélange dichlorométhane-méthanol (90-10 en volumes), filtrée sur verre fritté puis concentrée à sec sous pression réduite (2,7 kPa) à une température voisine de 40°C. On obtient ainsi 116 mg de 1, 2, 2', 3, 3', 4, 4'-O, O, O, O, O, O, O-heptaformyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une laque qui cristallise partiellement. La partie insoluble de la suspension précédente est reprise avec du dichlorométhane et purifiée par chromatographie préparative sur 3 plaques de gel de sllice 60F254 Merck (épaisseur = 1 mm, 20x20 cm) en éluant par un mélange dichlorométhane-acétate d'éthyle (6-4 en volumes). La fraction ne contenant que le produit cherché est extraite par de l'acétate d'éthyle, filtrée sur verre fritté puis concentrée à sec sous pression réduite (2,7 kPa) à une température voisine de 40°C. On obtient ainsi 43 mg d'une seconde fraction de 1, 2, 2', 3, 3', 4, 4'-O, O, O, O, O, O, O-heptaformyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une laque incolore. Les deux fractions précédentes de 1, 2, 2', 3, 3', 4, 4'-O, O, O, O, O, O, O-heptaformyl-2-[(1R, 2S, 3R) (1, 2, 3, 4-tétrahydroxybutyl)] 5-[(2'S, 3'R) (2', 3', 4'-trihydroxybutyl)] pyrazine sont regroupées avec une autre fraction de ce même produit préparé dans des conditions identiques pour former un lot unique représentant 232 mg.

Le produit obtenu possède les caractéristiques suivantes :

Spectre de R.M.N. ¹ H (400 MHz, (CD₃)₂SO d6, δ en ppm): 3,22 (dd, J = 15 et 9,5 Hz, 1H : 1H du CH₂ 5α) ; de 3,30 à 3,40 (mt : 1H correspondant à l'autre H du CH₂ 5α) ; 4.24 (dd, J = 12 et 6 Hz, 1H : 1H du CH₂ 2δ) ; 4,32 (dd, J = 12 et 7,5 Hz, 1H : 1H du CH₂ 5δ) ; 4,49 (dd, J = 12 et 2,5 Hz, 1H : l'autre H du CH₂ 2δ) ; 4,53 (dd, J = 12 et 3 Hz, 1H : l'autre H du CH₂ 5δ) ; 5,33 (mt, 1H : CH 2γ) ; 5,40 (mt, 1H : CH 5γ) ; 5,62 (mt, 1H : CH 5β) ; 5,74 (dd, J = 7 et 4 Hz, 1H : CH 2β) ; 6,18 (d, J = 4 Hz, 1H : CH 2α) ; 8,10 - 8,16 - 8,25 - 8,28 - 8,30 - 8,34 et 8,43 (7 s, 1H chacun : les 7 HC=O) ; 8,63 (s large, 1H : =CH en 6) ; 8,66 (s large, 1H : =CH en 3).

### Exemple 13

### 1, 2, 2', 3, 3', 4, 4'-O, O, O, O, O, O, O-heptamonosuccinate de méthyle-2-[(1R, 2S, 3R) (1, 2, 3, 4-tétrahydroxybutyl)] 5-[(2'S, 3'R) (2', 3', 4'-trihydroxybutyl)] pyrazine

A 50 mg de 2-[(1R, 2S, 3R) (1, 2, 3, 4-tétrahydroxybutyl)] 5-[(2'S, 3'R) (2', 3', 4'-trihydroxybutyl)] pyrazine, 50 mg de tamis moléculaire 4Å en poudre et 228 mg de monosuccinate de méthyle en suspension dans 1 cm³ de N,N-diméthylformamide anhydre, sont additionnés successivement, à une température voisine de 24°C, sous atmosphère d'Argon, 368 mg de dicyclohexylcarbodiimide et 6 mg de 4-(N,N-diméthylamino)-pyridine. Après 5 heures à une température voisine de 24°C, le mélange réactionnel est dilué avec 30 cm³ d'acétate d'éthyle, lavé avec 3 fois 8 cm³ d'eau, 8 cm³ d'une solution aqueuse saturée en chlorure de sodium, puis séché sur sulfate de magnésium, filtré sur verre fritté, et concentré à sec sous pression réduite (0,5 kPa) à une température voisine de 42°C. On obtient ainsi 608 mg d'un solide rosé que l'on purifie par chromatographie sous pression atmosphérique sur 40 g de gel de silice 60F254 Merck (0,063-0,200 mm) contenus dans une colonne de diamètre 2,5 cm. On utilise un gradient d'élution constitué d'un mélange dichlorométhane-méthanol 99-1 en volumes (0,25 l) puis 98-2 en volumes (0,25 1) en recueillant des fractions de 12 cm³. Les fractions ne contenant que le produit cherché sont réunies et concentrées à sec sous pression réduite (environ 2,7 kPa) à une température voisine de 40°C. On obtient ainsi 166,5 mg 1, 2, 2', 3, 3', 4, 4'-O, O, O, O, O, O, O-heptamonosuccinate de méthyle-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile incolore.

Le produit obtenu possède les caractéristiques suivantes :

Spectre de R.M.N. ¹ H (400 MHz, (CD₃)₂SO d6, δ en ppm) : de 2,25 à 2,85 (mt, 28H : les 7 COCH₂CH₂CO) ; 3,15 (dd, J = 14 et 9 Hz, 1H : 1H du CH₂ 5α) ; de 3,20 à 3,35 (mt : 1H correspondant à l'autre H du CH₂ 5α) ; 3,62 et 3,65 (2 s, 21H en totalité : les 7 COOCH₃) ; 4,16 (dd, J = 12 et 5,5 Hz, 1H : 1H du CH₂O 2δ) ; 4,25 (dd, J = 12 et 7 Hz, 1H : 1H du CH₂O 5δ) ; 4,38 (mt, 2H : l'autre H du CH₂O 2δ et l'autre H du CH₂O 5δ) ; 5,25 (mt, 2H : CH 2γ et CH 5γ) 5,47 (mt, 1H : CH 5β) 5,59 (dd, J = 8 et 4 Hz, 1H : CH 2β) ; 6,00 (d, J = 4 Hz, 1H : CH 2α) ; 8,58 (s, 2H : =CH en 6 et =CH en 3).

### Exemple 14

### 1, 2, 2', 3, 3', 4, 4'-O, O, O, O, O, O, O-heptamonoglutarate d'éthyle-2-[(1R, 2S, 3R) (1, 2, 3, 4-tétrahydroxybutyl)] 5-[(2'S, 3'R) (2', 3', 4'-trihydroxybutyl)] pyrazine

A 500 mg de 2-[(1R, 2S, 3R) (1, 2, 3, 4-tétrahydroxybutyl)] 5-[(2'S, 3'R) (2', 3', 4'-trihydroxybutyl)] pyrazine, 2 g de tamis moléculaire 4Å en poudre et 2,76 g de monoglutarate d'éthyle en suspension dans 10 cm³ de N,N-diméthylformamide anhydre, sont additionnés successivement, à une température voisine de 24°C, sous atmosphère d'Argon, 3,68 g de dicyclohexylcarbodiimide et 60 mg de 4-(N,N-diméthylamino)-pyridine. Après 13 heures à une température voisine de 22°C, le mélange réactionnel est filtré sur verre fritté. Le verre fritté est rincé avec 60 cm³ d'acétate d'éthyle. La phase organique est lavée avec 2 fois 30 cm³ d'une solution aqueuse saturée en hydrogénocarbonate de sodium, 6 fois 30 cm³ d'eau et 30 cm³ d'une solution aqueuse saturée en chlorure de sodium, puis séchée sur sulfate de magnésium, filtrée sur verre fritté, et concentrée à sec sous pression réduite (0,6 kPa) à une température voisine de 42°C. On obtient ainsi 2,16 g d'une huile brune dont on purifie un échantillon de 200 mg par chromatographie préparative sur 8 plaques de gel de silice 60F254 Merck (épaisseur = 0,5 mm, 20x20 cm) en éluant par un mélange dichlorométhane-méthanol (97-3 en volumes). La fraction ne contenant que le produit cherché est extraite par un mélange dichlorométhane-méthanol (85-15 en volumes), filtrée sur verre fritté puis concentrée à sec sous pression réduite (2,7 kPa) à une température voisine de 40°C. Cm obtient ainsi 82,6 mg 1, 2, 2', 3, 3', 4, 4'-O, O, O, O, O, O, O-heptamonoglutarate d'éthyle-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile incolore.

Le produit obtenu possède les caractéristiques suivantes :

Spectre de R.M.N. ¹ H (400 MHz, (CD₃)₂SO d6, δ en ppm) : 1,17 (t, J = 8 Hz, 21H : les 7 CH₃) ; de 1,50 à 1,85 (mt, 14H : les 7 CH₂ centraux) ; de 2,10 à 2,60 (mt, 28H : les 14 COCH₂) ; 3,11 (dd, J = 14 et 9 Hz, 1H : 1H du CH₂ 5α) ; 3,22 (dd, J = 14 et 3,5 Hz, 1H : l'autre H du CH₂5α) ; 4,05 (mt, 14H : les 7 COOCH₂) ; de 4,10 à 4,25 (mt, 2H : 1H du CH₂ 2δ et 1H du CH₂ 5δ) ; 4,31 et 4,39 (d large et dd, respectivement J = 12 Hz et J = 12 et 3 Hz, 1H chacun : l'autre H du CH₂ 2δ et l'autre H du CH₂ 5δ) ; 5,23 (mt, 2H : CH 2γ et CH 5γ) ; 5,44 (mt, 1H : CH 5β) ; 5,54 (dd, J = 7 et 4 Hz, 1H : CH 2β) ; 5,98 (d, J = 4 Hz, 1H : CH 2α) ; 8,53 (s large, 2H : =CH en 3 et =CH en 6)

### Exemple 15

### 1, 2-O, O 3, 4-O, O 3', 4'-O, O-tricarbonyl-2-[(1R, 2S, 3R) (1, 2, 3, 4-tétrahydroxybutyl)] 5-[(2'S, 3'R) (2',3', 4'-trihydroxybutyl)] pyrazine

A 1,52 g de 2-[(1R, 2S, 3R) (1, 2, 3, 4-tétrahydroxybutyl)] 5-[(2'S, 3'R) (2', 3', 4'-trihydroxybutyl)] pyrazine en suspension dans 50 cm³ de pyridine anhydre, à une température voisine de 20°C, sous atmosphère d'Argon, est additionné 1,567 cm³ de chloroformiate de phényle. Après 66 heures à une température voisine de 20°C, le mélange réactionnel est concentré à sec sous pression réduite à une température voisine de 40°C. Le résidu obtenu est repris et agité avec un mélange de 50 cm³ de dichlorométhane et 25 cm³ d'eau. Après décantation, la phase organique est séchée sur sulfate de magnésium, filtrée sur verre fritté, et concentrée à sec sous pression réduite à une température voisine de 40°C. On obtient ainsi 2 g d'une huile beige épaisse. La phase aqueuse est filtrée sur verre fritté, et le résidu solide séché à l'air fournit 54 mg de 1, 2-O, O 3, 4-O, O 3', 4'-O, O-tricarbonyl-2-[(1R, 2S, 3R) (1, 2, 3, 4-tétrahydroxybutyl)] 5-[(2'S, 3'R) (2', 3', 4'-trihydroxybutyl)] pyrazine. Le filtrat est concentré à sec sous pression réduite à une température voisine de 40°C. On obtient ainsi 1,2 g d'une huile brun clair que l'on purifie par chromatographie sous pression atmosphérique sur une hauteur de 20 cm de gel de silice 60F254 Merck (0,063-0,040 mm) contenus dans une colonne de diamètre 1,5 cm, en utilisant un éluant constitué d'un mélange dichlorométhane-méthanol (95-5 en volumes) et en recueillant des fractions de 15 cm³. Les fractions ne contenant que le produit cherché sont réunies et concentrées à sec sous pression réduite (environ 2,7 kPa) à une température voisine de 40°C. On obtient ainsi 0,34 g de 1, 2-O, O 3, 4-O, O 3', 4'-O, O-tricarbonyl-2-[(1R, 2S, 3R) (1, 2, 3, 4-tétrahydroxybutyl)] 5-[(2'S, 3'R) (2',3',4'-trihydroxybutyl)] pyrazine sous forme d'un solide pâteux jaune. Les échantillons de 54 mg et 0,34 g de 1, 2-O, O 3, 4-O, O 3', 4'-O, O-tricarbonyl-2-[(1R, 2S, 3R) (1, 2, 3, 4-tétrahydroxybutyl)] 5-[(2'S, 3'R) (2', 3', 4'-trihydroxybutyl)] pyrazine précédents sont réunis, dissous dans 10 cm³ de diméthylsulfoxyde. La solution obtenue est filtrée sur verre fritté et concentrée sous pression réduite (environ 0,2 kPa) à une température voisine de 75°C. L'huile brun clair obtenue est aussitôt dissoute dans 4 cm3 de méthanol. Le produit insoluble formé est filtré sur verre fritté, lavé avec 3 fois 1 cm3 de méthanol, séché à l'air, puis séché sous pression réduite (1 kPa) à une température voisine de 40°C. On obtient ainsi 0,252 g de 1, 2-O, O 3, 4-O, O 3', 4'-O, O-tricarbonyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une poudre beige.

Le produit obtenu possède les caractéristiques suivantes :

Spectre de R.M.N. ¹ H (400 MHz, (CD₃)₂SO d6, δ en ppm) : 2,91 et 3,00 (2dd, respectivement J = 14 et 9 Hz et J = 14 et 4 Hz, 1H chacun : CH₂ 5α) ; 4,22 (mt, 1H : CH 5β) ; de 4,40 à 4,55 (mt, 2H : 1H du CH₂O 2δ et 1H du CH₂O 5δ) ; 4,56 (t, J *=* 8,5 Hz, 1H : l'autre H du CH₂O 5δ) ; 4,70 (t, J = 8,5 Hz, 1H : l'autre H du CH₂O 2δ) ; 4,82 (mt, 1H : CH 5γ) ; de 5,25 à 5,40 (mt, 2H : CH 2γ et CH 2β) ; 5,65 (d, J = 5,5 Hz, 1H : OH en 5β) ; 6,12 (d, J = 5 Hz, 1H : CH 2α) ; 3,71 (d, J = 1 Hz, 1H : =CH en 6) ; 8,83 (d, J = 1 Hz, 1H : =CH en 3).

### Exemple 16

### 3, 4-O, O 3', 4'-O, O-bis-carbonyl-2-[(1R, 2S, 3R) (1, 2, 3, 4-tétrahydroxybutyl)] 5-[(2'S, 3'R) (2', 3', 4'-trihydroxybutyl)] pyrazine

A 1,52 g de 2-[(1R, 2S, 3R) (1, 2, 3, 4-tétrahydroxybutyl)] 5-[(2'S, 3'R) (2', 3', 4'-trihydroxybutyl)] en suspension dans 50 cm³ de pyridine anhydre, à une température voisine de 20°C, sous atmosphère d'Argon, sont additionnés 2,19 cm³ de chloroformiate de phényle. Après 72 heures à une température voisine de 20°C, le mélange réactionnel est concentré à sec sous pression réduite (1 kPa) à une température voisine de 40°C. L'huile beige clair obtenue est reprise et agitée avec un mélange de 25 cm³ d'acétate d'éthyle et 25 cm³ d'eau. Après décantation, la phase organique est lavée avec 2 fois 5 cm³ d'eau, tandis que la phase aqueuse est lavée avec 5 cm³ d'acétate d'éthyle. Les phases aqueuses sont rassemblées et concentrées à sec sous pression réduite (0,5 kPa) à une température voisine de 50°C. On obtient ainsi 2,74 g d'un solide pâteux jaune clair. Les phases organiques sont rassemblées, séchées sur sulfate de magnésium, filtrées sur verre fritté, et concentrées à sec sous pression réduite (0,5 kPa) à une température voisine de 50°C. On obtient ainsi 2,86 g d'une huile beige épaisse. Le solide pâteux jaune clair précédent (2,74 g) est purifié par chromatographie sous pression atmosphérique sur une hauteur de 20 cm de gel de silice 60F254 Merck (0,063-0,040 mm) contenus dans une colonne de diamètre 2,5 cm, en utilisant un éluant constitué d'un mélange dichlorométhane-méthanol (95-5 puis 90-10 en volumes) et en recueillant des fractions de 15 cm³. Les fractions ne contenant que le produit cherché sont réunies et concentrées à sec sous pression réduite (environ 2,7 kPa) à une température voisine de 40°C. Le solide pâteux blanc obtenu (1,4 g) est repris avec 5 cm³ de méthanol, agité quelques minutes, filtré sur verre fritté, lavé avec 3 fois 2 cm³ de méthanol, et séché à l'air. On obtient ainsi 0,183 g d'une poudre blanche, que l'on réunit avec un autre lot préparé de manière semblable (70 mg). Le mélange obtenu est dissous dans 4 cm³ de diméthylsulfoxyde, filtré sur verre fritté, et concentré sous pression réduite (0, kPa) à une température voisine de 70°C. On obtient ainsi 0,5 g d'une huile beige que l'on dissout dans 1 cm³ de méthanol. Après addition goutte à goutte de 5 cm³ d'eau, le mélange est placé à une température voisine de 4°C pendant 48 heures, puis filtré sur verre fritté. Le produit insoluble formé est lavé avec 2 cm3 d'eau, séché à l'air, puis séché sous pression réduite (0, kPa) à une température voisine de 40°C. On obtient ainsi 188 mg de 3, 4-O, O 3', 4'-O, O-bis-carbonyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une poudre beige.

Le produit obtenu possède les caractéristiques suivantes :

Spectre de R.M.N. ¹ H (400 MHz, (CD₃)₂SO d6, δ en ppm) : 2,84 et 2,92 (2dd, respectivement J = 14 et 9 Hz et J = 14 et 5 Hz, 1H chacun : CH₂ 5α) ; 4,11 (mt, 1H : CH 2β) ; 4,20 (mt, 1H : CH 5β) ; 4,48 (dd, J = 9 et 6,5 Hz, 1H : 1H du CH₂O 5δ) ; de 4,50 à 4,60 (mt, 3H : CH₂O 2δ et l'autre H du CH₂O 5δ) ; 4,78 (s large, 1H : CH 2α) ; 4,80 (mt, 1H : CH 5γ) ; 5,02 (mt, 1H : CH 2γ) ; de 5,40 à 6,00 (3 mfs, 1H chacun : OH en 2α - OH en 2β et OH en 5β) ; 8,51 (s, 1H : =CH en 6) ; 8,68 (s, 1H : =CH en 3).

### Exemple 17

### 1, 2, 2', 3, 3', 4, 4'-O, O, O, O, O, O, O-heptamonofumarate d'éthyle-2-[(1R, 2S, 3R) (1, 2, 3, 4-tétrahydroxybutyl)] 5-[(2'S, 3'R) (2', 3', 4'-trihydroxybutyl)] pyrazine

A 304 mg de 2-[(1R, 2S, 3R) (1, 2, 3, 4-tétrahydroxybutyl)] 5-[(2'S, 3'R) (2', 3', 4'-trihydroxybutyl)] pyrazine, 1,51 g de monofumarate d'éthyle, 2,27 g de dicyclohexylcarbodiimide et 37 mg de 4-(N,N-diméthylamino)-pyridine, est ajouté, à une température voisine de 20°C, sous atmosphère d'Argon, 6 cm³ de N,N-diméthylformamide anhydre. Après 24 heures à une température voisine de 22°C, le mélange réactionnel est dilué avec 10 cm³ d'eau et 25 cm³ d'acétate d'éthyle, agité et filtré sur verre fritte. Le verre fritté est rincé avec 5 cm³ d'acétate d'éthyle. Après décantation du filtrat, la phase organique est lavée avec 6 fois 20 cm³ d'eau, séchée sur sulfate de magnésium, filtrée sur verre fritté, et concentrée à sec sous pression réduite à une température voisine de 40°C. On obtient ainsi 2,1 g d'une huile brune que l'on purifie par chromatographie sous pression atmosphérique sur une hauteur de 20 cm de gel de silice 60F254 Merck (0,063-0,040 mm) contenus dans une colonne de diamètre 1 cm, en utilisant un éluant constitué d'un mélange dichlorométhane-méthanol (99-1 en volumes) et en recueillant des fractions de 12 cm³. Les fractions ne contenant que le produit cherché sont réunies et concentrées à sec sous pression réduite (environ 2,7 kPa) à une température voisine de 40°C. On obtient ainsi 1,4 g d'une huile épaisse quasi-incolore, dont on purifie 1,3 g par chromatographie sous pression atmosphérique sur une hauteur de 30 cm de gel de silice 60F254 Merck (0,063-0,040 mm) contenus dans une colonne de diamètre 1,5 cm, en utilisant un éluant constitué d'un mélange dichlorométhane-méthanol (99-1 en volumes) et en recueillant des fractions de 12 cm³. Les fractions ne contenant que le produit cherché sont réunies et concentrées à sec sous pression réduite (environ 2,7 kPa) à une température voisine de 40°C. On obtient ainsi 305 mg d'une pâte beige pâle que l'on purifie par chromatographie préparative sur 10 plaques de gel de silice 60F254 Merck (épaisseur = 0,5 mm, 20x20 cm) en éluant par un mélange dichlorométhane-méthanol (99-1 en volumes). La fraction ne contenant que le produit cherché est extraite par un mélange dichlorométhane-méthanol (90-10 en volumes), filtrée sur verre fritté puis concentrée à sec sous pression réduite (2,7 kPa) à une température voisine de 40°C. On obtient ainsi 205 mg d'une huile incolore qui est reprise avec 5 cm³ de dichlorométhane. La solution obtenue est filtrée sur coton, et concentrée à sec sous pression réduite (environ 2,7 kPa) à une température voisine de 30°C. On obtient ainsi 203 mg de 1, 2, 2', 3, 3', 4, 4'-O, O, O, O, O, O, O-heptamonofumarate d'éthyle-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile incolore.

Le produit obtenu possède les caractéristiques suivantes :

Spectre de R.M.N. ¹ H (400 MHz, (CD₃)₂SO d6, δ en ppm) : 1,27 (mt, 21H : les CH₃ des éthyles) ; de 3,20 à 3,40 (mt : les 2H correspondant aux CH₂ 5β) ; 4,21 (mt, 14H : les OCH₂ des éthyles) ; 4,41 (dd, J = 13 et 5 Hz, 1H : 1H du CH₂O 2δ) ; 4.48 (dd, J = 12 et 7 Hz, 1H : 1H du CH₂O 5δ) ; 4,57 (dd, J = 13 et 2 Hz, 1H : l'autre H du CH₂O 2δ) ; 4,63 (dd, J = 12 et 3 Hz, 1H : l'autre H du CH₂O 5δ) ; 5,46 (mt, 1H : CH 5γ) ; 5,53 (mt, 1H : CH 2γ) ; 5,66 (mt, 1H : CH 5β) ; 5,79 (dd, J = 8 et 3,5 Hz, 1H : CH 2β) ; 6,23 (d, J = 3,5 Hz, 1H : CH 2α) ; de 6,50 à 6,95 (mt, 14H : les CH=CH) ; 8,61 (s large, 1H : =CH en 6) ; 8,71 (s large, 1H : =CH en 3).

### Exemple 18

### 1, 2, 2'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine

### Première voie :

### Préparation de : 3, 4-O, O -3', 4'-O, O-bis-pentylidène-2-[(1R, 2S, 3S) (1, 2, 3, 4-tétrahydroxybutyl)] 5-[(2'S, 3'S) (2', 3', 4'-trihydroxybutyl)] pyrazine

A 500 mg de 2-[(1R, 2S, 3R) (1, 2, 3, 4-tétrahydroxybutyl)] 5-[(2'S, 3'R) (2', 3', 4'-trihydroxybutyl)] pyrazine en suspension, dans 12 cm³ de diméthylformamide, sont successivement additionnés à une température voisine de 20°C, 2,18 cm³ de cyclopentanone et 31 mg d'acide para-toluènesulfonique monohydrate. La solution obtenue après 30 minutes d'agitation à une température voisine de 20°C est de nouveau agitée pendant 2 heures 30 minutes à une température voisine de 20°C. On additionne ensuite du sulfate de magnésium et le milieu réactionnel est agité pendant 16 heures supplémentaires à une température voisine de 20°C. Le milieu est ensuite chauffé à une température voisine de 60°C pendant 4 heures. Le milieu réactionnel est laissé revenir à une température voisine de 20°C et est dilué avec un mélange de 10 cm³ d'eau distillée et 10 cm³ d'acétate d'éthyle. La phase organique, après décantation, est lavée avec deux fois 10 cm³ d'eau distillée. Les phases aqueuses sont réunies et extraite avec une fois 10 cm³ d'acétate d'éthyle. Les phases organiques réunies sont séchées sur sulfate de magnésium, filtrées sur verre fritté puis concentrées à sec sous pression réduite (0,27 kPa) à une température voisine de 30°C. On obtient ainsi 224 mg d'une laque jaune qui est purifiée par chromatographie sur 4 plaques de gel de silice 60F254 Merck (épaisseur = 1 mm, 20x20 cm) en éluant avec de l'acétate d'éthyle. La fraction ne contenant que le produit cherché est extraite par un mélange dichlorométhane-méthanol (90-10 en volumes), filtrée sur verre fritté puis concentrée à sec sous pression réduite (2,7 kPa) à une température voisine de 20°C.

On obtient ainsi 77 mg de 3, 4-O, O -3', 4'-O, O-bis-cyclopentylidène-2-[(1R, 2S, 3S) (1, 2, 3, 4-tétrahydroxybutyl)] 5-[(2'S, 3'S) (2', 3', 4'-trihydroxybutyl)] pyrazine sous forme d'une huile jaune pâle.

Le produit obtenu possède les caractéristiques suivantes :

Spectre de R.M.N. ¹ H (400 MHz, (CD₃)₂SO d6, δ en ppm) : de 1,50 à 1,80 (mf, 16H : les 4 CH₂ des 2 cyclopentyles) ; 2,76 (dd, J = 14 et 9 Hz, 1H : 1H du CH₂ 5α) ; 3,03 (dd, J = 14 et 3,5 Hz, 1H : l'autre H du CH₂ 5α) ; 3,62 (t large, J = 7 Hz, 1H : CH 2β) ; 3,77 (mt, 1H : CH 5β) ; de 3,70 à 4,00 (2 mts, respectivement 3H et 2H : CH 5γ - CH₂O 2δ et CH₂O 5δ) ; 4,15 (mt, 1H : CH 2γ) ; 4,78 (d large, J = 6 Hz, 1H : CH 2α) ; 4,82 (d, J = 8 Hz, 1H : OH en 2β) ; 5,04 (d, J = 6,5 Hz, 1H : OH en 5β) ; 5,56 (d, J = 6 Hz, 1H : OH en 2α) ; 8,43 (s, 1H : =CH en 6) ; 8,65 (s, 1H : =CH en 3).

### Préparation de : 1, 2, 2'-O, O, O-tribenzoyl 3, 4-O, O -3', 4'-O, O-bis-cyclopentylidène-2-[(1R, 2S, 3R) (1, 2, 3, 4-tétrahydroxybutyl)] 5-[(2'S, 3'R) (2', 3', 4'-trihydroxybutyl)] pyrazine

A une solution de 730 mg de 3, 4-O, O -3', 4'-O, O-bis-cyclopentylidène-2-[(1R, 2S, 3R) (1, 2, 3, 4-tétrahydroxybutyl)] 5-[(2'S, 3'R) (2', 3', 4'-trihydroxybutyl)] pyrazine dans 8 cm³ de pyridine anhydre, sous atmosphère d'Argon, à une température voisine de 23°C, on ajoute goutte à goutte 0,97 cm³ de chlorure de benzoyle. Après 3 heures à une température voisine de 23°C, le mélange réactionnel est dilué par 10 cm³ de dichlorométhane et 5 cm³ d'eau, agité 5 minutes puis décanté. La phase organique est lavée par 5 cm³ d'eau, séchée sur sulfate de magnésium, filtrée sur papier puis concentrée à sec et coévaporée avec du toluène sous pression réduite (2 kPa) à une température voisine de 40°C. On obtient 1,9 g d'une huile jaune qui est purifiée par chromatographie sous pression atmosphérique sur 200 g de gel de silice 60F254 Merck (0,063-0,200 mm) contenus dans une colonne de diamètre 4 cm, en utilisant un gradient d'élution (dichlorométhane-méthanol 100-0 puis 98-2 en volumes) et en recueillant des fractions de 30 cm³. Les fractions ne contenant que le produit cherché sont réunies et concentrées à sec sous pression réduite (environ 2,7 kPa) à une température voisine de 40°C. La meringue obtenue est reprise dans de l'éther éthylique et concentrée à sec sous pression réduite (environ 2,7 kPa) à une température voisine de 40°C. On obtient ainsi 1,14 g de 1, 2, 2'-O, O, O-tribenzoyl 3, 4-O, O -3', 4'-O, O-bis-cyclopentylidène-2-[(1R, 2S, 3R) (1, 2, 3, 4-tétrahydroxybutyl)] 5-[(2'S, 3'R) (2', 3', 4'-trihydroxybutyl)] pyrazine sous forme d'un solide amorphe de couleur ivoire.

Le produit obtenu possède les caractéristiques suivantes :

Spectre de R.M.N. ¹ H (400 MHz, (CD₃)₂SO d6, δ en ppm) : de 1,40 à 1,70 (mf, 16H : les CH₂ des cyclopentyles) ; 3,14 et 3,23 (2 dd, respectivement J = 14 et 8 Hz et J = 14 et 5 Hz, 1H chacun : CH₂ 5α) ; 3,89 (dd, J = 9 et 6 Hz, 1H : 1H du CH 5δ) ; de 3,90 à 4,05 (mt, 3H : CH₂O 2δ et l'autre H du CH₂O 5δ) : 4,25 (mt, 1H : CH 5γ) ; 4,39 (mt, 1H : CH 2γ) ; 5,48 (mt, 1H : CH 5β) ; 5,82 (t, J = 5 Hz, 1H : CH 2β) ; 6,29 (d, J = 5 Hz, 1H : CH 2α) ; de 7,35 à 8,05 (mt, 15H : H aromatiques) ; 8,56 (d, J = 1Hz, 1H : =CH en 6) ; 8,63 (d, J = 1Hz, 1H : =CH en 3).

### Préparation de : 1, 2, 2'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine

A une solution de 500 mg de 1, 2, 2'-O, O, O-tribenzoyl 3, 4-O, O -3', 4'-O, O-bis-cyclopentylidène-2-[(1R, 2S, 3R) (1, 2, 3, 4-tétrahydroxybutyl)] 5-[(2'S, 3'R) (2', 3', 4'-trihydroxybutyl)] pyrazine dans 4 cm³ de dichlorométhane, à une température voisine ce 20°C, on ajoute lentement 0,614 cm³ d'acide trifluoroacétique (acide trifluoroacétique à 80% en section aqueuse). Après 80 minutes à une température voisine de 20°C, on additionne au mélange réactionnel 6 cm³ d'une solution aqueuse saturée en bicarbonate de sodium en limitant le dégagement gazeux. Après décantation, la phase aqueuse est réextraite avec 3 cm³ de dichlorométhane. Les phases organiques sont rassemblées, séchées sur sulfate de magnésium, filtrées puis concentrées à sec sous pression réduite (2 kPa) à une température voisine de 40°C. On obtient 400 mg d'une meringue ivoire. La moitié de cette meringue ivoire est purifiée par chromatographie préparative sur 8 plaques de gel de silice 60F254 Merck (épaisseur = 0,5 mm, 20x20 cm) en éluant par un mélange dichlorométhane-méthanol (95-5 en volumes). La fraction ne contenant que le produit cherché est extraite par un mélange dichlorométhane-méthanol (90-10 en volumes), filtrée sur verre fritté puis concentrée à sec sous pression réduite (2,7 kPa) à une température voisine de 40°C. On obtient ainsi 116 mg de 1, 2, 2'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue blanche. Le solde de la meringue ivoire précédente est purifié selon la même méthode et conduit à 124 mg de 1, 2, 2'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue blanche.

Le produit obtenu possède les caractéristiques suivantes :

Spectre de R.M.N. ¹ H (400 MHz, (CD₃)₂SO d6, δ en ppm) : 3,21 (AB limite, 2H : CH₂ 5α) ; de 3,30 à 3,55 (mt : 4H correspondant aux CH₂ 2δ et CH₂ 5δ) ; 3,74 (mt, 1H : CH 5γ) ; 3,84 (mt, 1H : CH 2γ) ; 4,71 (mt, 2H : OH en 2δ et OH en 5δ) ; 5,16 (d, J = 5,5 Hz, 1H : OH en 5γ) ; 5,39 (mt, 2H : CH 5β et OH en 2γ) ; 5,67 (dd, J = 7 et 4 Hz, 1H: CH 2β) ; 6,43 (d, J = 4 Hz, 1H : CH 2α) ; de 7,30 à 8,05 (mt, 15H : H aromatiques) ; 8,45 (d, J = 1 Hz, 1H : =CH en 6) ; 8,53 (d, J = 1 Hz, 1H : =CH en 3).

### Deuxième voie:

La 1, 2, 2'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine peut être également obtenue, dans les mêmes conditions que ci-dessus (déprotection dans un milieu dichlorométhane-acide trifluoroacétique, à une température comprise entre 0°C et 40°C) à partir du dérivé 1, 2, 2'-O, O, O-tribenzoyl 3, 4-O, O -3', 4'-O, O-bis-cyclohexylidène-2-[(1R, 2S, 3R) (1, 2, 3, 4-tétrahydroxybutyl)] 5-[(2'S, 3'R) (2', 3', 4'-trihydroxybutyl)] pyrazine, qui peut être préparé de la façon suivante :

### Préparation de : 3, 4-O, O - 3', 4'-O, O-bis-cyclohexylidène-2-[(1R, 2S, 3S) (1, 2, 3, 4-tétrahydroxybutyl)] 5-[(2'S, 3'S) (2', 3', 4'-trihydroxybutyl)] pyrazine

### Première méthode

A 500 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 12 cm³ de diméthylformamide, sont successivement additionnés à une température voisins de 20°C, 2,54 cm³ de cyclohexanone et 31 mg d'acide para-toluènesulfonique monohydrate. La solution obtenue après 15 mn d'agitation à une température voisine de 20°C est de nouveau agitée pendant 2 heures 30 minutes à une température voisine de 20°C. On additionne ensuite du sulfate de magnésium et le milieu réactionnel est agité pendant 16 heures supplémentaires à une température voisine de 20°C. Le milieu est ensuite chauffé à une température voisine de 60°C pendant queiques minutes et devient blanc, laiteux. Le milieu réactionnel est laissé revenir à une température voisine de 20°C et est dilué avec un mélange de 10 cm³ d'eau distillée et 10 cm³ d'acétate d'éthyle. La phase organique, après décantation, est lavée avec deux fois 10 cm³ d'eau distillée. Les phases aqueuses sont réunies et extraite avec une fois 10 cm³ d'acétate d'éthyle. Les phases organiques réunies sont séchées sur sulfate de magnésium, filtrées sur verre fritté puis concentrées à sec sous pression réduite (0,27 kPa) à une température voisine de 30°C. On obtient ainsi 910 mg d'une meringue jaune pâle qui est reprise par 10 cm³ d'éther éthylique. Après 10 minutes d'agitation à une température voisine de 20°C, l'insoluble est filtré sur verre fritté, fincé avec 5 cm³ d'éther éthylique pour donner un produit blanc floconneux qui est séché à une température voisine de 40°C sous presion réduite (0,27 kPa).

On obtient ainsi 417 mg de 3, 4-O, O -3', 4'-O, O-bis-cyclohexylidène-2-[(1R, 2S, 3S) (1, 2, 3, 4-tétrahydroxybutyl)] 5-[(2'S, 3'S) (2', 3', 4'-trihydroxybutyl)] pyrazine sous forme d'un solide blanc.

### Deuxième méthode :

A 300 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 4 cm³ de diméthylformamide, sont successivement additionnés à une température voisine de 20°C, 0,153 cm³ de cyclohexanone, 0,244 cm³ de triéthylorthoformiate et 0,05 cm³ de tétrahydrofurane saturé en acide chlorhydrique (titre ≥ 10M). La solution obtenue après 5 minutes d'agitation à une température voisine de 20°C est agité pendant cinq jours supplémentaires à une température voisine de 20°C. Le mileu réactionnel est repris avec un mélange de 10 cm³ d'eau distillée et 10 cm³ d'acétate d'éthyle. La phase organique, après décantation, est lavée avec deux fois 10 cm³ d'eau distillée. Les phases aqueuses sont réunies et extraite avec une fois 12 cm³ d'acétate d'éthyle. Les phases organiques réunies sont séchées sur sulfate de magnésium, filtrées sur verre fritté puis concentrées à sec sous pression réduite (1,33 kPa) à une température voisine de 30°C. On obtient ainsi 324 mg d'une laque incolore qui est purifiée, après dissolution dans un minimum de dichlorométhane avec une trace de méthanol, par chromatographie sur 6 plaques de gel de silice 60F254 Merck (épaisseur = 1 mm, 20x20 cm) en éluant avec de l'acétate d'éthyle. La fraction ne contenant que le produit cherché est extraite par un mélange dichlorométhane-méthanol (90-10 en volumes), filtrée sur verre fritté puis concentrée à see sous pression réduite (2,7 kPa) à une température voisine de 40°C. Le solide blanc ainsi obtenu est repris avec de l'éther éthylique puis filtré sur verre fritté pour fournir 116 mg de 3, 4-O, O -3', 4'-O, O-bis-cyclohexylidène-2-[(1R, 2S, 3S) (1, 2, 3, 4-tétrahydroxybutyl)] 5-[(2'S, 3'S) (2', 3', 4'-trihydroxybutyl)] pyrazine.

Le produit obtenu possède les caractéristiques suivantes :

Spectre de R.M.N. ¹ H (400 MHz, (CD₃)₂SO d6, δ en ppm) : de 1,25 à 1,65 (mf, 20H : les 10 CH₂ des 2 cyclohexyles) ; 2,76 (dd, J = 14 et 9 Hz, 1H : 1H du CH₂ 5α) ; 3,04 (dd, J = 14 et 3,5 Hz, 1H : l'autre H du CH₂ 5α) ; 3,61 (dt, J = 7 et 1,5 Hz, 1H : CH 2β) ; 3,77 (mt, 1H : CH 5β) ; de 3,80 à 3,95 et de 3,95 à 4,10 (2 mts, respectivement 3H et 2H : CH 5γ - CH₂O 2δ et CH₂O 5δ) ; 4,18 (mt, 1H : CH 2γ) ; 4,80 (mt, 2H : CH 2α et OH en 2β) ; 5,02 (d, J = 7 Hz, 1H : OH en 5β) ; 5,54 (d, J = 6,5 Hz, 1H : OH en 2α) ; 8,43 (s, 1H : =CH en 6) ; 8,65 (s, 1H : =CH en 3).

### Préparation de : 1, 2, 2'-O, O, O-tribenzoyl 3, 4-O, O -3', 4'-O, O-bis-cyclohexylidène-2-[(1R, 2S, 3R) (1, 2, 3, 4-tétrahydroxybutyl)] 5-[(2'S, 3'R) (2', 3', 4'-trihydroxybutyl)] pyrazine

A une solution de 200 mg de 3, 4-O, O -3', 4'-O, O-bis-cyclohexylidène-2-[(1R, 2S, 3R) (1, 2, 3, 4-tétrahydroxybutyl)] 5-[(2'S, 3'R) (2', 3', 4'-trihydroxybutyl)] pyrazine dans 5 cm³ de pyridine anhydre, sous atmosphère d'Argon, à une température voisine de 23°C, on ajoute goutte à goutte 0,175 cm³ de chlorure de benzoyle. Après 1,25 heure à une température voisine de 23°C, le mélange réactionnel est concentrées à sec sous pression réduite (environ 2,7 kPa) à une température voisine de 40°C. Le solide blanc cassé obtenu est repris par du dichlorométhane. La suspension obtenue est filtrée sur verre fritté, et le filtrat est purifié par dépôt sur chromatographie préparative (4 plaques de gel de silice 60F254 Merck, épaisseur = 1 mm, 20x20 cm) en éluant par un mélange cyclohexane-acétate d'éthyle (50-50 en volumes). Les fractions ne contenant que les produits cherchés sont extraites par de l'acétate d'éthyle, filtrées sur verre fritté puis concentrées à sec sous pression réduite (2,7 kPa) à une température voisine de 40°C. On obtient ainsi :
64 mg de 1, 2'-O, O-dibenzoyl 3, 4-O, O -3', 4'-O, O-bis-cyclohexylidène-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue blanche,
et 210 mg de 1, 2, 2'-O, O, O-tribenzoyl 3, 4-O, O -3', 4'-O, O-bis-cyclohexylidène-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue blanche.

Les produits obtenus possèdent les caractéristiques suivantes :
1, 2'-O, O-dibenzoyl 3, 4-O, O -3', 4'-O, O-bis-cyclohexylidène-2-[(1R, 2S, 3R) (1, 2, 3, 4-tétrahydroxybutyl)] 5-[(2'S, 3'R) (2', 3', 4'-trihydroxybutyl)] pyrazine :

Spectre de R.M.N. ¹ H (400 MHz, (CD₃)₂SO d6, δ en ppm) : de 1,20 à 1,60 (mf, 20H : les CH₂ des cyclohexyles) ; 3,18 et 3,25 (2 dd, respectivement J = 14 et 8 Hz et J = 14 et 5 Hz, 1H chacun : CH₂ 5α) ; 3,84 (t dédoublé, J = 8 et 3 Hz, 1H : CH 2β) ; 3,91 et 4,01 (2 dd, respectivement J = 8,5 et 5 Hz et J = 8,5 et 7 Hz, 1H chacun : CH₂O 2δ) ; 3,96 (dd, J = 8 et 5,5 Hz, 1H : 1H du CH₂O 5δ) ; de 4,05 à 4,15 (mt, 2H : CH 2γ et l'autre H du CH₂O 5δ) ; 4,35 (mt, 1H : CH 5γ) ; 5,54 (d, J = 8 Hz, 1H : OH en 2β) ; 5,53 (mt, 1H : CH 5β) ; 6,03 (d, J = 3 Hz, 1H : CH 2α) ; 7,47 et 7,58 (2t, J = 7,5 Hz, 2H chacun : H aromatiques en méta du C=O) ; 7,62 et 7,72 (2 t, J = 7,5 Hz, 1H chacun : H aromatique en para du C=O) ; 7,87 et 8,13 (2 d, J = 7,5 Hz, 2H chacun : H aromatiques en ortho du C=O) ; 8,50 (s large, 1H : =CH en 6) ; 8,59 (s large, 1H ; =CH en 3).

### 1, 2, 2'-O, O, O-tribenzoyl 3, 4-O, O -3', 4'-O, O-bis-cyclohexylidène-2-[(1R, 2S, 3R) (1, 2, 3, 4-tétrahydroxybutyl)] 5-[(2'S, 3'R) (2', 3', 4'-trihydroxybutyl)] pyrazine :

Spectre de R.M.N. ¹ H (400 MHz, (CD₃)₂SO d6, δ en ppm) : de 1,20 à 1,60 (mf, 20H : les CH₂ des cyclohexyles) ; 3,14 et 3,23 (2 dd, respectivement J = 14 et 8 Hz et J = 14 et 5 Hz, 1H chacun : CH₂ 5α) ; 3,88 (dd, J = 9 et 6 Hz, 1H : 1H du CH₂O 55) ; de 3,95 à 4,10 (mt, 3H : CH₂O 2δ et l'autre H du CH₂O 5δ) ; 4,29 (mt, 1H : CH 5γ) ; 4,43 (mt, 1H : CH 2γ) ; 5,49 (mt, 1H : CH 5β) ; 5,83 (t, J = 5 Hz, 1H : CH 2β) ; 6,31 (d, J = 5 Hz, 1H : CH 2α) ; de 7,35 à 8,10 (mts, 15H : H aromatiques) ; 8,56 (s, 1H : =CH en 6) ; 8,64 (s, 1H : =CH en 3).

### Exemple 19

### 1, 2'-O, O-dibenzoyl-2-[(1R, 2S, 3R) (1, 2, 3, 4-tétrahydroxybutyl)] 5-[(2'S, 3'R) (2', 3', 4'-trihydroxybutyl)] pyrazine

La 1, 2'-O, O-dibenzoyl-2-[(1R, 2S, 3R) (1, 2, 3, 4-tétrahydroxybutyl)] 5-[(2'S, 3'R) (2', 3', 4'-trihydroxybutyl)] pyrazine peut être obtenue dans les mêmes conditions que celles utilisées pour la préparation de la 1, 2, 2'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine mais à partir du dérivé 1, 2'-O, O-dibenzoyl 3, 4-O, O -3', 4'-O, O-bis-cyclohexylidène-2-[(1R, 2S, 3R) (1, 2, 3, 4-tétrahydroxybutyl)] 5-[(2'S, 3'R) (2', 3', 4'-trihydroxybutyl)] pyrazine, préparé tel que décrit dans l'exemple précédent.

### Exemple 20

20a) 1, 2, 2', 4, 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine
20b) 1, 2, 2', 3, 4'- O, O, O, O, O -pentabenzoyl-2-[(1R, 2S, 3R) (1, 2, 3, 4-tétrahydroxybutyl)] 5-[(2'S, 3'R) (2', 3',4'-trihydroxybutyl)] pyrazine,
20c) 1, 2, 2', 3', 4- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine
20d) 1, 2, 2', 3, 3'- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine

### Préparation de : 1, 2, 2', 3, 3'-O, O, O, O, O-pentabenzoyl-4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine.

A une solution de 600 mg de 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine dans 6 cm³ de pyridine anhydre, sous atmosphère d'Argon, à une température voisine de 25°C, on ajoute goutte à goutte 0,892 cm³ de chlorure de benzoyle. Après 20 heures à une température voisine de 25°C, le mélange réactionnel est dilué par 50 cm³ d'acétate d'éthyle, lavé avec 2 fois 10 cm³ d'eau, puis 10 cm³ d'une solution aqueuse saturée en chlorure de sodium. Après décantation, la phase organique est séchée sur sulfate de magnésium, filtrée sur verre fritté puis concentrée à sec sous pression réduite (2 kPa) à une température voisine de 40°C. On obtient 1,81 g d'une huile jaune qui est purifiée par chromatographie sous pression atmosphérique sur 100 g de gel de silice 60F254 Merck (0,063-0,200 mm) contenus dans une colonne de diamètre 3 cm, en éluant avec du dichlorométhane et en recueillant des fractions de 25 cm³. Les fractions ne contenant que le produit cherché sont réunies et concentrées à sec sous pression réduite (environ 2,7 kPa) à une température voisine de 40°C. On obtient ainsi 838 mg de 1, 2, 2', 3, 3'-O, O, O, O, O-pentabenzoyl-4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue blanche.

### Le dérivé 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine peut être préparé tel que décrit dans l'exemple 10.

### Préparation de :

20a) 1, 2, 2', 4, 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine
20b) 1, 2, 2', 3, 4'- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine
20c) 1, 2, 2', 3', 4- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine
20d) 1, 2, 2', 3, 3'- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine

A une solution, de 30 mg de 1, 2, 2', 3, 3'-O, O, O, O, O-pentabenzoyl-4,4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine dans 0,5 cm³ de dichlorométhane, sous atmosphère d'Argon, à une température voisine de 29°C, on ajoute lentement 0,0375 cm³ de complexe acide fluorhydrique-triéthylamine (3HF,Et₃N). Après 5 heures à une température voisine de 29°C, on ajoute lentement 0,075 cm³ de complexe acide fluorhydrique-triéthylamine supplémentaire. Après 15 heures à une température voisine de 29°C, le mélange réactionnel brut est purifié directement par dépôt sur chromatographie préparative (2 plaques de gel de silice 60F254 Merck, épaisseur = 0,5 mm, 20x20 cm) en éluant par un mélange dichlorométhane-méthanol (97-3 en volumes). Les fractions ne contenant que les produits cherchés sont extraites par un mélange dichlorométhane-méthanol (85-15 en volumes), filtrées sur verre fritté puis concentrées à sec sous pression réduite (2,7 kPa) à une température voisine de 40°C. On obtient ainsi :
2,3 mg de 1, 2, 2', 4, 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'un solide blanc,
1,5 mg de 1, 2, 2', 3, 4'- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'un solide blanc,
1,8 mg de 1, 2, 2', 3', 4- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'un solide blanc,
et 3,0 mg de 1, 2, 2', 3, 3'- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'un solide blanc.

Les produits obtenus possèdent les caractéristiques suivantes : 20a) 1, 2, 2', 4, 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine :

Spectre de R.M.N. ¹H (400 MHz, (CD₃)₂SO d6, δ en ppm) : 3.26 (dd, J = 14 et 8.5 Hz, 1H : 1H du CH₂ 5α) ; de 3,30 à 3,40 (mt : 1H correspondant à l'autre H du CH₂ 5α) ; 4,07 (mt, 1H : CH 5γ) ; de 4,20 à 4,45 (mt, 5H : CH₂ 2δ - CH₂ 5δ et CH 2γ) ; 5,46 (mt, 1H : CH 5β) ; 5,73 (d, J = 6 Hz, 1H : OH en 5γ) ; 5,76 (dd, J = 8 et 3,5 Hz, 1H : CH 2β) ; 5,99 (d, J = 6,5 Hz, 1H : OH en 2γ) ; 6,48 (d, J = 3,5 Hz, 1H : CH 2α) ; de 7,30 à 8,15 (mt, 25H : H aromatiques) ; 8,51 (s large, 1H : =CH en 6) ; 8,57 (s large, 1H : =CH en 3).

### 20b) 1, 2, 2', 3, 4'- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine :

Spectre de R.M.N. ¹ H (400 MHz, (CD₃)₂SO d6, δ en ppm) : de 3,20 à 3,40 (mt : 2H correspondant au CH₂ 5α) ; 3,70 (mt, 1H : 1H du CH₂ 2δ) ; 3,86 (mt, 1H : l'autre H du CH₂ 2δ) ; 4,11 (mt, 1H : CH 5γ) ; 4,31 (dd, J = 11,5 et 5,5 Hz, 1H : 1H du CH₂ 5δ) ; 4,39 (dd, J = 11,5 et 4,5 Hz, 1H : l'autre H du CH₂ 5δ) ; 5,06 (t, J = 6 Hz, 1H : OH en 2δ) ; 5,40 (mt, 1H : CH 2γ) ; 5,48 (mt, 1H : CH 5β) ; 5,74 (d, J = 6 Hz, 1H : OH en 5γ) ; 6,13 (t, J = 5,5 Hz, 1H : CH 2β) ; 6,47(d, J = 5,5 Hz, 1H : CH 2α) ; de 7,30 à 8,00 (mt, 25H : H aromatiques) ; 8,60 (s large, 1H : =CH en 6) ; 8,64 (s large, 1H : =CH en 3).

### 20c) 1, 2, 2', 3', 4- O, O, O, O, O -pentabenzoyl-2-[(1R, 2S, 3R) (1, 2, 3, 4-tétrahydroxybutyl)] 5-[(2'S, 3'R) (2', 3', 4'-trihydroxybutyl)] pyrazine :

Spectre de R.M.N. ¹H (400 MHz, (CD₃)₂SO d6, δ en ppm) : de 3,25 à 3,45 (mt : 2H correspondant au CH₂ 5α) ; 3,75 (mt, 1H : 1H du CH₂ 5δ ) ; 3,86 (mt, 1H : l'autre H du CH₂ 5δ) ; 4.26 (dd, J = 12 et 4 Hz, 1H : 1H du CH₂ 2δ) ; 4,32 (mt, 1H : CH 2γ) ; 4,40 (dd, J = 12 et 3 Hz, 1H : l'autre H du CH₂ 2δ) ; 5,14 (t, J = 6 Hz, 1H : OH 5δ) ; 5,43 (mt, 1H : CH 5γ) ; de 5,70 à 5,80 (mt, 2H : CH 2β et CH 5β) ; 5,98 (d, J = 6,5 Hz, 1H : OH en 2γ) ; 6,48 (d, J = 3 Hz, 1H : CH 2α) ; de 7,30 à 8,15 (mt, 25H : H aromatiques) ; 8,51 (s large, 1H : =CH en 6) ; 8,61 (s large, 1H : =CH en 3).

### 20d) 1, 2, 2', 3, 3'- O, O, O, O, O -pentabenzoyl-2-[(1R, 2S, 3R) (1, 2, 3, 4-tétrahydroxybutyl)] 5-[(2'S, 3'R) (2',3',4'-trihydroxybutyl)] pyrazine :

Spectre de R.M.N. ¹ H (400 MHz, (CD₃)₂SO d6, δ en ppm) : de 3,25 à 3,45 (mt : 2H correspondant au CH₂ 5α) ; de 3,65 à 3,80 et de 3,80 à 3,90 (mt, 2H chacun : CH₂ 2δ et CH₂ 5δ) ; 5,16 (t, J = 6 Hz, 2H : OH en 2δ et OH en 5δ) ; 5,40 (mt, 1H : CH 2γ) ; 5,44 (mt, 1H : CH 5γ) ; 5,77 (mt, 1H : CH 5β) ; 6,10 (dd, J = 6,5 et 5 Hz, 1H : CH 2β) ; 6,46 (d, J = 5 Hz, 1H : CH 2α) ; de 7,30 à 8,00 (mt, 25H : H aromatiques) ; 8,64 (s, 2H : =CH en 6 et =CH en 3).

### Exemple 21

### 1, 2-O, O-carbonyl 2', 3, 3'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine

### Préparation de : 1, 2-O, O-carbonyl-4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine

A une solution limpide de 2 g de 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine dans 40 cm³ de dichlorométhane et 6,7 cm³ de diisopropyléthylamine, sous atmosphère d'Argon, à une température voisine de 20°C, on ajoute goutte à goutte 5,5 cm³ de chloroformiate de benzyle. Après 3 heures à une température voisine de 20°C, on ajoute au milieu réactionnel 10 cm³ d'eau. Après 5 minutes d'agitation, et décantation, la phase aqueuse est réextraite par 5 cm³ de dichlorométhane. Les phases organiques sont rassemblées, séchées sur sulfate de magnésium, filtrées sur papier puis concentrées à sec sous pression réduite (2 kPa) à une température voisine de 40°C. On obtient 6,2 g d'une huile jaunâtre qui est purifiée par chromatographie sous pression atmosphérique sur 600 g de gel de silice 60F254 Merck (0,063-0,200 mm) contenus dans un colonne de diamètre 6 cm. On utilise un gradient d'élution constitué d'un mélange dichlorométhane-méthanol (de 100-0 à 98-2 en volumes) en recueillant des fractions de 120 cm³. Les fractions ne contenant que le produit cherché sont réunies et concentrées à sec sous pression réduite (environ 2,7 kPa) à une température voisine de 40°C. On obtient ainsi 619 mg de 1, 2-O, O-carbonyl-4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue beige.

Le produit obtenu possède les caractéristiques suivantes :

Spectre de R.M.N. ¹ H (400 MHz, (CD₃)₂SO d6, δ en ppm) : 0,83 et 1,02 (2 s, 9H chacun : les 2 SiC(CH₃)₃) ; 2,88 et 3.01 (2 dd, respectivement J = 14 et 9 Hz et J = 14 et 2,5 Hz, 1H chacun : CH₂ 5α) ; de 3,55 à 3,75 et 3,81 (respectivement mt et dd, J = 9 et 4 Hz, 4H et 1H : CH₂O 2δ - CH₂O 5δ et CH 5γ) ; 3,94 (mt, 1H : CH en 5β) ; 4,12 (mt, 1H : CH en 2γ) ; 4,75 (d, J = 7 Hz, 1H : OH en 5β) ; 4,96 (d, J = 5 Hz, 1H : OH en 5γ) ; 5,22 (dd, J = 5 et 3,5 Hz, 1H : CH 2β) ; 5,95 (s large, 1H : OH en 2γ) ; 6,01 (d, J = 5 Hz, 1H : CH 2α) ; de 7,30 à 7,75 (mt, 20H : H aromatiques) ; 8,65 (s large, 1H : =CH en 6) ; 8,82 (s large, 1H : =CH en 3).

### Le dérivé 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine peut être préparé tel que décrit dans l'exemple 10.

### Préparation de : 1, 2-O, O-carbonyl 2', 3, 3'-O, O, O-tribenzoyl -4, 4'-O, O-di(diphényl-tert-butylsilyl)-2-[(1R, 2S, 3R) (1, 2, 3, 4-tétrahydroxybutyl)] 5-[(2'S, 3'R) (2', 3',4'-trihydroxybutyl)] pyrazine

A une solution limpide de 560 mg de 1, 2-O, O-carbonyl-4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine dans 6 cm³ de pyridine, sous atmosphère d'Argon, à une température voisine de 20°C, on ajoute lentement 0,403 cm³ de chlorure de benzoyle. Après 3 heures et 45 minutes à une température voisine de 20°C, on ajoute lentement 0,161 cm³ de chlorure de benzoyle. Après 2,5 heures à une température voisine de 20°C, on ajoute au milieu réactionnel 10 cm³ de dichlorométhane et 7 cm³ d'eau. Après décantation, la phase organique est lavée avec 4 cm³ d'eau, séchée sur sulfate de magnésium, filtrée puis concentrée à sec sous pression réduite (2 kPa) à une température voisine de 40°C. On obtient 1,12 g d'une huile caramel qui est purifiée par chromatographie sous pression atmosphérique sur 100 g de gel de silice 60F254 Merck (0,063-0,200 mm) contenus dans une colonne de diamètre 3,5 cm. On utilise un gradient d'élution constitué d'un mélange dichlorométhane-méthanol en recueillant des fractions de 25 cm³. Les fractions ne contenant que le produit cherché sont réunies et concentrées à sec sous pression réduite (environ 2,7 kPa) à une température voisine de 40°C. On obtient ainsi 0,6 g de 1, 2-O, O-carbonyl 2', 3, 3'-O, O, O-tribenzoyl -4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue crème.

Le produit obtenu possède les caractéristiques suivantes :

Spectre de R.M.N. ¹ H (400 MHz, (CD₃)₂SO d6, δ en ppm) : 0,81 et 0,93 (2 s, 9H chacun : les 2 SiC(CH₃)₃) ; 3,50 (d, J = 6,5 Hz, 2H : CH₂ 5α) ; 3,97 - 4,02 et 4,09 (respectivement 2 dd, J = 12 et 4,5 Hz et AB limite, 1H - 1H et 2H : CH₂O 2δ et CH₂O 5δ) ; 5,51 (t, J = 4,5 Hz,1H : CH en 2β) ; de 5,60 à 5,70 (mt, 2H : CH 2γ et CH 5γ) : 6,01 (mt, 1H : CH 5β) ; 6,30 (d, J = 4,5 Hz, 1H : CH 2α) ; de 7,15 à 8,05 (mt, 35H : H aromatiques) ; 8,74 (s large, 1H : =CH en 6) ; 8,76 (s large, 1H : =CH en 3).

### Préparation de : 1, 2-O, O-carbonyl 2', 3, 3'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine

A une solution de 600 mg de 1, 2-O, O-carbonyl 2', 3, 3'-O, O, O-tribenzoyl -4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine dans 5 cm³ de dichlorométhane, sous atmosphère d'Argon, à une température voisine de 22°C, on ajoute lentement 6,55 cm³ de complexe acide fluorhydrique-triéthylamine (3HF.Et₃N). Après 4 heures à une température voisine de 22°C, on ajoute lentement 25 cm³ d'une solution aqueuse saturée en hydrogénocarbonate de sodium. Après décantation, la phase organique est lavée avec 7 cm³ d'eau. Les phases aqueuses sont réunies, et réextraites avec quelques cm³ de dichlorométhane. Les phases organiques sont rassemblées, séchées sur sulfate de magnésium, filtrées sur papier puis concentrées à sec sous pression réduite (2 kPa) à une température voisine de 40°C. On obtient 600 mg d'une meringue jaune pâle que l'on purifie par chromatographie préparative sur 10 plaques de gel de silice 60F254 Merck (épaisseur = 1 mm, 20x20 cm) en éluant par un mélange dichlorométhane-méthanol (95-5 en volumes). La fraction ne contenant que le produit cherché est extraite par un mélange dichlorométhane-méthanol (90-10 en volumes), filtrée sur verre fritté puis concentrée à sec sous pression réduite (2,7 kPa) à une température voisine de 40°C. On obtient au total ainsi 161 mg de 1, 2-O, O-carbonyl 2', 3, 3'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue blanche.

Le produit obtenu possède les caractéristiques suivantes :

Spectre de R.M.N. ¹ H (400 MHz, (CD₃)₂SO d6, δ en ppm) : 3,51 (d, J = 6,5 Hz, 2H : CH₂ 5α) ; de 3,70 à 3,85 et de 3.85 à 4,00 (2 mts, respectivement 3H et 1H : CH₂O 2δ et CH₂O 5δ) ; 5,20 et 5,26 (2 mfs, 1H chacun : OH 2δ et OH 5δ) ; 5,30 (dd, J = 5 et 3 Hz, 1H : CH en 2β) ; 5,44 (mt, 1H : CH 2γ) ; 5,50 (mt, 1H : CH 5γ) ; 5,90 (mt, 1H : CH 5β) ; 6,30 (d, J = 5 Hz, 1H : CH 2α) ; de 7,40 à 8,05 (mt, 15H : H aromatiques) ; 8,76 (s large, 1H : =CH en 6) ; 8,79 (s large, 1H : =CH en 3).

### Exemple 22

### 1, 2, 2', 3, 3', 4, 4'-O, O, O, O, O, O, O-heptamonosuccinoyl-2-[(1R, 2S, 3R) (1, 2, 3, 4-tétrahydroxybutyl)] 5-[(2'S, 3'R) (2', 3', 4'-trihydroxybutyl)] pyrazine

### Préparation de : 1, 2, 2', 3, 3', 4, 4'-O, O, O, O, O, O, O-heptamonosuccinate de benzyle-2-[(1R, 2S, 3R) (1, 2, 3, 4-tétrahydroxybutyl)] 5-[(2'S, 3'R) (2', 3', 4'-trihydroxybutyl)] pyrazine

A 500 mg de 2-[(1R, 2S, 3R) (1, 2, 3, 4-tétrahydroxybutyl)] 5-[(2'S, 3'R) (2', 3', 4'-trihydroxybutyl)] pyrazine et 500 mg de tamis moléculaire 4Å en poudre en suspension dans 10 cm³ de N,N-diméthylformamide anhydre, sont additionnés successivement, à une température voisine de 26°C, sous atmosphère d'Argon, 3,59 g de monosuccinate de benzyle, 3,68 g de dicyclohexylcarbodiimide et 60 mg de 4-(N,N-diméthylamino)-pyridine. Après 16 heures à une température voisine de 26°C, le mélange réactionnel est dilué avec 60 cm³ d'acétate d'éthyle et filtré sur verre fritté. Le filtrat est lavé avec 2 fois 10 cm³ d'eau, 10 cm³ d'une solution aqueuse saturée en hydrogénocarbonate de sodium, 3 fois 10 cm³ d'eau, 10 cm³ d'une solution aqueuse saturée en chlorure de sodium, puis séché sur sulfate de magnésium, filtré sur verre fritté, et concentré à sec sous pression réduite (0,7 kPa) à une température voisine de 42°C. On obtient ainsi 4,4 g d'une huile marron dont on purifie par chromatographie sous pression atmosphérique sur 250 g de gel de silice 60F254 Merck (0,063-0,200 mm) contenus dans une colonne de diamètre 4 cm. On utilise un gradient d'élution constitué d'un mélange cyclohexane-acétate d'éthyle 80-20 en volumes (11) puis 60-40 en volumes (2 I) en recueillant des fractions de 50 cm³. Les fractions ne contenant que le produit cherché sont réunies et concentrées à sec sous pression réduite (environ 2,7 kPa) à une température voisine de 40°C. On obtient ainsi 2,6 g de 1, 2, 2', 3, 3', 4, 4'-O, O, O, O, O, O, O-heptamonosuccinate de benzyle-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile jaune pâle.

Le produit obtenu possède les caractéristiques suivantes :

Spectre de R.M.N. ¹ H (400 MHz, (CD₃)₂SO d6, δ en ppm) : de 2,25 à 2,80 (mt, 28H : les 7 COCH₂CH₂CO) ; 3,05 et 3,15 (2dd, respectivement J = 14 et 9 Hz, et J = 14 et 4 Hz, 1H chacun : CH₂ 5α) ; 4,09 (dd, J = 12 et 5,5 Hz, 1H : 1H du CH₂O 2δ) ; 4,17 (dd, J = 12 et 8 Hz, 1H : 1H du CH₂O 5δ) 4,30 (mt, 2H : l'autre H du CH₂O 2δ et l'autre H du CH₂O 5δ) ; 4,53 et 5,07 (2 s, 14H en totalité : les 7 OCH_{2α}r) ; 5,20 (mt, 2H : CH 2γ et CH 5γ) ; 5,44 (mt, 1H : CH 5β) ; 5,58 (dd, J = 8 et 4 Hz, 1H : CH 2β) ; 5,99 (d, J = 4 Hz, 1H : CH 2α) ; 7,33 (mt, 35H : H aromatiques) ; 8,48 (s large, 1H : =CH en 6) ; 8,55 (s large, 1H : =CH en 3).

Le monosuccinate de benzyle peut être préparé de la facon suivante :
A 5 g d'anhydride succinique dans 10 cm³ d'acétate d'éthyle sont additionnés successivement, à une température voisine de 20°C, sous atmosphère d'Argon, 4,65 cm³ d'alcool benzylique et 0,1 g de 4-(N,N-diméthylamino)-pyridine. Le mélange réactionnel est chauffé à une température voisine de 60°C pendant 15 heures, puis ramené à une température voisine de 20°C, dilué avec 50 cm³ d'acétate d'éthyle et lavé avec 2 fois 30 cm³ d'une solution aqueuse saturée en hydrogénocarbonate de sodium. Les phases aqueuses sont rassemblées, lavées avec 2 fois 20 cm³ d'acétate d'éthyle, refroidies à une température voisine de 0°C, puis acidifiées jusqu'à un pH voisin de 2 par addition d'une solution d'acide chlorhydrique concentré (37% minimum). L'insoluble blanc formé est filtré sur verre fritté, séché sous flux d'air, puis à l'étuve sous pression réduite (0,1 kPa) à une température voisine de 40°C. On obtient ainsi 5,9 g de monosuccinate de benzyle sous forme d'une poudre blanche.

### Préparation de : 1, 2, 2', 3, 3', 4, 4'-O, O, O, O, O, O, O-heptamonosuccinoyl-2-[(1R, 2S, 3R) (1, 2, 3, 4-tétrahydroxybutyl)] 5-[(2'S, 3'R) (2', 3', 4'-trihydroxybutyl)] pyrazine

A une solution de 2,6 g de 1, 2, 2', 3, 3', 4, 4'-O, O, O, O, O, O, O-heptamonosuccinate de benzyle-2-[(1R, 2S, 3R) (1, 2, 3, 4-tétrahydroxybutyl)] 5-[(2'S, 3'R) (2', 3', 4'-trihydroxybutyl)] pyrazine dans 52 cm³ de dioxane et 20 cm³ d'eau distillée, on ajoute 520 mg de Pd(OH)₂ sur charbon (20% de Pd en poids). La suspension obtenue est agitée vivement sous atmosphère d'hydrogène (environ 1 atm) pendant 2 heures, à une température voisine de 22°C, puis filtrée sur verre fritté garni de Célite. Après rinçage du verre fritté avec 2 fois 20 cm³ d'eau distillée, puis concentration du filtrat à sec sous pression réduite (environ 2,7 kPa) à une température voisine de 42°C, le résidu obtenu est repris avec 20 cm³ d'eau distillée. Après filtration, et concentration du filtrat à sec sous pression réduite (environ 2,7 kPa) à une température voisine de 42°C, on obtient 1,278 g de 1, 2, 2', 3, 3', 4, 4'-O O, O, O, O, O, O-heptamonosuccinoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une mousse blanche.

Le produit obtenu possède les caractéristiques suivantes :

Spectre de R.M.N. ¹ H (400 MHz, (CD₃)₂SO d6, δ en ppm) : de 2,25 à 2,80 (mt, 28H : les 7 COCH₂CH₂CO) ; 3,13 et 3,25 (2dd, respectivement J = 14 et 9 Hz et J = 14 et 4 Hz, 1H chacun : CH₂ 5α) ; 4,16 (dd, J = 12 et 5,5 Hz, 1H : 1H du CH₂O 2δ) ; 4,24 (dd, J = 12 et 7 Hz, 1H : 1H du CH₂O 5δ) ; 4,37 (mt, 2H : l'autre H du CH₂O 2δ et l'autre H du CH₂O 5δ) ; 5,23 (mt, 2H : CH 2γ et CH 5γ) ; 5,46 (mt, 1H : CH 5β) ; 5,61 (dd, J = 8 et 4 Hz, 1H : CH 2β) ; 5,99 (d, J = 4 Hz, 1H : CH 2α) ; 8,57 et 8,59 (2 s, 1H chacun : =CH en 3 et =CH en 6)

### Exemple 23

### 1, 2, 2', 3, 3', 4, 4'-O, O, O, O, O, O, O-heptamonoglutaroyl-2-[(1R, 2S, 3R) (1, 2, 3, 4-tétrahydroxybutyl)] 5-[(2'S, 3'R) (2', 3', 4'-trihydroxybutyl)] pyrazine

La 1, 2, 2', 3, 3', 4, 4'-O, O, O, O, O, O, O-heptamonoglutaroyl-2-[(1R, 2S, 3R) (1, 2, 3, 4-tétrahydroxybutyl)] 5-[(2'S, 3'R) (2', 3', 4'-trihydroxybutyl)] pyrazine peut être préparé selon le mode opératoire tel que décrit dans l'exemple 22 pour la préparation de la 1, 2, 2', 3, 3', 4, 4'-O, O, O, O, O, O, O-heptamonosuccinoyl-2-[(1R, 2S, 3R) (1, 2, 3, 4-tétrahydroxybutyl)] 5-[(2'S, 3'R) (2', 3', 4'-trihydroxybutyl)] pyrazine, à partir de 2-[(1R, 2S, 3R) (1, 2, 3, 4-tétrahydroxybutyl)] 5-[(2'S, 3'R) (2', 3', 4'-trihydroxybutyl)] pyrazine, mais en utilisent l'anhydride glutarique, et en préparant successivement le monoglutarate de benzyle, puis la
1, 2, 2', 3, 3', 4, 4'-O, O, O, O, O, O, O-heptamonoglutarate de benzyle-2-[(1R, 2S, 3R) (1, 2, 3, 4-tétrahydroxybutyl)] 5-[(2'S, 3'R) (2', 3', 4'-trihydroxybutyl)] pyrazine
et enfin la
1, 2, 2', 3, 3', 4, 4'-O, O, O, O, O, O, O-heptamonoglutaroyl-2-[(1R, 2S, 3R) (1, 2, 3, 4-tétrahydroxybutyl)] 5-[(2'S, 3'R) (2', 3', 4'-trihydroxybutyl)] pyrazine.

### Exemple 24

### 4, 4'-O, O-di(éthoxycarbonyl)-2-[(1R, 2S, 3R) (1, 2, 3, 4-tétrahydroxybutyl)] 5-[(2'S, 3'R) (2', 3', 4'-trihydroxybutyl)] pyrazine

A une suspension de 500 mg de 2-[(1R, 2S, 3R) (1, 2, 3, 4-tétrahydroxybutyl)] 5-[(2'S, 3'R) (2', 3', 4'-trihydroxybutyl)] pyrazine dans 10 cm³ de pyridine anhydre, à une température voisine de 20°C, on ajoute goutte à goutre 0,472 cm³ de chloroformiate d'éthyle. Après 45 heures à une température voisine de 20°C, le mélange réactionnel est concentré à sec sous pression réduite (environ 0,5 kPa) à une température voisine de 40°C. Le résidu obtenu est purifié par chromatographie préparative sur plaques de gel de silice 60F254 Merck (épaisseur = 2 mm, 20x20 cm) en éluant par un mélange dichlorométhane-méthanol (90-10 en volumes). La fraction ne contenant que le produit cherché est extraite par un mélange dichlorométhane-méthanol (80-20 en volumes), filtrée sur verre fritté puis concentrée à sec sous pression réduite (0.5 kPa) à une température voisine de 40°C. On obtient ainsi 71 mg de 4, 4'-O, O-di(éthoxycarbonyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une poudre blanche.

Le produit obtenu possède les caractéristiques suivantes :

Spectre de R.M.N. ¹ H (400 MHz, (CD₃)₂SO d6, δ en ppm) : 1,24 (t, J = 7,5 Hz, 6H : CH₃ éthyle) ; 2,75 et 3,14 (2dd, respectivement J = 14 et 9 Hz et J = 14 et 3 Hz, 1H chacun : CH₂ 5α) ; 3,56 (mt, 1H : CH 5γ) ; 3,60 (t large , J = 9 Hz , 1H : CH 2β) ; 3,78 (mt, 1H : CH 5β) ; 3,85 (mt, 1H : CH 2γ) ; de 4,00 à 4,25 (mt, 6H : 1H du CH₂O 2δ - 1H du CH₂O 5δ et OCH₂ éthyle) ; 4,32 (mt, 2H : l'autre H du CH₂O 2δ et l'autre H du CH₂O 5δ) ; 4,67 (d, J = 8,5 Hz, 1H : OH en 2β) ; 4,90 (d, J = 6,5 Hz, 1H : OH en 5β) ; 4,95 (d large, J = 6 Hz, 1H : CH 2α) ; 5,18 (d, J = 6 Hz, 1H : OH en 2γ) ; 5,22 (d, J = 6 Hz, 1H : OH en 5γ) ; 5,43 (d, J = 6 Hz, 1H : OH en 2α) ; 8,42 (s, 1H : =CH en 6) ; 8,66 (s, 1H : =CH en 3).

### Exemple 25

### 1, 2, 2', 3, 3', 4, 4'-O, O, O, O, O, O, O-heptapentanoyl-2-[(1R, 2S, 3R) (1, 2, 3, 4-tétrahydroxybutyl)] 5-[(2'S, 3'R) (2', 3', 4'-trihydroxybutyl)] pyrazine

A une suspension de 500 mg de 2-[(1R, 2S, 3R) (1, 2, 3, 4-tétrahydroxybutyl)] 5-[(2'S, 3'R) (2', 3', 4'-trihydroxybutyl)] pyrazine dans 6 cm³ de pyrdine anhydre, sont ajoutés goutte à goutte, à une température voisine de 0°C, 2.97 cm³ de chlorure de n.pentanoyle. Après 17 heures, à une température voisine de 20°C, le mélange réactionnel est concentré à sec sous pression réduite à une température voisine de 35°C. Le résidu brut obtenu est purifié par chromatographie sous pression (1,4 bar) sur un volume de 100 cm³ de gel de silice 60F254 Merck (0,063-0,040 mm) contenus dans une colonne de diamètre 2 cm, en utilisant du dichlorométhane comme éluant. Les fractions ne contenant que le produit cherché sont réunies et concentrées à sec sous pression réduite (environ 2,7 kPa) à une température voisine de 35°C. On obtient ainsi 1,832 g d'une huile orange que l'on purifie par chromatographie sous pression (1,4 bar) sur un volume de 200 cm³ de gel de silice 60F254 Merck (0,063-0,040 mm) contenus dans une colonne de diamètre 3 cm, en utilisant un mélange éluant acétate d'éthyle-cyclohexane (20-80 en volumes). Les fractions ne contenant que le produit cherché sont réunies et concentrées à sec sous pression réduite (environ 2,7 kPa) à une température voisine de 35°C. On obtient ainsi 538 mg de 1, 2, 2', 3, 3', 4, 4'-O, O, O, O, O, O, O-heptapentanoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile transparente.

Le produit obtenu possède les caractéristiques suivantes :

Spectre de R.M.N. ¹ H (400 MHz, (CD₃)₂SO d6, δ en ppm) : de 0,75 à 0,95 (mt, 21H : les CH₃ des pentanoyles) ; de 1,05 à 1,60 (mt, 28H en totalité : les CH₂ centraux des pentanoyles) ; de 2,00 à 2,50 (mt, 14H en totalité : les COCH₂ des pentanoyles) ; 3,11 et 3,21 (2dd, respectivement J = 14 et 10 Hz et J = 14 et 4 Hz, 1H chacum : CH₂ 5α) ; 4,10 (dd, J = 12 et 5 Hz, 1H : 1H du CH₂O 2δ) ; 4,20 (dd, J = 12 et 8 Hz, 1H : 1H du CH₂O 5δ) ; 4,30 (dd, J = 12 et 2,5 Hz, 1H : l'autre H du CH₂O 2δ) ; 4,39 (dd, J = 12 et 3 Hz, 1H : l'autre H du CH₂O 5δ) ; 5,23 (mt, 2H : CH 2γ et CH 5γ ) ; 5,45 (mt, 1H : CH 5β) ; 5,55 (dd, J = 8 et 3 Hz, 1H : CH 2β) ; 5,97 (d, J = 3 Hz, 1H : CH 2α) ; 8,53 (s, 1H : =CH en 6) ; 8,54 (s, 1H : =CH en 3).

### Exemple 26

a) 4-O-benzoyl-2-[(1R, 2S, 3R) (1, 2, 3, 4-tétrahydroxybutyl)] 5-[(2'S, 3'R) (2', 3', 4'-trihydroxybutyl)] pyrazine
b) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine
c) 3, 4, 4'-O, O, O-tribenzoyl-2-[(1R, 2S, 3R) (1, 2, 3, 4-tétrahydroxybutyl)] 5-[(2'S, 3'R) (2', 3',4'-trihydroxybutyl)]pyrazine
d) 3, 4, 2', 3', 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine

### Préparation de :

1, 2-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3',4'-trihydroxybutyl)] pyrazine
2, 3-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine
1, 3-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine

A 3,9 g de 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine en solution dans 190 cm³ de N,N-diméthylformamide anhydre, sous atmosphère inerte d'Argon, à une température voisine de 20°C, sont additionnés 47,5 mg d'acide paratoluènesulfonique (monohydrate) et 18,4 cm³ de 2,2-diméthoxypropane, Le mélange réactionnel est porté à une température voisine de 60°C pendnat 40 heures, concentré à sec sous pression réduite (0,5 kPa) à une température voisine de 50°C, puis repris avec 100 cm³ de dichlorométhane. La phase organique obtenue est lavée avec 2 fois 25cm³ d'une solution aqueuse saturée en hydrogénocarbonate de sodium, 2 fois 50 cm³ d'eau, puis séchée sur sulfate de magnésium, filtrée sur papier et concentre à sec sous pression réduite (0,5 kPa) à une température voisine de 30°C. On obtient 4,5 g d'une huile épaisse marron qui est mise en solution dans 50 cm³ d'un mélange cyclohexane-acétate d'éthyle (90-10 en volumes) et purifiée par chromatographie sous pression (environ 130 bar) sur 500 g de gel de silice 60 Merck (0,015-0,040 mm), en utilisant un gradient d'élution (cyclohexane-acétate d'éthyle 90-10 puis 50-50 en volumes) et en recueillant des fractions de 70 cm³. Les fractions ne contenant que les produits cherchés sont réunies et concentrées à sec sous pression réduite (environ 1 kPa) à une température voisine de 40°C. On obtient ainsi 1,05 g de 1, 2-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile jaune claire et 1,53 g d'un mélange contenant la 2, 3-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine et la 1, 3-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine. Ce dernier mélange de 1,53 g est repurifié par chromatographie sous pression (environ 130 bar) sur 500 g de gel de silice 60 Merck (0,015-0,040 mm), en utilisant un mélange éluant cyclohexane-acétate d'éthyle (90-10 en volumes) et en recueillant des fractions de 70 cm³. Les fractions ne contenant que les produits cherchés sont réunies et concentrées à sec sous pression réduite (environ 1 kPa) à une température voisine de 40°C. On obtient ainsi 187 mg de 2, 3-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile épaisse incolore et 300 mg de 1, 3-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile épaisse incolore.

Les produits obtenus possèdent les caractéristiques suivantes :

1, 2-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine : spectre de R.M.N. ¹ H (400 MHz, (CD₃)₂SO d6, δ en ppm) : 0,90 et 1,01 (2 s, 9H chacun : les 2 C(CH₃)₃) ; 1,22 - 1,35 - 1,44 et 1,46 (4 s, 3H chacun : les 4 CH₃) ; 3,08 (dd, J = 14 et 9 Hz, 1H : 1H du CH₂ 5α) ; 3,15 (dd, J = 14 et 3,5 Hz, 1H : l'autre H du CH₂ 5α) ; 3,60 (AB limite, 2H : CH₂O 2δ) ; 3,71 (dd, J = 11 et 5 Hz, 1H : 1H du CH₂O 5δ) ; de 3,80 à 3,95 (mt, 2H : l'autre H du CH₂ 5δ et CH 2γ) ; 4,32 (q, J = 5 Hz, 1H : CH 5γ) ; 4,48 (6d, J = 7 et 5 Hz, 1H : CH 2β) ; 4,70 (mt, 1H : CH 5β) ; 5,14 (d, J = 7 Hz, 1H ; CH 2α) ; 5,19 (d, J = 5 Hz, 1H : OH en 2γ) ; de 7,35 à 7,80 (mt, 20H : H aromatiques des 4 phényles) ; 8,53 (s, 1H : =CH en 6) ; 8,68 (s, 1H : =CH en 3).

2, 3-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine : spectre de R.M.N. ¹ H (400 MHz, (CD₃)₂SO d6, δ en ppm) : de 0,90 à 1,00 (mt, 18H : SiC(CH₃)₃) ; de 1,10 à 1,60 (mt, 12H : C(CH₃)₂) ; 3,09 (mt, 2H : CH₂ 5α) 3,73 (dd, J = 11 et 5,5 Hz, 1H : 1H du CH₂O 5δ) ; 3,82 (dd, J = 10 et 5,5 Hz, 1H : 1H du CH₂O 2δ) ; 3,88 (dd, J = 11 et 5,5 Hz, 1H : l'autre H du CH₂O 5δ) ; 3,99 (dd, J = 10 et 5,5 Hz, 1H : l'autre H du CH₂O 2δ) ; de 4,25 à 4,40 (mt, 2H : CH 2γ et CH 5γ) ; 4,58 (dd, J = 7 et 4 Hz, 1H : CH 2β) ; 4,70 (mt, 1H : CH 5β) ; 4,88 (mf, 1H : CH 2α) ; 5,53 (mf, 1H : OH en 2α) ; de 7,30 à 7,75 (mt, 20H : H aromatiques) ; 8,43 (s, 1H : =CH en 6) ; 8,64 (s, 1H : =CH en 3).

1, 3-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine : spectre de R.M.N. ¹ H (400 MHz, (CD₃)₂SO d6, δ en ppm) : 1,01 et 1,05 (2s; 9H chacun : les 2 C(CH₃)₃) ; 1,24 - 1,36 et 1,50 (3 s, respectivement 3H - 6H et 3H : les 4 CH₃) ; 3,03 (dd, J = 14 et 9 Hz, 1H : 1H du CH₂ 5α) ; 3,12 (dd, J = 14 et 3,5 Hz, 1H : l'autre H du CH₂ 5α) ; 3,70 (dd, J = 11 et 5,5 Hz, 1H : 1H du CH2O 5δ) ; de 3,75 à 3,95 (mt, 4H : CH 2γ - CH₂O 2δ et l'autre H du CH₂O 56) ; 4,03 (mt, 1H : CH 2β) ; 4,32 (mt, 1H : CH 5γ) ; 4,70 (mt, 1H : CH 5β) ; de 4,95 à 5,05 (mt, 2H : CH 2α et OH 2β) ; de 7,35 à 7,55 et de 7,60 à 7,80 (2 mts, respectivement 12H et 8H : H aromatiques des 4 phényles) ; 8,46 (s, 1H : =CH en 6) ; 8,53 (s, 1H : =CH en 3).

### La préparation du dérivé 4,4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazinepeut être préparé tel que décrit dans l'exemple 10.

### Préparation de

### 1, 2-O, O-2', 3'-O, O-di(diméthylacétonide) 3-O-benzoyl-4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine

A 0,086 g de 1, 2-O, O-2', 3'-O, O-di(diméthylacétonide) 4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine en solution dans 0,5 cm³ de pyridine anhydre, sous atmosphère inerte d'Argon, à une température voisine de 20°C, est additionné 0,0175 cm³ de chlorure de benzoyle. Après 20 heures à une température voisine de 20°C, le mélange réactionnel est concentré à sec sous courant d'Argon, à une température voisine de 20°C, puis purifié par chromatographie préparative sur 4 plaques de gel de silice 60F254 Merck (épaisseur = 0,5 mm, 20x20 cm) en éluant par un mélange cyclohexane-acétate d'éthyle (75-25 en volumes). Les fractions ne contenant que les produits cherchés sont extraites par un mélange dichlorométhane-méthanol (80-20 en volumes), filtrées sur verre fritté puis concentrées à sec sous pression réduite (0,5 kPa) à une température volsine de 30°C. On obtient ainsi 62 mg de 1, 2-O, O-2', 3'-O, O-di(diméthylacétomde) 3-O-benzoyl-4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue blanche.

Le produit obtenu possède les caractéristiques suivantes :

Spectre de R.M.N. ¹ H (400 MHz, (CD₃)₂SO d6, δ en ppm) : 0,88 et 1,02 (2 s, 9H chacun : les SiC(CH₃)₃) ; 1,22 - 1,35 - 1,39 et 1,47 (4 s, 3H chacun : les C(CH₃)₂) ; 3,07 (d, J = 7 Hz, 2H : CH₂ 5α) ; 3,69 et 3,86 (2dd, respectivement J = 10 et 5 Hz et J = 11 et 6,5 Hz, 1H chacun : CH₂O 5δ) ; 3,90 (AB limite, 2H : CH₂O 2δ) ; 4,30 (mt, 1H : CH 5γ) ; 4,64 (mt, 1H : CH 5β) ; 4,82 (dd, J = 8 et 7 Hz, 1H : CH 2β) ; 5,18 (d, J = 8 Hz, 1H : CH 2α) ; 5,49 (mt, 1H : CH 2γ) ; de 7,25 à 7,75 (mt, 25H : H aromatiques) ; 8,43 (d, J = 1 Hz, 1H : =CH en 6) ; 8,62 (d, J = 1 Hz, 1H : =CH en 3).

### Préparation de

### 1, 2-O, O-2', 3'-O, O-di(diméthylacétoride) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine

A 0,05 g de 1, 2-O, O-2', 3'-O, O-di(diméthylacétonide) 3-O-benzoyl-4, 4'-O, O-di(diphényl-tert-butylsilyl)-2-[(1R, 2S, 3R) (1, 2, 3, 4-tétrahydroxybutyl)] 5-[(2'S, 3'R) (2', 3', 4'-trihydroxybutyl)] pyrazine en solution dans 0,25 cm³ de dichlorométhane, à une température voisine de 20°C, est additionné 0,632 cm³ de complexe acide fluorhydrique-triéthylamine (3HF.Et₃N). Après 20 heures à une température voisine de 20°C, le mélange réactionnel est additionné de 2 cm³ de dichlorométhane et 5 cm³ de tampon phosphate. Après décantation, la phase organique est lavée avec 5 fois 2 cm³ d'eau, séchée sur sulfate de magnésium, concentrée à sec sous pression, réduite (environ 1 kPa) à une température voisine de 30°C. On obtient 41 mg d'une huile épaisse jaune-beige que l'on purifie par chromatographie préparative sur 2 plaques de gel de silice 60F254 Merck (épaisseur = 0,5 mm, 20x20 cm) en éluant par un mélange dichlorométhane-méthanol (80-20 en volumes). Les fractions ne contenant que les produits cherchés sont extraites par un mélange dichlorométhane-méthanol (80-20 en volumes), filtrées sur verre fritté puis concentrées à sec sous pression réduite (0,5 kPa) à une température voisine de 25°C. On obtient ainsi 16 mg de 1, 2-O, O-2', 3'-O, O-di(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une meringue blanche.

Le produis obtenu possède les caractéristiques suivantes :

Spectre de R.M.N. ¹ H (400 MHz, (CD₃)₂SO d6, δ en ppm) : 1,24 - 1,39 et 1,47 (3 s, respectivement 3H - 6H et 3H : les 2 C(CH₃)₃) ; 2,97 (AB limite, 2H : CH₂ 5α) ; 3,57 (AB limite, 2H : CH₂O 5δ) ; 3,61 et 3,71 (respectivement mt et d large, J = 12 Hz, 1H chacun : CH₂O 2δ) ; 4,19 (mt, 1H : CH 5γ) ; 4,56 (mt, 1H : CH 5β) ; 4,69 (dd, J = 8 et 6,5 Hz, 1H : CH 2β) ; 4,87 (t large, J = 5 Hz, 1H : OH en 5δ) ; 4,94 (t large, J = 5 Hz, 1H : OH en 2δ) ; 5,18 (d, J = 8 Hz, 1H : CH 2α) ; 5,32 (mt, 1H : CH 2γ) ; 7,48 (t, J = 7,5 Hz, 2H : H aromatiques en méta du CO) ; 7,65 (t, J = 7,5 Hz, 1H : H aromatique en para du CO) ; 7,75 (t, J = 7,5 Hz, 2H : H aromatiques en ortho du CO) ; 8,49 (s, 1H : =CH en 6) ; 8,65 (s, 1H : =CH en 3).

### Préparation de

### 1, 2-O, O-(diméthylacétonide) 4-O-benzoyl-2-[(1R, 2S, 3R) (1, 2, 3, 4-tétrahydroxybutyl)] 5-[(2'S, 3'R) (2', 3', 4'-trihydroxybutyl)] pyrazine 1, 2-O, O-(diméthylacétonide) 3-O-benzoyl-2-[(1R, 2S, 3R) (1, 2, 3, 4-tétrahydroxybutyl)] 5-[(2'S, 3'R) (2', 3',4'-trihydroxybutyl)] pyrazine

A 0,015 g de 1, 2-O, O-2', 3'-O, O-di(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine en solution dans 0,2 cm³ de dichlorométhane, à une température voisine de 20°C, est additionné 0,03 cm³ d'un mélange acide trifluoroacétique-eau (80-20 en volumes). Après 1 heure à une température voisine de 20°C, le mélange réactionnel est neutralisé par l'ajout de de 0,45 cm³ d'une solution aqueuse saturée en hydrogénocarbonate de sodium. Après décantation, la phase organique est purifiée par chromatomaphie préparative sur 1 plaque de gel de silice 60F254 Merck (épaisseur = 0,25 mm, 20x20 cm) en éluant par un mélange dichlorométhane-méthanol (90-10 en volumes). Les fractions ne contenant que les produits cherchés sont extraites par un mélange dichlorométhane-méthanol (80-20 en volumes), filtrées sur verre fritte puis concentrées à sec sous pression réduite (2,7 kPa) à une température voisine de 40°C. On obtient ainsi 3,5 mg de 1, 2-O, O-(diméthylacétonide) 4-O-benzoyl-2-[(1R, 2S, 3R) (1, 2, 3, 4-tétrahydroxybutyl)] 5-[(2'S, 3'R) (2', 3', 4'-trihydroxybutyl)] pyrazine et 5 mg de 1, 2-O, O-(diméthylacétonide) 3-O-benzoyl-2-[(1R, 2S, 3R) (1, 2, 3, 4-tétrahyrdroxybutyl)] 5-[(2'S, 3'R) (2', 3', 4'-trihydroxybutyl)] pyrazine.

Les produits obtenus possèdent les caractéristiques suivantes :
1, 2-O, O-(diméthylacétonide) 4-O-benzoyl-2-[(1R, 2S, 3R) (1, 2,- 3, 4-tétrahydroxybutyl)] 5-[(2'S, 3'R) (2', 3', 4'-trihydroxybutyl)] pyrazine ; spectre de R.M.N. ¹ H (400 MHz, (CD₃)₂SO d6 avec ajout de quelques gouttes de CF₃COOD, δ en ppm) : 1,44 et 1,47 (2 s, 3H chacun : C(CH₃)₂) ; 2,77 et 3,10 (2dd, respectivement J = 14 et 10 Hz et J = 14 et 3 Hz, 1H chacun : CH₂ 5α) ; de 3,35 à 3,45 (mt, 2H : 1H du CH₂O 5δ et CH 5γ) ; 3,58 (dd, J = 9 et 2,5 Hz, 1H : l'autre H du CH₂O 5δ) ; 3,78 (mt, 1H : CH 5β) ; 4,09 (mt, 1H : CH 2γ) ; 4,23 (dd, J = 11 et 7 Hz, 1H : 1H du CH₂O 2δ) ; de 4,35 à 4,45 (mt, 2H : l'autre H du CH₂O 2δ et CH 2β) ; 5,16 (d, J = 7 Hz, 1H : CH 2α) ; 7,53 (t, J = 8 Hz, 2H : H aromatiques en méta du CO) ; 7,67 (t, J = 8 Hz, 1H : H aromatique en para du CO) ; 7,97 (d, J = 8 Hz, 2H : H aromatiques en ortho du CO) ; 8,53 (d, J = 1 Hz, 1H : =CH en 6) ; 8,67 (d, J = 1 Hz, 1H : =CH en 3).
1, 2-O, O-(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine ; spectre de R.M.N. ¹ H (400 MHz, (CD₃)₂SO d6, δ en ppm) : 1,39 et 1,47 (2 s, 3H chacun : C(CH₃)₂) ; 2,77 et 3,06 (2dd, respectivement J = 14 et 9 Hz et J = 14 et 3 Hz, 1H chacun : CH₂ 5α) ; de 3,30 à 3,40 (mt : les 2H correspondants aux CH 5γ et 1H du CH₂O 5δ) ; de 3,50 à 3,65 (mt, 2H : 1H du CH₂O 2δ et l'autre H du CH₂O 5δ) ; 3,70 (d large, J = 13 Hz, 1H : l'autre H du CH₂O 2δ) ;
3,77 (mt, 1H : CH 5β) ; de 4,30 à 4,95 (3 s, 4H correspondant aux 4 OH) ; 4,69 (dd, J = 8 et 6,5 Hz, 1H : CH 2β) ; 5,19 (d, J = 8 Hz, 1H : CH 2α) ; 5.33 (mt, 1H : CH 2γ) ; 7,48 (t, J = 7,5 Hz, 2H ; H aromatiques en méta du CO) ; 7,64 (t, J = 7,5 Hz, 1H : H aromatique en para du CO) ; 7,76 (d, J = 7,5 Hz, 2H : H aromatiques en ortho du CO) ; 8,46 (s large, 1H : =CH en 6) ; 8,62 (s large, 1H : =CH en 3).

### Préparation de

### 4-O-benzoyl-2-[(1R, 2S, 3R) (1, 2, 3, 4-tétrahydroxybutyl)] 5-[(2'S, 3'R) (2', 3', 4'-trihydroxybutyl)] pyrazine

A partir de la 1, 2-O, O-(diméthylacétonide) 4-O-benzoyl-2-[(1R, 2S, 3R) (1, 2, 3, 4-tétrahydroxybutyl)] 5-[(2'S, 3'R) (2', 3', 4'-trihydroxybutyl)] pyrazine, en suivant le mode opératoire de la préparation de la 1, 2-O, O-(diméthylacétonide) 4-O-benzoyl-2-[(1R, 2S, 3R) (1, 2, 3, 4-tétrahydroxybutyl)] 5-[(2'S, 3'R) (2', 3', 4'-trihydroxybutyl)] pyrazine (mise en solution de la 1, 2-O, O-(diméthylacétozide) 4-O-benzoyl-2-[(1R, 2S, 3R) (1, 2, 3, 4-tétrahydroxybutyl)] 5-[(2'S, 3'R) (2', 3', 4'-trihydroxybutyl)] pyrazine dans un mélange dichlorométhane-acide trifluoroacétique-eau à une température voisine de 20°C), on obtient la 4-O-benzoyl-2-[(1R, 2S, 3R) (1, 2, 3, 4-tétrahydroxybutyl)] 5-[(2'S, 3'R) (2', 3', 4'-trihydroxybutyl)] pyrazine.

### Préparation de

### 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine

A partir de la 1, 2-O, O-(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine, en suivant le mode opératoire de la préparation de la 1, 2-O, O-(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine (mise en solution de la 1, 2-O, O-(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine dans un mélange dichlouméthane-acide trifluoroacétique-eau à une température voisine de 20°C), on obtient la 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine.

### Préparation de

### 3, 4, 4'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine

En faisant réagir la 1, 2-0, O-(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine en solution dans la pyridine anhydre, sous atmosphère inerte, à une température voisine de 20°C, avec le chlorure de benzoyle (2,5 éq.), on obtient la 1, 2-O, O-(diméthylacétonide) 3, 4, 4'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine. A partir de la 1, 2-O, O-(diméthylacétonide) 3, 4, 4'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine en suivant le mode opératoire de la préparation de la 1, 2-O, O-(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine (mise en solution de la 1, 2-O, O-(diméthylacétonide) 3, 4, 4'-O, O, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine dans un mélange dichlorométhane-acide trifluoroacétique-eau à une température voisine de 20°C), on obtient la 3, 4, 4'-O, 0, O-tribenzoyl-2-[(1R, 2S, 3R) (1, 2, 3, 4-tétrahydroxybutyl)] 5-[(2'S, 3'R) (2', 3', 4'-trihydroxybutyl)] pyrazine.

### Préparation de

### 3, 4, 2', 3', 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine

En faisant réagir la 1, 2-O, O-(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine en solution dans la pyridine anhydre, sous atmosphère inerte, à une température voisine de 20°C, avec le chlorure de benzoyle (8,6 éq.), on obtient la 1, 2-O, O-(diméthylacétonide) 3, 4, 2', 3', 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine. A partir de la 1, 2-O, O-(diméthylacétonide) ) 3, 4, 2', 3', 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine en suivant le mode opératoire de la préparation de la 1, 2-0, O-(diméthylacétonide) 3-O-benzoyl-2-[(1R, 2S, 3R) (1, 2, 3, 4-tétrahydroxybutyl)] 5-[(2'S, 3'R) (2', 3', 4'-trihydroxybutyl)] pyrazine (mise en solution de 1, 2-O, O-(diméthylacétonide) 3, 4, 2', 3', 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine dans un mélange dichlorométhane-acide trifluoroacétique-eau à une température voisine de 20°C), on obtient la 3, 4, 2', 3', 4'-O, O, O, O, O-pentabenzoyl-2-[(1R, 2S, 3R) (1, 2, 3, 4-tétrahydroxybutyl)] 5-[(2'S, 3'R) (2',3',4'-trihydroxybutyl)] pyrazine.

La 3, 4, 2', 3', 4'-O, O, O, O, O-pentabenzoyl-2-[(1R, 2S, 3R) (1, 2, 3, 4-tétrahydroxybutyl)] 5-[(2'S, 3'R) (2', 3', 4'-trihydroxybutyl)] pyrazine.peut être également obtenue selon le même mode opératoire que décrit ci-dessus à partir de la 1, 2-O, O-(diméthylacétonide) 4-O-benzoyl-2-[(1R, 2S, 3R) (1, 2, 3, 4-tétrahydroxybutyl)] 5-[(2'S, 3'R) (2',3',4'-trihydroxybutyl)] pyrazine.

### Exemple 27

### 2'-O-benzoyl-2-[(1R, 2S, 3R) (1, 2, 3, 4-tétrahydroxybutyl)] 5-[(2'S, 3'R) (2', 3', 4'-trihydroxybutyl)] pyrazine

### Préparation de

### 1, 2-O, O 3, 4-O, O 3', 4'-O, O-tri(diméthylacétonide) 2'-O-benzoyl-2-[(1R, 2S, 3R) (1, 2, 3, 4-tétrahydroxybutyl)] 5-[(2'S, 3'R) (2', 3', 4'-trihydroxybutyl)] pyrazine

A une solution de 1 g de 1, 2-O, O 3, 4-O, O 3', 4'-O, O-tri(diméthylacétonide)-2-[(1R, 2S, 3R) (1, 2, 3, 4-tétrahydroxybutyl)] 5-[(2'S, 3'R) (2', 3', 4'-trihydroxybutyl)] pyrazine dans 15 cm³ de pyridine anhydre, à une température voisine de 20°C, on ajoute goutte à goutte 0,492 cm³ de chlorure de benzoyle. Après 18 heures à une température voisine de 20°C, on ajoute 0,272 cm³ de chlorure de benzoyle supplémentaire. Après 2 heures à une température voisine de 20°C, le mélange réactionnel est filtré, concentré à sec sous pression réduite à une température voisine de 35°C. On obtient ainsi 1,3 g d'un résidu brut que l'on purifie par chromatographie sous pression (1,4 bar) sur un volume de 150 cm³ de gel de silice 60F254 Merck (0,063-0,040 mm) contenus dans une colonne de diamètre 3 cm, en utilisant un mélange éluant acétate d'éthyle-cyclohexane (20-80 en volumes). Les fractions ne contenant que le produit cherché sont réunies et concentrées à sec sous pression réduite (environ 2,7 kPa) à une température voisine de 35°C. On obtient ainsi 884 mg de 1, 2-O, O 3, 4-O, O 3', 4'-O, O-tri(diméthylacétonide) 2'-O-benzoyl-2-[(1R, 2S, 3R) (1, 2, 3, 4-tétrahydroxybutyl)] 5-[(2'S, 3'R) (2', 3', 4'-trihydroxybutyl)] pyrazine.

Le produit obtenu possède les caractéristiques suivantes :

Spectre de R.M.N. ¹ H (400 MHz, (CD₃)₂SO d6, δ en ppm) : 0,86 - 1,10 - 1,26 - 1,29 - 1,37 et 1,40 (6 s, 3H chacun : les 3 C(CH₃)₂) ; 3,18 (dd, J = 14 et 9 Hz, 1H : 1H du CH₂ 5α) ; de 3,20 à 3,40 (mt : 1H correspondant à l'autre H du CH₂ 5α) ; 3,78 - de 3,95 à 4,05 et 4,11 (respectivement dd, J = 9 et 4 Hz - mt et dd, J = 7,5 Hz, 1H - 2H et 1H : CH₂O 5δ et CH₂O 2δ) ; 4,20 et 4,37 (2 mts, respectivement 2H et 1H : CH 2β - CH 5γ et CH 2γ) ; 4,95 (d, J = 6,5 Hz, 1H : CH 2α) ; 5,57 (mt, 1H : CH 5β) ; 7,50 (t, J = 7 Hz, 2H : H aromatiques en méta du CO) ; 7,64 (t, J = 7,5 Hz, 1H : H aromatique en para du CO) ; 7,87 (d, J = 7,5 Hz, 2H : H aromatiques en ortho du CO) ; 8,60 (s, 1H : =CH en 6) ; 8,63 (s, 1H : =CH en 3).

La 1, 2-O, O 3, 4-O, O 3', 4'-O, O-tri(diméthylacétonide)-2-[(1R, 2S, 3R) (1, 2, 3, 4-tétrahydroxybutyl)] 5-[(2'S, 3'R) (2', 3', 4'-trihydroxybutyl)] pyrazine peut être préparée de la façon suivante : A une solution de 10 g de désoxyfructosazine dans 250 cm³ de diméthytformamide sous agitation on ajoute 81 cm³ de 2,2-diméthoxypropane puis 0,3 g d'acide para-toluènesulfonique. Le mélange réactionnel est agité à une température d'environ 25°C pendant 20 heures, puis 10 cm³ de 2,2-diméthoxypropane sont rajoutés et l'agitation est poursuivie pendant 3 heures. Le mélange est alors chauffé à une température de 50°C pendant 21 heures. Après concentration sous pression réduite (2,7 kPa) à une température de 60°C, l'huile résiduelle est dissoute dans 300 cm³ de dichlorométhane et lavée 2 fois avec 100 cm³ d'une solution aqueuse de bicarbonate de sodium à 5% puis 2 fois avec 200 cm³ d'eau. La phase organique est séchée sur sulfate de magnésium puis concentrée sous pression réduite (2,7 kPa) à une température de 40°C. L'huile obtenue est chromatographiée sur une colonne de silice (0,020-0,045 mm) éluée avec un mélange acétate d'éthyle/cyclohexane (1:1 en volumes) à une pression d'environ 1,5x10⁵ Pa. Les fractions contenant les produits attendus sont réunies et concentrées sous pression réduite (2,7 kPa) à une température de 40°C. On obtient ainsi 5,2 g de 1, 2-O, O 3, 4-O, O 3', 4'-O, O-tri(diméthylacétonide)-2-[(1R, 2S, 3R) (1, 2, 3, 4-tétrahydroxybutyl)] 5-[(2'S, 3'R) (2', 3', 4'-trihydroxybutyl)] pyrazine sous forme d'un solide blanc ainsi que 2,4 g du même produit impur. Ce dernier est recristallisé dans un mélange eau/éthanol absolu (5:1 en volumes). On isole ainsi 0,6 g de 1, 2-O, O 3, 4-O, O 3', 4'-O, O-tri(diméthylacétonide)-2-[(1R, 2S, 3R) (1, 2, 3, 4-tétrahydroxybutyl)] 5-[(2'S, 3'R) (2', 3', 4'-trihydroxybutyl)] pyrazine sous forme de cristaux blancs fondant à 74°C [Spectre de R.M.N. ¹H (400 MHz, (CD₃)₂SO d6, δ en ppm) : 1,04 - 1,20 - 1,28 - 1,33 et 1,44 (5 s, respectivement 3H-3H-3H-3H et 6H : les 5 CH₃); 2,79 (dd, J=13 et 9 Hz, 1H : 1H au CH₂ 5α), 3,06 (dd, J=13 et 2,5 Hz, 1H : l'autre H du CH₂ 5α); 3,79 (mf, 1H : CH 5β); de 3,80 à 3,90 (mt, 2H : 1H du CH₂ 2δ et 1H du CH₂ 5δ); 3,91 (mt, 1H : CH 5γ); 4,00 (t, J= 7 Hz, 1H : l'autre H du CH₂ 5δ); 4,06 (t, J = 7,5 Hz, 1H : l'autre H du CH₂ 2δ); 4,28 (mt, 1H : CH 2γ); 4,33 (t, J = 7 Hz, 1H : CH 2β); 4,99 (d, J = 7,5 Hz, 1H : CH 2α); 5,07 (d large, J = 5 Hz, 1H : OH en 5β); 8,54 (s, 1H : =CH en 6); 8,66 ( s, 1H : =CH en 3); α_{D}²⁰=+6,7° ± 1,1 (c=0,5/ dichlorométhane); (Rf=0,36; chromatographie sur couche mince de gel de silice; éluant mélange acétate d'éthyle/cyclohexane 1:1 en volumes)].

### La désoxyfructosazine peut être préparée selon la méthode décrite car K. Sumoto et al. dans Chem. Pharm. Bull., 39, 792 (1991).

### 2'-O-benzoyl-2-[(1R, 2S, 3R) (1, 2, 3, 4-tétrahydroxybutyl)] 5-[(2'S, 3'R) (2', 3', 4'-trihydroxybutyl)] pyrazine

A 50 mg de 1, 2-O, O 3, 4-O, O 3', 4'-O, O-tri(diméthylacétonide) 2'-O-benzoyl-2-[(1R, 2S, 3R) (1, 2, 3, 4-tétrahydroxybutyl)] 5-[(2'S, 3'R) (2', 3', 4'-trihydroxybutyl)] pyrazine, à une température voisine de 20°C, on ajoute succesivement un mélange de 0, 67 cm³ d'acide trifluoroacétique et 0,018 cm³ d'eau, puis 1 cm³ de dichlorométhane. Après 23 heures à une température voisine de 20°C, on ajoute 0,033 cm³ d'acide trifluoroacétique supplémentaire. Après 3 heures à une température voisine de 20°C, le mélange réactionnel est concentré à sec sous pression réduite à une température voisine de 35°C. Le résidu brut obtenu est purifié par chromatographie préparative sur 1 plaque de gel de silice 60F254 Merck (épaisseur = 1 mm, 20x20 cm) en éluant par un mélange dichlorométhane-méthanol (90-10 en volumes). Les fractions ne contenant que les produits cherchés sont extraites par un mélange dichlorométhane-méthanol (80-20 en volumes), filtrées sur verre fritté puis concentrées à sec sous pression réduite à une température voisine de 35°C. On obtient ainsi 13 mg de 2'-O-benzoyl-2-[(1R, 2S, 3R) (1, 2, 3, 4-tétrahydroxybutyl)] 5-[(2'S, 3'R) (2', 3', 4'-trihydroxybutyl)] pyrazine.

Le produit obtenu possède les caractéristiques suivantes :

Spectre de R.M.N. ¹ H (400 MHz, (CD₃)₂SO d6, δ en ppm) : 3,25 (AB limite, 2H : CH₂ 5α) ; de 3,35 à 3,65 (mt, 6H : CH 2β - CH 2γ - CH₂O 2δ et CH₂O 5δ) ; 3,80 (mt, 1H : CH 5γ) ; 4,36 (t, J = 5,5 Hz, 1H : OH en 2δ) ; 4,39 (d, J = 8 Hz, 1H : OH en 2γ) ; 4,63 (d, J = 5 Hz, 1H : OH en 2β) ; 4,74 (t, J = 5,5 Hz, 1H : OH en 5δ) ; 4,91 (d large, J = 6,5 Hz, 1H : CH 2α) ; 5,21 (d, J = 7 Hz, 1H : OH en 5γ) ; 5,30 (d, J = 7 Hz, 1H : OH en 2α) ; 5,48 (mt, 1H : CH 5β) ; 7,51 (t, J = 8 Hz, 2H : H aromatiques en méta du CO) ; 7,64 (t, J = 8 Hz, 1H : H aromatique en para du CO) ; 7,89 (d, J = 8 Hz, 2H : H aromatiques en ortho du CO) ; 8,47 (d, J = 1 Hz, 1H : =CH en 6) ; 8,60 (d, J = 1 Hz, 1H : =CH en 3).

### Exemple 28

### 4'-O-pentanoyl-2-[(1R, 2S, 3R) (1, 2, 3, 4-tétrahydroxybutyl)] 5-[(2'S, 3'R) (2', 3', 4'-trihydroxybutyl)] pyrazine

### Préparation de

### 1, 2-O, O 3, 4-O, O 3', 4'-O, O-tri(diméthylacétomide) 2'-O-pentanoyl-2-[(1R, 2S, 3R) (1, 2, 3, 4-tétrahydroxybutyl)] 5-[(2'S, 3'R) (2', 3', 4'-trihydroxybutyl)] pyrazine

A une solution de 2 g de 1, 2-O, O 3, 4-O, O 3', 4'-O, O-tri(diméthylacétonide)-2-[(1R, 2S, 3R) (1, 2, 3, 4-tétrahydroxybutyl)] 5-[(2'S, 3'R) (2', 3', 4'-trihydroxybutyl)] pyrazine dans 30 cm³ de pyridine anhydre, à une température voisine de 20°C, on ajoute goutte à goutte 0,672 cm³ de chlorure de pentanoyle. Après 17 heures à une température voisine de 20°C, on ajoute 0,28 cm³ de chlorure de pentanoyle supplémentaire. Après 23 heures à une température voisine de 20°C, le mélange réactionnel est concentré à sec sous pression réduite à une température voisine de 35°C. On obtient ainsi 3,8 g d'une huile orange que l'on purifie par chromatographie sous pression (1,4 bar) sur un volume de 300 cm³ de gel de silice 60F254 Merck (0,063-0,040 mm) contenus dans une colonne de diamètre 4 cm, en utilisant un mélange éluant acétate d'éthyle-cyclohexane (40-60 en volumes). Les fractions ne contenant que le produit cherché sont réunies et concentrées à sec sous pression réduite à une température voisine de 35°C. On obtient ainsi 2,107 g de 1, 2-O, O 3, 4-O, O 3', 4'-O, O-tri(diméthylacétonide) 2'-O-pentanoyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile jaune.

Le produit obtenu possède les caractéristiques suivantes :

Spectre de R.M.N. ¹ H (400 MHz, (CD₃)₂SO d6, δ en ppm) : 0,80 (t, J = 7,5 Hz, 3H : CH₃ du pentanoyle) ; 1,03 - 1,20 - 1,30 - 1,35 - 1,42 et 1,45 (6 s, 3H chacun : les 3 C(CH₃)₂); 1,13 et 1,35 (2 mts, 2H chacun : CH₂ centraux du pentancyle) ; 2,18 (Ab limite, 2H : CH₂CO du pentanoyle) ; 3,01 et 3,16 (2dd, respectivement J = 14 et 9 Hz et J = 14 et 4 Hz, 1H chacun : CH₂ 5α) ; 3,80 (mt, 2H : 1H du CH₂O 5δ et 1H du CH₂O 2δ) ; 4,05 (mt, 2H : l'autre H du CH₂O 2δ et l'autre H du CH₂O 5δ) ; de. 4,15 à 4,35 (mt, 3H : CH 2β - CH 2γ et CH 5γ) ; 5,00 (d, J = 8 Hz, 1H : CH 2α) ; 5,29 (mt, 1H : CH 5β) ; 8,57 (d, J = 1 Hz, 1H : =CH en 6) ; 8,67 (d, J = 1 Hz, 1H : =CH en 3).

La préparation de la 1, 2-O, O 3, 4-O, O 3', 4'-O, O-tri(diméthylacétonide)-2-[(1R, 2S, 3R) (1, 2, 3, 4-tétrahydroxybutyl)] 5-[(2'S, 3'R) (2', 3', 4'-trihydroxybutyl)] pyrazine est décrite dans l'exemple 27.

### Préparation de

### 4'-O- pentanoyl-2-[(1R, 2S, 3R) (1, 2, 3, 4-tétrahydroxybutyl)] 5-[(2'S, 3'R) (2', 3', 4'-trihydroxybutyl)] pyrazine

A une solution de 500 mg de 1, 2-O, O 3, 4-O, O 3', 4'-O, O-tri(diméthylacétonide) 2'-O-penranoyl-2-[(1R, 2S, 3R) (1, 2, 3, 4-tétrahydroxybutyl)] 5-[(2'S, 3'R) (2', 3', 4'-trihydroxybutyl)] pyrazine, à une température voisine de 20°C, dans 5 cm³ de dichlorométhane, on ajoute successivement 0,775 cm³ d'acide trifluoroacétique et 0,194 cm³ d'eau. Après environ 23 heures à une température voisine de 20°C, le mélange réactionnel est concentré à sec sous pression réduite à une température voisine de 35°C. Le résidu brut obtenu (865 mg) est purifié par chromatographie préparative sur plaques de gel de silice 60F254 Merck (épaisseur = 1 mm, 20x20 cm) en éluant par un mélange dichlorométhane-méthanol (85-15 en volumes). La fraction ne contenant que le produit cherché est extraite par un mélange dichlorométhane-méthanol (80-20 en volumes), filtrée sur verre fritté puis concentrée à sec sous pression réduite à une température voisine de 35°C. Le produit impur ainsi obtenu est rassemblé avec 3 autres échantillons préparés de manière identique (24, 78 et 57 mg), et ce mélange est purifié par chromatographie préparative sur 2 plaques de gel de silice 60F254 Merck (épaisseur = 1 mm, 20x20 cm) en éluant par un mélange dichlorométhane-méthanol (85-15 en volumes). La fraction ne contenant que le produit cherché est extraite par un mélange dichlorométhane-méthanol (80-20 en volumes), filtrée sur verre fritté puis concentrée à sec sous pression réduite à une température voisine de 35°C. On obtient ainsi 45 mg de 4'-O-pentanoyl-2-[(1R, 2S, 3R) (1, 2, 3, 4-tétrahydroxybutyl)] 5-[(2'S, 3'R) (2', 3', 4'-trihydroxybutyl)] pyrazine.

Le produit obtenu possède les caractéristiques suivantes :

Spectre de R.M.N. ¹ H (400 MHz, (CD₃)₂SO d6, δ en ppm) : 0,90 (t, J = 7 Hz, 3H : CH₃ du pentanoyle) ; 1,32 et 1,55 (2 mis, 2H chacun : CH₂ centraux du pentanoyle) ; 2,34 (t, J = 7,5 Hz, 2H : COCH₂ du pentanoyle) ; 2,77 et 3,13 (2dd, respectivement J = 14 et 10 Hz et J = 14 et 3 Hz, 1H chacun : CH₂ 5α) ; de 3,20 à 3,70 (mt : les 5H correspondant aux CH 2β - CH 2γ - CH₂O 2δ et CH 5γ) ; 3,78 (mt, 1H : CH en 5β) ; 4,00 et 4,26 (2dd, J = 11 et 7 Hz et J = 11 et 3 Hz, 1H chacun : CH₂O 5δ) ; 4,38 (t, J = 6 Hz, 1H : OH en 2δ) ; 4,42 (d, J = 8 Hz, 1H : OH en 2γ) ; 4,65 (d, J = 5 Hz, 1H : OH en 2β) ; 4,86 (d, J = 7 Hz, 1H : OH en 5β) ; 4,97 (d, J = 6,5 Hz, 1H : CH 2α) ; 5,10 (d, J = 6 Hz, 1H ; OH en 5γ) ; 5,31 (d, J = 6,5 Hz, 1H : OH en 2α) ; 8,42 (s large, 1H : =CH en 6) ; 8,66 (s large, 1H : =CH en 3).

### Exemple 29

### 4'-O-acétyl-2-[(1R, 2S, 3R) (1, 2, 3, 4-tétrahydroxybutyl)] 5-[(2'S, 3'R) (2', 3', 4'-trihydroxybutyl)] pyrazine

### Préparation de

### 1, 2-O, O 3, 4-O, O 3', 4'-O, O-tri(diméthylacétonide) 2'-O-acétyl-2-[(1R, 2S, 3R) (1, 2, 3, 4-tétrahydroxybutyl)] 5-[(2'S, 3'R) (2', 3', 4'-trihydroxybutyl)] pyrazine

A une solution de 0,5 g de 1, 2-O, O 3, 4-O, O 3', 4'-O, O-tri(diméthylacétonide)-2-[(1R, 2S, 3R) (1, 2, 3, 4-tétrahydroxybutyl)] 5-[(2'S, 3'R) (2', 3', 4'-trihydroxybutyl)] pyrazine dans 10 cm³ de pyridine anhydre, à une température voisine de 20°C, on ajoute goutte à goutte 0,133 cm³ d'anhydride acétique. Après 42 heures à une température voisine de 20°C, le mélange réactionnel est concentré à sec sous pression réduite à une température voisine de 35°C. Le résidu brut obtenu est purifié par chromatographie sous pression (1,4 bar) sur 30 g de gel de silice 60F254 Merck (0,063-0,040 mm) contenus dans une colonne de diamètre 2 cm, en utilisant un mélange éluant acétate d'éthyle-cyclohexane (50-50 en volumes). Les fractions ne contenant que le produit cherché sont réunies et concentrées à sec sous pression réduite à une température voisine de 35°C. On obtient ainsi 0,408 g de 1, 2-O, 0 3, 4-O, O 3', 4'-O, O-tri(diméthylacétonide) 2'-O-acétyl-2-[(1R, 2S, 3R) (1, 2, 3, 4-tétrahydroxybutyl)] 5-[(2'S, 3'R) (2', 3', 4'-trihydroxybutyl)] pyrazine sous forme d'une huile jaune clair.

Le produit obtenu possède les caractéristiques suivantes :

Spectre de R.M.N. ¹ H (400 MHz, (CD₃)₂SO d6, δ en ppm) : 1,00 - 1,20 - 1,23 - 1,35 - 1,42 et 1,45 (6 s, 3H chacun : les 6 CH₃) ; 1,90 (s, 3H : CH₃CO en 5β) ; 3,00 et 3,15 (2dd, respectivement J = 14 et 9 Hz et J = 14 et 4 Hz, 1H chacun : CH₂ 5α) ; 3,80 (mt, 2H : 1H du CH₂O 2δ et 1H du CH₂O 5δ) ; 4,04 (mt, 2H : l'autre H du CH₂O 2δ et l'autre H du CH₂O 5δ) ; de 4,15 à 4,35 (mt, 3H : CH 2γ - CH 2β et CH 5γ) ; 4,99 (d, J = 7 Hz, 1H : CH 2α) ; 5,25 (mt, 1H : CH 5β) ; 8,57 (d, J = 1 Hz, 1H : =CH en 6) ; 8,67 (d, J = 1 Hz, 1H : =CH en 3). La préparation de la 1, 2-O, O 3, 4-O, O 3', 4'-O, O-tri(diméthylacétonide)-2-[(1R, 2S, 3R) (1, 2, 3, 4-tétrahydroxybutyl)] 5-[(2'S, 3'R) (2', 3', 4'-trihydroxybutyl)] pyrazine est décrite dans l'exemple 27.

### Préparation de

### 4'-O-acétyl-2-[(1R, 2S, 3R) (1, 2, 3, 4-tétrahydroxybutyl)] 5-[(2'S, 3'R) (2', 3', 4'-trihydroxybutyl)] pyrazine

A 100 mg de 1, 2-O, O 3, 4-O, O 3', 4'-O, O-tri(diméthylacétonide) 2'-O-acétyl-2-[(1R, 2S, 3R) (1, 2, 3, 4-tétrahydroxybutyl)] 5-[(2'S, 3'R) (2', 3', 4'-trihydroxybutyl)] pyrazine, à une température voisine de 20°C, on ajoute un mélange de 19,68 cm³ d'acide trifluoroacétique et 4,9 cm³ d'eau. Après environ 3,5 heures à une température voisine de 20°C, le mélange réactionnel est concentré à sec sous pression réduite à une température voisine de 35°C. On obtient ainsi 199 mg d'une huile orange qui est purifiée par chromatographie préparative sur 4 plaques de gel de silice 60F254 Merck (épaisseur = 1 mm, 20x20 cm) en éluant par un mélange dichlorométhane-méthanol (90-10 en volumes). La fraction ne contenant que le produit cherché est extraite par un mélange dichlorométhane-méthanol (80-20 en volumes), filtrée sur verre fritté puis concentrée à sec sous pression réduite à une température voisine de 35°C. On obtient ainsi 18 mg de 4'-O-acétyl-2-[(1R, 2S, 3R) (1, 2, 3, 4-tétrahydroxybutyl)] 5-[(2'S, 3'R) (2', 3', 4'-trihydroxybutyl)] pyrazine.

Le produit obtenu possède les caractéristiques suivantes :

Spectre de R.M.N. ¹ H (400 MHz, (CD₃)₂SO d6, δ en ppm) : 2,04 (s, 3H : CH₃CO) ; 2,76 et 3,13 (2dd, respectivement J = 14 et 9 Hz et J = 14 et 3 Hz, 1H chacun : CH₂ 5α) ; 3,45 (mt, 1H : 1H du CH₂O 2δ) ; de 3,50 à 3,75 (mt, 4H : CH 2β - CH 2γ - l'autre H du CH₂O 2δ et CH 5γ) ; 3,78 (mt, 1H : CH 5β) ; 3,99 et 4,25 (2dd, respectivement J = 11 et 7 Hz et J = 11 et 3 Hz, 1H chacun : CH₂O 5δ) ; 4,40 (t, J = 5,5 Hz, 1H : OH en 2δ) ; 4,45 (d, J = 7,5 Hz, 1H : OH en 2) ; 4,68 (d, J = 5 Hz, 1H : OH en 2) ; 4,89 (d, J = 6,5 Hz, 1H : OH en 5β) ; 4,96 (d large, J = 6,5 Hz, 1H : CH 2α) ; 5,15 (d, J = 6,5 Hz, 1H : OH en 5γ) ; 5,34 (d, J = 6,5 Hz, 1H : OH en 2α) ; 8,42 (s large, 1H : =CH en 6) ; 8,66 (s,1H : =CH en 3).

### Exemple 30

### 4, 4'-O, O-di(4-méthyl-benzoyl)-2-[(1R, 2S, 3R) (1, 2, 3, 4-tétrahydroxybutyl)] 5-[(2'S, 3'R) (2', 3', 4'-trihydroxybutyl)] pyrazine

A 300 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 7,5 cm³ de pyridine anhydre est additionné, goutte à goutte, à une température voisine de 20°C sous atmosphère d'argon, 0,326 cm³ de chlorure de 4-méthyl-benzoyle. Après 15 heures d'agitation, à une température voisine de 20°C, le mélange réactionnel est concentré à sec sous pression réduite (0,4 kPa) à une température voisine de 40°C. Le résidu brut ainsi obtenu est purifié par chromatographie sous pression atmosphérique sur 40 g de gel de silice 60F254 Merck (0,063-0,200 mm) contenus dans une colonne de diamètre 3,5 cm, en éluant par un gradient d'élution constitué d'un mélange dichlorométhane-méthanol (de100-0 à 90-10 en volumes) en recueillant des fractions de 10 cm³. Les fractions ne contenant que le produit cherché sont réunies et concentrées à sec sous pression réduite (environ 0,5 kPa) à une température voisine de 40°C. On obtient ainsi 139 mg de 4, 4'-O, O-di(4-méthyl-benzoyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'un solide blanc.

Le produit obtenu possède les caractéristiques suivantes :

Spectre de R.M.N. ¹ H (400 MHz, (CD₃)₂SO d6, δ en ppm) : 2,41 (s, 6H : ArCH₃) ; 2,82 et 3,21 (2 dd, respectivement J = 14 et 9,5 Hz et J = 14 et 3 Hz, 1H chacun : CH₂ 5α) ; de 3,65 à 3,75 (mt, 1H : CH 5γ) ; 3,72 (t large, J = 8 Hz, 1H : CH 2β) ; 3,90 (mt, 1H : CH 5β) ; 4,01 (mt, 1H : CH 2γ) ; 4,25 et 4,49 (2 dd, respectivement J = 12 et 6,5 Hz et J = 12 et 3 Hz , 1H chacun : CH₂O 5δ) ; 4,28 et 4,52 (2 dd, respectivement J = 12 et 6,5 Hz et J = 12 et 3 Hz , 1H chacun : CH₂O 2δ) ; 4,74 (d, J = 8 Hz, 1H : OH en 2β) ; 4,97 (d, J = 7 Hz, 1H : OH en 5β) ; 5,04 (d large, J = 6 Hz, 1H : CH 2α) ; 5.25 (d, J = 6 Hz, 1H : OH en 2γ) ; 5,29 (d, J = 6 Hz, 1H : OH en 5γ) ; 5,46 (d, J = 6 Hz, 1H : OH en 2α) ; 7,36 (d, J = 8 Hz, 4H : H aromatiques en méta du CO) ; 7,94 (mt, 4H : H aromatiques en ortho du CO) ; 8,47 (d, J = 1 Hz, 1H : =CH en 6) : 8,70 (s large, 1H : =CH en 3).

En opérant selon le mode opératoire précédent, les composés suivant peuvent être obtenus d'une manière semblable :

### Exemple 31

### 4, 4'-O, O-di(4-méthoxy-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,82 et 3,20 (respectivement dd et d large, J = 14 et 9 Hz et J = 14 Hz, 1H chacun : CH₂ 5α) ; de 3,65 à 3,75 (mt, 1H : CH 5γ) ; 3,72 (t large, J = 8 Hz, 1H : CH 2β) ; 3,87 (s, 6H : ArOCH₃) ; de 3,80 à 3,95 (mt, 1H : CH 5β) ; 4,00 (mt, 1H : CH 2γ) ; de 4,15 à 4,30 (mt, 2H : 1H du CH₂O 5δ et 1H du CH₂O 2δ) ; 4,47 et 4,50 (respectivement dd et d large, J = 12 et 3 Hz et J = 12 Hz, 1H chacun : l'autre H du CH₂O 5δ et l'autre du CH₂O 2δ) 4,72 (d, J = 8 Hz, 1H : OH en 2β) ; 4,94 (d, J = 6,5 Hz, 1H : OH en 5β) ; 5,03 (d large, J = 5,5 Hz, 1H : CH 2α) ; 5,22 (d, J = 6 Hz, 1H : OH en 2γ) ; 5,25 (d, J = 6 Hz, 1H : OH en 5γ) ; 5,44 (d, J = 5,5 Hz, 1H : OH en 2α) ; 7,08 (d, J = 8,5 Hz, 4H : H aromatiques en méta du CO) ; 8,00 (d large, J = 8,5 Hz, 4H : H aromatiques en ortho du CO) ; 8,47 (s large, 1H : en 6) ; 8,70 (s large, 1H : =CH en 3).

### Exemple 32

### 4, 4'-O, O-di(3-méthyl-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,40 (s, 6H : ArCH₃) ; 2,83 et 3,20 (2 dd, respectivement J = 14 et 9 Hz et J = 14 et 3 Hz, 1H chacun : CH₂ 5α) ; de 3,65 à 3,30 (mt, 2H : CH 5γ et CH 2β) ; 3,89 (mt, 1H : CH 5β) ; 4,01 (mt, 1H : CH 2γ) ; de 4,20 à 4,30 (mt, 2H : 1H du CH₂O 5δ et 1H du CH₂O 2δ) ; 4,50 et 4,54 (2 dd, J = 12 et 3 Hz, 1H chacun : respectivement l'autre H du CH₂O 5δ et l'autre du CH₂O 2δ) ; 4,74 (d, J = 8 Hz, 1H : OH en 2β) ; 4,96 (d, J = 7 Hz, 1H : OH en 5β) ; 5,02 (s large, 1H : CH 2α) ; 5,27 (mt, 2H : OH en 2γ et OH en 5γ) ; 5,47 (mf, 1H : OH en 2α) ; 7,43 (t, J = 7,5 Hz, 2H : H aromatiques en 5) ; 7,48 (d large, J = 7,5 Hz, 2H : H aromatiques en 4) ; 7,83 (d large, J = 7,5 Hz, 2H : H aromatiques en 6) ; 7,96 (s large, 2H : H aromatiques en 2) ; 8,47 (d , J = 1 Hz, 1H : =CH en 6) ; 8,70 (s large, 1H : =CH en 3).

### Exemple 33

### 4, 4'-O, O-di(4-fluoro-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,82 et 3,21 (2 dd, respectivement J = 14 et 9,5 Hz et J = 14 et 3,5 Hz, 1H chacun : CH₂ 5α) ; de 3,65 à 3,75 (mt, 1H : CH 5γ) ; 3,72 (t large, J = 8 Hz, 1H : CH 2β) ; 3,90 (mt, 1H : CH 5β) ; 4,01 (mt, 1H : CH 2γ) ; 4,26 et 4,50 (2 dd, respectivement J = 12 et 6,5 Hz et J = 12 et 3 Hz , 1H chacun : CH₂O 5δ) ; 4,28 et 4,53 (2 dd, respectivement J = 12 et 6,5 Hz et J = 12 et 3 Hz , 1H chacun : CH₂O 2δ) ; 4,74 (d, J = 8 Hz, 1H : OH en 2β) ; 4,97 (d, J = 6,5 Hz, 1H : OH en 5β) ; 5,01 (d large, J = 6 Hz, 1H : CH 2α) ; 5,26 (d, J = 6 Hz, 1H : OH en 2γ) ; 5,30 (d, J = 6 Hz, 1H : OH en 5γ) ; 5,45 (d, J = 6 Hz, 1H : OH en 2α) ; 7,38 (t, J = 8,5 Hz, 4H : H aromatiques en ortho du F) ; 8,10 (mt, 4H : H aromatiques en ortho du CO) ; 8,47 (s large, 1H : =CH en 6) ; 8,70 (s large, 1H : =CH en 3).

### Exemple 34

### 4, 4'-O, O-di-thiénoyl-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,81 et 3,19 (2 dd, respectivement J = 14 et 9,5 Hz et J = 14 et 3 Hz, 1H chacun : CH₂ 5α) ; de 3,65 à 3,75 (mt, 1H : CH 5γ) ; 3,71 (t large, J = 8 Hz, 1H : CH 2β) ; 3,87 (mt, 1H : CH 5β) ; 3,98 (mt, 1H : CH 2γ) ; 4,25 et 4,49 (2 dd, respectivement J = 12 et 7 Hz et J = 12 et 3 Hz , 1H chacun : CH₂O 55) ; 4,29 et 4,52 (2 dd, respectivement J = 12 et 7 Hz et J = 12 et 3 Hz, 1H chacun : CH₂O 2δ) ; 4,75 (d, J = 8 Hz, 1H : OH en 2β) ; 4,97 (d, J = 7 Hz, 1H : OH en 5β) ; 5,01 (d large, J = 6 Hz, 1H : CH 2α) ; 5,25 (d, J = 6 Hz, 1H : OH en 2γ) ; 5,28 (d, J = 6 Hz, 1H : OH en 5γ) ; 5,47 (d, J = 6 Hz, 1H : OH en 2α) ; 7,25 (mt, 2H : H4 du thiényle) ; 7,87 et 7,97 (2 mts, 2H chacun : H3 et H5 du thiényle) ; 8,47 (s, 1H : =CH en 6) ; 8,71 (s, 1H : =CH en 3).

### Exemple 35

### 4, 4'-O, O-di(4-nitro-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,82 et 3,22 (2 dd, respectivement J = 14 et 9 Hz et J = 14 et 3 Hz, 1H chacun : CH₂ 5α) ; de 3,70 à 3,80 (mt, 1H : CH 5γ) ; 3,74 (t large, J = 8 Hz, 1H : CH 2β) ; 3,90 (mt, 1H : CH 5β) ; 4,03 (mt, 1H : CH 2γ) ; 4,35 et 4,56 (2 dd, respectivement J = 12 et 7 Hz et J = 12 et 3 Hz, 1H chacun : CH₂O 5δ) ; 4,37 et 4,59 (2 dd, respectivement J = 12 et 7 Hz et J = 12 et 3 Hz, 1H chacun : CH₂O 2δ) ; 4,80 (d, J = 8 Hz, 1H : OH en 2β) ; de 5,00 à 5,10 (mt, 2H : OH en 5β et CH 2α) ; 5,34 (d, J = 6 Hz, 1H : OH en 2γ) ; 5,38 (d, J = 6 Hz, 1H : OH en 5γ) ; 5,50 (d, J = 6 Hz, 1H : OH en 2α) ; 8,23 (mt, 4H : H aromatiques en ortho du CO) ; 8,40 (d, J = 8 Hz, 4H : H aromatiques en ortho du NO₂) ; 8,47 (s, 1H : =CH en 6) ; 8,71 (s, 1H : =CH en 3).

### Exemple 36

### 4, 4'-O, O-di(4-chloro-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,80 et 3,20 (2 dd, respectivement J = 14 et 9,5 Hz et J = 14 et 3,5 Hz, 1H chaoun : CH₂ 5α) ; de 3,65 à 3,75 (mt, 1H : CH 5γ) ; 3,72 (t large, J = 8 Hz, 1H : CH 2β) ; 3,89 (mt, 1H : CH 5β) ; 4,00 (mt, 1H : CH 2γ) ; 4,28 et 4,50 (2 dd, respectivement J = 12 et 6 Hz et J = 12 et 3 Hz, 1H chacun : CH₂O 5δ) ; 4,31 et 4,53 (2 dd, respectivement J = 12 et 6 Hz et J = 12 et 3 Hz , 1H chacun : CH₂O 2δ) ; 4,76 (d, J = 8 Hz, 1H : OH en 2β) ; 4,99 (d, J = 7 Hz, 1H : OH en 5β) ; 5,02 (d large, J = 6 Hz, 1H : CH 2α) ; 5,30 (d, J = 6 Hz, 1H : OH en 2γ) ; 5,32 (d, J = 6 Hz, 1H : OH en 5γ) ; 5,48 (d, J = 6 Hz, 1H : OH en 2α) ; 7,63 (d, J = 8 Hz, 4H : H aromatiques en ortho du Cl) ; 8,05 (mt, 4H : H aromatiques en ortho du CO) ; 8,47 (d, J = 1 Hz, 1H : =CH en 6) ; 8,70 (s large, 1H : =CH en 3).

### Exemple 37

### 4, 4'-O, O-di(4-N,N-diméthylamino-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,82 et 3,19 (2 dd, respectivement J = 14 et 9 Hz et J = 14 et 3 Hz, 1H chacun : CH₂ 5α) ; 3,03 (s, 12H : ArN(CH₃)₂) ; de 3,65 à 3,80 (mt, 1H : CH 5γ) ; 3,71 (t large, J = 8 Hz, 1H : CH 2β) ; 3,89 (mt, 1H : CH 5β) ; 3,98 (mt, 1H : CH 2γ) ; de 4,15 à 4,25 (mt, 2H : 1H du CH₂O 5δ et 1H du CH₂O 2δ) ; de 4,40 à 4,55 (mt, 2H : l'autre H du CH₂O 5δ et l'autre du CH₂O 2δ) ; 4,69 (d, J = 8 Hz, 1H : OH en 2β) ; 4,92 (d, J = 6,5 Hz, 1H : OH en 5β) ; 5,03 (d large, J = 6 Hz, 1H : CH 2α) ; 5,17 et 5,21 (2 d, J = 6 Hz, 1H chacun : OH en 2γ et OH en 5γ) ; 5,43 (d, J = 6 Hz, 1H : OH en 2α) ; 6,76 (d, J = 8,5 Hz, 4H : H aromatiques en méta du CO) ; 7,85 (mt, 4H : H aromatiques en ortho du CO) ; 8,47 (s large, 1H : =CH en 6) ; 8,70 (s large, 1H : =CH en 3).

Le dérivé 4, 4'-O, O-di(4-amino-benzoyl)-2-[(1R, 2S, 3R) (1, 2, 3, 4-tétrahydroxybutyl)] 5-[(2'S, 3'R) (2', 3', 4'-trihydroxybutyl)] pyrazine peut être préparé en utilisant un mode opératoire semblable.

### Exemple 38

### 4, 4'-O, O-di(4-benzyloxy-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,81 et 3,21 (2 dd, respectivement J = 14 et 9 Hz et J = 14 et 3 Hz, 1H chacun : CH₂ 5α) ; de 3,65 à 3,80 (mt, 1H : CH 5γ) ; 3,71 (t large, J = 8 Hz, 1H : CH 2β) ; 3,89 (mt, 1H : CH 5β) ; 4,00 (mt, 1H : CH 2γ) ; 4,22 et 4,51 (2 dd, respectivement J = 12 et 7 Hz et J = 12 et 3 Hz, 1H chacun : CH₂O 5δ) ; 4,25 et 4,51 (2 dd, respectivement J = 12 et 7 Hz et J = 12 et 2,5 Hz, 1H chacun : CH₂O 2.5) ; 4,75 (d, J = 8 Hz, 1H : OH en 2β) ; 4,97 (d, J = 6,5 Hz, 1H : OH en 5β) ; 5,03 (mt, 1H : CH 2α) ; de 5,20 à 5,35 (mt, 2H : OH en 2γ et OH en 5γ) ; 5,22 (s, 4H : ArCH₂O) ; 5,47 (d large, J = 5,5 Hz, 1H : OH en 2α) ; 7,17 (d, J = 8,5 Hz, 4H : H aromatiques en méta du CO) ; 7,38 (t, J = 7,5 Hz, 2H : H aromatiques en para pour les benzyles) ; 7,44 (t, J = 7,5 Hz, 4H : H aromatiques en méta pour les benzyles) ; 7,50 (d, J = 7,5 Hz, 4H : H aromatiques en ortho pour les benzyles) ; 8,00 (mt, 4H : H aromatiques en ortho du CO) ; 8,47 (d, J = 1 Hz, 1H : =CH en 6) ; 8,70 (s large, 1H : =CH en 3).

### Exemple 39

### 4, 4'-O, O-di(3,5-dichloro-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,81 et 3,22 (2 dd, respectivement J = 14 et 9 Hz et J = 14 et 3 Hz, 1H chacun : CH₂ 5α) ; de 3,65 à 3,75 (mt, 1H : CH 5γ) ; 3,72 (t large, J = 9 Hz, 1H : CH 2β) ; 3,88 (mt, 1H : CH 5β) ; 4,02 (mt, 1H : CH 2γ) ; de 4,20 à 4,35 (mt, 2H : 1H du CH₂O 5δ et 1H du CH₂O 2δ) ; 4,51 (dd, J = 12 et 3 Hz, 1H : l'autre H du CH₂O 5δ) ; 4,53 (dd, J = 12 et 3 Hz, 1H : l'autre du CH₂O 2δ) ; 4,79 (d, J = 9 Hz, 1H : OH en 2β) ; de 5,00 à 5,10 (mt, 2H: OH en 5β et CH 2α) ; 5,38 (d, J = 6 Hz, 1H : OH en 2γ) ; 5,41 (d, J = 6 Hz, 1H : OH en 5γ) ; 5,50 (d, J = 5,5 Hz, 1H : OH en 2α) ; de 8,00 à 8,10 (mt, 6H : H aromatiques) ; 8,47 (d, J = 1 Hz, 1H : =CH en 6) ; 8,71 (d, J = 1 Hz, 1H : =CH en 3).

### Exemple 40

### 4,4'-O, O-di-phénylacétyl-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 2,77 et 3,14 (2 dd, respectivement J = 14 et 9 Hz et J = 14 et 3 Hz, 1H chacun : CH₂ 5α) ; de 3,55 à 3,65 (mt, 1H : CH 5γ) ; 3,64 (t large, J = 9 Hz, 1H : CH 2β) ; 3,71 (s, 4H : OCOCH_{2α}r) ; 3,80 (mt, 1H : CH 5β) ; 3,88 (mt, 1H : CH 2γ) ; de 4,00 à 4,15 (mt, 2H : 1H du CH₂O 5δ et 1H du CH₂O 2δ) ; 4,30 (dd, J = 12 et 3 Hz, 1H : l'autre H du CH₂O 5δ) ; 4,34 (dd, J = 12 et 2,5 Hz, 1H : l'autre H du CH₂O 2δ) ; 4,65 (d, J = 9 Hz, 1H : OH en 2β) ; 4,88 (d, J = 7 Hz, 1H : OH en 5β) ; 4,99 (s large, 1H : CH 2α) ; 5,13 (d, J = 6 Hz, 1H : OH en 2γ) ; 5,16 (d, J = 6 Hz, 1H : OH en 5γ) ; 5,45 (mf, 1H ; OH en 2α) ; de 7,20 à 7,45 (mt, 10H : H aromatiques) ; 8,44 (s large, 1H : =CH en 6) ; 8,68 (s large, 1H : =CH en 3).

### Exemple 41

### 4, 4'-O, O-di(4-hydroxy-benzoyl)-2-[(1R, 2S, 3R) (1, 2, 3, 4-tétrahydroxybutyl)] 5-[(2'S, 3'R) (2', 3', 4'-trihydroxybutyl)] pyrazine

A une solution de 158 mg de 4, 4'-O, O-di(4-benzyloxy-benzoyl)-2-[(1R, 2S, 3R) (1, 2, 3, 4-tétrahydroxybutyl)] 5-[(2'S, 3'R) (2', 3', 4'-trihydroxybutyl)] pyrazine dans 5 cm³ de méthanol, est ajouté, à une température voisine de 20°C, 80 mg de palladium sur charbon (10% en poids de palladium). La suspension obtenue est agitée sous atmosphère d'hydrogène (300 kPa), à une température voisine de 26°C, pendant 3 heures. Le milieu réactionnel est alors filtré sur Célite, La Célite est rincée avec du méthanol puis avec un mélange méthanol-dichlorométhane (10-90 en volumes). Le filtrat est concentré à sec sous pression réduite à une température voisine de 40°C. On obtient ainsi 89 mg de 4, 4'-O, O-di(4-hydroxy-benzoyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une laque jaune.

Le produit obtenu possède les caractéristiques suivantes :

Spectre de R.M.N. ¹ H (400 MHz, (CD₃)₂SO d6, δ en ppm) : : 2,82 et 3,20 (respectivement dd et d large, J = 14 et 9 Hz et J = 14 Hz, 1H chacun : CH₂ 5α) ; de 3,65 à 3,75 (mt, 1H : CH 5γ) ; 3,71 (t large, J = 9 Hz, 1H : CH 2β) ; 3,90 (mt, 1H : CH 5β) ; 3,98 (mt, 1H : CH 2γ) ; de 4,15 à 4,25 (mt, 2H : 1H du CH₂O 5δ et 1H du CH₂O 2δ) ; 4,45 (dd, J = 12 et 3 Hz, 1H : l'autre H du CH₂O 5δ) ; 4,49 (d large, J = 12 Hz, 1H : l'autre du CH₂O 2δ) ; 4,73 (d, J = 9 Hz, 1H : OH en 2β) ; 4,95 (d, J = 6,5 Hz, 1H : OH en 5β) ; 5,03 (d large, J = 5,5 Hz, 1H : CH 2α) ; 5,22 (d, J = 6 Hz, 1H : OH en 2γ) ; 5,26 (d, J = 6 Hz, 1H : OH en 5γ) ; 5,46 (d, J = 5,5 Hz, 1H : OH en 2α) ; 6,88 (d, J = 8,5 Hz, 4H : H aromatiques en méta du CO) ; 7,90 (d large, J = 8,5 Hz, 4H : H aromatiques en ortho du CO) ; 8,47 (s large, 1H : =CH en 6) ; 8,71 (s large, 1H : =CH en 3) ; 10,30 (mf étalé, 2H : ArOH).

Les produits ci-dessous peuvent également être préparés, en opérant selon le même mode opératoire à partir de la désoxyfructosazine, et en utilisant les produits de départ correspondants (acides ou chlorures d'acyle) convenablement choisis :

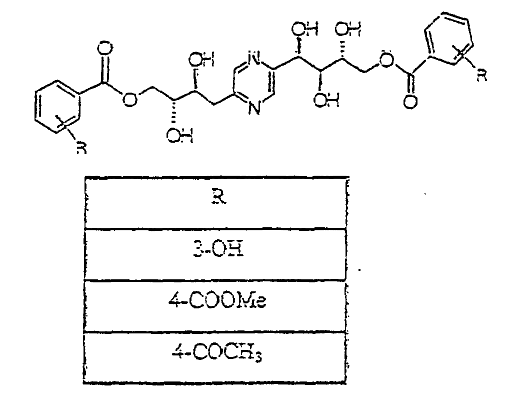

### Exemple 42

### 4, 4'-O, O-di(4-(N,N-di-n-propyl-aminométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine

### 4-(N,N-di-n-propyl-aminométhylène)-benzoate de méthyle

A une suspension de 2,29 g de 4-(bromométhyl) benzoate de méthyle, et 1,52 g de carbonate de potassium, dans 40 cm³ d'acétonitrile, à une température voisine de 20°C, on ajoute 1,37 cm³ de N,N-di-n.propylamine. Le mélange réactionnel est porté au reflux pendant environ 16 heures, puis après refroidissement jusqu'à une température voisine de 20°C, il est filtré sur verre fritté. Le verre fritté est rincé avec 3 fois 30 cm³ d'acétate d'éthyle. Le filtrat est concentré à sec sous pression réduite à une température voisine de 40°C. Après dissolution du résidu dans 100 cm³ d'acétate d'éthyle, la phase organique est lavée successivement par 3 fois 100 cm³ d'eau distillée, 100 cm³ d'une solution aqueuse saturés en chlorure de sodium, séchée sur sulfate de magnésium, et concentrée à sec sous pression réduite (environ 1 kPa) à une température voisine de 40°C. On obtient ainsi 2,5 g de 4-(N,N-di-n.propylaminométhylène)-benzoate de méthyle sous forme d'une huile jaune

Acide 4-(N,N-di-n.propyl-aminométhylène)-benzoique (monochlorhydrate)

A une suspension huileuse de 2,4 g de 4-(N,N-di-n.propyl-aminométhylène)-benzoate de méthyle dans 9 cm³ d'acide chlorhydrique concentré (36% minimum), à une température voisine de 20°C, on ajoute 9 cm³ d'eau distillée. Le mélange réactionnel est porté au reflux pendant 24 heures, puis additionné de noir 3S, et filtré à chaud sur un verre fritté. Le filtrat est concentré à sec sous pression réduite (environ 1 kPa) à une température voisine de 40°C. Le solide blanc ainsi obtenu est filtré sur verre fritté, rincé avec 3 fois 10 cm³ d'acétonitrile, 10 cm³ d'oxyde de diisopropyle, séché à l'air puis sous pression réduite (environ 0,2 kPa) à une température voisine de 50°C. On obtient ainsi 1,8 g d'acide 4-(N,N-di-n.propyl-aminométhylène)-benzoïque (monochlorhydrate) sous forme de cristaux blancs.

### Chlorure de 4-(N,N-di-n.propyl-aminométhylène)-benzoyle (monochlorhydrate)

A une suspension de 0,748 g de monochlorhydrate de l'acide 4-(N,N-di-n.propyl-aminométhylène)-benzoïque dans 20 cm³ de dichlorométhane, on ajoute successivement, à une température voisine de 20°C, 0,283 cm³ de chlorure d'oxalyle puis une goutte de diméthylformamide. Le milieu réactionnel est laissé sous agitation à une température voisine de 20°C jusqu'à la fin du dégagement gazeux, puis concentré à sec sous pression réduite (environ 0,5 kPa) à une température voisine de 35°C. Le solide blanc ainsi obtenu est utilisé tel quel pour la préparation de la 4, 4'-O, O-di(4-(N,N-di-n.propyl-aminométhylène)-benzoyl)-2-[(1R, 25, 3R) (1, 2, 3, 4-tétrahydroxybutyl)] 5-[(2'S, 3'R) (2', 3', 4'-trihydroxybutyl)] pyrazine.

### 4, 4'-O, O-di(4-(N,N-di-n.propyl-aminométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine

A 304 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 10 cm³ de pyridine anhydre est addidonné, par petites portions, à une température voisine de 20°C sous atmosphère d'argon, 0,00275 mole de chlorure de 4-(N,N-di-n.propyl-aminométhylène)-benzoyle (sous forme de monochlorhydrate). Après environ 17 heures d'agitation à une température voisine de 20°C, le milieu réactionnel est concentré à sec sous pression réduite (environ 0,5 kPa) à une température voisine de 35°C. La pâte jaune obtenue est redissous dans 30 cm³ d'eau, lavée avec 3 fois 20 cm³ d'acétate d'éthyle. La phase aqueuse est concentrée à sec sous pression réduite (environ 0,5 kPa) à une température voisine de 40°C. On obtient 1,3 g d'une meringue jaune pâle que l'on purifie par chromatographie sous pression (180 kPa) sur un volume de 120 cm³ de gel de silice 60F254 Merck (0,063-0,040 mm) contenus dans une colonne de diamètre 3 cm, en éluant par un gradient d'élution constitué d'un mélange chloroforme-méthanol-ammoniaque (12-3-0 puis 12-3-0,2 en volumes) en recueillant des fractions de 20 cm³. Les fractions ne contenant que le produit cherché sont réunies et concentrées à sec sous pression réduite (environ 0,5 kPa) à une température voisine de 40°C. On obtient ainsi 280 mg d'un solide que l'on repurifie par chromatographie sous pression (180 kPa) sur un volume de 90 cm³ de gel de silice 60F254 Merck (0,063-0,040 mm) contenus dans une colonne de diamètre 3 cm, en éluant par un gradient d'élution constitué d'un mélange chloroforme-méthanol-ammoniaque (80-20-0 puis 80-20-1 en volumes). Les fractions ne contenant que le produit cherché sont réunies et concentrées à sec sous pression réduite (environ 0,5 kPa) à une température voisine de 40°C. On obtient ainsi 170 mg de 4, 4'-O, O-di(4-(N,N-di-n.propyl-aminométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine sous forme d'une poudre cristalline blanche.

Le produit obtenu possède les caractéristiques suivantes :

Spectre de R.M.N. ¹ H (400 MHz, (CD₃)₂SO d6, δ en ppm) : 0,83 (t, J = 7,5 Hz, 12H : CH₃ des propyles) ; 1,44 (mt, 8H : CH₂ central des propyles) ; 2,34 (mt, 8H : NCH₂ des propyles) ; 2,82 et 3,20 (2 dd, respectivement J = 14 et 9,5 Hz et J = 14 et 3 Hz, 1H chacun : CH₂ 5α) ; 3,60 (s, 4H : ArCH₂N) ; de 3,65 à 3,75 (mt, 1H : CH 5γ) ; 3,71 (t, J = 9 Hz, 1H : CH 2β) ; 3,89 (mt, 1H : CH 5β) ; 3,99 (mt, 1H : CH 2γ) ; de 4,20 à 4,30 (mt, 2H : 1H du CH₂O 5δ et 1H du CH₂O 25) ; 4,48 (dd, J = 12 et 3 Hz, 1H : l'autre H du CH₂O 5δ) ; 4,51 (dd, J = 12 et 3 Hz, 1H : l'autre H du CH₂O 2δ) ; 4,74 (d, J = 9 Hz, 1H : OH en 2β) ; 4,96 (d, J = 7 Hz, 1H : OH en 5β) ; 5,01 (d large, J = 6 Hz, 1H : CH 2α) ; 5,23 (d, J = 7 Hz, 1H : OH en 2γ) ; 5,27 (d, J = 7 Hz, 1H : OH en 5γ) ; 5,46 (d, J = 6 Hz, 1H : OH en 2α) ; 7,48 (d, J = 8 Hz, 4H : H aromatiques en méta du CO) ; 7,98 (d, J = 8 Hz, 4H : H aromatiques en ortho du CO) ; 8,47 (s large, 1H : =CH en 6) ; 8,70 (s large, 1H : =CH en 3).

### Exemple 43

### trichlorhydrate de (4, 4'-O, O-di(4-(N,N-di-n.propyl-aminométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine)

A une solution de 170 mg de 4, 4'-O, O-di(4-(N,N-di-n.propylaminométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine dans 10 cm³ d'eau, on ajoute, à une température voisine de 20°C, 7,3 cm³ d'une solution d'acide chlorhydrique aqueux de concentration 0,1N. Après 30 minutes d'agitation à une température voisine de 20°C, le milieu réactionnel est lyophilisé sous pression réduite. On obtient ainsi 182 mg de trichlorhydrate de (4, 4'-O, O-di(4-(N,N-di-n.propyl-aminométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine) sous forme d'une poudre blanche.

Le produit obtenu possède les caractéristiques suivantes :

Spectre de R.M.N. ¹ H (400 MHz, (CD₃)₂SO d6, δ en ppm) : 0,88 (t, J = 7,5 Hz, 12H : CH₃ des propyles) ; 1,72 (mt, 8H : CH₂ central des propyles) ; 2,81 (dd, J = 14 et 9,5 Hz, 1H : 1H du CH₂ 5α) ; 2,96 (mt, 8H : NCH₂ des propyles) ; 3,21 (dd, J = 14 et 3 Hz, 1H : l'autre H du CH₂ 5α) ; de 3,65 à 3,80 (mt, 2H : CH 5γ et CH 2β) ; 3,89 (mt, 1H : CH 5β) ; 4,02 (mt, 1H : CH 2γ) ; de 4,25 à 4,35 (mt, 2H : 1H du CH₂O 5δ et 1H du CH₂O 2δ) ; 4,42 (d, J = 5,5 Hz, 4H : ArCH₂N) ; 4,51 (dd, J = 12 et 3 Hz, 1H : l'autre H du CH₂O 5δ) ; 4,55 (dd, J = 12 et 3 Hz, 1H : l'autre H du CH₂O 2δ) ; 5,03 (s large, 1H : CH 2α) ; 7,78 (d, J = 8 Hz, 4H : H aromatiques en méta du CO) ; 7,97 (d, J = 8 Hz, 4H : H aromatiques en ortho du CO) ; 8,47 (s large, 1H : =CH en 6) ; 8,70 (s large, 1H : =CH en 3) ; 11,28 (mf, 2H : NH⁺ Cl⁻).

En opérant selon le mode opératoire de l'exemple 42 et 43, les composés suivant peuvent être obtenus d'une manière semblable :

### Exemple 44

### 4, 4'-O, O-di(4-(N,N-diéthyl-aminométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : 1,01 (t, J = 7,5 Hz, 12H : CH₃ des éthyles) ; 2,48 (q, J = 7,5 Hz, 8H : NCH₂ des éthyles) ; 2,82 et 3,20 (2 dd respectivement J = 14 et 9,5 Hz et J = 14 et 3 Hz, 1H chacun : CH₂ 5α) ; 3,62 (s, 4H : ArCH₂N) ; de 3,65 à 3,80 (mt, 1H : CH 5γ) ; 3,72 (t, J = 8,5 Hz, 1H : CH 2β) ; 3,88 (mt, 1H : CH 5β) ; 3,99 (mt, 1H : CH 2γ) ; de 4,20 à 4,35 (mt, 2H : 1H du CH₂O 5δ et 1H du CH₂O 2δ) ; 4,49 (dd, J = 12 et 3 Hz, 1H : l'autre H du CH₂O 5δ) ; 4,53 (dd, J = 12 et 3 Hz, 1H : l'autre H du CH₂O 2δ) ; 4,75 (d, J = 8,5 Hz, 1H : OH en 2β) ; 4,97 (d, J = 7 Hz, 1H : OH en 5β) ; 5,02 (d large, J = 6 Hz, 1H : CH 2α) ; 5,25 (d, J = 7 Hz, 1H : OH en 2γ) , 5,28 (d, J = 7 Hz, 1H : OH en 5γ) ; 5,47 (d, J = 6 Hz, 1H : OH en 2α) ; 7,48 (d, J = 8 Hz, 4H : H aromatiques en méta du CO) ; 7,99 (d, J = 8 Hz, 4H : H aromatiques en ortho du CO) ; 8,48 (d, J = 1 Hz, 1H : =C H en 6) ; 8,72 (s large, 1H : =CH en 3).

### Exemple 45

### Trichlorhydrate de (4, 4'-O, O-di(4-(N,N-diéthyl -aminométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine)

Spectre de R.M.N. ¹ H (500 MHz, (CD₃)₂SO d6, δ en ppm) : 1,30 (t, J = 7,5 Hz, 12H : CH₃ des éthyles) ; 2,92 et 3,22 (2 dd, respectivement J = 14 et 9,5 Hz et J = 14 et 3 Hz, 1H chacun : CH₂ 5α) ; 3,11 (mt, 8H : NCH₂ des éthyles) ; 3,79 (mt, 1H : CH 5γ) ; 3,83 (d large, J = 3,5 Hz, 1H : CH 2β) ; 3,99 (mt, 1H : CH 5β) ; 4,04 (mt, 1H : CH 2γ) ; de 4,30 à 4,45 (mt, 2H : 1H du CH₂O 5δ et 1H du CH₂O 2δ) ; 4,38 (s, 4H : ArCH₂N) ; 4,55 (dd, J = 12 et 3 Hz, 1H : l'autre H du CH₂O 5δ) ; 4,60 (dd, J = 12 et 3 Hz, 1H : l'autre H du CH₂O 2δ) ; 5,04 (s large, 1H : CH 2α) ; 7,82(d, J 3 Hz, 4H : H aromatiques en méta du CO) ; 8,09 (d large, J = 8 Hz, 4H : H aromatiques en ortho du CO) ; 8,49 (s large, 1H : =CH en 6) ; 8,72 (s large, 1H : =CH en 3) ; 10.70 (mf étalé, 2H : NH⁺Cl⁻).

### Example 46

### 4, 4'-O, O-di(4-(pipéridinométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (400 MHz, (CD₃)₂SO d6, δ en ppm) : de 1,30 à 1,60 (mt, 12H : CH₂CH₂CH₂ des pipéridyles) ; 2,34 (mf, 8H : CH₂NCH₃ des pipéridyles) ; 2,80 et 3,19 (2 dd, respectivement J = 14 et 9,5 Hz et J = 14 et 3,5 Hz, 1H chacun : CH₂ 5α) ; 3,52 (mf, 4H : ArCH₂N) ; de 3,65 à 3,75 (mt, 2H : CH 5γ et CH 2β) ; 3,86 (mt, 1H : CH 5β) ; 3,98 (mt, 1H : CH 2γ) ; de 4,15 à 4,30 (mt, 2H : 1H du CH₂O 5δ et 1H du CH₂O 2δ) ; 4,46 (dd, J = 12 et 3 Hz, 1H ; l'autre H du CH₂O 5δ); 4.49 (dd, J = 12 et 2,5 Hz, 1H : l'autre H du CH₂O 2δ) ; 4,72 (d, J = 8,5 Hz, 1H : OH en 2β) ; 4,96 (d, J = 6,5 Hz, 1H : OH en 5β) ; 5,00 (d large, J = 6 Hz, 1H : CH 2α) ; 5,23 (d, J = 6,5 Hz, 1H : OH en 2γ) ; 5,26 (d, J = 6,5 Hz, 1H : OH en 5γ) ; 5,44 (d, J = 6 Hz, 1H : OH en 2α) ; 7,45 (d large, J = 8 Hz, 4H : H aromatiques en méta du CO) ; 7,97 (d, J = 8 Hz, 4H : H aromatiques en ortho du CO) ; 8,45 (s, 1H : =CH en 6) ; 8,69 (s, 1H : =CH en 3).

### Exemple 47

### 4, 4'-O, O-di(4-(morpholinométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (500 MHz, (CD₃)₂SO d6, δ en ppm) : 2,38 (mt, 8H : CH₂NCH₂ des morpholines) ; 2,82 et 3,20 (2 dd, respectivement J = 14 et 9,5 Hz et J = 14 et 3,5 Hz, 1H chacun : CH₂ 5α) ; 3,56 (s, 4H : ArCH₂N) ; 3,60 (mt, 8H : CH₂OCH₂ des morpholines) ; de 3,65 à 3,75 (mt, 1H : CH 5γ) ; 3,73 ( t large, J = 8,5 Hz, 1H : CH 2β) ; 3,89 (mt, 1H : CH 5β) ; 4,01 (mt, 1H : CH 2γ) ; 4,27 (mt, 2H : 1H du CH₂O 5δ et 1H du CH₂O 2δ) ; 4,49 (dd, J = 12 et 3 Hz, 1H : l'autre H du CH₂O 5δ) ; 4,52 (dd, J = 12 et 2,5 Hz, 1H : l'autre H du CH₂O 2δ) ; 4,70 (d, J = 8,5 Hz, 1H : OH en 2β) ; 4,93 (d, J = 6,5 Hz, 1H : OH en 5β) ; 5,02 (d large, J = 6 Hz, 1H : CH 2α) ; 5,21 (d, J = 6,5 Hz, 1H : OH en 2γ) ; 5,24 (d, J = 6,5 Hz, 1H : OH en 5γ) ; 5,44 (d, J = 6 Hz, 1H : OH en 2α) ; 7,49 (d large, J = 8 Hz, 4H : H aromatiques en méta du CO) ; 8,00 (d large, J = 8 Hz, 4H : H aromatiques en ortho du CO) ; 8,46 (s large, 1H : =CH en 6) ; 8,70 (s, 1H : =CH en 3).

### Exemple 48

### 4, 4'-O, O-di(4-(N,N-diméthyl-aminométhylène)-benzoyl)-2-[(1R, 2S, 3R) (1, 2, 3, 4-tétrahydroxybutyl)] 5-[(2'S, 3'R) (2', 3', 4'-trihydroxybutyl)] pyrazine

Spectre de R.M.N. ¹ H (500 MHz, (CD₃)₂SO d6, δ en ppm) : 2,15 (s, 12H : NCH₃) ; 2,81 et 3,20 (2 dd, respectivement J = 14 et 9,5 Hz et J = 14 et 3 Hz, 1H chacun : CH₂ 5α) ; 3,48 (s, 4H : ArCH₂N) ; de 3,65 à 3,80 (mt, 1H : CH 5γ) ; 3,71 (t, J = 8,5 Hz, 1H : CH 2β) ; 3,89 (mt, 1H : CH 5β) ; 4,00 (mt, 1H : CH 2γ) ; 4,27 (mt, 2H : 1H du CH₂O 5δ et 1H du CH₂O 2δ) ; 4,49 (dd, J = 12 et 3 Hz, 1H : l'autre H du CH₂O 5δ) ; 4,52 (dd, J = 12 et 3 Hz, 1H : l'autre H du CH₂O 2δ) ; 4,72 (d, J = 8,5 Hz, 1H : OH en 2β) ; 4,95 (d, J = 7 Hz, 1H : OH en 5β) ; 5,02 (d large, J = 6 Hz, 1H : CH 2α) ; 5,23 (d, J = 7 Hz, 1H : OH en 2γ) ; 5,26 (d, J = 7 Hz, 1H : OH en 5γ) ; 5,43 (d, J = 6 Hz, 1H : OH en 2α) ; 7,45 (d, J = 8 Hz, 4H : H aromatiques en méta du CO) ; 7,99 (d large, J = 8 Hz, 4H : H aromatiques en ortho du CO) ; 8,47 (d, J = 1 Hz, 1H : =CH en 6) ; 8,70 (s large, 1H : =CH en 3).

### Exemple 49

### 4, 4'-O, O-di(N-phénylcarbamoyl)-2-[(1R, 2S, 3R) (1, 2, 3, 4-tétrahydroxybutyl)] 5-[(2'S, 3'R) (2', 3', 4'-trihydroxybutyl)] pyrazine

A une suspension de 400 mg de 2-[(1R, 2S, 3R) (1, 2, 3, 4-tétrahydroxybutyl)] 5-[(2'S, 3'R) (2', 3', 4'-trihydroxybutyl)] pyrazine dans 10 cm³ de pyridine anhydre est additionné, à une température voisine de 20°C sous atmosphère d'argon, 0,36 cm³ d'isocyanate de phényle. Après environ 22 heures d'agitation à une température voisine de 20°C, le milieu réactionnel est additionné de 10 cm³ d'un mélange constitué de méthanol-dichlorométhane (5-95 en volumes), puis purifié par chromatographie sous pression (180 kPa) sur un volume de 100 cm³ de gel de silice 60F254 Merck (0,063-0,040 mm) contenus dans une colonne de dismètre 2,5 cm, en éluant par un gradient d'élution constitué d'un mélange dichlorométhane-méthanol (90-10 en volumes) en recueillant des fractions de 20 cm³. Les fractions ne contenant que le produit cherché sont réunies et concentrées à sec sous pression réduite (environ 0,5 kPa) à une température voisine de 40°C. On obtient ainsi 280 mg d'un solide que l'on repurifie par chromatographie sous pression (180 kPa) sur un volume de 90 cm³ de gel de silice 60F254 Merck (0,063-0,040 mm) contenus dans une colonne de diamètre 3 cm, en éluant par un gradient d'élution constitué d'un mélange chloroforme-méthanol-ammoniaque (80-20-0 puis 80-20-1 en volumes). Les fractions ne contenant que le produit cherché sont réunies et concentrées à sec sous pression réduite (environ 0,5 kPa) à une température voisine de 40°C. On obtient ainsi 2 fractions (solides) contenant la 4, 4'-O, O-di(N-phénylcarbamoyl)-2-[(1R, 2S, 3R) (1, 2, 3, 4-tétrahydroxybutyl)] 5-[(2'S, 3'R) (2', 3', 4'-trihydroxybutyl)] pyrazine. Ces deux solides sont lavés avec de l'eau distillée puis avec de l'acétate d'éthyle, et enfin séchés sous pression réduite (environ 1 kPa) à une température voisine de 40°C. On obtient ainsi 47,4 mg et 65,2 mg de 4, 4'-O, O-di(N-phénylcarbamoyl)-2-[(1R, 2S, 3R) (1, 2, 3, 4-tétrahydroxybutyl)] 5-[(2'S, 3'R) (2', 3',4'-trihydroxybutyl)] pyrazine sous forme d'une poudre blanche.

Le produit obtenu possède les caractéristiques suivantes :

Spectre de R.M.N. ¹ H (400 MHz, (CD₃)₂SO d6, δ en ppm) : 2,79 et 3,16 (respectivement dd et d large, J = 14 et 9 Hz et J = 14 Hz, 1H chacun : CH₂ 5α) ; 3,62 (mt, 1H : CH 5γ) ; 3,70 (t large, J = 8 Hz, 1H : CH 2β) ; de 3,80 à 4,00 (mt, 2H : CH 5β et CH 2γ) ; de 4,05 à 4,20 (mt, 2H : 1H du CH₂O 5δ et 1H du CH₂O 2δ) ; de 4,30 à 4,45 (mt, 2H chacun : l'autre H du CH₂O 5δ et l'autre du CH₂O 2δ) ; 4,66 (d, J = 8 Hz, 1H : OH en 2β) ; 4,89 (d, J = 6,5 Hz, 1H : OH en 5β) ; 5,00 (d large, J = 5,5 Hz, 1H : CH 2α) ; 5,08 (d, J = 6 Hz, 1H : OH en 2γ) ; 5,13 (d, J = 6 Hz, 1H : OH en 5γ) ; 5,44 (d, J = 5,5 Hz, 1H : OH en 2α) ; 6,98 (t large, J = 7,5 Hz, 2H : H aromatiques en para du NHCO) ; 7,27 (t large, 4H : H aromatiques en méta du NHCO) ; 7,49 (d large, J = 7,5 Hz, 4H : H aromatiques en ortho du NHCO) ; 8,46 (s large, 1H : =CH en 6) ; 8,68 (s large, 1H : =CH en 3) ; 9,63 et 9,66 (2 s larges, 1H chacun : NHCO).

### Exemple 50

### 4, 4'-O, O-di(N-benzylcarbamoyl)-2-[(1R, 2S, 3R) (1, 2, 3, 4-tétrahydroxybutyl)] 5-[(2'S, 3'R) (2', 3', 4'-trihydroxybutyl)]pyrazine

A une suspension de 200 mg de 2-[(1R, 2S, 3R) (1, 2, 3, 4-tétrahydroxybutyl)] 5-[(2'S, 3'R) (2', 3', 4'-trihydroxybutyl)] pyrazine dans 5 cm³ de pyridine anhydre est additionné, à une température voisine de 20°C sous atmosphère d'argon, 0,207 cm³ diisocyanate de benzyle. Le milieu réactionnel est agité pendant environ 1 heure à une température voisine de 50°C. Après refroidissement du milieu réactionnel jusqu'à une température voisine de 20°C, on ajoute 5 cm³ d'un mélange constitué de méthanol-dichlorométhane (5-95 en volumes). L'insoluble formé est filtré sur verre fritté, lavé à l'eau, à l'acétate d'éthyle, puis séché sous pression réduite (environ 0,5 kPa) à une température voisine de 40°C. On obtient ainsi 39 mg de 4, 4'-O, O-di(N-benzylcarbamoyl)-2-[(1R, 2S, 3R) (1, 2, 3, 4-tétrahydroxybutyl)] 5-[(2'S, 3'R) (2', 3', 4'-trihydroxybutyl)]pyrazine.

Le produit obtenu possède les caractéristiques suivantes :

Spectre de R.M.N. ¹ H (400 MHz, (CD₃)₂SO d6, δ en ppm) : 2,80 et 3,13 (2 dd, respectivement J = 14 et 9,5 Hz et J = 14 et 3 Hz, 1H chacun : CH₂ 5α) ; 3,58 (mt, 1H : CH 5γ) ; 3,67 (t large, J = 8 Hz, 1H : CH 2β) ; de 3,80 à 3,90 (mt, 2H : CH 5β et CH 2γ) ; 4,00 et 4,24 (2 dd, respectivement J = 12 et 7,5 Hz et J = 12 et 3 Hz, 1H chacun : CH₂O 5δ) ; 4,03 et 4,30 (2 dd, respectivement J = 12 et 7,5 Hz et J = 12 et 2,5 Hz, 1H chacun : CH₂O 2δ) ; 4,22 (d, J = 6 Hz, 4H : NCH₂Ar) ; 4,47 (d, J = 8 Hz, 1H : OH en 2β) ; 4,71 (d, J = 6,5 Hz, 1H : OH en 5β) ; 4,86 (d, J = 6 Hz, 1H : OH en 2γ) ; 4,90 (d, J = 6 Hz, 1H : OH en 5γ) ; 4,99 (s large, 1H : CH 2α) ; 5,25 (mf, 1H : OH en 2α) ; de 7,20 à 7,40 (mt, 10H : H aromatiques) ; de 7,50 à 7,65 (mt, 2H : les 2 NHCO) ; 8,44 (d, J = 1 Hz, 1H : =CH en 6) ; 8,68 (s large, 1H : =CH en 3).

### Exemple 51

### 1, 1', 2, 2', 3, 3', 4, 4'-O, O, O, O, O, O, O, O-octabenzoyl-2-[(1R, 2S, 3R) (1, 2, 3, 4-tétrahydroxybutyl)] 5-[(1'R, 2'S, 3'R) (1', 2', 3', 4'-tétrahydroxybutyl)] pyrazine

A 20 mg de 2-[(1R, 2S, 3R) (1, 2, 3, 4-tétrahydroxybutyl)] 6-[(1'R, 2'S, 3'R) (1', 2', 3', 4'-tétrahydroxybutyl)] pyrazine en suspension dans 0,5 cm³ de anhydre, est additionné, goutte à goutte à une température voisine de 20 °C sous atmosphère d'argon, 0,124 cm³ de chlorure de benzoyle. Le mélange réactionnel est agité 6 heures à une température voisine de 20 °C. Le solvant est évaporé sous flux d'air à une température voisine de 20°C pendant environ 16 heures. Le résidu obtenu est purifié par chromatographie préparative sur 2 plaques de gel de silice 60F254 Merck (épaisseur = 0,5 mm, 20x20 cm) en éluant par un mélange dichlorométhane-méthanol (98-2 en volumes). La fraction ne contenant que le produit cherché est extraite par un mélange dichlorométhane-méthanol (85-15 en volumes), filtrée sur verre fritté puis concentrée à sec sous pression réduite (0,5 kPa) à une température voisine de 40°C. On obtient ainsi 63,5 mg de 1, 1', 2, 2', 3, 3', 4, 4'-O, O, O, O, O, O, O, O-octabenzoyl-2-[(1R, 2S, 3R) (1, 2, 3, 4-tétrahydroxybutyl)] 5-[(1'R, 2'S, 3'R) (1', 2', 3', 4'-tétrahydroxybutyl)] pyrazine sous la forme d'une meringue blanche.

Le produit obtenu possède les caractéristiques suivantes :

Spectre de R.M.N. ¹H (400 MHz, (CD₃)₂SO d6, δ en ppm) : 4,60 et 4,82 (2 dd, respectivement J = 12,5 et 5,5 Hz et J = 12,5 et 2 Hz, 2H chacun : CH₂O δ) ; 5,86 (mt, 2H : CH γ) ; 6,18 (dd, J = 7,5 et 4 Hz, 2H : CH β) ; 6,50 (d, J = 4 Hz, 2H : CH α) ; de 7,30 à 8,00 (mt, 40H : H aromatiques) ; 8,78 (s, 2H : =CHN).

### Exemple 52

### 1, 2, 2', 3, 3', 4, 4'-O, O, O, O, O, O, O-heptabenzoyl-2-[(1R, 2S, 3R) (1, 2, 3,4-tétrahydroxybutyl)] 6-[(2'S, 3'R) (2', 3', 4'-trihydroxybutyl)] pyrazine

A 20 mg de 2-[(1R, 2S, 3R) (1, 2, 3, 4-tétrahydroxybutyl)] 6-[(2'S, 3'R) (2', 3', 4'-trihydroxybutyl)] pyrazine en suspension dans 0,5 cm³ de pyridine anhydre, est additionné, goutte à goutte à une température voisine de 20 °C sous atmosphère d'argon, 0,115 cm³ de chlorure de benzoyle. Le mélange réactionnel est agité 6 heures à une température voisine de 20 °C. Le solvant est évaporé sous flux d'air à une température voisine de 20°C pendant environ 16 heures. Le résidu obtenu est purifié par chromatographie préparative sur 2 plaques de gel de silice 60F254 Merck (épaisseur = 0,5 mm, 20x20 cm) en éluant par un mélange dichlorométhane-méthanol (98-2 en volumes). La fraction ne contenant que le produit cherché est extraite par un mélange dichlorométhane-méthanol (85-15 en volumes), filtrée sur verre fritté puis concentrée à sec sous pression réduite (0,5 kPa) à une température voisine de 40°C. On obtient ainsi 56,3 mg de 1, 2, 2', 3, 3', 4, 4'-O, O, O, O, O, O, O-heptabenzoyl-2-[(1R, 2S, 3R) (1, 2, 3, 4-tétrahydroxybutyl)] 6-[(2'S, 3'R) (2', 3', 4'-trihydroxybutyl)] pyrazine sous la forme d'une meringue blanche.

Le produit obtenu possède les caractéristiques suivantes :

Spectre de R.M.N. ¹H (400 MHz, (CD₃)₂SO d6, δ en ppm) : 3,41 et 3,51 (2 dd, respectivement J = 14 et 7,5 Hz et J = 14 et 4,5 Hz, 1H chacun : CH₂ 6α) ; 4,63 et 4,86 (2 mts, 2H chacun : CH₂O 2δ et CH₂O 6δ) ; 5,94 (mt, 2H : CH 6γ et CH 2γ) ; 6,02 (mt, 1H : CH 6β) ; 6,40 (dd, J = 7,5 et 4 Hz, 1H : CH 2β) ; 6,47 (d, J = 4 Hz, 1H : CH 2α) ; de 7,35 à 8,10 (mt, 35H : H aromatiques) ; 8,52 (s, 1H : =CH en 5) ; 8,60 (s, 1H : =CH en 3).

### Exemple 53

### 4, 4'-O, O-dibenzoyl-2-[(1R, 2S, 3R) (1, 2, 3, 4-tétrahydroxybutyl)] 6-[(2'S, 3'R) (2', 3', 4'-trihydroxybutyl)] pyrazine

A 20 mg de 2-[(1R, 2S, 3R) (1, 2, 3, 4-tétrahydroxybutyl)] 6-[(2'S, 3'R) (2', 3', 4'-trihydroxybutyl)] pyrazine en suspension dans 0,5 cm³ de pyridine anhydre, est additionné, goutte à goutte à une température voisine de 20 °C sous atmosphère d'argon, 0,0191 cm³ de chlorure de benzoyle. Le mélange réactionnel est agité 22 heures à une température voisine de 20 °C. Le solvant est évaporé sous flux d'air à une température voisine de 20°C pendant environ 72 heures. Le résidu obtenu est purifié par chromatographie préparative sur 2 plaques de gel de silice 60F254 Merck (épaisseur = 0,5 mm, 20x20 cm) en éluant par un mélange dichlorométhane-méthanol (90-10 en volumes). La fraction ne contenant que le produit cherché est extraite par un mélange dichlorométhane-méthanol (85-15 en volumes), filtrée sur verre fritté puis concentrée à sec sous pression réduite (0,5 kPa) à une température voisine de 40°C. On obtient ainsi 12 mg de 1, 2, 2', 3, 3', 4, 4'-O, O, O, O, O, O, O-heptabenzoyl-2-[(1R, 2S, 3R) (1, 2, 3, 4-tétrahydroxybutyl)] 6-[(2'S, 3'R) (2', 3', 4'-trihydroxybutyl)] pyrazine sous la forme d'une laque incolore.

Le produit obtenu possède les caractéristiques suivantes :

Spectre de R.M.N. ¹H (400 MHz, (CD₃)₂SO d6, δ en ppm) : 2,81 et 3,21 (2 dd, respectivement J = 14 et 9,5 Hz et J = 14 et 3 Hz, 1H chacun : CH₂ 6α) ; 3,72 (mt, 1H : CH 6γ) ; 3,75 (t large, J = 8 Hz, 1H : CH 2β) ; 3,89 (mt, 1H : CH 6β) ; 4,03 (mt, 1H : CH 2γ) ; 4,28 et 4,52 (2 dd, respectivement J = 12 et 2,5 Hz et J = 12 et 3 Hz , 1H chacun : CH₂O 6δ) ; 4,30 et 4,56 (2 dd, respectivement J = 12 et 2,5 Hz et J = 12 et 3 Hz , 1H chacun : CH₂O 2δ) ; 4,74 (d, J = 8 Hz, 1H : OH en 2β) ; 4,96 (d, J = 7 Hz, 1H : OH en 6β) ; 5,04 (d large, J = 6 Hz, 1H : CH 2α) ; 5,31 (mt, 2H : OH en 2γ et OH en 6γ) ; 5,48 (d, J = 6 Hz, 1H : OH en 2α) ; 7,54 (mt, 4H : H aromatiques en méta du CO) ; 7,67 (mt, 2H : H aromatiques en para du CO) ; 8,05 (d, J = 8 Hz, 4H : H aromatiques en ortho du CO) ; 8,41 (s, 1H : =CH en 5) ; 8,61 (s, 1H : =CH en 3).

## Revendications

1. Médicaments contenant en tant que principe actif au moins un composé de formule générale (V), (VI), (VII) dans laquelle :
A - (V) est un composé de formule générale : 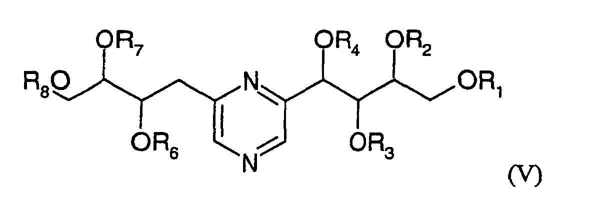 pour laquelle
(1) au moins un des substituants R₁, R₂, R₃, R₄, R₆, R₇, R₈ représente un radical choisi parmi la liste (L) suivante, les autres R₁, R₂, R₃, R₄, R₆, R₇, R₈ représentant un atome d'hydrogène :
(L) : acétyl, 2,2-diméthylpropanoyl, benzoyl, 4-diméthylamino-benzoyl, 4-aminobenzoyl, 4-benzyloxyloxybenzoyl, 4-hydroxybenzoyl, 4-méthoxybenzoyl, 4-méthylbenzoyl, 3-méthylbenzoyl, 4-fluorobenzoyl, 3-hydroxybenzoyl, 4-chlorobenzoyl, 4-méthoxycarbonylbenzoyl, (4-acétyl)benzoyl, 4-nitro-benzoyl, 3,5-dichloro-benzoyl, N,N-diisopropylaminométhylènebenzoyl, N,N-diéthylaminométhylènebenzoyl, pentanoyl, (2-acétyloxy)-benzoyl, phénylacétyl, formyl, butanoyl, méthoxy-acétyl, méthoxycarbonylpropanoyl, carboxypropanoyl, carboxybutanoyl, ethoxycarbonylpropènoyl, éthoxycarbonylbutanoyl, benzyloxycarbonylpropanoyl, benzyloxycarbonylbutanoyl, N(phényl)aminocarbonyle, N(benzyl)aminocarbonyle, 2-thiénylcarbonyle, 1-pipéridinylméthylènebenzoyl, N-morpholinylméthylènebenzoyl, N,N-diméthylaminométhylènebenzoyl, N-4méthylpipérazinylméthylènebenzoyl, N(hydroxy-2 éth-1)ylaminométhylènebenzoyl, N-carbamoylméthylaminométhylènebenzoyl, N-éthylaminométhylènebenzoyl, aminométhylènebenzoyl, N-(2-hydroxyéthyl) N-(méthyl) aminométhylènebenzoyl, 2-furanylcarbonyle, phénoxyacétyle, éthoxycarbonyle, N-phénylcarbamoyle, N-benzylcarbamoyle, 4-diméthylaminobutanoyl.
(1i) : soit un des substituants R₁ ou R₈ représente un radical choisi parmi la liste (L), et les autres R₁, R₂, R₃, R₄, R₆, R₇, R₈ représentent un atome d'hydrogène;
(1ii) : soit les 2 substituants R₁ et R₈ représentent chacun un radical identique choisi parmi la liste (L), et les autres R₂, R₃, R₄, R₆, R₇ représentent un atome d'hydrogène;
(1iii) : soit R₁, R₂, R₃, R₄, R₆, R₇, R₈ représentent chacun un radical identique choisi parmi la liste (L);
(2) soit R₁ et R₂ forment ensemble le groupe -CO-, et R₃, R₄, R₆, R₇, R₈ représentent un atome d'hydrogène;
(3) soit R₇ et R₈ forment ensemble le groupe -CO-, et R₁, R₂, R₃, R₄, R₆ représentent un atome d'hydrogène
(4) soit R₁ et R₂ d'une part et R₇ et R₈ d'autre part forment deux à deux le groupe -CO-et R₃, R₄, R₆ représentent des atomes d'hydrogène;
(5) soit R₁ et R₂, R₃ et R₄, R₇ et R₈ forment deux à deux le groupe -CO- et R₆ représente un atome d'hydrogène;
B - (VI) est un composé de formule générale : 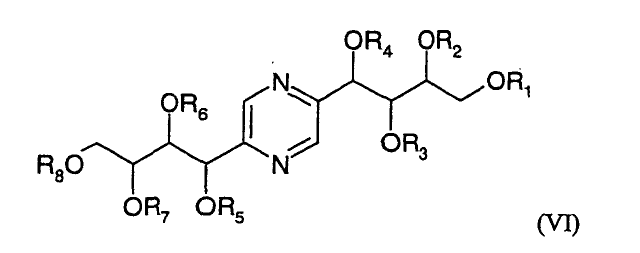 pour laquelle :
(1) au moins un des substituants R₁, R₂, R₃, R₄, R₅, R₆, R₇, R₈ représente un radical choisi parmi la liste (L) citée ci-dessus, et les autres substituants R₁, R₂, R₃, R₄, R₅, R₆, R₇, R₈ représentent un atome d'hydrogène;
(1i) : soit un des substituants R₁ ou R₈ représente un radical choisi parmi la liste (L), et les autres R₁, R₂, R₃, R₄, R₅, R₆, R₇, R₈ représentent un atome d'hydrogène;
(1ii) : soit les 2 substituants R₁ et R₈ représentent chacun un radical identique choisi parmi la liste (L), et les autres R₂, R₃, R₄, R₅, R₆, R₇ représentent un atome d'hydrogène;
(1iii) : soit R₁, R₂, R₃, R₄, R₅, R₆, R₇, R₈ représentent chacun un radical identique choisi parmi la liste (L),
(2) soit R₁ et R₂ forment ensemble le groupe -CO-, et R₃, R₄, R₅, R₆, R₇, R₈ représentent un atome d'hydrogène;
(3) soit R₇ et R₈ forment ensemble le groupe -CO-, et R₁, R₂, R₃, R₄, R₅, R₆ représentent un atome d'hydrogène
(4) soit R₁ et R₂ d'une part et R₇ et R₈ d'autre part forment deux à deux le groupe -CO-et R₃, R₄, R₅, R₆ représentent des atomes d'hydrogène;
(5) soit R₁ et R₂, R₃ et R₄, R₇ et R₈ forment deux à deux le groupe -CO- et R₅, R₆ représente un atome d'hydrogène;
(6) soit R₁ et R₂, R₃ et R₄, R₅ et R₆, R₇ et R₈ forment deux à deux le groupe -CO-
C - (VII) est un composé de formule générale : 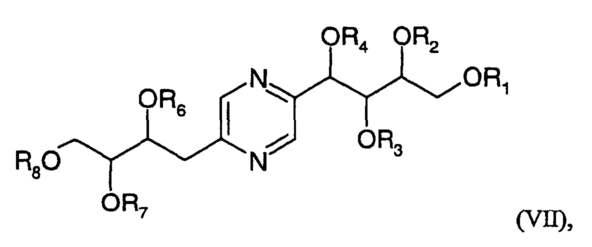 pour laquelle
(1) au moins un des substituants R₁, R₂, R₃, R₄, R₆, R₇, R₈ représente un radical choisi parmi la liste (L) citée ci-dessus, et les autres subsubstituants R₁, R₂, R₃, R₄, R₆, R₇, R₈ représentent un atome d'hydrogène;
(1i) : soit un des substituants R₁ ou R₈ représente un radical choisi parmi la liste (L), et les autres R₁, R₂, R₃, R₄, R₆, R₇, R₈ représentent un atome d'hydrogène;
(1ii) : soit les 2 substituants R₁ et R₈ représentent chacun un radical identique choisi parmi la liste (L), et les autres R₂, R₃, R₄, R₆, R₇ représentent un atome d'hydrogène;
(1iii) : soit R₁, R₂, R₃, R₄, R₆, R₇, R₈ représentent chacun un radical identique choisi parmi la liste (L);
(2) soit R₁ et R₂ forment ensemble le groupe -CO-, et R₃, R₄, R₆, R₇, R₈ représentent un atome d'hydrogène;
(3) soit R₇ et R₈ forment ensemble le groupe -CO-, et R₁, R₂, R₃, R₄, R₆ représentent un atome d'hydrogène
(4) soit R₁ et R₂ d'une part et R₇ et R₈ d'autre part forment deux à deux le groupe -CO-et R₃, R₄, R₆ représentent des atomes d'hydrogène;
(5) soit R₁ et R₂, R₃ et R₄, R₇ et R₈ forment deux à deux le groupe -CO- et R₆ représente un atome d'hydrogène.

2. Médicaments selon la revendication 1 pour lesquels les substituants de la liste (L) sont choisis parmi la liste (L') suivante :
(L') : acétyl, 2,2-diméthylpropanoyl, benzoyl, 4-diméthylamino-benzoyl, 4-benzyloxyloxybenzoyl, 4-hydroxybenzoyl, 4-méthoxybenzoyl, 4-méthylbenzoyl, 3-méthylbenzoyl, 4-fluorobenzoyl, 4-chlorobenzoyl, , 4-nitro-benzoyl, 3,5-dichlorobenzoyl, 4(N,N-diisopropylaminométhylène)benzoyl, 4(N,N-diéthylaminométhylène)benzoyl, 4(1-pipéridinylméthylène)benzoyl, 4(N-morpholinylméthylène)benzoyl, 4(N,N-diméthylaminométhylène)benzoyl, pentanoyl, (2-acétyloxy)-benzoyl, phénylacétyl, formyl, butanoyl, méthoxy-acétyl, méthoxycarbonylpropanoyl, carboxypropanoyl, carboxybutanoyl, ethoxycarbonylpropènoyl, éthoxycarbonylbutanoyl, benzyloxycarbonylpropanoyl, benzyloxycarbonylbutanoyl, N(phényl)aminocarbonyle, N(benzyl)aminocarbonyle, 2-thiénylcarbonyle.

3. Médicaments selon l'une quelconque des revendications précédentes pour lesquels les composés de formule (V), (VI), (VII) représentent respectivement les stéréoisomères de formule (VIII), (IX), (X) suivants : 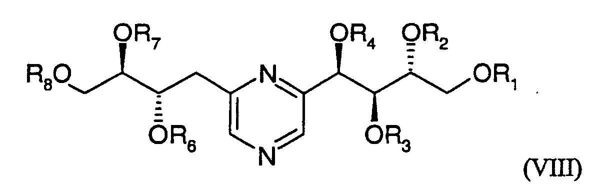 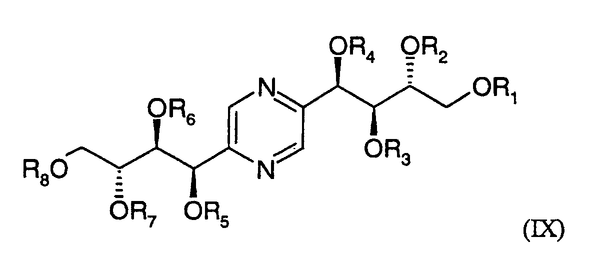 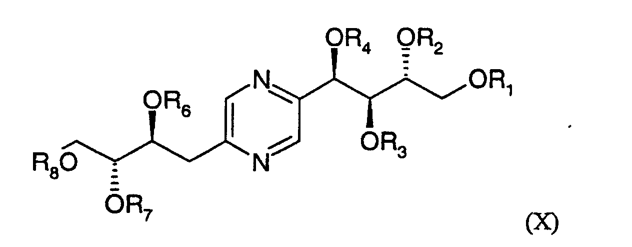 dans lesquelles les substituants R₁, R₂, R₃, R₄, R₅, R₆, R₇, R₈ ont les mêmes significations que dans les revendications 1 à 2,
ou les sels de tels composés avec un acide organique ou minéral.

4. Médicaments selon l'une quelconque des revendications précédentes pour lesquels les composés sont les composés de formule (X) tels que définis précédemment pour lesquels :
soit R₁ et R₂, R₃ et R₄, R₇ et R₈ forment deux à deux le groupe -CO- et R₆ représente un atome d'hydrogène;
soit R₁ et R₂, R₇ et R₈ forment deux à deux le groupe -CO- et R₃ , R₄, R₆ représentent un atome d'hydrogène;
soit ou R₁ ,R₂ ,R₆ ou R₄, R₆ ou R₁, R₃, R₄, R₆, R₈ ou R₂, R₃, R₄, R₆, ou R₁, R₃, R₄, R₆, R₇ ou R₂, ou R₁, R₂, R₈, ou R₁, R₂, R₆, R₇, R₈ ou R₆ représentent un radical benzoyl et les autres R₁, R₂, R₃, R₄, R₆, R₇, R₈ représentent un atome d'hydrogène;
soit R₂, R₆, R₇ représentent un radical benzoyl et R₃, R₄ forment ensemble le radical -CO- et R₁, R₈ représentent un atome d'hydrogène;
soit R₈ représente un radical pentanoyl ou acétyl et les autres R₁, R₂, R₃, R₄, R₆, R₇ représentent un atome d'hydrogène;
soit les 2 substituants R₁ et R₈ représentent chacun un radical identique choisi parmi les radicaux acétyl, 2,2-diméthylpropanoyl, benzoyl, 4-diméthylamino-benzoyl, 4-benzyloxyloxybenzoyl, 4-hydroxybenzoyl, 4-méthoxybenzoyl, 4-méthylbenzoyl, 3-méthylbenzoyl, 4-fluorobenzoyl, 4-chlorobenzoyl, 4-nitro-benzoyl, 3,5-dichlorobenzoyl, 4(N,N-diisopropylaminométhylène)benzoyl, 4(N,N-diéthylaminométhylène)benzoyl, 4(1-pipéridinylméthylène)benzoyl, 4(N-morpholinylméthylène)benzoyl, 4(N,N-diméthylaminométhylène)benzoyl, pentanoyl, (2-acétyloxy)-benzoyl, phénylacétyl, formyl, butanoyl, méthoxy-acétyl, méthoxycarbonylpropanoyl, carboxypropanoyl, carboxybutanoyl, ethoxycarbonylpropènoyl, éthoxycarbonylbutanoyl, benzyloxycarbonylpropanoyl, benzyloxycarbonylbutanoyl, N(phényl)aminocarbonyle, N(benzyl)aminocarbonyle, 2-thiénylcarbonyle;
et les autres R₂, R₃, R₄, R₆, R₇ représentent un atome d'hydrogène;
soit R₁, R₂, R₃, R₄, R₆, R₇, R₈ représentent chacun un radical identique choisi parmi les radicaux benzoyl, acétyl, 2,2-diméthylpropanoyl, (2-acétyloxy)-benzoyl, méthoxyacétyl, pentanoyl, formyl, méthoxycarbonylpropanoyl, carboxypropanoyl, éthoxycarbonylbutanoyl, carboxybutanoyl, éthoxycarbonylpropènoyl, benzyloxycarbonylpropanoyl, benzyloxybutanoyl;

5. Composés de formule (V), (VI), (VII) dans lesquels : 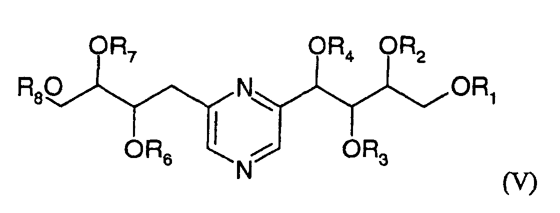 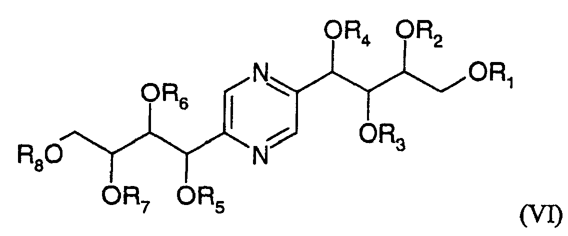 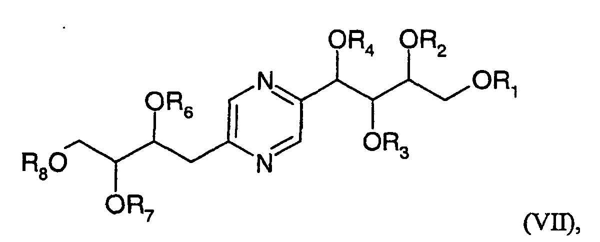
pour lesquels les substituants R₁, R₂, R₃, R₄, R₅, R₆, R₇, R₈ ont les mêmes significations que dans les revendications 1 à 4
à l'exception de
1,2,2',3,3',4,4'-O,O,O,O,O,O,O-heptaacétyle-2-[(1R,2S,3R)(1,2,3,4-tétrahydroxybutyl)] 5-[(2'S,3'R)(2',3',4'-trihydroxybutyl)]pyrazine,
1,1',2,2',3,3',4,4'-O,O,O,O,O,O,O,O-octaacétyle-2-[(1R,2S,3R)(1,2,3,4-tétrahydroxybutyl)] 5-[(1'R,2'S,3'R)(1',2',3',4'-tétrahydroxybutyl)]pyrazine,
1,1',2,2',3,3',4,4'-O,O,O,O,O,O,O,O-octabenzoyle-2-[(1R,2S,3R)(1,2,3,4-tétrahydroxybutyl)] 5-[(1R,2'S,3'R)(1',2',3',4'-tétrahydroxybutyl)]pyrazine
1,2,2',3,3',4,4'-O,O,O,O,O,O,O-heptaacétyle-2-[(1R,2S,3R)(1,2,3,4-tétrahydroxybutyl)] 6-[(2'S,3'R)(2',3',4'-trihydroxybutyl)]pyrazine
1,2,2',3,3',4,4'-O,O,O,O,O,O,O-heptaacétyle-2-[(1R,2S,3S)(1,2,3,4-tétrahydroxybutyl)] 6-[(2'S,3'S)(2',3',4'-trihydroxybutyl)]pyrazine,
ou leurs stéréoisomères ou les sels de tels composés avec un acide organique ou minéral.

6. Composés selon la revendication 5 pour lesquels les composés de formule (V), (VI), (VII) sont choisis parmi les stéréoisomères : 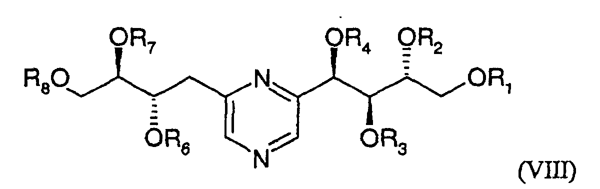 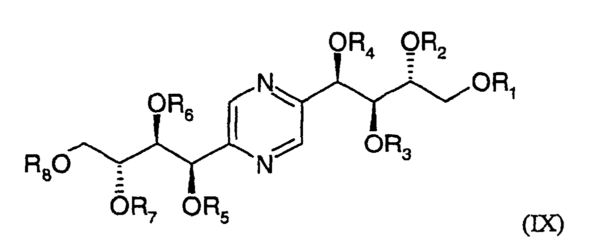 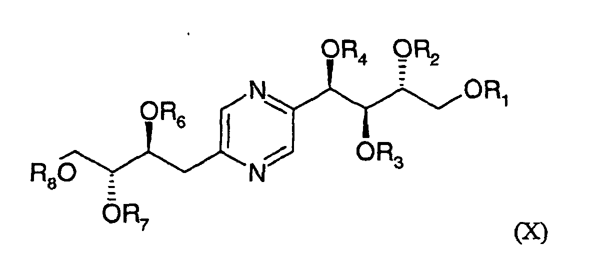
pour lesquels les substituants R_{1,} R_{2,} R_{3,} R_{4,} R_{5,} R_{6,} R_{7,} R₈ ont les mêmes significations que dans les revendications 1 à 5,
à l'exception de
1,2,2',3,3',4,4'-O,O,O,O,O,O,O-heptaacétyle-2-[(1R,2S,3R)(1,2,3,4-tétrahydroxybutyl)] 5-[(2'S,3'R)(2',3',4'-trihydroxybutyl)]pyrazine,
1,1',2,2',3,3',4,4'-O,O,O,O,O,O,O,O-octaacétyle-2-[(1R,2S,3R)(1,2,3,4-tétrahydroxybutyl)] 5-[(1'R,2'S,3'R)(1',2',3',4'-tétrahydroxybutyl)]pyrazine,
1,1',2,2',3,3',4,4'-O,O,O,O,O,O,O,O-octabenzoyle-2-[(1R,2S,3R)(1,2,3,4-tétrahydroxybutyl)] 5-[(1'R,2'S,3'R)(1',2',3',4'-tétrahydroxybutyl)]pyrazine
1,2,2',3,3',4,4'-O,O,O,O,O,O,O-heptaacétyle-2-[(1R,2S,3R)(1,2,3,4-tétrahydroxybutyl)] 6-[(2'S,3'R)(2',3',4'-trihydroxybutyl)]pyrazine
1,2,2',3,3',4,4'-O,O,O,O,O,O,O-heptaacétyle-2-[(1R,2S,3S)(1,2,3,4-tétrahydroxybutyl)] 6-[(2'S,3'S)(2',3',4'-trihydroxybutyl)]pyrazine,
ou les sels de tels composés avec un acide organique ou minéral.

7. Procédé de préparation des composés de formule (V), (VI), (VII) selon la revendication 5 **caractérisé en ce que** on fait réagir sur les composés de formule (XI), (XII), (XIII) 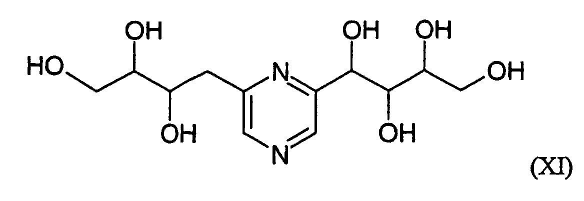 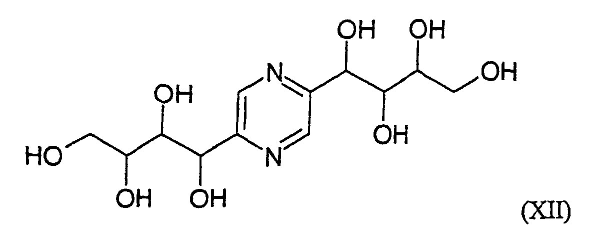 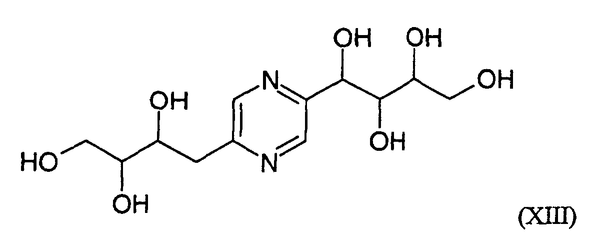
pour lesquels les fonctions hyroxyles sont éventuellement protégées par des groupes protecteurs,
- soit un composé de formule R-X (XIV) dans laquelle R représente de préférence les groupements -COR_{9 ,} -COOR_{10 ,} -CR₁₁R₁₂OCOR_{13 ,} -CR₁₁R₁₂OR_{13 ,} -CONR₁₄R₁₅ et X représente un atome d'halogène de préférence le chlore ou le brome
- soit un composé de formule (R₉CO)₂O (XV)
- soit un composé de formule R₁₄N=C=O (XVI)
- soit un composé de formule CR₁₁R₁₂(OR₁₃)₂ (XVII),
pour lesquels R₉, R₁₀, R₁₁, R₁₂, R₁₃, R₁₄, R₁₅ sont choisis parmi la liste (L) de la revendication 1,
suivi éventuellement d'une déprotection totale, ou suivi éventuellement d'une déprotection sélective et d'une ou plusieurs autres fonctionnalisations à l'aide d'un des réactifs de formule (XIV), (XV), (XVI), (XVII) identiques ou différents du premier, les étapes de fonctionnalisation et de déprotection pouvant être répétées plusieurs fois, étant entendu que les réactifs de formule (XIV), (XV), (XVI) et (XVII) peuvent être identiques ou différents à chaque fonctionnalisation,
isole le produit et le transforme éventuellement en sel avec un acide minéral ou organique.

8. Utilisation des composés de formule générale (V), (VI), (VII) 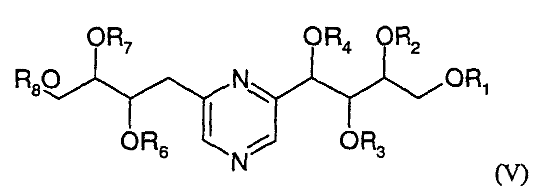 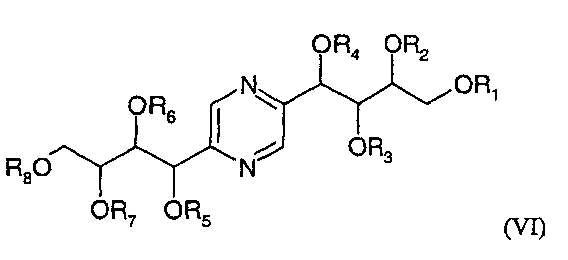 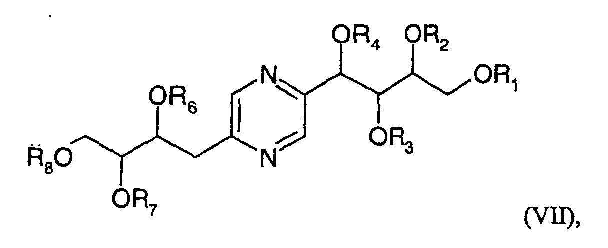 dans laquelle
R₁, R₂, R₃, R₄, R₅, R₆, R₇ et R₈ ont les mêmes significations que dans la revendication 1 pour 1a préparation de compositions pharmaceutiques utiles pour le traitement ou la prévention du diabète et les complications du diabète.

## Patentansprüche

1. Arzneimittel, enthaltend als Wirkstoff zumindest eine Verbindung der allgemeinen Formel (V), (VI), (VII), worin
A - (V) eine Verbindung der allgemeinen Formel 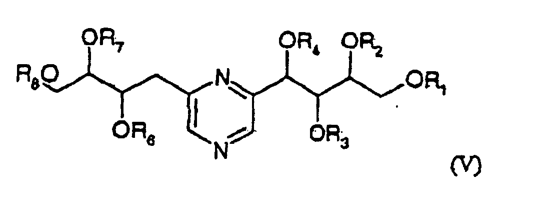 ist, worin
(1) zumindest einer der Substituenten R₁, R₂, R₃, R₄, R₆, R₇, R₈ einen Rest ausgewählt unter der folgenden Liste (L) bedeutet, wobei die anderen R₁, R₂, R₃, R₄, R₆, R₇, R₈ ein Wasserstoffatom bedeuten:
(L) Acetyl, 2,2-Dimethylpropanoyl, Benzoyl, 4-Dimethylaminobenzoyl, 4-Aminobenzoyl, 4-Benzyloxyloxybenzoyl, 4-Hydroxybenzoyl, 4-Methoxybenzoyl, 4-Methylbenzoyl, 3-Methylbenzoyl, 4-Fluorbenzoyl, 3-Hydroxybenzoyl, 4-Chlorbenzoyl, 4-Methoxycarbonylbenzoyl, (4-Acetyl)-benzoyl, 4-Nitrobenzoyl, 3,5-Dichlorbenzoyl, N,N-Diisopropylaminomethylenbenzoyl, N,N-Diethylaminomethylenbenzoyl, Pentanoyl, (2-Acetyloxy)-benzoyl, Phenylacetyl, Formyl, Butanoyl, Methoxyacetyl, Methoxycarbonylpropanoyl, Carboxypropanoyl, Carboxybutanoyl, Ethoxycarbonylpropenoyl, Ethoxycarbonylbutanoyl, Benzyloxycarbonylpropanoyl, Benzyloxycarbonylbutanoyl, N-(Phenyl)-aminocarbonyl, N-(Benzyl)-aminocarbonyl, 2-Thienylcarbonyl, 1-Piperidinylmethylenbenzoyl, N-Morpholinylmethylenbenzoyl, N,N-Dimethylaminomethylenbenzoyl, N-4-Methylpiperazinylmethylenbenzoyl, N-(Hydroxy-2-eth-1-)-yl-aminomethylenbenzoyl, N-Carbamoylmethylaminomethylenbenzoyl, N-Ethylaminomethylenbenzoyl, Aminomethylenbenzoyl, N-(2-Hydroxyethyl)-N-(methyl)-aminomethylenbenzoyl, 2-Furanylcarbonyl, Phenoxyacetyl, Ethoxycarbonyl, N-Phenylcarbamoyl, N-Benzylcarbamoyl, 4-Dimethylaminobutanoyl,
(1i): oder einer der Substituenten R₁ oder R₈ einen Rest ausgewählt aus der Liste (L) bedeutet, und die anderen R₁, R₂, R₃, R₄, R₆, R₇, R₈ ein Wasserstoffatom bedeuten,
(1ii): oder die 2 Substituenten R₁ und R₈ jeweils einen identischen Rest ausgewählt aus der Liste (L) bedeuten, und die anderen R₂, R₃, R₄, R₆, R₇ ein Wasserstoffatom bedeuten,
(1iii): oder R₁, R₂, R₃, R₄, R₆, R₇, R₈ jeweils einen identischen Rest ausgewählt aus der Liste (L) bedeuten;
(2) oder R₁ und R₂ gemeinsam die Gruppe -CO- bilden, und R₃, R₄, R₆, R₇, R₈ ein Wasserstoffatom bedeuten,
(3) oder R₇ und R₈ gemeinsam die Gruppe -CO- bilden, und R₁, R₂, R₃, R₄, R₆ ein Wasserstoffatom bedeuten,
(4) oder R₁ und R₂ einesteils und R₇ und R₈ andernteils jeweils zu zweien die Gruppe -CO- bilden und R₃, R₄, R₆ Wasserstoffatome bedeuten,
(5) oder R₁ und R₂, R₃ und R₄, R₇ und R₈ jeweils zu zweien die Gruppe -CO- bilden, und R₆ ein Wasserstoffatom bedeutet,
(B) - (VI) eine Verbindung der allgemeinen Formel 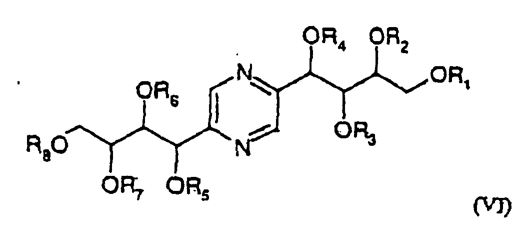 ist, worin
(1) zumindest einer der Substituenten R₁, R₂, R₃, R₄, R₅, R₆, R₇, R₈ einen Rest ausgewählt unter der vorstehenden Liste (L) bedeutet, und die anderen R₁, R₂, R₃, R₄, R₅, R₆, R₇, R₈ ein Wasserstoffatom bedeuten:
(1i): oder einer der Substituenten R₁ oder R₈ einen Rest ausgewählt aus der Liste (L) bedeutet, und die anderen R₁, R₂, R₃, R₄, R₅, R₆, R₇, R₈ ein Wasserstoffatom bedeuten,
(1ii): oder die 2 Substituenten R₁ und R₈ jeweils einen identischen Rest ausgewählt aus der Liste (L) bedeuten, und die anderen R₂, R₃, R₄, R₅, R₆, R₇ ein Wasserstoffatom bedeuten,
(1iii): oder R₁, R₂, R₃, R₄, R₅, R₆, R₇, R₈ jeweils einen identischen Rest ausgewählt aus der Liste (L) bedeuten;
(2) oder R₁ und R₂ gemeinsam die Gruppe -CO- bilden, und R₃, R₄, R₅, R₆, R₇, R₈ ein Wasserstoffatom bedeuten,
(3) oder R₇ und R₈ gemeinsam die Gruppe -CO- bilden, und R₁, R₂, R₃, R₄, R₅, R₆ ein Wasserstoffatom bedeuten,
(4) oder R₁ und R₂ einesteils und R₇ und R₈ andernteils jeweils zu zweien die Gruppe -CO- bilden und R₃, R₄, R₅, R₆ Wasserstoffatome bedeuten,
(5) oder R₁ und R₂, R₃ und R₄, R₇ und R₈ jeweils zu zweien die Gruppe -CO- bilden, und R₅, R₆ ein Wasserstoffatom bedeuten,
(6) oder R₁ und R₂, R₃ und R₄, R₅ und R₆, R₇ und R₈ jeweils zu zweien die Gruppe -CO- bilden,
C - (VII) eine Verbindung der allgemeinen Formel 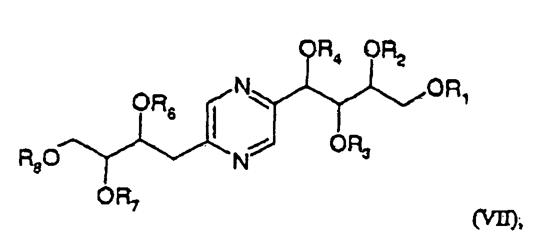 ist, worin
(1) zumindest einer der Substituenten R₁, R₂, R₃, R₄,R₆, R₇, R₈ einen Rest ausgewählt unter der vorstehenden Liste (L) bedeutet, und die anderen Sub-Substituenten R₁, R₂, R₃, R₄, R₆, R₇, R₈ ein Wasserstoffatom bedeuten:
(1i): oder einer der Substituenten R₁ oder R₈ einen Rest ausgewählt aus der Liste (L) bedeutet, und die anderen R₁, R₂, R₃, R₄,R₆, R₇, R₈ ein Wasserstoffatom bedeuten,
(1ii): oder die 2 Substituenten R₁ und R₈ jeweils einen identischen Rest ausgewählt aus der Liste (L) bedeuten, und die anderen R₂, R₃, R₄, R₆, R₇ ein Wasserstoffatom bedeuten,
(1iii): oder R₁, R₂, R₃, R₄, R₆, R₇, R₈ jeweils einen identischen Rest ausgewählt aus der Liste (L) bedeuten;
(2) oder R₁ und R₂ gemeinsam die Gruppe -CO- bilden, und R₃, R₄, R₆, R₇, R₈ ein Wasserstoffatom bedeuten,
(3) oder R₇ und R₈ gemeinsam die Gruppe -CO- bilden, und R₁, R₂, R₃, R₄, R₆ ein Wasserstoffatom bedeuten,
(4) oder R₁ und R₂ einesteils und R₇ und R₈ andernteils jeweils zu zweien die Gruppe -CO- bilden und R₃, R₄, R₆ Wasserstoffatome bedeuten,
(5) oder R₁ und R₂, R₃ und R₄, R₇ und R₈ jeweils zu zweien die Gruppe -CO- bilden, und R₆ ein Wasserstoffatom bedeutet,

2. Arzneimittel gemäß Anspruch 1, worin die Substituenten der Liste (L) ausgewählt sind aus der folgenden Liste (L'):
(L'): Acetyl, 2,2-Dimethylpropanoyl, Benzoyl, 4-Dimethylaminobenzoyl, 4-Benzyloxyloxybenzoyl, 4-Hydroxybenzoyl, 4-Methoxybenzoyl, 4-Methylbenzoyl, 3-Methylbenzoyl, 4-Fluorbenzoyl, 4-Chlorbenzoyl, 4-Nitrobenzoyl, 3,5-Dichlorbenzoyl, 4-(N,N-Diisopropylaminomethylen)-benzoyl, 4-(N,N-Diethylaminomethylen)-benzoyl, 4-(1-Piperidinylmethylen)-benzoyl, 4-(N-Morpholinylmethylen)-benzoyl, 4-(N,N-Dimethylaminomethylen)-benzoyl, Pentanoyl, (2-Acetyloxy)-benzoyl, Phenylacetyl, Formyl, Butanoyl, Methoxyacetyl, Methoxycarbonylpropanoyl, Carboxypropanoyl, Carboxybutanoyl, Ethoxycarbonylpropenoyl, Ethoxycarbonylbutanoyl, Benzyloxycarbonylpropanoyl, Benzyloxycarbonylbutanoyl, N-(Phenyl)-aminocarbonyl, N-(Benzyl)-aminocarbonyl, 2-Thienylcarbonyl.

3. Arzneimittel gemäß einem der vorhergehenden Ansprüche, worin die Verbindungen der Formel (V), (VI) bzw. (VII) Stereoisomere der folgenden Formeln (VIII), (IX) bzw. (X) sind: 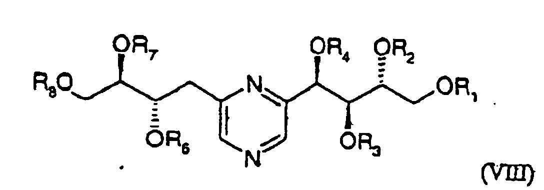 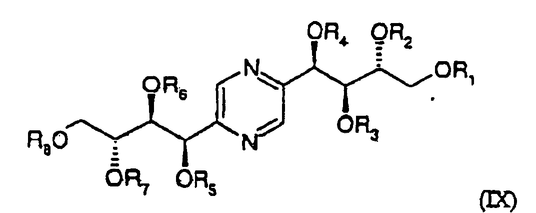 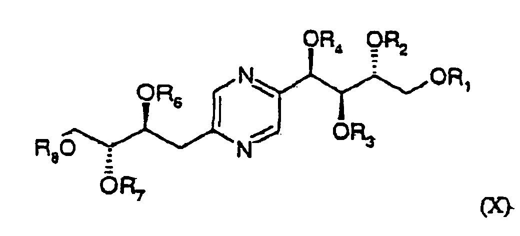 worin die Substituenten R₁, R₂, R₃, R₄, R₅, R₆, R₇, R₈ die in den Ansprüchen 1 bis 2 angegebenen Bedeutungen besitzen,
oder die Salze derartiger Verbindungen mit einer organischen oder Mineralsäure.

4. Arzneimittel gemäß einem der vorhergehenden Ansprüche, worin die Verbindungen Verbindungen der Formel (X), wie vorstehend definiert, sind, worin:
entweder R₁ und R₂, R₃ und R₄, R₇ und R₈ jeweils zu zweien die Gruppe -CO- bilden, und R₆ ein Wasserstoffatom bedeutet,
oder R₁ und R2, R₇ und R₈ jeweils zu zweien die Gruppe -CO- bilden, und R₃, R₄, R₆ ein Wasserstoffatom bedeuten,
oder entweder R₁, R₂, R₆ oder R₄, R₆ oder R₁, R₃, R₄, R₆, R₈ oder R₂, R₃, R₄, R₆ oder R₁, R₃, R₄, R₆, R₇ oder R₂, oder R₁, R₂, R₈ oder R₁, R₂, R₆, R₇, R₈ oder R₆ einen Benzoylrest bedeuten, und die anderen R₁, R₂, R₃, R₄, R₆, R₇, R₈ ein Wasserstoffatom bedeuten,
oder R₂, R₆, R₇ einen Benzoylrest bedeuten und R₃, R₄ gemeinsam den Rest -CObilden und R₁, R₈ ein Wasserstoffatom bedeuten,
oder R₈ einen Pentanoyl- oder Acetylrest bedeutet und die anderen R₁, R₂, R₃, R₄, R₆, R₇ ein Wasserstoffatom bedeuten,
oder die 2 Substituenten R₁ und R₈ jeweils einen identischen Rest bedeuten, ausgewählt unter den Resten Acetyl, 2,2-Dimethylpropanoyl, Benzoyl, 4-Dimethylaminobenzoyl, 4-Benzyloxyloxybenzoyl, 4-Hydroxybenzoyl, 4-Methoxybenzoyl, 4-Methylbenzoyl, 3-Methylbenzoyl, 4-Fluorbenzoyl, 4-Chlorbenzoyl, 4-Nitrobenzoyl, 3,5-Dichlorbenzoyl, 4-(N,N-Diisopropylaminomethylen)-benzoyl, 4-(N,N-Diethylaminomethylen)-benzoyl, 4-(1-Piperidinylmethylen)-benzoyl, 4-(N-Morpholinylmethylen)-benzoyl, 4-(N,N-Dimethylaminomethylen)-benzoyl, Pentanoyl, (2-Acetoxy)-benzoyl, Phenylacetyl, Formyl, Butanoyl, Methoxyacetyl, Methoxycarbonylpropanoyl, Carboxypropanoyl, Carboxybutanoyl, Ethoxycarbonylpropenoyl, Ethoxycarbonylbutanoyl, Benzyloxycarbonylpropanoyl, Benzyloxycarbonylbutanoyl, N-(Phenyl)-aminocarbonyl, N-(Benzyl)-aminocarbonyl, 2-Thienylcarbonyl,
und die anderen R₂, R₃, R₄, R₆, R₇ ein Wasserstoffatom bedeuten,
oder R₁, R₂, R₃, R₄, R₆, R₇, R₈ jeweils einen identischen Rest bedeuten, ausgewählt unter den Resten Benzoyl, Acetyl, 2,2-Dimethylpropanoyl, (2-Acetyloxy)-benzoyl, Methoxyacetyl, Pentanoyl, Formyl, Methoxycarbonylpropanoyl, Carboxypropanoyl, Ethoxycarbonylbutanoyl, Carboxybutanoyl, Ethoxycarbonylpropenoyl, Benzyloxycarbonylpropanoyl, Benzyloxybutanoyl.

5. Verbindungen der Formel (V), (VI), (VII), worin: 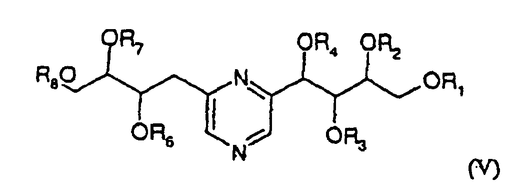 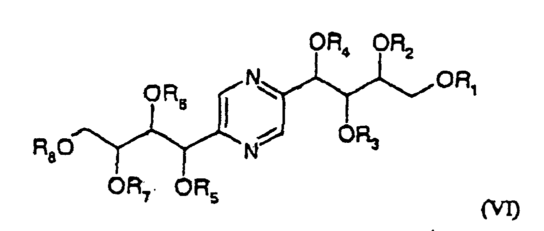 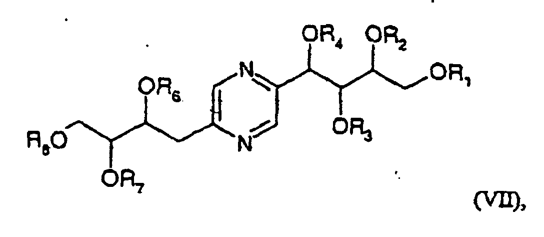 worin die Substituenten R₁, R₂, R₃, R₄, R₅, R₆, R₇, R₈ die gleichen Bedeutungen wie in den Ansprüchen 1 bis 4 besitzen, mit der Ausnahme von
1,2,2',3,3',4,4'-O,O,O,O,O,O,O-Heptaacetyl-2-[(1R,2S,3R)-(1,2,3,4-tetrahydroxybutyl)]-5-[(2'S,3'R)-(2',3',4'-trihydroxybutyl)]-pyrazin,
1,1',2,2',3,3',4,4'-O,O,O,O,O,O,O,O-Octaacetyl-2-[(1R,2S,3R)-(1,2,3,4-tetrahydroxybutyl)]-5-[(1'R,2'S,3'R)-(1',2',3',4'-tetrahydroxybutyl)]-pyrazin,
1,1',2,2',3,3',4,4'-O,O,O,O,O,O,O,O-Octabenzoyl-2-[(1R,2S,3R)-(1,2,3,4-tetrahydroxybutyl)]-5-[(1'R,2'S,3'R)-(1',2',3',4'-tetrahydroxybutyl)]-pyrazin,
1,2,2',3,3',4,4'-O,O,O,O,O,O,O-Heptaacetyl-2-[(1R,2S,3R)-(1,2,3,4-tetrahydroxy-butyl)]-6-[(2'S,3'R)-(2',3',4'-trihydroxybutyl)]-pyrazin,
1,2,2',3,3',4,4'-O,O,O,O,O,O,O-Heptaacetyl-2-[( 1R,2S,3S)-(1,2,3,4-tetrahydroxy-butyl)]-6-[(2'S,3'S)-(2',3',4'-trihydroxybutyl)]-pyrazin,
oder deren Stereoisomere oder die Salze derartiger Verbindungen mit einer organischen oder Mineralsäure.

6. Verbindungen gemäß Anspruch 5, worin die Verbindungen der Formel (V), (VI), (VII) ausgewählt sind unter den Stereoisomeren: 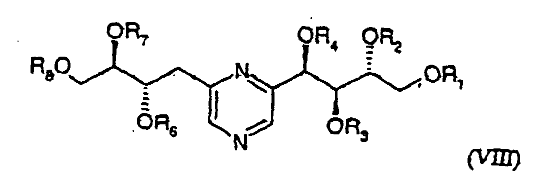 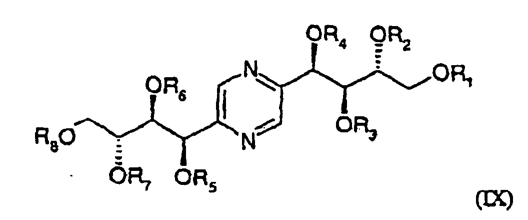 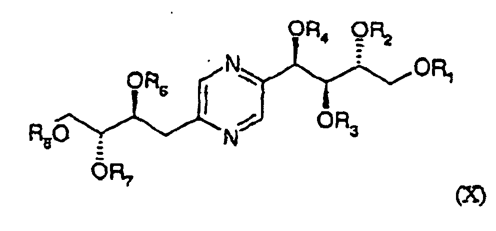 worin die Substituenten R₁, R₂, R₃, R₄, R₅, R₆, R₇, R₈ die gleichen Bedeutungen wie in den Ansprüchen 1 bis 5 besitzen, mit der Ausnahme von
1,2,2',3,3',4,4'-O,O,O,O,O,O,O-Heptaacetyl-2-[(1R,2S,3R)-(1,2,3,4-tetrahydroxybutyl)]-5-[(2'S,3'R)-(2',3',4'-trihydroxybutyl)]-pyrazin,
1,1',2,2',3,3',4,4'-O,O,O,O,O,O,O,O-Octaacetyl-2-[(1R,2S,3R)-(1,2,3,4-tetrahydroxybutyl)]-5-[(1'R,2'S,3'R)-(1',2',3',4'-tetrahydroxybutyl)]-pyrazin,
1,1',2,2',3,3',4,4'-O,O,O,O,O,O,O,O-Octabenzoyl-2-[(1R,2S,3R)-(1,2,3,4-tetrahydroxybutyl)]-5-[(1'R,2'S,3'R)-(1',2',3',4'-tetrahydroxybutyl)]-pyrazin,
1,2,2',3,3',4,4'-O,O,O,O,O,O,O-Heptaacetyl-2-[(1R,2S,3R)-(1,2,3,4-tetrahydroxy-butyl)]-6-[(2'S,3'R)-(2',3',4'-trihydroxybutyl)]-pyrazin,
1,2,2',3,3',4,4'-O,O,O,O,O,O,O-Heptaacetyl-2-[(1R,2S,3S)-(1,2,3,4-tetrahydroxy-butyl)]-6-[(2'S,3'S)-(2',3',4'-trihydroxybutyl)]-pyrazin,
oder deren Stereoisomere oder die Salze derartiger Verbindungen mit einer organischen oder Mineralsäure.

7. Verfahren zur Herstellung der Verbindungen der Formel (V), (VI), (VII) gemäß Anspruch 5, **dadurch gekennzeichnet, dass** man mit den Verbindungen der Formel (XI), (XII), (XIII) 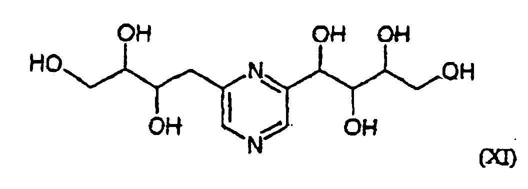 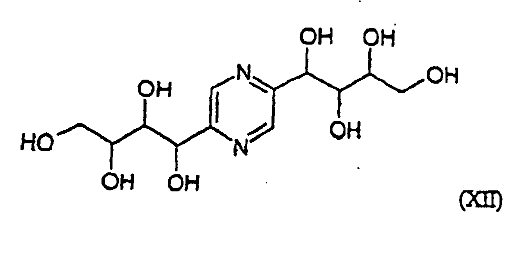 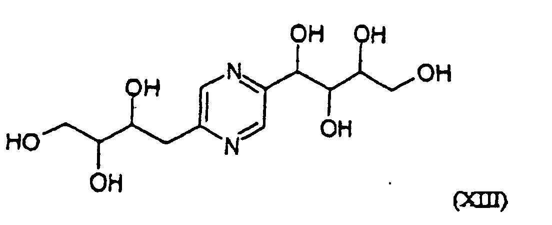 worin die
Hydroxylfunktionen gegebenenfalls durch Schutzgruppen geschützt sind, umsetzt,
- entweder eine Verbindung der Formel R-X (XIV), worin R vorzugsweise die Grup pen -COR₉, -COOR₁₀, -CR₁₁R₁₂OCOR₁₃, -CR₁₁R₁₂OR₁₃, -CONR₁₄R₁₅ darstellt und X für ein Halogenatom vorzugsweise Chlor oder Brom steht,
- oder eine Verbindung der Formel (R₉CO)₂O (XV),
- oder eine Verbindung der Formel R₁₄N=C=O (XVI),
- oder eine Verbindung der Formel CR₁₁R₁₂(OR₁₃)₂ (XVII),
worin R₉, R₁₀, R₁₁, R₁₂, R₁₃, R₁₄, R₁₅ ausgewählt sind aus der Liste (L) von Anspruch 1,
gegebenenfalls gefolgt von einer vollständigen Schutzgruppenabspaltung oder gegebenenfalls gefolgt von einer selektiven Schutzgruppenabspaltung und einer oder mehreren weiteren Funktionalisierungen mit Hilfe einer der Reaktionskomponenten der Formel (XIV), (XV), (XVI), (XVII), die identisch sind mit oder verschieden sind von der ersten, wobei die Stufen der Funktionalisierung und der Schutzgruppenabspaltung mehrfach wiederholt werden können, wobei die Reaktionskomponenten der Formel (XIV, (XV), (XVI) und (XVII) bei jeder Funktionalisierung identisch oder verschieden sein können,
das Produkt isoliert und es gegebenenfalls mit einer Mineral- oder organischen Säure in ein Salz überführt.

8. Verwendung der Verbindungen der allgemeinen Formel (V), (VI), (VII) 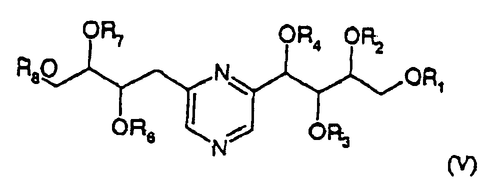 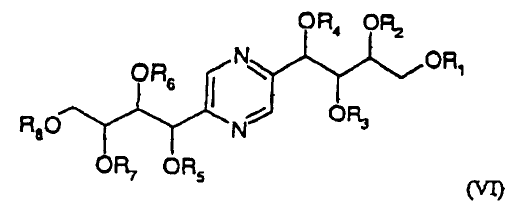 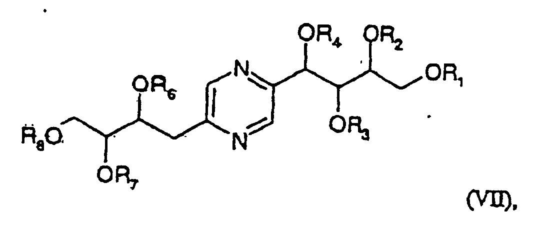 worin R₁, R₂, R₃, R₄, R₅, R₆, R₇ und R₈ die gleichen Bedeutungen wie in Anspruch 1 besitzen, für die Herstellung von pharmazeutischen Zusammensetzungen, die verwendbar sind bei der Behandlung oder der Prävention von Diabetes und den Komplikationen von Diabetes.

## Claims

1. Medicaments containing, as active ingredient, at least one compound of general formula (V), (VI), (VII) in which:
A - (V) is a compound of general formula: 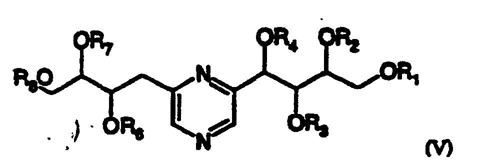 for which
(1) at least one of the substituents R₁, R₂, R₃, R₄, R₆, R₇, R₈ represents a radical chosen from the following list (L), the other substituents R₁, R₂, R₃, R₄, R₆, R₇, R₈ representing a hydrogen atom:
(L): acetyl, 2,2-dimethylpropanoyl, benzoyl, 4-dimethylaminobenzoyl, 4-aminobenzoyl, 4-benzyloxyloxybenzoyl, 4-hydroxybenzoyl, 4-methoxybenzoyl, 4-methylbenzoyl, 3-methylbenzoyl, 4-fluorobenzoyl, 3-hydroxybenzoyl, 4-chlorobenzoyl, 4-methoxycarbonylbenzoyl, (4-acetyl)benzoyl, 4-nitrobenzoyl, 3,5-dichlorobenzoyl, N,N-diisopropylaminomethylenebenzoyl, N,N-diethylaminomethylenebenzoyl, pentanoyl, (2-acetyloxy)benzoyl, phenylacetyl, formyl, butanoyl, methoxyacetyl, methoxycarbonylpropanoyl, carboxypropanoyl, carboxybutanoyl, ethoxycarbonylpropenoyl, ethoxycarbonylbutanoyl, benzyloxycarbonylpropanoyl, benzyloxycarbonylbutanoyl, N-(phenyl)aminocarbonyl, N-(benzyl)aminocarbonyl, 2-thienylcarbonyl, 1-piperidinylmethylenebenzoyl, N-morpholinylmethylenebenzoyl, N,N-dimethylaminomethylenebenzoyl, N-4-methylpiperazinylmethylenebenzoyl, N-(2-hydroxy-1-ethyl)-aminomethylenebenzoyl, N-carbamoylmethylaminomethylenebenzoyl, N-ethylaminomethylenebenzoyl, aminomethylenebenzoyl, N-(2-hydroxyethyl)-N-(methyl)aminomethylenebenzoyl, 2-furanylcarbonyl, phenoxyacetyl, ethoxycarbonyl, N-phenylcarbamoyl, N-benzylcarbamoyl, 4-dimethylaminobutanoyl;
(1i): either one of the substituents R₁ or R₈ represents a radical chosen from the list (L), and the other substituents R₁, R₂, R₃, R₄, R₆, R₇, R₈ represent a hydrogen atom;
(1ii): or the 2 substituents R₁ and R₈ each represent an identical radical chosen from the list (L), and the
other substituents R₂, R₃, R₄, R₆, R₇ represent a
hydrogen atom;
(1iii) : or R₁, R₂, R₃, R₄, R₆, R₇, R₈ each represent an identical radical) chosen from the list (L);
(2) or R₁ and R₂ together form the group -CO-, and R₃, R₄, R₆, R₇, R₈ represent a hydrogen atom;
(3) or R₇ and R₈ together form the group -CO-, and R₁, R₂, R₃, R₄, R₆ represent a hydrogen atom;
(4) or R₁ and R₂, on the one hand, and R₇ and R₈, on the other hand, form in pairs the group -CO- and R₃, R₄, R₆ represent hydrogen atoms;
(5) or R₁ and R₂, R₃ and R₄, R₇ and R₈ form in pairs the group -CO- and R₆ represents a hydrogen atom;
B - (VI) is a compound of general formula: 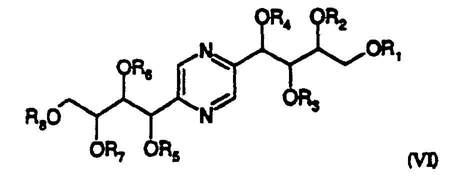 for which:
(1) at least one of the substituents R₁, R₂, R₃, R₄, R₅, R₆, R₇, R₈ represents a radical chosen from the abovementioned list (L), and the other substituents R₁, R₂, R₃, R₄, R₅, R₆, R₇, R₈ represent a hydrogen atom;
(1i): either one of the substituents R₁ or R₈ represents a radical chosen from the list (L), and the other substituents R₁, R₂, R₃, R₄, R₅, R₆, R₇, R₈ represent a hydrogen atom;
(1ii): or the 2 substituents R₁ and R₈ each represent an identical radical chosen from the list (L), and the other substituents R₂, R₃, R₄, R₅, R₆, R₇ represent a hydrogen atom;
(1iii) : or R₁, R₂, R₃, R₄, R₅, R₆, R₇, R₈ each represent an identical radical chosen from the list (L);
(2) or R₁ and R₂ together form the group -CO-, and R₃, R₄, R₅, R₆, R₇, R₈ represent a hydrogen atom;
(3) or R₇ and R₈ together form the group -CO-, and R₁, R₂, R₃, R₄, R₅, R₆ represent a hydrogen atom;
(4) or R₁ and R₂, on the one hand, and R₇ and R₈, on the other hand, form in pairs the group -CO- and R₃, R₄, R₅, R₆ represent hydrogen atoms;
(5) or R₁ and R₂, R₃ and R₄, R₇ and R₈ form in pairs the group -CO- and R₅) R₆ represents a hydrogen atom;
(6) or R₁ and R₂, R₃ and R₄, R₅ and R₆, R₇ and R₈ form in pairs the group -CO-;
C - (VII) is a compound of general formula: 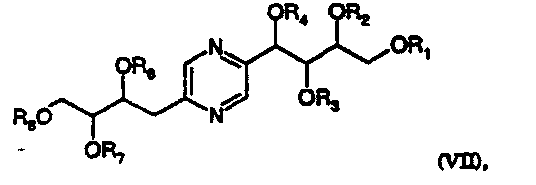 for which
(1) at least one of the substituents R₁, R₂, R₃, R₄, R₆, R₇, R₈ represents a radical chosen from the abovementioned list (L), and the other substituents R₁, R₂, R₃, R₄, R₆, R₇, R₈ represent a hydrogen atom;
(1i): either one of the substituents R₁ or R₈ represents a radical chosen from the list (L), and the other substituents R₁, R₂, R₃, R₄, R₆, R₇, R₈ represent a hydrogen atom;
(1ii): or the 2 substituents R₁ and R₈ each represent an identical radical chosen from the list (L), and the other substituents R₂, R₃, R₉, R₆, R₇ represent a hydrogen atom;
(1iii) : or R₁, R₂, R₃, R₄, R₆, R₇, R₈ each represent an identical radical chosen from the list (L);
(2) or R₁ and R₂ together f orm the group -CO-, and R₃, R₄, R₆, R₇, R₈ represent a hydrogen atom;
(3) or R₇ and R₈ together f orm the group -CO-, and R₁, R₂, R₃, R₄, R₆ represent a hydrogen atom;
(4) or R₁ and R₂, on the one hand, and R₇ and R₈, on the other hand, form in pairs the group -CO- and R₃, R₄, R₆ represent hydrogen atoms;
(5) or R₁ and R₂, R₃ and R₄, R₇ and R₈ form in pairs the group -CO- and R₆ represents a hydrogen atom.

2. Medicaments according to Claim 1, for which the substituents of the list (L) are chosen from the following list (L'):
(L'): acetyl, 2,2-dimethylpropanoyl, benzoyl, 4-dimethylaminobenzoyl, 4-benzyloxyloxybenzoyl, 4-hydroxybenzoyl, 4-methoxybenzoyl, 4-methylbenzoyl, 3-methylbenzoyl, 4-fluorobenzoyl, 4-chlorobenzoyl, 4-nitrobenzoyl, 3,5-dichlorobenzoyl, 4-(N,N-di-isopropylaminomethylene)benzoyl, 4-(N,N-diethylaminomethylene)benzoyl, 4-(1-piperidinylmethylene)benzoyl, 4-(N-morpholinylmethylene)benzoyl, 4-(N,N-dimethylaminomethylene)benzoyl, pentanoyl, (2-acetyloxy)-benzoyl, phenylacetyl, formyl, butanoyl, methoxyacetyl, methoxycarbonylpropanoyl, carboxypropanoyl, carboxybutanoyl, ethoxycarbonylpropenoyl, ethoxycarbonylbutanoyl, benzyloxycarbonylpropanoyl, benzyloxycarbonylbutanoyl, N-(phenyl)aminocarbonyl, N-(benzyl)-aminocarbonyl, 2-thienylcarbonyl.

3. Medicaments according to either one of the preceding claims, for which the compounds of formula (V), (VI), (VII) represent respectively the following stereoisomers of formula (VIII), (IX), (X): 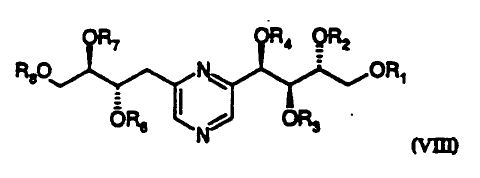 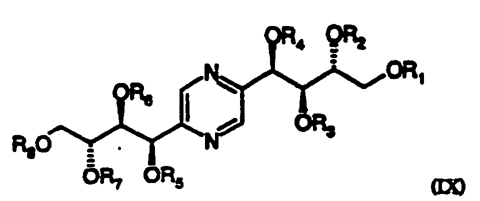 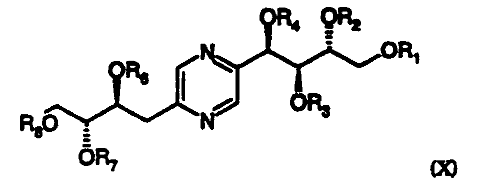 in which the substituents R₁, R₂, R₃, R₄, R₅, R₆, R₇, R₈ have the same meanings as in Claims 1 and 2, or the salts of such compounds with an organic or inorganic acid.

4. Medicaments according to any one of the preceding claims, for which the compounds are the compounds of formula (X) as defined above for which:
either R₁ and R₂, R₃ and R₄, R₇ and R₈ form in pairs the group -CO- and R₆ represents a hydrogen atom;
or R₁ and R₂, R₇ and R₈ form in pairs the group -CO- and R₃, R₄, R₆ represent a hydrogen atom;
either R₁, R₂, R₆ or R₄, R₆ or R₁, R₃, R₄, R₆, R₈ or R₂, R₃, R₄, R₆ 'or R₁, R₃, R₄, R₆, R₇ or R₂, or R₁, R₂, R₈, or R₁, R₂, R₆, R₇, R₈ or R₆ represent a benzoyl radical and
the other substituents R₁, R₂, R₃, R₄, R₆, R₇, R₈ represent a hydrogen atom;
or R₂, R₆, R₇ represent a benzoyl radical and R₃, R₄ together form the radical -CO- and R₁, R₈ represent a hydrogen atom;
or R₈ represents a pentanoyl or acetyl radical and the other substituents R₁, R₂, R₃, R₄, R₆, R₇ represent a hydrogen atom;
or the 2 substituents R₁ and R₈ each represent an identical radical chosen from the radicals acetyl, 2,2-dimethylpropanoyl, benzoyl, 4-dimethylaminobenzoyl, 4-benzyloxyloxybenzoyl, 4-hydroxybenzoyl, 4-methoxybenzoyl, 4-methylbenzoyl, 3-methylbenzoyl, 4-fluorobenzoyl, 4-chlorobenzoyl, 4-nitrobenzoyl, 3,5-dichlorobenzoyl, 4-(N,N-diisopropylaminomethylene)benzoyl, 4-(N,N-diethylaminomethylene)benzoyl, 4-(1-piperidinylmethylene)benzoyl, 4-(N-morpholinylmethylene)benzoyl, 4-(N,N-dimethylaminomethylene)benzoyl, pentanoyl, (2-acetyloxy)benzoyl, phenylacetyl, formyl, butanoyl, methoxyacetyl, methoxycarbonylpropanoyl, carboxypropanoyl, carboxybutanoyl, ethoxycarbonylpropenoyl, ethoxycarbonylbutanoyl, benzyloxycarbonylpropanoyl, benzyloxycarbonylbutanoyl, N-(phenyl)aminocarbonyl, N-(benzyl)aminocarbonyl, 2-thienylcarbonyl; and the other substitutents R₂, R₃, R₄, R₆, R₇ represent a hydrogen atom;
or R₁, R₂, R₃, R₄, R₆, R₇, R₈ each represent an identical radical chosen from the radicals benzoyl, acetyl, 2,2-dimethylpropanoyl, (2-acetyloxy)benzoyl, methoxyacetyl, pentanoyl, formyl, methoxycarbonylpropanoyl, carboxypropanoyl, ethoxycarbonylbutanoyl, carboxybutanoyl, ethoxycarbonylpropenoyl, benzyloxycarbonylpropanoyl, benzyloxybutanoyl.

5. Compounds of formula (V), (VI), (VII) in which: 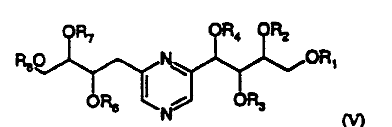 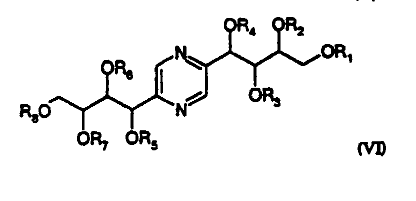 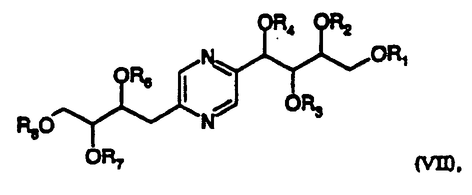 for which the substituents R₁, R₂, R₃, R₄, R₅, R₆, R₇, R₈ have the same meanings as in Claims 1 to 4,
with the exception of
1,2,2',3,3',4,4'-O,O,O,O,O,O,O-heptaacetyl-2-[(1R,2S,3R)(1,2,3,4-tetrahydroxybutyl)]-5-[(2'S,3'R)(2',3',4'-trihydroxybutyl)]pyrazine,
1,1',2,2',3,3',4,4'-O,O,O,O,O,O,O,O-octaacetyl-2-[(1R,2S,3R)(1,2,3,4-tetrahydroxybutyl)]-5- [(1'R,2'S,3'R)(1',2',3',4'-tetrahydroxybutyl)]pyrazine,
1,1',2,2',3,3',4,4'-O,O,O,O,O,O,O,O-octabenzoyl-2-[(1R,2S,3R)(1,2,3,4-tetrahydroxybutyl)]-5-[(1'R,2'S,3'R)(1',2',3',4'-tetrahydroxybutyl)]pyrazine
1,2,2',3,3',4,4'-O,O,O,O,O,O,O-heptaacetyl-2-[(1R,2S,3R)(1,2,3,4-tetrahydroxybutyl)]-6-[(2'S,3'R)(2',3',4'-trihydroxybutyl)]pyrazine
1,2,2',3,3',4,4'-O,O,O,O,O,O,O-heptaacetyl-2-[(1R,2S,3S)(1,2,3,4-tetrahydroxybutyl)]-6-[(2'S,3'S)(2',3',4'-trihydroxybutyl)]pyrazine,
or their stereoisomers or the salts of such compounds with an organic or inorganic acid.

6. Compounds according to Claim 5, for which the compounds of formula (V), (VI), (VII) are chosen from the stereoisomers: 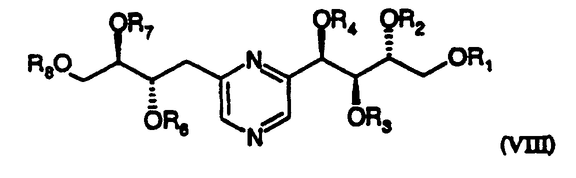 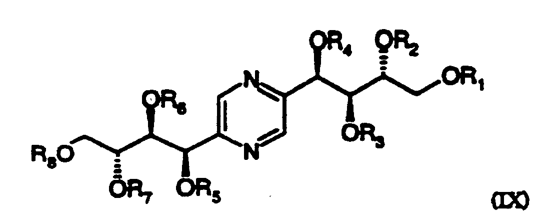 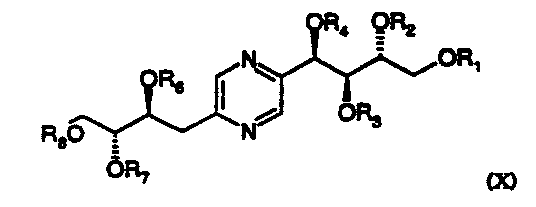 for which the substituents R₁, R₂, R₃, R₄, R₅, R₆, R₇, R₈ have the same meanings as in Claims 1 to 5,
with the exception of
1,2,2',3,3',4,4'-O,O,O,O,O,O,O-heptaacetyl-2-[(1R,2S,3R)(1,2,3,4-tetrahydroxybutyl)]-5- [(2'S,3'R)(2',3',4'-trihydroxybutyl)]pyrazine,
1,1',2,2',3,3',4,4'-O,O,O,O,O,O,O,O-octaacetyl-2-[(1R,2S,3R)(1,2,3,4-tetrahydroxybutyl)]-5- [(1'R,2'S,3'R) (1',2',3',4'-tetrahydroxybutyl)]pyrazine,
1, 1', 2 , 2' , 3 , 3' , 4 , 4'-O, O, O, O, O, O, O, O-octabenzoyl-2-[(1R,2S,3R)(1,2,3,4-tetrahydroxybutyl)]-5-[(1'R,2'S,3'R)(1',2',3',4'-tetrahydroxybutyl)]pyrazine
1,2,2',3,3',4,4'-O,O,O,O,O,O,O-heptaacetyl-2-[(1R,2S,3R)(1,2,3,4-tetrahydroxybutyl)]-6- [(2'S,3'R)(2',3',4'-trihydroxybutyl)]pyrazine
1, 2, 2', 3, 3', 4, 4'-O, O, O, O, O, O, O-heptaacetyl-2-[(1R,2S,3S)(1,2,3,4-tetrahydroxybutyl)]-6-[(2'S,3'S)(2',3',4'-trihydroxybutyl)]pyrazine,
or the salts of such compounds with an organic or inorganic acid.

7. Process for preparing the compounds of formula (V), (VI), (VII) according to Claim 5, **characterized in that** the compounds of formula (XI), (XII), (XIII) 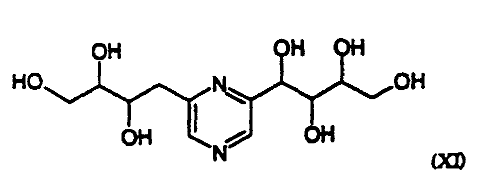 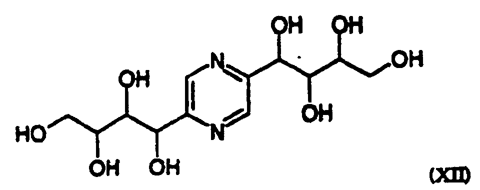 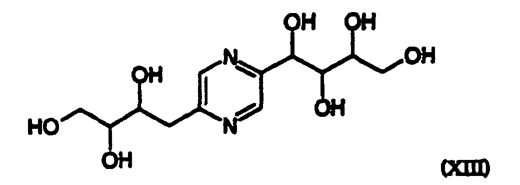 for which the hydroxyl functional groups are optionally protected with protecting groups, are reacted
- either with a compound of formula R-X (XIV) in which R preferably represents the groups -COR₉, -COOR₁₀,
- CR₁₁R₁₂OCOR₁₃, -CR₁₁R₁₂OR₁₃, -CONR₁₄R₁₅ and X represents a halogen atom, preferably chlorine or bromine
- or with a compound of formula (R₉CO)₂O (XV)
- or with a compound of formula R₁₄N=C=O (XVI)
- or with a compound of formula CR₁₁R₁₂(OR₁₃)₂ (XVII),
for which R₉, R₁₀, R₁₁, R₁₂, R₁₃, R₁₄, R₁₅ are chosen from list (L) in Claim 1,
optionally followed by complete deprotection, or optionally followed by selective deprotection and by one or more other functionalizations with the aid of one of the reagents of formula (XIV), (XV), (XVI), (XVII) which are identical to or different from the first, it being possible to repeat the functionalization and deprotection steps several times, it being understood that the reagents of formula (XIV), (XV), (XVI) and (XVII) may be identical or different on each functionalization,
the product is isolated and optionally converted to a salt with an inorganic or organic acid.

8. Use of the compounds of general formula (V), (VI), (VII) 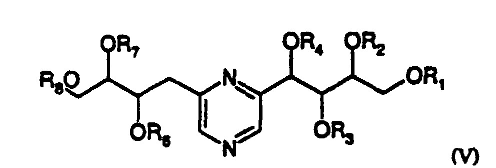 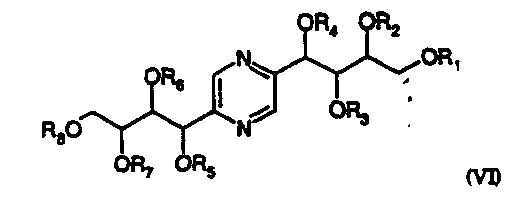 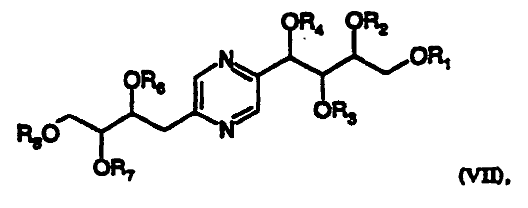 in which
R₁, R₂, R₃, R₄, R₅, R₆, R₇ and R₈ have the same meanings as in Claim 1, for the preparation of pharmaceutical compositions used for the treatment or prevention of diabetes and the complications of diabetes.
